(19) Europäisches Patentamt / European Patent Office / Office européen des brevets

(11) EP 3 313 443 B1

(12) **EUROPEAN PATENT SPECIFICATION**

(45) Date of publication and mention of the grant of the patent:
**07.06.2023 Bulletin 2023/23**

(21) Application number: **16815285.8**

(22) Date of filing: **23.06.2016**

(51) International Patent Classification (IPC):
***A61K 39/395*** *(2006.01)* ***A61P 35/00*** *(2006.01)*
***C07K 16/30*** *(2006.01)* ***A61K 31/357*** *(2006.01)*
***A61P 35/02*** *(2006.01)* ***A61P 35/04*** *(2006.01)*
***A61P 43/00*** *(2006.01)* ***C07K 16/28*** *(2006.01)*
***A61K 39/00*** *(2006.01)* ***A61K 47/68*** *(2017.01)*
***A61K 45/06*** *(2006.01)* ***A61K 31/337*** *(2006.01)*
***A61K 31/502*** *(2006.01)* ***A61K 31/519*** *(2006.01)*
***A61K 31/52*** *(2006.01)*

(52) Cooperative Patent Classification (CPC):
(C-Sets available)
**C07K 16/30; A61K 31/337; A61K 31/357; A61K 31/502; A61K 31/519; A61K 31/52; A61K 39/39558; A61K 45/06; A61K 47/6803; A61K 47/6849; A61K 47/6851; A61K 47/6853; A61P 35/00; A61P 35/02; A61P 35/04;** (Cont.)

(86) International application number:
**PCT/US2016/038986**

(87) International publication number:
**WO 2016/210108 (29.12.2016 Gazette 2016/52)**

(54) **COMBINING ANTI-HLA-DR OR ANTI-TROP-2 ANTIBODIES WITH MICROTUBULE INHIBITORS, PARP INHIBITORS, BRUTON KINASE INHIBITORS OR PHOSPHOINOSITIDE 3-KINASE INHIBITORS SIGNIFICANTLY IMPROVES THERAPEUTIC OUTCOME IN CANCER**

KOMBINATION VON ANTI-HLA-DR- ODER ANTI-TROP-2-ANTIKÖRPERN MIT MIKROTUBULI-INHIBITOREN, PARP-INHIBITOREN, BRUTON-KINASE-INHIBITOREN ODER PHOSPHOINOSITOL-3-KINASE-INHIBITOREN ZUR SIGNIFIKANTEN VERBESSERUNG DES THERAPEUTISCHEN ERGEBNISSES BEI KREBS

COMBINAISON D'ANTICORPS ANTI-HLA-DR OU ANTI-TROP-2 AVEC DES INHIBITEURS DE MICROTUBULE, DES INHIBITEURS DE PARP, DES INHIBITEURS DE KINASE DE BRUTON OU DES INHIBITEURS DE PHOSPHOINOSITIDE 3-KINASE AMÉLIORANT CONSIDÉRABLEMENT UN RÉSULTAT THÉRAPEUTIQUE DE CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**25.06.2015 US 201562184331 P**
**05.08.2015 US 201562201361 P**
**04.11.2015 US 201562250715 P**
**04.12.2015 US 201562263134 P**
**14.03.2016 US 201615069208**

(43) Date of publication of application:
**02.05.2018 Bulletin 2018/18**

(73) Proprietor: **Immunomedics, Inc.**
**Morris Plains, NJ 07950 (US)**

(72) Inventors:
- **GOLDENBERG, David M.**
  **Morris Plains, New Jersey 07950 (US)**
- **CARDILLO, Thomas, M.**
  **Morris Plains, New Jersey 07950 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

(74) Representative: **Bohmann, Armin K.**
**Bohmann**
**Anwaltssozietät**
**Nymphenburger Straße 1**
**80335 München (DE)**

(56) References cited:
**WO-A1-2014/124227** **WO-A1-2015/047510**
**WO-A1-2015/047510** **WO-A2-2015/069430**
**US-A1- 2011 171 126** **US-A1- 2014 219 914**
**US-A1- 2015 366 988**

- **THOMAS M. CARDILLO ET AL: "Synthetic Lethality Exploitation by an Anti-Trop-2-SN-38 Antibody-Drug Conjugate, IMMU-132, Plus PARP Inhibitors in BRCA1/2 -wild-type Triple-Negative Breast Cancer", CLINICAL CANCER RESEARCH, vol. 23, no. 13, 9 January 2017 (2017-01-09), pages 3405-3415, XP055441910, US ISSN: 1078-0432, DOI: 10.1158/1078-0432.CCR-16-2401**

(52) Cooperative Patent Classification (CPC): (Cont.)
**A61P 43/00; C07K 16/2803; C07K 16/2887; C07K 16/3007;** A61K 2039/505; A61K 2039/545; C07K 2317/24; C07K 2317/73; C07K 2317/732; C07K 2317/94

C-Sets
**A61K 31/337, A61K 2300/00;**
**A61K 31/357, A61K 2300/00;**
**A61K 31/502, A61K 2300/00;**
**A61K 31/519, A61K 2300/00;**
**A61K 31/52, A61K 2300/00;**
**A61K 39/39558, A61K 2300/00**

## Description

## BACKGROUND OF THE INVENTION

### FIELD OF THE INVENTION

The present invention relates to an immunoconjugate that binds to Trop-2 for use in a method of treating Trop-2 expressing cancer; and at least one therapeutic agent selected from the group consisting of a PARP inhibitor and a microtubule inhibitor, for use in a method of treating Trop-2 expressing cancer. Said combination therapy is more effective than the immunoconjugate alone, drug alone, or the combined effects of drug and immunoconjugate alone. Preferably, the combination exhibits synergistic effects. Preferably, immunoconjugates are administered at specific dosages and/or specific schedules of administration that optimize their therapeutic effect. The optimized dosages and schedules of administration of antibody-drug conjugates (ADCs) for human therapeutic use disclosed herein show unexpected superior efficacy that could not have been predicted from animal model studies, allowing effective treatment of cancers that are resistant to standard anti-cancer therapies, including irinotecan (CPT-11), paclitaxel or other compounds that induce DNA strand breaks. Surprisingly, combination therapy with antibody-SN38 immunoconjugates and microtubule inhibitors or PARP inhibitors shows unexpected synergistic effects. In a particularly preferred embodiment, an SN-38 conjugated antibody of use is an anti-Trop-2 antibody, such as IMMU-132 (sacitizumab govitecan, also known as hRS7-CL2A-SN-38 or IMMU-132). More preferably, the methods are of use in treating triple-negative breast cancer (TNBC), metastatic colorectal cancer, SCLC or NSCLC, as well as other Trop-2-expressing cancers.

### BACKGROUND OF THE INVENTION

For many years it has been an aim of scientists in the field of specifically targeted drug therapy to use monoclonal antibodies (MAbs) for the specific delivery of toxic agents to human cancers. Conjugates of MAbs that target tumor-associated antigens (TAA) and suitable toxic agents have been developed, but have had mixed success in the therapy of cancer in humans, and virtually no application in other diseases, such as infectious and autoimmune diseases. The toxic agent is most commonly a chemotherapeutic drug, although particle-emitting radionuclides, or bacterial or plant toxins, have also been conjugated to MAbs, especially for the therapy of cancer (Sharkey and Goldenberg, CA Cancer J Clin. 2006 Jul-Aug;56(4):226-243) and, more recently, with radioimmunoconjugates for the preclinical therapy of certain infectious diseases (Dadachova and Casadevall, Q J Nucl Med Mol Imaging 2006;50(3): 193-204).

The advantages of using MAb-chemotherapeutic drug conjugates are that (a) the chemotherapeutic drug itself is structurally well defined; (b) the chemotherapeutic drug is linked to the MAb protein using very well-defined conjugation chemistries, often at specific sites remote from the MAbs' antigen-binding regions; (c) MAb-chemotherapeutic drug conjugates can be made more reproducibly and usually with less immunogenicity than chemical conjugates involving MAbs and bacterial or plant toxins, and as such are more amenable to commercial development and regulatory approval; and (d) the MAb-chemotherapeutic drug conjugates are orders of magnitude less toxic systemically than radionuclide MAb conjugates, particularly to the radiation-sensitive bone marrow.

Camptothecin (CPT) and its derivatives are a class of potent antitumor agents. Irinotecan (also referred to as CPT-11) and topotecan are CPT analogs that are approved cancer therapeutics (Iyer and Ratain, Cancer Chemother. Phamacol. 42: S31-S43 (1998)). CPTs act by inhibiting topoisomerase I enzyme by stabilizing topoisomerase I-DNA complex (Liu, et al. in The Camptothecins: Unfolding Their Anticancer Potential, Liehr J.G., Giovanella, B.C. and Verschraegen (eds), NY Acad Sci., NY 922:1-10 (2000)). CPTs present specific issues in the preparation of conjugates. One issue is the insolubility of most CPT derivatives in aqueous buffers. Second, CPTs provide specific challenges for structural modification for conjugating to macromolecules. For instance, CPT itself contains only a tertiary hydroxyl group in ring-E. The hydroxyl functional group in the case of CPT must be coupled to a linker suitable for subsequent protein conjugation; and in potent CPT derivatives, such as SN-38, the active metabolite of the chemotherapeutic CPT-11, and other C-10-hydroxyl-containing derivatives such as topotecan and 10-hydroxy-CPT, the presence of a phenolic hydroxyl at the C-10 position complicates the necessary C-20-hydroxyl derivatization. Third, the lability under physiological conditions of the $\delta$-lactone moiety of the E-ring of camptothecins results in greatly reduced antitumor potency. Therefore, the conjugation protocol is performed such that it is carried out at a pH of 7 or lower to avoid the lactone ring opening. However, conjugation of a bifunctional CPT possessing an amine-reactive group such as an active ester would typically require a pH of 8 or greater. Fourth, an intracellularly-cleavable moiety preferably is incorporated in the linker/spacer connecting the CPTs and the antibodies or other binding moieties.

WO 2015/047510 A1 is related to anti-Trop-2 antiobdy-drug conjuages and uses thereof.

A need exists for more effective methods of preparing and administering antibody-drug conjugates, such as antibody-SN-38 conjugates, and more effective combination therapy with antibodies or immunoconjugates and drugs, such as microtubule inhibitors, PARP inhibitors, Bruton kinase inhibitors or PI3Ks inhibitors.

SUMMARY OF THE INVENTION

The problem underlying the present invention is solved by the subject matter of the attached independent claims. Preferred embodiments may be taken from the attached dependenet claims.

More specifically, the problem underlying the present invention is solved in a first aspect by an immunoconjugate that binds to Trop-2 for use in a method of treating Trop-2 expressing cancer, wherein the method comprises:

a) administering to a human subject with the cancer the immunoconjugate; and

b) administering to the subject at least one therapeutic agent selected from the group consisting of a PARP inhibitor and a microtubule inhibitor

wherein the immunoconjugate comprises an anti-Trop-2 antibody conjugated to SN-38.

More specifically, the problem underlying the present invention is solved in a second aspect by at least one therapeutic agent selected from the group consisting of a PARP inhibitor and a microtubule inhibitor, for use in a method of treating Trop-2 expressing cancer, wherein the method comprises:

a) administering to a human subject with the cancer the immunoconjugate; and
b) administering to the subject at least one therapeutic agent selected from the group consisting of a PARP inhibitor and a microtubule inhibitor,

wherein the immunoconjugate comprises an anti-Trop-2 antibody conjugated to SN-38.

In an embodiment of the first and the second aspect, the combination of antibody or immunoconjugate and therapeutic agent has a synergistic effect in inhibiting tumor growth.

In an embodiment of the first and the second aspect, the anti-Trop-2 antibody is hRS7.

In an embodiment of the first and the second aspect, the PARP inhibitor is selected from the group consisting of olaparib, talazoparib (BMN-673), rucaparib, veliparib, CEP 9722, MK 4827, BGB-290, ABT-888, AG014699, BSI-201, CEP-8983 and 3-aminobenzamide; or the microtubule inhibitor is selected from the group consisting of a vinca alkaloid, a taxane, a maytansinoid, an auristatin, vincristine, vinblastine, paclitaxel, mertansine, demecolcine, nocodazole, epothilone, docetaxel, disodermolide, colchicine, combrestatin, podophyllotoxin, CI-980, phenylahistins, steganacins, curacins, 2-methoxy estradiol, E7010, methoxy benzenesuflonamides, vinorelbine, vinflunine, vindesine, dolastatins, spongistatin, rhizoxin, tasidotin, halichondrins, hemiasterlins, cryptophycin 52, MMAE and eribulin mesylate.

In an embodiment of the first and the second aspect, the PARP inhibitor is olaparib; or the microtubule inhibitor is paclitaxel or eribulin mesylate.

In an embodiment of the first and the second aspect, the cancer is a solid tumor and the treatment results in a reduction in tumor size of at least 15%, at least 20%, at least 30%, or at least 40%.

In an embodiment of the first and the second aspect, the cancer is metastatic and the method further comprises reducing in size or eliminating the metastases.

In an embodiment of the first and the second aspect, the cancer is refractory to other therapies but responds to the combination of antibody-drug conjugate and therapeutic agent.

In an embodiment of the first and the second aspect, the patient has failed to respond to therapy with irinotecan, prior to treatment with the antibody-drug conjugate.

In an embodiment of the first and the second aspect, the method further comprises administering to the subject one or more additional therapeutic modalities selected from the group consisting of unconjugated antibodies, radiolabeled antibodies, drug-conjugated antibodies, toxin-conjugated antibodies, gene therapy, chemotherapy, therapeutic peptides, cytokine therapy, oligonucleotides, localized radiation therapy, surgery and interference RNA therapy.

In an embodiment of the first and the second aspect, the therapeutic modality comprises treatment with an agent selected from the group consisting of 5-fluorouracil, afatinib, aplidin, azaribine, anastrozole, anthracyclines, axitinib, AVL-101, AVL-291, bendamustine, bleomycin, bortezomib, bosutinib, bryostatin-1, busulfan, calicheamycin, camptothecin, carboplatin, 10-hydroxycamptothecin, carmustine, celebrex, chlorambucil, cisplatin (CDDP), Cox-2 inhibitors, irinotecan (CPT-11), SN-38, carboplatin, cladribine, camptothecans, cyclophosphamide, crizotinib, cytarabine, dacarbazine, dasatinib, dinaciclib, docetaxel, dactinomycin, daunorubicin, doxorubicin, 2-pyrrolinodoxorubicine (2P-DOX), cyano-morpholino doxorubicin, doxorubicin glucuronide, epirubicin glucuronide, erlotinib, estramustine, epidophyllotoxin, erlotinib, entinostat, estrogen receptor binding agents, etoposide (VP16), etoposide glucuronide, etoposide phosphate, exemestane, fingolimod, flavopiridol, floxuridine (FUdR), 3',5'-O-dioleoyl-FudR (FUdR-dO), fludarabine, flutamide, farnesyl-protein transferase inhibitors, fostamatinib, ganetespib, GDC-0834, GS-1101, gefitinib, gemcitabine, hydroxyurea, ibrutinib, idarubicin, idelalisib, ifosfamide, imatinib, L-asparaginase, lapatinib, lenolidamide, leucovorin, LFM-A13, lomustine, mechlorethamine, melphalan, mercaptopurine, 6-mercaptopurine, methotrexate, mitoxantrone, mithramycin, mitomycin, mitotane, navelbine, neratinib, nilotinib, nitrosurea, olaparib, plicomycin, procarbazine, paclitaxel, PCI-32765, pentostatin, PSI-341, raloxifene, semustine, sorafenib, streptozocin, SU11248, sunitinib, tamoxifen, temazolomide (an aqueous form of DTIC), transplatinum, thalidomide, thioguanine, thiotepa, teniposide, topotecan, uracil mustard, vatalanib, vinorelbine, vinblastine, vincristine, vinca alkaloids and ZD1839.

In an embodiment of the first and the second aspect, the cancer is selected from the group consisting of triple-negative breast cancer, breast cancer, ovarian cancer, endometrial cancer, urothelial cancer, uterine cancer, non-small-cell lung cancer, small-cell lung cancer, stomach cancer, urinary bladder cancer, renal cancer, prostate cancer and colorectal cancer.

Preferably, the combination therapy according to the present invention as defined in the claims is more effective than antibody or immunoconjugate alone, drug alone, or the sum of the effects of antibody or immunoconjugate and drug. Most preferably, the combination exhibits synergistic effects in human subjects.

As used herein, the abbreviation "CPT" may refer to camptothecin or any of its derivatives, such as SN-38, unless expressly stated otherwise. The present invention as defined in the claims provides improved methods and compositions for preparing and administering CPT-antibody immunoconjugates. Examplary diseases or conditions that may be treated with the antibodies or immunoconjugates include, for example, cancer or autoimmune disease.

The antibody used in accordance with the present invention as defined in the claims can be of various isotypes, preferably human IgG1, IgG2, IgG3 or IgG4, more preferably comprising human IgG1 hinge and constant region sequences. The antibody or fragment thereof can be a chimeric human-mouse, a chimeric human-primate, a humanized (human framework and murine hypervariable (CDR) regions), or fully human antibody, as well as variations thereof, such as half-IgG4 antibodies (referred to as "unibodies"), as described by van der Neut Kolfschoten et al. (Science 2007; 317:1554-1557). More preferably, the antibody or fragment thereof may be designed or selected to comprise human constant region sequences that belong to specific allotypes, which may result in reduced immunogenicity when the antibody or immunoconjugate is administered to a human subject. Preferred allotypes for administration include a non-G1m1 allotype (nG1m1), such as G1m3, G1m3,1, G1m3,2 or G1m3,1,2. More preferably, the allotype is selected from the group consisting of the nG1m1, G1m3, nG1m1, 2 and Km3 allotypes.

Antibodies of use may bind to any disease-associated antigen known in the art. Where the disease state is cancer, for example, many antigens expressed by or otherwise associated with tumor cells are known in the art, including but not limited to, carbonic anhydrase IX, alpha-fetoprotein (AFP), $\alpha$-actinin-4, A3, antigen specific for A33 antibody, ART-4, B7, Ba 733, BAGE, BrE3-antigen, CA125, CAMEL, CAP-1, CASP-8/m, , CCL19, CCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD44, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4/m, CDKN2A, CTLA-4, CXCR4, CXCR7, CXCL12, HIF-1$\alpha$, colon-specific antigen-p (CSAp), CEA (CEACAM5), CEACAM6, c-Met, DAM, EGFR, EGFRvIII, EGP-1 (Trop-2), EGP-2, ELF2-M, Ep-CAM, fibroblast growth factor (FGF), Flt-1, Flt-3, folate receptor, G250 antigen, GAGE, gp100, GRO-$\beta$, HLA-DR, HM1.24, human chorionic gonadotropin (HCG) and its subunits, HER2/neu, HMGB-1, hypoxia inducible factor (HIF-1), HSP70-2M, HST-2, Ia, IGF-1R, IFN-$\gamma$, IFN-$\alpha$, IFN-$\beta$, IFN-$\lambda$, IL-4R, IL-6R, IL-13R, IL-15R, IL-17R, IL-18R, IL-2, IL-6, IL-8, IL-12, IL-15, IL-17, IL-18, IL-23, IL-25, insulin-like growth factor-1 (IGF-1), IGF-1R, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, pancreatic cancer mucin, PD-1 receptor, PD-L1 receptor, placental growth factor, p53, PLAGL2, prostatic acid phosphatase, PSA, PRAME, PSMA, P1GF, ILGF, ILGF-1R, IL-6, IL-25, RS5, RANTES, T101, SAGE, S100, survivin, survivin-2B, TAC, TAG-72, tenascin, TRAIL receptors, TNF-$\alpha$, Tn antigen, Thomson-Friedenreich antigens, tumor necrosis antigens, VEGFR, ED-B fibronectin, WT-1, 17-1A-antigen, complement factors C3, C3a, C3b, C5a, C5, an angiogenesis marker, bcl-2, bcl-6, Kras, an oncogene marker and an oncogene product (see, e.g., Sensi et al., Clin Cancer Res 2006, 12:5023-32; Parmiani et al., J Immunol 2007, 178:1975-79; Novellino et al. Cancer Immunol Immunother 2005, 54:187-207). Preferably, the antibody binds to CEACAM5, CEACAM6, EGP-1 (Trop-2), MUC-16, AFP, MUC5a,c, CD74, CD19, CD20, CD22 or HLA-DR. More preferably, the antibody binds to Trop-2 or HLA-DR.

Exemplary antibodies that may be utilized include, but are not limited to, hR1 (anti-IGF-1R, U.S. Patent Application Serial No. 13/688,812, filed 11/29/12), hPAM4 (anti-mucin, U.S. Patent No. 7,282,567), hA20 (anti-CD20, U.S. Patent No. 7, 151,164), hA19 (anti-CD19, U.S. Patent No. 7,109,304), hIMMU31 (anti-AFP, U.S. Patent No. 7,300,655), hLL1 (anti-CD74, U.S. Patent No. 7,312,318), hLL2 (anti-CD22, U.S. Patent No. 5,789,554), hMu-9 (anti-CSAp, U.S. Patent No. 7,387,772), hL243 (anti-HLA-DR, U.S. Patent No. 7,612,180), hMN-14 (anti-CEACAM5, U.S. Patent No. 6,676,924), hMN-15 (anti-CEACAM6, U.S. Patent No. 8,287,865), hRS7 (anti-EGP-1, U.S. Patent No. 7,238,785), hMN-3 (anti-CEACAM6, U.S. Patent No. 7,541,440), Ab124 and Ab125 (anti-CXCR4, U.S. Patent No. 7,138,496). More preferably, the antibody is IMMU-31 (anti-AFP), hRS7 (anti-Trop-2), hMN-14 (anti-CEACAM5), hMN-3 (anti-CEACAM6), hMN-15 (anti-CEACAM6), hLL1 (anti-CD74), hLL2 (anti-CD22), hL243 or IMMU-114 (anti-HLA-DR), hA19 (anti-CD19) or hA20 (anti-CD20). As used herein, the terms epratuzumab and hLL2 are interchangeable, as are the terms veltuzumab

and hA20, hL243g4P, hL243gamma4P and IMMU-114. In a most preferred embodiment, the antibody is an anti-Trop-2 antibody, such as hRS7, or an anti-HLA-DR antibody, such as hL243.

Alternative antibodies of use include, but are not limited to, abciximab (anti-glycoprotein IIb/IIIa), alemtuzumab (anti-CD52), bevacizumab (anti-VEGF), cetuximab (anti-EGFR), gemtuzumab (anti-CD33), ibritumomab (anti-CD20), panitumumab (anti-EGFR), rituximab (anti-CD20), tositumomab (anti-CD20), trastuzumab (anti-ErbB2), lambrolizumab (anti-PD-1 receptor), atezolizumab (anti-PD-L1), MEDI4736 (anti-PD-L1), nivolumab (anti-PD-1 receptor), ipilimumab (anti-CTLA-4), abagovomab (anti-CA-125), adecatumumab (anti-EpCAM), atlizumab (anti-IL-6 receptor), benralizumab (anti-CD125), obinutuzumab (GA101, anti-CD20), CC49 (anti-TAG-72), AB-PG1-XG1-026 (anti-PSMA, U.S. Patent Application 11/983,372, deposited as ATCC PTA-4405 and PTA-4406), D2/B (anti-PSMA, WO 2009/130575), tocilizumab (anti-IL-6 receptor), basiliximab (anti-CD25), daclizumab (anti-CD25), efalizumab (anti-CD11a), GA101 (anti-CD20; Glycart Roche), muromonab-CD3 (anti-CD3 receptor), natalizumab (anti-α4 integrin), omalizumab (anti-IgE); anti-TNF-α antibodies such as CDP571 (Ofei et al., 2011, Diabetes 45:881-85), MTNFAI, M2TNFAI, M3TNFAI, M3TNFABI, M302B, M303 (Thermo Scientific, Rockford, IL), infliximab (Centocor, Malvern, PA), certolizumab pegol (UCB, Brussels, Belgium), anti-CD40L (UCB, Brussels, Belgium), adalimumab (Abbott, Abbott Park, IL), Benlysta (Human Genome Sciences); antibodies for therapy of Alzheimer's disease such as Alz 50 (Ksiezak-Reding et al., 1987, J Biol Chem 263:7943-47), gantenerumab, solanezumab and infliximab; anti-fibrin antibodies like 59D8, T2G1s, MH1; anti-CD38 antibodies such as MOR03087 (MorphoSys AG), MOR202 (Celgene), HuMax-CD38 (Genmab) or daratumumab (Johnson & Johnson); (anti-HIV antibodies such as P4/D10 (U.S. Patent 8,333,971), Ab 75, Ab 76, Ab 77 (Paulik et al., 1999, Biochem Pharmacol 58:1781-90), as well as the anti-HIV antibodies described and sold by Polymun (Vienna, Austria), also described in U.S. Patent 5,831,034, U.S. Patent 5,911,989, and Vcelar et al., AIDS 2007; 21(16):2161-2170 and Joos et al., Antimicrob. Agents Chemother. 2006; 50(5):1773-9.

In accordance with the present invention as defined in the claims, a drug moiety conjugated to an antibody is selected from camptothecin (CPT) and its analogs and derivatives and is more preferably SN-38. However, other drug moieties that may be utilized include taxanes (e.g., baccatin III, taxol), auristatins (e.g., MMAE), calicheamicins, epothilones, anthracyclines (e.g., doxorubicin (DOX), epirubicin, morpholinodoxorubicin (morpholino-DOX), cyanomorpholino-doxorubicin (cyanomorpholino-DOX), 2-pyrrolinodoxorubicin (2-PDOX), a prodrug form of 2-PDOX (pro-2-PDOX), topotecan, etoposide, cisplatinum, oxaliplatin or carboplatin; *see, e.g.,* Priebe W (ed.), ACS symposium series 574, published by American Chemical Society, Washington D.C., 1995 (332pp) and Nagy et al., Proc. Natl. Acad. Sci. USA 93:2464-2469, 1996). Alternatively, the drug may be a Bruton kinase inhibitor, such as ibrutinib (PCI-32765), PCI-45292, CC-292 (AVL-292), ONO-4059, GDC-0834, LFM-A13 or RN486, or a PI3K inhibitor, such as idelalisib, Wortmannin, demethoxyviridin, perifosine, PX-866, IPI-145 (duvelisib), BAY 80-6946, BEZ235, RP6530, TGR1202, SF1126, INK1117, GDC-0941, BKM120, XL147, XL765, Palomid 529, GSK1059615, ZSTK474, PWT33597, IC87114, TG100-115, CAL263, PI-103, GNE477, CUDC-907, AEZS-136 or LY294002. Preferably, the antibody or fragment thereof links to at least one chemotherapeutic moiety; preferably 1 to about 5 drug moieties; more preferably 6 to 8 drug moieties, most preferably about 6 to about 12 drug moieties.

An example of a water soluble CPT derivative is CPT-11. Extensive clinical data are available concerning CPT-11's pharmacology and its *in vivo* conversion to the active SN-38 (Iyer and Ratain, Cancer Chemother Pharmacol. 42:S31-43 (1998); Mathijssen et al., Clin Cancer Res. 7:2182-2194 (2002); Rivory, Ann NY Acad Sci. 922:205-215, 2000)). The active form SN-38 is about 2 to 3 orders of magnitude more potent than CPT-1 1. In specific preferred embodiments, the immunoconjugate may be an hMN-14-SN-38, hMN-3-SN-38, hMN-15-SN-38, IMMU-31-SN-38, hRS7-SN-38, hA20-SN-38, hL243-SN-38, hLL1-SN-38 or hLL2-SN-38 conjugate.

In accordance with the present invention as defined in the claims, cancer includes but is not limited to non-Hodgkin's lymphomas, B-cell acute and chronic lymphoid leukemias, Burkitt lymphoma, Hodgkin's lymphoma, acute large B-cell lymphoma, hairy cell leukemia, acute myeloid leukemia, chronic myeloid leukemia, acute lymphocytic leukemia, chronic lymphocytic leukemia, T-cell lymphomas and leukemias, multiple myeloma, Waldenstrom's macroglobulinemia, carcinomas, melanomas, sarcomas, gliomas, bone, and skin cancers. The carcinomas may include carcinomas of the oral cavity, esophagus, gastrointestinal tract, pulmonary tract, lung, stomach, colon, rectum, breast, ovary, prostate, uterus, endometrium, cervix, urinary bladder, pancreas, bone, brain, connective tissue, thyroid, liver, gall bladder, urinary bladder (urothelial), kidney, skin, central nervous system and testes.

In certain embodiments of the present invention as defined in the claims, , the immunoconjugate may be used in combination with surgery, radiation therapy, chemotherapy, immunotherapy with naked antibodies, including checkpoint-inhibiting antibodies, radioimmunotherapy, immunomodulators, vaccines, and the like. These combination therapies can allow lower doses of each therapeutic to be given in such combinations, thus reducing certain severe side effects, and potentially reducing the courses of therapy required. When there is no or minimal overlapping toxicity, full doses of each can also be given.

Preferred optimal dosing of immunoconjugates may include a dosage of between 1 mg/kg and 20 mg/kg, more preferably 4 to 16 mg/kg, most preferably 8 to 10 mg/kg, preferably given either weekly, twice weekly, every other week, or every third week. The optimal dosing schedule may include treatment cycles of two consecutive weeks of therapy

followed by one, two, three or four weeks of rest, or alternating weeks of therapy and rest, or one week of therapy followed by two, three or four weeks of rest, or three weeks of therapy followed by one, two, three or four weeks of rest, or four weeks of therapy followed by one, two, three or four weeks of rest, or five weeks of therapy followed by one, two, three, four or five weeks of rest, or administration once every two weeks, once every three weeks or once a month. Treatment may be extended for any number of cycles, preferably at least 2, at least 4, at least 6, at least 8, at least 10, at least 12, at least 14, or at least 16 cycles. Exemplary dosages of use may include 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, and 18 mg/kg. The person of ordinary skill will realize that a variety of factors, such as age, general health, specific organ function or weight, as well as effects of prior therapy on specific organ systems (e.g., bone marrow) may be considered in selecting an optimal dosage of immunoconjugate, and that the dosage and/or frequency of administration may be increased or decreased during the course of therapy. The dosage may be repeated as needed, with evidence of tumor shrinkage observed after as few as 4 to 8 doses. The optimized dosages and schedules of administration disclosed herein show unexpected superior efficacy and reduced toxicity in human subjects, which could not have been predicted from animal model studies. Surprisingly, the superior efficacy allows treatment of tumors that were previously found to be resistant to one or more standard anti-cancer therapies, including the parental compound, CPT-11, from which SN-38 is derived *in vivo.*

The present invention as defined in the claims may include use of CT and/or PET/CT, or MRI, to measure tumor response at regular intervals. Blood levels of tumor markers, such as CEA (carcinoembryonic antigen), CA19-9, AFP, CA 15.3, or PSA, may also be monitored. Dosages and/or administration schedules may be adjusted as needed, according to the results of imaging and/or marker blood levels.

A surprising result with the present invention as defined in the claims is the unexpected tolerability of high doses of antibody-drug conjugate when given to patients, even with repeated infusions, with only relatively low-grade toxicities of nausea and vomiting observed, or manageable neutropenia. Preferably, the incidence of grade 3 or higher side effects, such as anemia, diarrhea, nausea, vomiting or neutropenia, is limited to 26% or less of the treated population. More preferably, the incidence of grade 3 or higher diarrhea and neutropenia occurs in 26% or less of the treated population. A further surprising result is the lack of accumulation of the antibody-drug conjugate, unlike other products that have conjugated SN-38 to albumin, PEG or other carriers, to nontarget tissues or target tissues where the MAb is not accessible. The lack of accumulation is associated with improved tolerability and lack of serious toxicity even after repeated or increased dosing. These surprising results allow optimization of dosage and delivery schedule, with unexpectedly high efficacies and low toxicities; i.e., a higher therapeutic index than when the parental drug to SN-38, irinotecan, is given alone or in combination with other drugs. This is underscored by the black box warning on the product labeling of irinotecan, required by FDA, stating the high severe early and late diarrhea resulting from therapy with irinotecan. This is not experienced to the same extent and frequency with these SN-38-containing ADCs. Even febrile neutropenia is much lower with the SN-38-ADCs described herein than with irinotecan therapy.

The present invention as defined in the claims provide for shrinkage of solid tumors, in individuals with previously resistant cancers, of 15% or more, preferably 20% or more, preferably 30% or more, more preferably 40% or more in size (as measured by summing the longest diameter of target lesions, as per RECIST or RECIST 1.1). The person of ordinary skill will realize that tumor size may be measured by a variety of different techniques, such as total tumor volume, maximal tumor size in any dimension or a combination of size measurements in several dimensions. This may be with standard radiological procedures, such as computed tomography, magnet resonance imaging, ultrasonography, and/or positron-emission tomography. The means of measuring size is less important than observing a trend of decreasing tumor size with antibody or immunoconjugate treatment, preferably resulting in elimination of the tumor. However, to comply with RECIST guidelines, CT or MRI is preferred on a serial basis, and should be repeated to confirm measurements.

While the antibody or immunoconjugate may be administered as a periodic bolus injection, in alternative embodiments of the invention as defined in the claims the antibody or immunoconjugate may be administered by continuous infusion. In order to increase the Cmax and extend the PK of the antibody or immunoconjugate in the blood, a continuous infusion may be administered for example by indwelling catheter. Such devices are known in the art, such as HICKMAN®, BROVIAC® or PORT-A-CATH® catheters (see, e.g., Skolnik et al., Ther Drug Monit 32:741-48, 2010) and any such known indwelling catheter may be used. A variety of continuous infusion pumps are also known in the art and any such known infusion pump may be used. The dosage range for continuous infusion may be between 0.1 and 3.0 mg/kg per day. More preferably, these immunoconjugates can be administered by intravenous infusions over relatively short periods of 2 to 5 hours, more preferably 2-3 hours.

In particularly preferred embodiments of the present invention as defined in the claims, the antibodies or immunoconjugates and dosing schedules may be efficacious in patients resistant to standard therapies. For example, an hRS7-SN-38 immunoconjugate may be administered to a patient who has not responded to prior therapy with irinotecan, the parent agent of SN-38. Surprisingly, the irinotecan-resistant patient may show a partial or even a complete response to hRS7-SN-38. The ability of the immunoconjugate to specifically target the tumor tissue may overcome tumor resistance

by improved targeting and enhanced delivery of the therapeutic agent. A specific preferred subject may be a patient with a Trop-2-positive breast, ovarian, cervical, endometrial, lung, prostate, colon, rectum, stomach, esophageal, bladder (urothelial), renal, pancreatic, brain, thyroid, epithelial or head-and-neck cancer. Preferably, the cancer is metastatic cancer. More preferably, the patient has previously failed treatment with at least one standard anti-cancer therapy. Most preferably, the patient has previously failed therapy with irinotecan (CPT-11), the parent compound of SN-38. In alternative preferred embodiments, the cancer is TNBC, non-TNBC, endometrial, lung, or ovarian cancer.

In certain preferred embodiments of the present invention as defined in the claims, an immunoconjugate, such as sacituzumab govitecan, may be used in combination therapy with at least one microtubule inhibitor. A number of microtubule inhibitors are known in the art, such as vinca alkaloids (e.g., vincristine, vinblastine), taxanes (e.g., paclitaxel), maytansinoids (e.g., mertansine) and auristatins. Other known microtubule inhibitors include demecolcine, nocodazole, epothilone, docetaxel, discodermolide, colchicine, combrestatin, podophyllotoxin, CI-980, phenylahistins, steganacins, curacins, 2-methoxy estradiol, E7010, methoxy benzenesuflonamides, vinorelbine, vinflunine, vindesine, dolastatins, spongistatin, rhizoxin, tasidotin, halichondrins, hemiasterlins, cryptophycin 52, MMAE and eribulin mesylate (see, e.g., Dumontet & Jordan, 2010, Nat Rev Drug Discov 9:790-803). Any such known microtubule inhibitor may be used in combination with an antibody or antibody-drug conjugate (ADC). Preferably, the microtubule inhibitor is one that exhibits synergistic effects when used in combination with an antibody or ADC. One potent example is SN-38-conjugated antibody, such as sacituzumab govitecan or labetuzumab govitecan (targeting CEACAMS) expressed by many solid cancers. Most preferably, the microtubule inhibitor is paclitaxel or eribulin mesylate.

In other preferred embodiments of the present invention as defined in the claims, the ADC may be used in combination therapy with at least one PARP inhibitor. One such ADC is comprised of SN-38 conjugated to a tumor-targeting antibody, such as sacituzumab govitecan or labetuzumab govitecan. A number of PARP inhibitors are known in the art, such as olaparib, talazoparib (BMN-673), rucaparib, veliparib, niraparib, iniparib, CEP 9722, MK 4827, BGB-290, ABT-888, AG014699, BSI-201, CEP-8983 and 3-aminobenzamide (see, e.g., Rouleau et al., 2010, Nat Rev Cancer 10:293-301, Bao et al., 2015, Oncotarget [Epub ahead of print, September 22, 2015]). Any such known PARP inhibitor may be used in combination with an antibody or ADC, such as, for example, an SN-38-antibody conjugate or a P2PDox-antibody conjugate. Preferably, the PARP inhibitor is one that exhibits synergistic effects when used in combination with the antibody or ADC. This has been validated when using an SN-38-conjugated antibody, such as sacituzumab govitecan. Most preferably, the PARP inhibitor is olaparib or rucaparib.

According to the instant disclosure, an antibody or immunoconjugate may be used in combination with a Bruton kinase inhibitor or PI3K inhibitor. Exemplary Bruton kinase inhibitors include, but are not limited to, ibrutinib (PCI-32765), PCI-45292, CC-292 (AVL-292), ONO-4059, GDC-0834, LFM-A13 or RN486. Exemplary PI3K inhibitors include, but are not limited to, idelalisib, Wortmannin, demethoxyviridin, perifosine, PX-866, IPI-145 (duvelisib), BAY 80-6946, BEZ235, RP6530, TGR1202, SF1126, INK1117, GDC-0941, BKM120, XL147, XL765, Palomid 529, GSK1059615, ZSTK474, PWT33597, IC87114, TG100-115, CAL263, PI-103, GNE477, CUDC-907, AEZS-136 or LY294002. Any Bruton kinase or PI3K inhibitors known in the art may be utilized in the combination therapy.

## BRIEF DESCRIPTION OF THE FIGURES

**FIG. 1A. Synergy of IMMU-132 plus Olaparib in BRCA1/2 w.t. TNBC (MDA-MB-468).** Dose/response curves for each agent alone were first tested to determine single agent $IC_{10}$, $IC_{20}$, or $IC_{30}$-values after a 96-h incubation. In combination assays, one agent (e.g., IMMU-132) was tested on a given cell line across a range of concentrations (i.e., dose/response curves). One set of wells only received IMMU-132. Another set received IMMU-132 as dose/response with a constant amount of olaparib (e.g., $IC_{10}$-concentration). Two other sets used olaparib at $IC_{20}$ or $IC_{30}$ concentrations. For each IMMU-132 dose/response curve, the $IC_{50}$-value was determined from these data. FIG. 1A shows changes in $IC_{50}$-values of olaparib when combined with constant amounts of IMMU-132 (0.6, 1.1 or 1.8 nM SN-38 equivalents).

**FIG. 1B. Synergy of IMMU-132 plus Olaparib in BRCA1/2 w.t. TNBC (MDA-MB-468).** Dose/response curves for each agent alone were first tested to determine single agent $IC_{10}$, $IC_{20}$, or $IC_{50}$-values after a 96-h incubation. In combination assays, one agent (e.g., IMMU-132) was tested on a given cell line across a range of concentrations (i.e., dose/response curves). One set of wells only received IMMU-132. Another set received IMMU-132 as dose/response with a constant amount of olaparib (e.g., $IC_{10}$-concentration). Two other sets used olaparib at $IC_{20}$ or $IC_{30}$ concentrations. For each IMMU-132 dose/response curve, the $IC_{50}$-value was determined from these data. FIG. 1B shows the same cell line showing changes in IMMU-132 $IC_{50}$-values when combined with constant amounts of olaparib (1, 2, or 3 mM).

**FIG. 1C. Synergy of IMMU-132 plus Olaparib in BRCA1/2 w.t. TNBC (MDA-MB-468).** Dose/response curves for each agent alone were first tested to determine single agent $IC_{10}$, $IC_{20}$, or $IC_{30}$-values after a 96-h incubation. In

combination assays, one agent (e.g., IMMU-132) was tested on a given cell line across a range of concentrations (i.e., dose/response curves). One set of wells only received IMMU-132. Another set received IMMU-132 as dose/response with a constant amount of olaparib (e.g., $IC_{10}$-concentration). Two other sets used olaparib at $IC_{20}$ or $IC_{30}$ concentrations. For each IMMU-132 dose/response curve, the $IC_{50}$-value was determined from these data. FIG. 1C shows an isobologram of normalized $IC_{50}$-values from three separate experiments testing the interaction of IMMU-132 and olaparib, clearly showing a synergistic interaction.

**FIG. 2A. Tumor growth inhibition of combined IMMU-132 and Olaparib in TNBC: BRCA1/2 and PTEN defective tumors.** Tumor-bearing mice (TV~0.3 $cm^3$) were treated with Olaparib (1 mg; ~50 mg/kg, i.p. on a M-F schedule; red arrows) or IMMU-132 (i.v. weekly, black arrows). A non-tumor-targeting anti-CD20 SN-38-ADC was used as a control. HCC1806 is a BRCAI/2-defective TNBC tumor line. Olaparib alone had no significant anti-tumor effects. IMMU-132 alone significantly inhibited tumor growth compared to all control groups (P<0.0106, AUC). IMMU-132 plus olaparib further improved anti-tumor responses significantly compared to all groups (P<0.0019; AUC). Mice in the combination group have yet to reach median survival (>80.5 days) which is more than 2- and 4-fold longer than IMMU-132 or olaparib monotherapy, respectively (P<0.0083).

**FIG. 2B. Tumor growth inhibition of combined IMMU-132 and Olaparib in TNBC: BRCA1/2 and PTEN defective tumors.** Tumor-bearing mice (*TV*~0.3 $cm^3$) were treated with Olaparib (1 mg; ~50 mg/kg, i.p. on a M-F schedule; light arrows) or IMMU-132 (i.v. weekly, dark arrows). A non-tumor-targeting anti-CD20 SN-38-ADC was used as a control. In the *BRCA1/2* w.t., PTEN-defective MDA-MB-468 tumors, IMMU-132 alone had significant anti-tumor effects compared to all control groups (P<0.0098; AUC). However, the combination of IMMU-132 plus olaparib inhibited tumor growth significantly better than either IMMU-132 or olaparib alone (P=0.004; AUC). This translates into a significant survival benefit when compared to all other groups (P<0.045).

**FIG. 3A. Tumor growth inhibition of combined IMMU-132 and microtubule inhibitors in human TNBC tumor xenografts.** Tumor-bearing mice (*TV*~0.3 $cm^3$) were treated with either paclitaxel or eribulin mesylate, as indicated, either alone or with IMMU-132. Therapy was administered as weekly injections for two weeks with one week off before repeating. A non-tumor-targeting anti-CD20 SN-38-ADC was used as a control ADC. HCC1806 tumor-bearing mice treated with the combination of IMMU-132 plus paclitaxel significantly inhibited tumor growth when compared to IMMU-132 alone (P<0.0195, AUC).

**FIG. 3B. Tumor growth inhibition of combined IMMU-132 and microtubule inhibitors in human TNBC tumor xenografts.** Tumor-bearing mice (*TV*~0.3 $cm^3$) were treated with either paclitaxel or eribulin mesylate, as indicated, either alone or with IMMU-132. Therapy was administered as weekly injections for two weeks with one week off before repeating. A non-tumor-targeting anti-CD20 SN-38-ADC was used as a control ADC. HCC1806 tumor-bearing mice treated with the combination of IMMU-132 plus paclitaxel showed a significant survival benefit when compared to all other treatments (P<0.006, log-rank).

**FIG. 3C. Tumor growth inhibition of combined IMMU-132 and microtubule inhibitors in human TNBC tumor xenografts.** Mice bearing MDA-MB-468 tumors demonstrated significant anti-tumor effects when IMMU-132 (100 or 200 mg) was combined with paclitaxel when compared to mice receiving monotherapy (P<0.0328, AUC).

**FIG. 3D. Tumor growth inhibition of combined IMMU-132 and microtubule inhibitors in human TNBC tumor xenografts.** Eribulin mesylate alone significantly inhibits MDA-MB-468 tumor growth (P=0.0019, AUC). Importantly, the combination of IMMU-132 plus eribulin mesylate resulted in significant tumor regressions with mean tumor volumes regressing to $0.026 \pm 0.033$ $cm^3$ *versus* $0.177 \pm 0.087$ $cm^3$ and $0.344 \pm 302 cm^3$ for IMMU-132 and eribulin monotherapy groups, respectively (P<0.0009 combo vs. all other therapy groups, AUC).

**FIG. 3E. Tumor growth inhibition of combined IMMU-132 and microtubule inhibitors in human TNBC tumor xenografts.** IMMU-132 plus eribulin mesylate produced significant anti-tumor effects when compared to all other treatment groups (P<0.0432; paired, two-tailed t-Test).

**FIG. 3F. Tumor growth inhibition of combined IMMU-132 and microtubule inhibitors in human TNBC tumor xenografts.** IMMU-132 plus eribulin mesylate provided a significant survival benefit compared to all groups, except the combination of eribulin mesylate plus control ADC (P<0.0284).

**FIG. 4. Synergistic activity of IMMU-132 plus Olaparib in various human TNBC** cell lines. Summary of calculated combination index (CI)-values for various cell lines shown. CI numbers are calculated using the following formula: CI=Da/Dxa + Db/Dxb, where

Da=$IC_{50}$-dose of IMMU-132 when used in combination with a constant amount of Olaparib
Db=$IC_{50}$-dose of Olaparib when used in combination with a constant amount of IMMU-132
Dxa=$IC_{50}$-dose of IMMU-132 when used alone
Dxb=$IC_{50}$-dose of Olaparib when used alone
[a]IMMU-132 $IC_{50}$-value (nM) when combined with olaparib.
[b]Olaparib $IC_{50}$-value (nM) when combined with IMMU-132.
[c]CI combination index number (CI<1.0 is indicative of synergy).

**FIG. 5A.** Effect of IMMU-114 on $IC_{50}$ of ibrutinib.

**FIG. 5B.** Effect of IMMU-114 on $IC_{50}$ of idelalisib.

**FIG. 6A.** Isobologram demonstrating an additive effect of combined IMMU-114 with a Bruton kinase inhibitor (ibrutinib) in a human CLL cell line. Human chronic B-cell leukemia cells (JVM-3) were incubated in 96-well plates with a Bruton kinase inhibitor (ibrutinib) at doses ranging from $1\times10^{-5}$ to $3.9\times10^{-9}$M. One set of cells received only the inhibitor while other sets received the inhibitor plus a constant amount of IMMU-114 (0.25, 0.5, 0.75 or 1 nM). Plates were incubated for 96 h. Cell viability was assessed by MTS assay and dose/response curves were generated for each condition. $IC_{50}$-values were determined using Prism Graph-Pad. Data were normalized and and isobologram generated, indicating an additive effect for ibrutinib when combined with IMMU-114

**FIG. 6B.** Isobologram demonstrating an additive effect of combined IMMU-114 with a PI3K inhibitor (idelalisib) in a human CLL cell line. The studies were performed as described in the legend to **FIG. 6A.** The isobologram indicated an additive effect for idelalisib when combined with IMMU-114.

**FIG. 7.** *In vivo* therapy of athymic nude mice, bearing Capan 1 human pancreatic carcinoma, with MAb-CL2A-SN-38 conjugates.

**FIG. 8.** *In vivo* therapy of athymic nude mice, bearing BxPC3 human pancreatic carcinoma, with MAb-CL2A-SN-38 conjugates.

**FIG. 9.** *In vivo* therapy of athymic nude mice, bearing LS174T human colon carcinoma, with hMN-14-CL2A-SN-38 conjugate.

**FIG. 10.** Survival curves of hMN14-CL-**SN-38** treated mice bearing GW-39 lung metastatic disease.

**FIG. 11A.** Therapeutic efficacy of hRS7-SN-38 ADC in mice bearing human non-small cell lung tumor xenografts. Mice bearing Calu-3 tumors (*N* = 5-7) were injected with hRS7-CL2-SN-38 every 4 days for a total of 4 injections (q4dx4). All the ADCs and controls were administered in the amounts indicated (expressed as amount of SN-38 per dose; long arrows = conjugate injections, short arrows = irinotecan injections).

**FIG. 11B.** Therapeutic efficacy of hRS7-SN-38 ADC in mice bearing human colorectal tumor xenografts. COLO 205 tumor-bearing mice 5) were injected 8 times (q4dx8) with the ADC or every 2 days for a total of 5 injections (q2dx5) with the MTD of irinotecan. All the ADCs and controls were administered in the amounts indicated (expressed as amount of SN-38 per dose; long arrows = conjugate injections, short arrows = irinotecan injections).

**FIG. 11C.** Therapeutic efficacy of hRS7-SN-38 ADC in mice bearing human pancreatic cancer xenografts. Capan-1 (*N* = 10) tumor-bearing mice (*N* = 10) were treated twice weekly for 4 weeks with the agents indicated. All the ADCs and controls were administered in the amounts indicated (expressed as amount of SN-38 per dose; long arrows = conjugate injections, short arrows = irinotecan injections).

**FIG. 11D.** Therapeutic efficacy of hRS7-SN-38 ADC in mice bearing human pancreatic cancer xenografts. BxPC-3 tumor-bearing mice (*N* = 10) were treated twice weekly for 4 weeks with the agents indicated. All the ADCs and controls were administered in the amounts indicated (expressed as amount of SN-38 per dose; long arrows = conjugate injections, short arrows = irinotecan injections).

**FIG. 11E.** Therapeutic efficacy of hRS7-SN-38 ADC in mice bearing human squamous cell lung carcinoma xenografts. In addition to ADC given twice weekly for 4 week, SK-MES-1 tumor-bearing (*N* = 8) mice received the MTD of CPT-11 (q2dx5). All the ADCs and controls were administered in the amounts indicated (expressed as amount of SN-38 per dose; long arrows = conjugate injections, short arrows = irinotecan injections).

**FIG. 12A.** Comparative efficacy of epratuzumab (Emab)-SN-38 and veltuzumab (Vmab)-SN-38 conjugates in the subcutaneous Ramos model. Nude mice (*N* = 10 per group) with tumors averaging approximately 0.35 $cm^3$ (0.20-0.55 $cm^3$) were administered 0.25 mg of Emab-SN-38 twice weekly for 4 weeks.

**FIG. 12B.** Comparative efficacy of epratuzumab (Emab)-SN-38 and veltuzumab (Vmab)-SN-38 conjugates in the subcutaneous Ramos model. Nude mice (*N* = 10 per group) with tumors averaging approximately 0.35 $cm^3$ (0.20-0.55 $cm^3$) were administered 0.25 mg of Vmab-SN-38 twice weekly for 4 weeks.

**FIG. 12C.** Comparative efficacy of epratuzumab (Emab)-SN-38 and veltuzumab (Vmab)-SN-38 conjugates in the subcutaneous Ramos model. Nude mice (*N* = 10 per group) with tumors averaging approximately 0.35 $cm^3$ (0.20-0.55 $cm^3$) were administered 0.5 mg of Emab-SN-38 twice weekly for 4 weeks.

**FIG. 12D.** Comparative efficacy of epratuzumab (Emab)-SN-38 and veltuzumab (Vmab)-SN-38 conjugates in the subcutaneous Ramos model. Nude mice (*N* = 10 per group) with tumors averaging approximately 0.35 $cm^3$ (0.20-0.55 $cm^3$) were administered 0.5 mg of Vmab-SN-38 twice weekly for 4 weeks.

**FIG. 13A.** Specificity of Emab anti-CD22-SN-38 conjugate (solid line) versus an irrelevant labetuzumab (Lmab)-SN-38 conjugate (dashed line) in nude mice bearing subcutaneous Ramos tumors. Animals were given twice weekly doses of 75 $\mu$g of each conjugate per dose (54.5 $\mu$g/kg of SN-38, based on average weight of 22 g) intraperitoneally for 4 weeks. Survival based on time-to-progression (TTP) to 3.0 $cm^3$, with tumors starting at an average size of 0.4 $cm^3$. *P* values comparing median survival (shown) for Emab-SN-38 to Lmab-SN-38 conjugate are shown in each panel. C, survival curves (solid gray) for another group of animals given weekly intraperitoneal injections of irinotecan (6.5 $\mu$g/dose; SN-38 equivalents approximately the same as the 250-$\mu$g dose of the Emab-SN-38 conjugate).

**FIG. 13B.** Specificity of Emab anti-CD22-SN-38 conjugate (solid line) versus an irrelevant labetuzumab (Lmab)-SN-38 conjugate (dashed line) in nude mice bearing subcutaneous Ramos tumors. Animals were given twice weekly doses of 125 μg of each conjugate per dose (91 μg/kg of SN-38, based on average weight of 22 g) intraperitoneally for 4 weeks. Survival based on time-to-progression (TTP) to 3.0 $cm^3$, with tumors starting at an average size of 0.4 $cm^3$. P values comparing median survival (shown) for Emab-SN-38 to Lmab-SN-38 conjugate are shown in each panel. C, survival curves (solid gray) for another group of animals given weekly intraperitoneal injections of irinotecan (6.5 μg/dose; SN-38 equivalents approximately the same as the 250-μg dose of the Emab-SN-38 conjugate).

**FIG. 13C.** Specificity of Emab anti-CD22-SN-38 conjugate (solid line) versus an irrelevant labetuzumab (Lmab)-SN-38 conjugate (dashed line) in nude mice bearing subcutaneous Ramos tumors. Animals were given twice weekly doses of 250 μg of each conjugate per dose (182 μg/kg of SN-38, based on average weight of 22 g) intraperitoneally for 4 weeks. Survival based on time-to-progression (TTP) to 3.0 $cm^3$, with tumors starting at an average size of 0.4 $cm^3$. P values comparing median survival (shown) for Emab-SN-38 to Lmab-SN-38 conjugate are shown in each panel. Survival curves (solid gray) are also shown for another group of animals given weekly intraperitoneal injections of irinotecan (6.5 μg/dose; SN-38 equivalents approximately the same as the 250-μg dose of the Emab-SN-38 conjugate).

**FIG. 14.** History of prior treatment of patient, before administering IMMU-130 (labetuzumab-SN-38). Prior treatment included stage IV CRC coloectomy/hepatectomy (partial lobe), radiofrequency ablation therapy of liver metasteses, wedge resection of lung metasteses, and chemotherapy with irinotecan/oxaliplatin, Folfirinox, Folfirinox + bevacizumab, bevacizumab + 5-FU/leucovorin, FolFiri, Folfiri + cetuximab, and cetuximab alone. The patient received doses of 16 mg/kg of IMMU-130 by slow IV infusion every other week for a total of 17 treatment doses.

## DETAILED DESCRIPTION OF THE INVENTION

### Definitions

In the description that follows, a number of terms are used and the following definitions are provided to facilitate understanding of the claimed subject matter. Terms that are not expressly defined herein are used in accordance with their plain and ordinary meanings.

Unless otherwise specified, a or an means "one or more."

The term about is used herein to mean plus or minus ten percent (10%) of a value. For example, "about 100" refers to any number between 90 and 110.

An antibody, as used herein, refers to a full-length (i.e., naturally occurring or formed by normal immunoglobulin gene fragment recombinatorial processes) immunoglobulin molecule (e.g., an IgG antibody) or an antigen-binding portion of an immunoglobulin molecule, such as an antibody fragment. An antibody or antibody fragment may be conjugated or otherwise derivatized within the scope of the claimed subject matter. Such antibodies include but are not limited to IgG1, IgG2, IgG3, IgG4 (and IgG4 subforms), as well as IgA isotypes. As used below, the abbreviation "MAb" may be used interchangeably to refer to an antibody, antibody fragment, monoclonal antibody or multispecific antibody.

An antibody fragment is a portion of an antibody such as $F(ab')_2$, $F(ab)_2$, Fab', Fab, Fv, scFv (single chain Fv), single domain antibodies (DABs or VHHs) and the like, including the half-molecules of IgG4 cited above (van der Neut Kolfschoten et al. (Science 2007; 317(14 Sept):1554-1557). Regardless of structure, an antibody fragment of use binds with the same antigen that is recognized by the intact antibody. The term "antibody fragment" also includes synthetic or genetically engineered proteins that act like an antibody by binding to a specific antigen to form a complex. For example, antibody fragments include isolated fragments consisting of the variable regions, such as the "Fv" fragments consisting of the variable regions of the heavy and light chains and recombinant single chain polypeptide molecules in which light and heavy variable regions are connected by a peptide linker ("scFv proteins"). The fragments may be constructed in different ways to yield multivalent and/or multispecific binding forms.

A naked antibody is generally an entire antibody that is not conjugated to a therapeutic agent. A naked antibody may exhibit therapeutic and/or cytotoxic effects, for example by Fc-dependent functions, such as complement fixation (CDC) and ADCC (antibody-dependent cell cytotoxicity). However, other mechanisms, such as apoptosis, anti-angiogenesis, anti-metastatic activity, anti-adhesion activity, inhibition of heterotypic or homotypic adhesion, and interference in signaling pathways, may also provide a therapeutic effect. Naked antibodies include polyclonal and monoclonal antibodies, naturally occurring or recombinant antibodies, such as chimeric, humanized or human antibodies and fragments thereof. In some cases a "naked antibody" may also refer to a "naked" antibody fragment. As defined herein, "naked" is synonymous with "unconjugated," and means not linked or conjugated to a therapeutic agent.

A chimeric antibody is a recombinant protein that contains the variable domains of both the heavy and light antibody chains, including the complementarity determining regions (CDRs) of an antibody derived from one species, preferably a rodent antibody, more preferably a murine antibody, while the constant domains of the antibody molecule are derived from those of a human antibody. For veterinary applications, the constant domains of the chimeric antibody

may be derived from that of other species, such as a primate, cat or dog.

A humanized antibody is a recombinant protein in which the CDRs from an antibody from one species; e.g., a murine antibody, are transferred from the heavy and light variable chains of the murine antibody into human heavy and light variable domains (framework regions). The constant domains of the antibody molecule are derived from those of a human antibody. In some cases, specific residues of the framework region of the humanized antibody, particularly those that are touching or close to the CDR sequences, may be modified, for example replaced with the corresponding residues from the original murine, rodent, subhuman primate, or other antibody.

A human antibody is an antibody obtained, for example, from transgenic mice that have been "engineered" to produce human antibodies in response to antigenic challenge. In this technique, elements of the human heavy and light chain loci are introduced into strains of mice derived from embryonic stem cell lines that contain targeted disruptions of the endogenous heavy chain and light chain loci. The transgenic mice can synthesize human antibodies specific for various antigens, and the mice can be used to produce human antibody-secreting hybridomas. Methods for obtaining human antibodies from transgenic mice are described by Green et al., Nature Genet. 7:13 (1994), Lonberg et al., Nature 368:856 (1994), and Taylor et al., Int. Immun. 6:579 (1994). A fully human antibody also can be constructed by genetic or chromosomal transfection methods, as well as phage display technology, all of which are known in the art. See for example, McCafferty et al., Nature 348:552-553 (1990) for the production of human antibodies and fragments thereof *in vitro,* from immunoglobulin variable domain gene repertoires from unimmunized donors. In this technique, human antibody variable domain genes are cloned in-frame into either a major or minor coat protein gene of a filamentous bacteriophage, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. In this way, the phage mimics some of the properties of the B cell. Phage display can be performed in a variety of formats, for their review, see e.g. Johnson and Chiswell, Current Opinion in Structural Biology 3:5564-571 (1993). Human antibodies may also be generated by *in vitro* activated B cells. See U.S. Patent Nos. 5,567,610 and 5,229,275.

A therapeutic agent is an atom, molecule, or compound that is useful in the treatment of a disease. Examples of therapeutic agents include, but are not limited to, antibodies, antibody fragments, immunoconjugates, drugs, cytotoxic agents, pro-apopoptotic agents, toxins, nucleases (including DNAses and RNAses), hormones, immunomodulators, chelators, boron compounds, photoactive agents or dyes, radionuclides, oligonucleotides, interference RNA, siRNA, RNAi, anti-angiogenic agents, chemotherapeutic agents, cyokines, chemokines, prodrugs, enzymes, binding proteins or peptides or combinations thereof.

An immunoconjugate is an antibody, antigen-binding antibody fragment, antibody complex or antibody fusion protein that is conjugated to at least one therapeutic agent. Conjugation may be covalent or non-covalent. Preferably, conjugation is covalent.

As used herein, the term antibody fusion protein is a recombinantly-produced antigen-binding molecule in which one or more natural antibodies, single-chain antibodies or antibody fragments are linked to another moiety, such as a protein or peptide, a toxin, a cytokine, a hormone, etc. In certain preferred embodiments, the fusion protein may comprise two or more of the same or different antibodies, antibody fragments or single-chain antibodies fused together, which may bind to the same epitope, different epitopes on the same antigen, or different antigens.

An immunomodulator is a therapeutic agent that when present, alters, suppresses or stimulates the body's immune system. Typically, an immunomodulator of use stimulates immune cells to proliferate or become activated in an immune response cascade, such as macrophages, dendritic cells, B-cells, and/or T-cells. However, in some cases an immunomodulator may suppress proliferation or activation of immune cells. An example of an immunomodulator as described herein is a cytokine, which is a soluble small protein of approximately 5-20 kDa that is released by one cell population (e.g., primed T-lymphocytes) on contact with specific antigens, and which acts as an intercellular mediator between cells. As the skilled artisan will understand, examples of cytokines include lymphokines, monokines, interleukins, and several related signaling molecules, such as tumor necrosis factor (TNF) and interferons. Chemokines are a subset of cytokines. Certain interleukins and interferons are examples of cytokines that stimulate T cell or other immune cell proliferation. Exemplary interferons include interferon-$\alpha$, interferon-$\beta$, interferon-y and interferon-$\lambda$.

CPT is an abbreviation for camptothecin, and as used in the present application CPT represents camptothecin itself or an analog or derivative of camptothecin, such as SN-38. The structures of camptothecin and some of its analogs, with the numbering indicated and the rings labeled with letters A-E, are given in formula 1 in Chart 1 below.

Chart 1

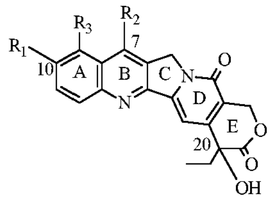

(1)

CPT: $R_1 = R_2 = R_3 = H$

10-Hydroxy-CPT: $R_1$ = OH; $R_2 = R_3 = H$

CPT-11: $R_1$ = 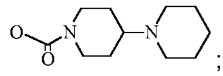 ; $R_2$ = ethyl; $R_3$ = H

SN-38: $R_1$ = OH; $R_2$ = ethyl; $R_3$ = H

Topotecan: $R_1$ = OH; $R_2$ = H; $R_3$ = $CH_2$-$N(CH_3)_2$

**General Antibody Techniques**

Techniques for preparing monoclonal antibodies against virtually any target antigen are well known in the art. *See,* for example, Köhler and Milstein, Nature 256: 495 (1975), and Coligan et al. (eds.), CURRENT PROTOCOLS IN IMMUNOLOGY, VOL. 1, pages 2.5.1-2.6.7 (John Wiley & Sons 1991). The person of ordinary skill will realize that, where human subjects are to be treated, the antibodies preferably bind to human antigens. Briefly, monoclonal antibodies can be obtained by injecting mice with a composition comprising an antigen, removing the spleen to obtain B-lymphocytes, fusing the B-lymphocytes with myeloma cells to produce hybridomas, cloning the hybridomas, selecting positive clones which produce antibodies to the antigen, culturing the clones that produce antibodies to the antigen, and isolating the antibodies from the hybridoma cultures.

MAbs can be isolated and purified from hybridoma cultures by a variety of well-established techniques. Such isolation techniques include affinity chromatography with Protein-A or Protein-G Sepharose, size-exclusion chromatography, and ion-exchange chromatography. See, for example, Coligan at pages 2.7.1-2.7.12 and pages 2.9.1-2.9.3. Also, see Baines et al., "Purification of Immunoglobulin G (IgG)," in METHODS IN MOLECULAR BIOLOGY, VOL. 10, pages 79-104 (The Humana Press, Inc. 1992).

After the initial raising of antibodies to the immunogen, the antibodies can be sequenced and subsequently prepared by recombinant techniques. Humanization and chimerization of murine antibodies and antibody fragments are well known to those skilled in the art, as discussed below.

The skilled artisan will realize that according to the invention as defined in the claims, a wide variety of antibodies known in the art may be used. Antibodies of use may be commercially obtained from a wide variety of known sources. For example, a variety of antibody secreting hybridoma lines are available from the American Type Culture Collection (ATCC, Manassas, VA). A large number of antibodies against various disease targets, including but not limited to tumor-associated antigens, have been deposited at the ATCC and/or have published variable region sequences and are available for use in the claimed methods and compositions. See, e.g., U.S. Patent Nos. 7,312,318; 7,282,567; 7,151,164; 7,074,403; 7,060,802; 7,056,509; 7,049,060; 7,045,132; 7,041,803; 7,041,802; 7,041,293; 7,038,018; 7,037,498; 7,012,133; 7,001,598; 6,998,468; 6,994,976; 6,994,852; 6,989,241; 6,974,863; 6,965,018; 6,964,854; 6,962,981; 6,962,813; 6,956,107; 6,951,924; 6,949,244; 6,946,129; 6,943,020; 6,939,547; 6,921,645; 6,921,645; 6,921,533; 6,919,433; 6,919,078; 6,916,475; 6,905,681; 6,899,879; 6,893,625; 6,887,468; 6,887,466; 6,884,594; 6,881,405; 6,878,812; 6,875,580; 6,872,568; 6,867,006; 6,864,062; 6,861,511; 6,861,227; 6,861,226; 6,838,282; 6,835,549; 6,835,370; 6,824,780; 6,824,778; 6,812,206; 6,793,924; 6,783,758; 6,770,450; 6,767,711; 6,764,688; 6,764,681; 6,764,679; 6,743,898; 6,733,981; 6,730,307; 6,720,155; 6,716,966; 6,709,653; 6,693,176; 6,692,908; 6,689,607; 6,689,362; 6,689,355; 6,682,737; 6,682,736; 6,682,734; 6,673,344; 6,653,104; 6,652,852; 6,635,482; 6,630,144; 6,610,833; 6,610,294; 6,605,441; 6,605,279; 6,596,852; 6,592,868; 6,576,745; 6,572;856; 6,566,076; 6,562,618; 6,545,130; 6,544,749; 6,534,058; 6,528,625; 6,528,269; 6,521,227; 6,518,404; 6,511,665; 6,491,915; 6,488,930; 6,482,598; 6,482,408; 6,479,247; 6,468,531; 6,468,529; 6,465,173; 6,461,823; 6,458,356; 6,455,044; 6,455,040, 6,451,310; 6,444,206; 6,441,143; 6,432,404; 6,432,402; 6,419,928; 6,413,726; 6,406,694; 6,403,770; 6,403,091; 6,395,276; 6,395,274; 6,387,350; 6,383,759; 6,383,484; 6,376,654; 6,372,215; 6,359,126; 6,355,481; 6,355,444; 6,355,245; 6,355,244; 6,346,246; 6,344,198; 6,340,571; 6,340,459; 6,331,175; 6,306,393; 6,254,868; 6,187,287; 6,183,744; 6,129,914; 6,120,767; 6,096,289; 6,077,499; 5,922,302; 5,874,540; 5,814,440; 5,798,229; 5,789,554; 5,776,456; 5,736,119; 5,716,595; 5,677,136; 5,587,459; 5,443,953, 5,525,338. These are exemplary only and a wide variety of other antibodies and their hybridomas are known in the art. The skilled artisan will realize that antibody sequences or antibody-secreting hybridomas against almost any disease-associated antigen may be obtained by a simple search of the ATCC, NCBI and/or USPTO databases for antibodies against a selected disease-associated target of interest. The antigen binding domains of the cloned antibodies may be amplified, excised, ligated into an expression vector, transfected into an adapted host cell and used for protein production, using standard techniques well known in the art. Isolated antibodies may be conjugated to therapeutic agents that induce DNA strand breaks, such as camptothecins or anthracyclines, using the techniques disclosed herein.

*Chimeric and Humanized Antibodies*

A chimeric antibody is a recombinant protein in which the variable regions of a human antibody have been replaced by the variable regions of, for example, a mouse antibody, including the complementarity-determining regions (CDRs) of the mouse antibody. Chimeric antibodies exhibit decreased immunogenicity and increased stability when administered to a subject. Methods for constructing chimeric antibodies are well known in the art (*e.g.,* Leung et al., 1994, Hybridoma 13:469).

A chimeric monoclonal antibody may be humanized by transferring the mouse CDRs from the heavy and light variable chains of the mouse immunoglobulin into the corresponding variable domains of a human antibody. The mouse framework regions (FR) in the chimeric monoclonal antibody are also replaced with human FR sequences. To preserve the stability and antigen specificity of the humanized monoclonal, one or more human FR residues may be replaced by the mouse counterpart residues. Humanized monoclonal antibodies may be used for therapeutic treatment of subjects. Techniques for production of humanized monoclonal antibodies are well known in the art. (See, *e.g.,* Jones et al., 1986, Nature, 321:522; Riechmann et al., Nature, 1988, 332:323; Verhoeyen et al., 1988, Science, 239:1534; Carter et al., 1992, Proc. Nat'l Acad. Sci. USA, 89:4285; Sandhu, Crit. Rev. Biotech., 1992, 12:437; Tempest et al., 1991, Biotechnology 9:266; Singer et al., J. Immun., 1993, 150:2844.)

Other embodiments of the present invention as defined in the claims may concern non-human primate antibodies. General techniques for raising therapeutically useful antibodies in baboons may be found, for example, in Goldenberg et al., WO 91/11465 (1991), and in Losman et al., Int. J. Cancer 46: 310 (1990). In another embodiment, an antibody may be a human monoclonal antibody. Such antibodies may be obtained from transgenic mice that have been engineered to produce specific human antibodies in response to antigenic challenge, as discussed below.

*Human Antibodies*

Methods for producing fully human antibodies using either combinatorial approaches or transgenic animals transformed with human immunoglobulin loci are known in the art (*e.g.,* Mancini et al., 2004, New Microbiol. 27:315-28; Conrad and Scheller, 2005, Comb. Chem. High Throughput Screen. 8:117-26; Brekke and Loset, 2003, Curr. Opin. Phamacol. 3:544-50). Such fully human antibodies are expected to exhibit even fewer side effects than chimeric or humanized antibodies and to function *in vivo* as essentially endogenous human antibodies. In certain embodiments of the present invention as defined in the claims, human antibodies produced by such techniques may be utilized.

In one alternative, the phage display technique may be used to generate human antibodies (*e.g.,* Dantas-Barbosa et al., 2005, Genet. Mol. Res. 4:126-40). Human antibodies may be generated from normal humans or from humans that exhibit a particular disease state, such as cancer (Dantas-Barbosa et al., 2005). The advantage to constructing human antibodies from a diseased individual is that the circulating antibody repertoire may be biased towards antibodies against disease-associated antigens.

In one non-limiting example of this methodology, Dantas-Barbosa et al. (2005) constructed a phage display library of human Fab antibody fragments from osteosarcoma patients. Generally, total RNA was obtained from circulating blood lymphocytes *(Id.)* Recombinant Fab were cloned from the $\mu$, $\gamma$ and $\kappa$ chain antibody repertoires and inserted into a phage display library *(Id.)* RNAs were converted to cDNAs and used to make Fab cDNA libraries using specific primers against the heavy and light chain immunoglobulin sequences (Marks et al., 1991, J. Mol. Biol. 222:581-97). Library construction was performed according to Andris-Widhopf et al. (2000, In: Phage Display Laboratory Manual, Barbas et al. (eds), 1st edition, Cold Spring Harbor Laboratory Press, Cold Spring Harbor, NY pp. 9.1 to 9.22). The final Fab fragments were digested with restriction endonucleases and inserted into the bacteriophage genome to make the phage display library. Such libraries may be screened by standard phage display methods. The skilled artisan will realize that this technique is exemplary only and any known method for making and screening human antibodies or antibody fragments by phage display may be utilized.

In another alternative, transgenic animals that have been genetically engineered to produce human antibodies may be used to generate antibodies against essentially any immunogenic target, using standard immunization protocols as discussed above. Methods for obtaining human antibodies from transgenic mice are described by Green et al., Nature Genet. 7:13 (1994), Lonberg et al., Nature 368:856 (1994), and Taylor et al., Int. Immun. 6:579 (1994). A non-limiting example of such a system is the XENOMOUSE® (e.g., Green et al., 1999, J. Immunol. Methods 231:11-23) from Abgenix (Fremont, CA). In the XENOMOUSE® and similar animals, the mouse antibody genes have been inactivated and replaced by functional human antibody genes, while the remainder of the mouse immune system remains intact.

The XENOMOUSE® was transformed with germline-configured YACs (yeast artificial chromosomes) that contained portions of the human IgH and Ig kappa loci, including the majority of the variable region sequences, along accessory genes and regulatory sequences. The human variable region repertoire may be used to generate antibody producing B cells, which may be processed into hybridomas by known techniques. A XENOMOUSE® immunized with a target antigen will produce human antibodies by the normal immune response, which may be harvested and/or produced

by standard techniques discussed above. A variety of strains of XENOMOUSE® are available, each of which is capable of producing a different class of antibody. Transgenically produced human antibodies have been shown to have therapeutic potential, while retaining the pharmacokinetic properties of normal human antibodies (Green et al., 1999). The skilled artisan will realize that the claimed compositions and methods are not limited to use of the XENOMOUSE® system but may utilize any transgenic animal that has been genetically engineered to produce human antibodies.

*Production of Antibody Fragments*

Some embodiments of the invention as defined in the claims may concern antibody fragments. Such antibody fragments may be obtained, for example, by pepsin or papain digestion of whole antibodies by conventional methods. For example, antibody fragments may be produced by enzymatic cleavage of antibodies with pepsin to provide a 5S fragment denoted $F(ab')_2$. This fragment may be further cleaved using a thiol reducing agent and, optionally, a blocking group for the sulfhydryl groups resulting from cleavage of disulfide linkages, to produce 3.5S Fab' monovalent fragments. Alternatively, an enzymatic cleavage using pepsin produces two monovalent Fab fragments and an Fc fragment. Exemplary methods for producing antibody fragments are disclosed in U.S. Pat. No. 4,036,945; U.S. Pat. No. 4,331,647; Nisonoff et al., 1960, Arch. Biochem. Biophys., 89:230; Porter, 1959, Biochem. J., 73:119; Edelman et al., 1967, METHODS IN ENZYMOLOGY, page 422 (Academic Press), and Coligan et al. (eds.), 1991, CURRENT PROTOCOLS IN IMMUNOLOGY, (John Wiley & Sons).

Other methods of cleaving antibodies, such as separation of heavy chains to form monovalent light-heavy chain fragments, further cleavage of fragments or other enzymatic, chemical or genetic techniques also may be used, so long as the fragments bind to the antigen that is recognized by the intact antibody. For example, Fv fragments comprise an association of $V_H$ and $V_L$ chains. This association can be noncovalent, as described in Inbar et al., 1972, Proc. Nat'l. Acad. Sci. USA, 69:2659. Alternatively, the variable chains may be linked by an intermolecular disulfide bond or cross-linked by chemicals such as glutaraldehyde. See Sandhu, 1992, Crit. Rev. Biotech., 12:437.

Preferably, the Fv fragments comprise $V_H$ and $V_L$ chains connected by a peptide linker. These single-chain antigen binding proteins (scFv) are prepared by constructing a structural gene comprising DNA sequences encoding the $V_H$ and $V_L$ domains, connected by an oligonucleotides linker sequence. The structural gene is inserted into an expression vector that is subsequently introduced into a host cell, such as E. coli. The recombinant host cells synthesize a single polypeptide chain with a linker peptide bridging the two V domains. Methods for producing scFvs are well-known in the art. See Whitlow et al., 1991, Methods: A Companion to Methods in Enzymology 2:97; Bird et al., 1988, Science, 242:423; U.S. Pat. No. 4,946,778; Pack et al., 1993, Bio/Technology, 11:1271, and Sandhu, 1992, Crit. Rev. Biotech., 12:437.

Another form of an antibody fragment is a single-domain antibody (dAb), sometimes referred to as a single chain antibody. Techniques for producing single-domain antibodies are well known in the art (see, e.g., Cossins et al., Protein Expression and Purification, 2007, 51:253-59; Shuntao et al., Molec Immunol 2006, 43:1912-19; Tanha et al., J. Biol. Chem. 2001, 276:24774-780). Other types of antibody fragments may comprise one or more complementarity-determining regions (CDRs). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See Larrick et al., 1991, Methods: A Companion to Methods in Enzymology 2:106; Ritter et al. (eds.), 1995, MONOCLONAL ANTIBODIES: PRODUCTION, ENGINEERING AND CLINICAL APPLICATION, pages 166-179 (Cambridge University Press); Birch et al., (eds.), 1995, MONOCLONAL ANTIBODIES: PRINCIPLES AND APPLICATIONS, pages 137-185 (Wiley-Liss, Inc.)

*Antibody Variations*

In certain embodiments, the sequences of antibodies, such as the Fc portions of antibodies, may be varied to optimize the physiological characteristics of the conjugates, such as the half-life in serum. Methods of substituting amino acid sequences in proteins are widely known in the art, such as by site-directed mutagenesis (e.g. Sambrook et al., Molecular Cloning, A laboratory manual, 2nd Ed, 1989). In preferred embodiments, the variation may involve the addition or removal of one or more glycosylation sites in the Fc sequence (e.g., U.S. Patent No. 6,254,868). In other preferred embodiments, specific amino acid substitutions in the Fc sequence may be made (e.g., Hornick et al., 2000, J Nucl Med 41:355-62; Hinton et al., 2006, J Immunol 176:346-56; Petkova et al. 2006, Int Immunol 18:1759-69; U.S. Patent No. 7,217,797).

## Target Antigens and Exemplary Antibodies

In a preferred embodiment of the invention as defined in the claims, antibodies are used that recognize and/or bind to human antigens that are expressed at high levels on target cells and that are expressed predominantly or

exclusively on diseased cells versus normal tissues. More preferably, the antibodies internalize rapidly following binding. An exemplary rapidly internalizing antibody is the LL1 (anti-CD74) antibody, with a rate of internalization of approximately $8 \times 10^6$ antibody molecules per cell per day (e.g., Hansen et al., 1996, Biochem J. 320:293-300). Thus, a "rapidly internalizing" antibody may be one with an internalization rate of about $1 \times 10^6$ to about $1 \times 10^7$ antibody molecules per cell per day. Antibodies of use in the claimed compositions and methods may include MAbs with properties as recited above. Exemplary antibodies of use for therapy of, for example, cancer include but are not limited to LL1 (anti-CD74), LL2 or RFB4 (anti-CD22), veltuzumab (hA20, anti-CD20), rituxumab (anti-CD20), obinutuzumab (GA101, anti-CD20), lambrolizumab (anti-PD-1 receptor), nivolumab (anti-PD-1 receptor), ipilimumab (anti-CTLA-4), RS7 (anti-epithelial glycoprotein-1 (EGP-1, also known as Trop-2)), PAM4 or KC4 (both anti-mucin), MN-14 (anti-carcinoembryonic antigen (CEA, also known as CD66e or CEACAM5), MN-15 or MN-3 (anti-CEACAM6), Mu-9 (anti-colon-specific antigen-p), Immu 31 (an anti-alpha-fetoprotein), R1 (anti-IGF-1R), A19 (anti-CD19), TAG-72 (e.g., CC49), Tn, J591 or HuJ591 (anti-PSMA (prostate-specific membrane antigen)), AB-PG1-XG1-026 (anti-PSMA dimer), D2/B (anti-PSMA), G250 (an anti-carbonic anhydrase IX MAb), L243 (anti-HLA-DR) alemtuzumab (anti-CD52), bevacizumab (anti-VEGF), cetuximab (anti-EGFR), gemtuzumab (anti-CD33), ibritumomab tiuxetan (anti-CD20); panitumumab (anti-EGFR); tositumomab (anti-CD20); PAM4 (aka clivatuzumab, anti-mucin) and trastuzumab (anti-ErbB2). Such antibodies are known in the art (e.g., U.S. Patent Nos. 5,686,072; 5,874,540; 6,107,090; 6,183,744; 6,306,393; 6,653,104; 6,730.300; 6,899,864; 6,926,893; 6,962,702; 7,074,403; 7,230,084; 7,238,785; 7,238,786; 7,256,004; 7,282,567; 7,300,655; 7,312,318; 7,585,491; 7,612,180; 7,642,239; and U.S. Patent Application Publ. No. 20050271671; 20060193865; 20060210475; 20070087001.) Specific known antibodies of use include hPAM4 (U.S. Patent No. 7,282,567), hA20 (U.S. Patent No. 7,151,164), hA19 (U.S. Patent No. 7,109,304), hIMMU-31 (U.S. Patent No. 7,300,655), hLL1 (U.S. Patent No. 7,312,318, ), hLL2 (U.S. Patent No. 5,789,554), hMu-9 (U.S. Patent No. 7,387,772), hL243 (U.S. Patent No. 7,612,180), hMN-14 (U.S. Patent No. 6,676,924), hMN-15 (U.S. Patent No.8,287,865), hR1 (U.S. Patent Application 14/061,1767), hRS7 (U.S. Patent No. 7,238,785), hMN-3 (U.S. Patent No. 7,541,440), AB-PG1-XG1-026 (U.S. Patent Application 11/983,372, deposited as ATCC PTA-4405 and PTA-4406) and D2/B (WO 2009/130575).

Other useful antigens that may be targeted using the described conjugates include carbonic anhydrase IX, B7, CCL19, CCL21, CSAp, HER-2/neu, BrE3, CD1, CD1a, CD2, CD3, CD4, CD5, CD8, CD11A, CD14, CD15, CD16, CD18, CD19, CD20 (e.g., C2B8, hA20, 1F5 MAbs), CD21, CD22, CD23, CD25, CD29, CD30, CD32b, CD33, CD37, CD38, CD40, CD40L, CD44, CD45, CD46, CD52, CD54, CD55, CD59, CD64, CD67, CD70, CD74, CD79a, CD80, CD83, CD95, CD126, CD133, CD138, CD147, CD154, CEACAM5, CEACAM6, CTLA-4, alpha-fetoprotein (AFP), VEGF (e.g., AVASTIN®, fibronectin splice variant), ED-B fibronectin (e.g., L19), EGP-1 (Trop-2), EGP-2 (e.g., 17-1A), EGF receptor (ErbB1) (e.g., ERBITUX®), ErbB2, ErbB3, Factor H, FHL-1, Flt-3, folate receptor, Ga 733,GRO-$\beta$, HMGB-1, hypoxia inducible factor (HIF), HM1.24, HER-2/neu, insulin-like growth factor (ILGF), IFN-$\gamma$, IFN-$\alpha$, IFN-$\beta$, IFN-$\lambda$, IL-2R, IL-4R, IL-6R, IL-13R, IL-15R, IL-17R, IL-18R, IL-2, IL-6, IL-8, IL-12, IL-15, IL-17, IL-18, IL-25, IP-10, IGF-1R, Ia, HM1.24, gangliosides, HCG, the HLA-DR antigen to which L243 binds, CD66 antigens, i.e., CD66a-d or a combination thereof, MAGE, mCRP, MCP-1, MIP-1A, MIP-1B, macrophage migration-inhibitory factor (MIF), MUC1, MUC2, MUC3, MUC4, MUC5ac, placental growth factor (PlGF), PSA (prostate-specific antigen), PSMA, PAM4 antigen, PD-1 receptor, NCA-95, NCA-90, A3, A33, Ep-CAM, KS-1, Le(y), mesothelin, S100, tenascin, TAC, Tn antigen, Thomas-Friedenreich antigens, tumor necrosis antigens, tumor angiogenesis antigens, TNF-$\alpha$, TRAIL receptor (R1 and R2), Trop-2, VEGFR, RANTES, T101, as well as cancer stem cell antigens, complement factors C3, C3a, C3b, C5a, C5, and an oncogene product.

A comprehensive analysis of suitable antigen (**C**luster **D**esignation, or CD) targets on hematopoietic malignant cells, as shown by flow cytometry and which can be a guide to selecting suitable antibodies for drug-conjugated immunotherapy, is Craig and Foon, Blood prepublished online January 15, 2008; DOL 10.1182/blood-2007-11-120535.

The CD66 antigens consist of five different glycoproteins with similar structures, CD66a-e, encoded by the carcinoembryonic antigen (CEA) gene family members, BCG, CGM6, NCA, CGM1 and CEA, respectively. These CD66 antigens (e.g., CEACAM6) are expressed mainly in granulocytes, normal epithelial cells of the digestive tract and tumor cells of various tissues. Also included as suitable targets for cancers are cancer testis antigens, such as NY-ESO-1 (Theurillat et al., Int. J. Cancer 2007; 120(11):2411-7), as well as CD79a in myeloid leukemia (Kozlov et al., Cancer Genet. Cytogenet. 2005; 163(1):62-7) and also B-cell diseases, and CD79b for non-Hodgkin's lymphoma (Poison et al., Blood 110(2):616-623). A number of the aforementioned antigens are disclosed in U.S. Provisional Application Serial No. 60/426,379, entitled "Use of Multi-specific, Non-covalent Complexes for Targeted Delivery of Therapeutics," filed November 15, 2002. Cancer stem cells, which are ascribed to be more therapy-resistant precursor malignant cell populations (Hill and Perris, J. Natl. Cancer Inst. 2007; 99:1435-40), have antigens that can be targeted in certain cancer types, such as CD 133 in prostate cancer (Maitland et al., Ernst Schering Found. Sympos. Proc. 2006; 5:155-79), non-small-cell lung cancer (Donnenberg et al., J. Control Release 2007; 122(3):385-91), and glioblastoma (Beier et al., Cancer Res. 2007; 67(9):4010-5), and CD44 in colorectal cancer (Dalerba er al., Proc. Natl. Acad. Sci. USA 2007; 104(24)10158-63), pancreatic cancer (Li et al., Cancer Res. 2007; 67(3):1030-7), and in head and neck squamous cell carcinoma (Prince et al., Proc. Natl. Acad. Sci. USA 2007; 104(3)973-8).

For multiple myeloma therapy, suitable targeting antibodies have been described against, for example, CD38 and CD138 (Stevenson, Mol Med 2006; 12(11-12):345-346; Tassone et al., Blood 2004; 104(12):3688-96), CD74 (Stein et al., *ibid.),* CS1 (Tai et al., Blood 2008; 112(4):1329-37, and CD40 (Tai et al., 2005; Cancer Res. 65(13):5898-5906).

Macrophage migration inhibitory factor (MIF) is an important regulator of innate and adaptive immunity and apoptosis. It has been reported that CD74 is the endogenous receptor for MIF (Leng et al., 2003, J Exp Med 197:1467-76). The therapeutic effect of antagonistic anti-CD74 antibodies on MIF-mediated intracellular pathways may be of use for treatment of a broad range of disease states, such as cancers of the bladder, prostate, breast, lung, colon and chronic lymphocytic leukemia (e.g., Meyer-Siegler et al., 2004, BMC Cancer 12:34; Shachar & Haran, 2011, Leuk Lymphoma 52:1446-54); autoimmune diseases such as rheumatoid arthritis and systemic lupus erythematosus (Morand & Leech, 2005, Front Biosci 10:12-22; Shachar & Haran, 2011, Leuk Lymphoma 52:1446-54); kidney diseases such as renal allograft rejection (Lan, 2008, Nephron Exp Nephrol. 109:e79-83); and numerous inflammatory diseases (Meyer-Siegler et al., 2009, Mediators Inflamm epub March 22, 2009; Takahashi et al., 2009, Respir Res 10:33; Milatuzumab (hLL1) is an exemplary anti-CD74 antibody of therapeutic use for treatment of MIF-mediated diseases.

Anti-TNF-$\alpha$ antibodies are known in the art and may be of use to treat immune diseases, such as autoimmune disease, immune dysfunction (e.g., graft-versus-host disease, organ transplant rejection) or diabetes. Known antibodies against TNF-$\alpha$ include the human antibody CDP571 (Ofei et al., 2011, Diabetes 45:881-85); murine antibodies MTNFAI, M2TNFAI, M3TNFAI, M3TNFABI, M302B and M303 (Thermo Scientific, Rockford, IL); infliximab (Centocor, Malvern, PA); certolizumab pegol (UCB, Brussels, Belgium); and adalimumab (Abbott, Abbott Park, IL). These and many other known anti-TNF-$\alpha$ antibodies may be used in the claimed methods and compositions. Other antibodies of use for therapy of immune dysregulatory or autoimmune disease include, but are not limited to, anti-B-cell antibodies such as veltuzumab, epratuzumab, milatuzumab or hL243; tocilizumab (anti-IL-6 receptor); basiliximab (anti-CD25); daclizumab (anti-CD25); efalizumab (anti-CD11a); muromonab-CD3 (anti-CD3 receptor); anti-CD40L (UCB, Brussels, Belgium); natalizumab (anti-$\alpha$4 integrin) and omalizumab (anti-IgE).

Type-1 and Type-2 diabetes may be treated using known antibodies against B-cell antigens, such as CD22 (epratuzumab and hRFB4), CD74 (milatuzumab), CD19 (hA19), CD20 (veltuzumab) or HLA-DR (hL243) *(see, e.g.,* Winer et al., 2011, Nature Med 17:610-18). Anti-CD3 antibodies also have been proposed for therapy of type 1 diabetes (Cernea et al., 2010, Diabetes Metab Rev 26:602-05).

In another preferred embodiment of the invention as defined in the claims, antibodies are used that internalize rapidly and are then re-expressed, processed and presented on cell surfaces, enabling continual uptake and accretion of circulating conjugate by the cell. An example of a most-preferred antibody/antigen pair is LL1, an anti-CD74 MAb (invariant chain, class II-specific chaperone, Ii) (see, e.g., U.S. Patent Nos. 6,653,104; 7,312,318). The CD74 antigen is highly expressed on B-cell lymphomas (including multiple myeloma) and leukemias, certain T-cell lymphomas, melanomas, colonic, lung, and renal cancers, glioblastomas, and certain other cancers (Ong et al., Immunology 98:296-302 (1999)). A review of the use of CD74 antibodies in cancer is contained in Stein et al., Clin Cancer Res. 2007 Sep 15;13(18 Pt 2):5556s-5563s.

The diseases that are preferably treated with anti-CD74 antibodies include, but are not limited to, non-Hodgkin's lymphoma, Hodgkin's disease, melanoma, lung, renal, colonic cancers, glioblastome multiforme, histiocytomas, myeloid leukemias, and multiple myeloma. Continual expression of the CD74 antigen for short periods of time on the surface of target cells, followed by internalization of the antigen, and re-expression of the antigen, enables the targeting LL1 antibody to be internalized along with any drug moiety it carries. This allows a high, and therapeutic, concentration of LL1-drug conjugate to be accumulated inside such cells. Internalized LL1-drug conjugates are cycled through lysosomes and endosomes, and the drug moiety is released in an active form within the target cells.

Antibodies of use to treat autoimmune disease or immune system dysfunctions (e.g., graft-versus-host disease, organ transplant rejection) are known in the art and may be conjugated to SN-38 using the disclosed methods and compositions. Antibodies of use to treat autoimmune/immune dysfunction disease may bind to exemplary antigens including, but not limited to, BCL-1, BCL-2, BCL-6, CD1a, CD2, CD3, CD4, CD5, CD7, CD8, CD10, CD11b, CD11c, CD13, CD14, CD15, CD16, CD19, CD20, CD21, CD22, CD23, CD25, CD33, CD34, CD38, CD40, CD40L, CD41a, CD43, CD45, CD55, CD74, TNF-alpha, interferon and HLA-DR. Antibodies that bind to these and other target antigens, discussed above, may be used to treat autoimmune or immune dysfunction diseases. Autoimmune diseases that may be treated with antibodies or immunoconjugates may include acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, diabetes mellitus, Henoch-Schonlein purpura, post-streptococcal nephritis, erythema nodosum, Takayasu's arteritis, ANCA-associated vasculitides, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitis obliterans, Sjogren's syndrome, primary biliary cirrhosis, Hashimoto's thyroiditis, thyrotoxicosis, scleroderma, chronic active hepatitis, polymyositis/dermatomyositis, polychondritis, bullous pemphigoid, pemphigus vulgaris, Wegener's granulomatosis, membranous nephropathy, amyotrophic lateral sclerosis, tabes dorsalis, giant cell arteritis/polymyalgia, pernicious anemia,

rapidly progressive glomerulonephritis, psoriasis or fibrosing alveolitis.

The antibodies discussed above and other known antibodies against disease-associated antigens may be used as CPT-conjugates, more preferably SN-38-conjugates, in the practice of the invention as defined in the claims.

## Bispecific and Multispecific Antibodies

Bispecific antibodies are useful in a number of biomedical applications. For instance, a bispecific antibody with binding sites for a tumor cell surface antigen and for a T-cell surface receptor can direct the lysis of specific tumor cells by T cells. Bispecific antibodies recognizing gliomas and the CD3 epitope on T cells have been successfully used in treating brain tumors in human patients (Nitta, et al. Lancet. 1990; 355:368-371). A preferred bispecific antibody is an anti-CD3 × anti-CD19 antibody. Alternatively, an anti-CD3 antibody or fragment thereof may be attached to an antibody or fragment against another B-cell associated antigen, such as anti-CD3 × anti-CD20, anti-CD3 × anti-CD22, anti-CD3 × anti-HLA-DR or anti-CD3 × anti-CD74. In certain embodiments of the invention as defined in the claims, the techniques and compositions for therapeutic agent conjugation disclosed herein may be used with bispecific or multispecific antibodies as the targeting moieties.

Numerous methods to produce bispecific or multispecific antibodies are known, as disclosed, for example, in U.S. Patent No. 7,405,320. Bispecific antibodies can be produced by the quadroma method, which involves the fusion of two different hybridomas, each producing a monoclonal antibody recognizing a different antigenic site (Milstein and Cuello, Nature, 1983; 305:537-540).

Another method for producing bispecific antibodies uses heterobifunctional crosslinkers to chemically tether two different monoclonal antibodies (Staerz, et al. Nature, 1985; 314:628-631; Perez, et al. Nature, 1985; 316:354-356). Bispecific antibodies can also be produced by reduction of each of two parental monoclonal antibodies to the respective half molecules, which are then mixed and allowed to reoxidize to obtain the hybrid structure (Staerz and Bevan. Proc Natl Acad Sci U S A. 1986; 83:1453-1457). Another alternative involves chemically cross-linking two or three separately purified Fab' fragments using appropriate linkers. (See, e.g., European Patent Application 0453082).

Other methods include improving the efficiency of generating hybrid hybridomas by gene transfer of distinct selectable markers via retrovirus-derived shuttle vectors into respective parental hybridomas, which are fused subsequently (DeMonte, et al. Proc Natl Acad Sci U S A. 1990, 87:2941-2945); or transfection of a hybridoma cell line with expression plasmids containing the heavy and light chain genes of a different antibody.

Cognate $V_H$ and $V_L$ domains can be joined with a peptide linker of appropriate composition and length (usually consisting of more than 12 amino acid residues) to form a single-chain Fv (scFv) with binding activity. Methods of manufacturing scFvs are disclosed in U.S. Pat. No. 4,946,778 and U.S. Pat. No. 5,132,405. Reduction of the peptide linker length to less than 12 amino acid residues prevents pairing of $V_H$ and $V_L$ domains on the same chain and forces pairing of $V_H$ and $V_L$ domains with complementary domains on other chains, resulting in the formation of functional multimers. Polypeptide chains of $V_H$ and $V_L$ domains that are joined with linkers between 3 and 12 amino acid residues form predominantly dimers (termed diabodies). With linkers between 0 and 2 amino acid residues, trimers (termed triabody) and tetramers (termed tetrabody) are favored, but the exact patterns of oligomerization appear to depend on the composition as well as the orientation of V-domains ($V_H$-linker-$V_L$ or $V_L$-linker-$V_H$), in addition to the linker length.

These techniques for producing multispecific or bispecific antibodies exhibit various difficulties in terms of low yield, necessity for purification, low stability or the labor-intensiveness of the technique. More recently, a technique known as "dock and lock" (DNL) has been utilized to produce combinations of virtually any desired antibodies, antibody fragments and other effector molecules (see, e.g., U.S. Patent Nos. 7,521,056; 7,527,787; 7,534,866; 7,550,143; 7,666,400; 7,858,070; 7,871,622; 7,906,121; 7,906,118; 8,163,291; 7,901,680; 7,981,398; 8,003,111 and 8,034,352). The technique utilizes complementary protein binding domains, referred to as anchoring domains (AD) and dimerization and docking domains (DDD), which bind to each other and allow the assembly of complex structures, ranging from dimers, trimers, tetramers, quintamers and hexamers. These form stable complexes in high yield without requirement for extensive purification. The DNL technique allows the assembly of monospecific, bispecific or multispecific antibodies. Any of the techniques known in the art for making bispecific or multispecific antibodies may be utilized in the practice of the invention as defined in the claims.

In various embodiments of the invention as defined in the claims, a conjugate as disclosed herein may be part of a composite, multispecific antibody. Such antibodies may contain two or more different antigen binding sites, with differing specificities. The multispecific composite may bind to different epitopes of the same antigen, or alternatively may bind to two different antigens. Some of the more preferred target combinations include those listed in **Table 1.** This is a list of examples of preferred combinations, but is not intended to be exhaustive.

**Table 1. Some Examples of multispecific antibodies.**

| First target | Second target |
|---|---|
| MIF | A second proinflammatory effector cytokine, especially HMGB-1, TNF-α, IL-1, or IL-6 |
| MIF | Proinflammatory effector chemokine, especially MCP-1, RANTES, MIP-1A, or MIP-1B |
| MIF | Proinflammatory effector receptor, especially IL-6R, IL-13R, and IL-15R |
| MIF | Coagulation factor, especially TF or thrombin |
| MIF | Complement factor, especially C3, C5, C3a, or C5a |
| MIF | Complement regulatory protein, especially CD46, CD55, CD59, and mCRP |
| MIF | Cancer associated antigen or receptor |
| HMGB-1 | A second proinflammatory effector cytokine, especially MIF, TNF-α, IL-1, or IL-6 |
| HMGB-1 | Proinflammatory effector chemokine, especially MCP-1, RANTES, MIP-1A, or MIP-1B |
| HMGB-1 | Proinflammatory effector receptor especially MCP-1, RANTES, MIP-1A, or MIP-1B |
| HMGB-1 | Coagulation factor, especially TF or thrombin |
| HMGB-1 | Complement factor, especially C3, C5, C3a, or C5a |
| HMGB-1 | Complement regulatory protein, especially CD46, CD55, CD59, and mCRP |
| HMGB-1 | Cancer associated antigen or receptor |
| TNF-α | A second proinflammatory effector cytokine, especially MIF, HMGB-1, TNF-α, IL-1, or IL-6 |
| TNF-α | Proinflammatory effector chemokine, especially MCP-1, RANTES, MIP-1A, or MIP-1B |
| TNF-α | Proinflammatory effector receptor, especially IL-6R IL-13R, and IL-15R |
| TNF-α | Coagulation factor, especially TF or thrombin |
| TNF-α | Complement factor, especially C3, C5, C3a, or C5a |
| TNF-α | Complement regulatory protein, especially CD46, CD55, CD59, and mCRP |
| TNF-α | Cancer associated antigen or receptor |
| LPS | Proinflammatory effector cytokine, especially MIF, HMGB-1, TNF-α, IL-1, or IL-6 |
| LPS | Proinflammatory effector chemokine, especially MCP-1, RANTES, MIP-1A, or MIP-1B |
| LPS | Proinflammatory effector receptor, especially IL-6R IL-13R, and IL-15R |
| LPS | Coagulation factor, especially TF or thrombin |
| LPS | Complement factor, especially C3, C5, C3a, or C5a |
| LPS | Complement regulatory protein, especially CD46, CD55, CD59, and mCRP |
| TF or thrombin | Proinflammatory effector cytokine, especially MIF, HMGB-1, TNF-α, IL-1, or IL-6 |
| TF or thrombin | Proinflammatory effector chemokine, especially MCP-1, RANTES, MIP-1A, or MIP-1B |
| TF or thrombin | Proinflammatory effector receptor, especially IL-6R IL-13R, and IL-15R |
| TF or thrombin | Complement factor, especially C3, C5, C3a, or C5a |
| TF or thrombin | Complement regulatory protein, especially CD46, CD55, CD59, and mCRP |
| TF or thrombin | Cancer associated antigen or receptor |

Still other combinations, such as are preferred for cancer therapies, include CD20 + CD22 antibodies, CD74 + CD20 antibodies, CD74 + CD22 antibodies, CEACAM5 (CEA) + CEACAM6 (NCA) antibodies, insulin-like growth factor (ILGF) + CEACAM5 antibodies, EGP-1 (e.g., RS-7) + ILGF antibodies, CEACAM5 + EGFR antibodies, IL6 + CEACAM6 antibodies, CD74 and HLA-DR antibodies, and CD22 and HLA-DR antibodies. Such antibodies need not only be used in combination, but can be combined as fusion proteins of various forms, such as IgG, Fab, scFv, and the like, as described in U.S. Patent Nos. 6,083,477; 6,183,744 and 6,962,702 and U.S. Patent Application Publication Nos.

20030124058; 20030219433; 20040001825; 20040202666; 20040219156; 20040219203; 20040235065; 20050002945; 20050014207; 20050025709; 20050079184; 20050169926; 20050175582; 20050249738; 20060014245 and 20060034759.

**DOCK-AND-LOCKO (DNLO)**

In preferred embodiments, a bivalent or multivalent antibody is formed as a DOCK-AND-LOCK® (DNL®) complex (see, e.g., U.S. Patent Nos. 7,521,056; 7,527,787; 7,534,866; 7,550,143; 7,666,400; 7,858,070; 7,871,622; 7,906,121; 7,906,118; 8,163,291; 7,901,680; 7,981,398; 8,003,111 and 8,034,352.) Generally, the technique takes advantage of the specific and high-affinity binding interactions that occur between a dimerization and docking domain (DDD) sequence of the regulatory (R) subunits of cAMP-dependent protein kinase (PKA) and an anchor domain (AD) sequence derived from any of a variety of AKAP proteins (Baillie *et al., FEBS Letters.* 2005; 579: 3264. Wong and Scott, *Nat. Rev. Mol. Cell Biol.* 2004; 5: 959). The DDD and AD peptides may be attached to any protein, peptide or other molecule. Because the DDD sequences spontaneously dimerize and bind to the AD sequence, the technique allows the formation of complexes between any selected molecules that may be attached to DDD or AD sequences.

Although the standard DNL® complex comprises a trimer with two DDD-linked molecules attached to one AD-linked molecule, variations in complex structure allow the formation of dimers, trimers, tetramers, pentamers, hexamers and other multimers. In some embodiments, the DNL® complex may comprise two or more antibodies, antibody fragments or fusion proteins which bind to the same antigenic determinant or to two or more different antigens. The DNL® complex may also comprise one or more other effectors, such as proteins, peptides, immunomodulators, cytokines, interleukins, interferons, binding proteins, peptide ligands, carrier proteins, toxins, ribonucleases such as onconase, inhibitory oligonucleotides such as siRNA, antigens or xenoantigens, polymers such as PEG, enzymes, therapeutic agents, hormones, cytotoxic agents, anti-angiogenic agents, pro-apoptotic agents or any other molecule or aggregate.

PKA, which plays a central role in one of the best studied signal transduction pathways triggered by the binding of the second messenger cAMP to the R subunits, was first isolated from rabbit skeletal muscle in 1968 (Walsh et al., J. Biol. Chem. 1968;243:3763). The structure of the holoenzyme consists of two catalytic subunits held in an inactive form by the R subunits (Taylor, J. Biol. Chem. 1989;264:8443). Isozymes of PKA are found with two types of R subunits (RI and RII), and each type has α and β isoforms (Scott, Pharmacol. Ther. 1991;50: 123). Thus, the four isoforms of PKA regulatory subunits are RIα, RIβ, RIIα and RIIβ. The R subunits have been isolated only as stable dimers and the dimerization domain has been shown to consist of the first 44 amino-terminal residues of RIIα (Newlon et al., Nat. Struct. Biol. 1999; 6:222). As discussed below, similar portions of the amino acid sequences of other regulatory subunits are involved in dimerization and docking, each located near the N-terminal end of the regulatory subunit. Binding of cAMP to the R subunits leads to the release of active catalytic subunits for a broad spectrum of serine/threonine kinase activities, which are oriented toward selected substrates through the compartmentalization of PKA via its docking with AKAPs (Scott et al., J. Biol. Chem. 1990;265;21561)

Since the first AKAP, microtubule-associated protein-2, was characterized in 1984 (Lohmann et al., Proc. Natl. Acad. Sci USA. 1984; 81:6723), more than 50 AKAPs that localize to various sub-cellular sites, including plasma membrane, actin cytoskeleton, nucleus, mitochondria, and endoplasmic reticulum, have been identified with diverse structures in species ranging from yeast to humans (Wong and Scott, Nat. Rev. Mol. Cell Biol. 2004;5:959). The AD of AKAPs for PKA is an amphipathic helix of 14-18 residues (Carr et al., J. Biol. Chem. 1991;266:14188). The amino acid sequences of the AD are quite varied among individual AKAPs, with the binding affinities reported for RII dimers ranging from 2 to 90 nM (Alto et al., Proc. Natl. Acad. Sci. USA. 2003;100:4445). AKAPs will only bind to dimeric R subunits. For human RIIα, the AD binds to a hydrophobic surface formed by the 23 amino-terminal residues (Colledge and Scott, Trends Cell Biol. 1999; 6:216). Thus, the dimerization domain and AKAP binding domain of human RIIα are both located within the same N-terminal 44 amino acid sequence (Newlon et al., Nat. Struct. Biol. 1999;6:222; Newlon et al., EMBO J. 2001;20:1651), which is termed the DDD herein.

We have developed a platform technology to utilize the DDD of human PKA regulatory subunits and the AD of AKAP as an excellent pair of linker modules for docking any two entities, referred to hereafter as **A** and **B,** into a noncovalent complex, which could be further locked into a DNL® complex through the introduction of cysteine residues into both the DDD and AD at strategic positions to facilitate the formation of disulfide bonds. The general methodology of the approach is as follows. Entity **A** is constructed by linking a DDD sequence to a precursor of **A,** resulting in a first component hereafter referred to as a. Because the DDD sequence would effect the spontaneous formation of a dimer, **A** would thus be composed of $\mathbf{a_2}$. Entity **B** is constructed by linking an AD sequence to a precursor of **B,** resulting in a second component hereafter referred to as **b.** The dimeric motif of DDD contained in $\mathbf{a_2}$ will create a docking site for binding to the AD sequence contained in **b,** thus facilitating a ready association of $\mathbf{a_2}$ and **b** to form a binary, trimeric complex composed of $\mathbf{a_2b}$. This binding event is made irreversible with a subsequent reaction to covalently secure the two entities via disulfide bridges, which occurs very efficiently based on the principle of effective local concentration because the initial binding interactions should bring the reactive thiol groups placed onto both the DDD and AD into

proximity (Chmura et al., Proc. Natl. Acad. Sci. USA. 2001;98:8480) to ligate site-specifically. Using various combinations of linkers, adaptor modules and precursors, a wide variety of DNL® constructs of different stoichiometry may be produced and used (see, e.g., U.S. Nos. 7,550,143; 7,521,056; 7,534,866; 7,527,787 and 7,666,400.)

By attaching the DDD and AD away from the functional groups of the two precursors, such site-specific ligations are also expected to preserve the original activities of the two precursors. This approach is modular in nature and potentially can be applied to link, site-specifically and covalently, a wide range of substances, including peptides, proteins, antibodies, antibody fragments, and other effector moieties with a wide range of activities. Utilizing the fusion protein method of constructing AD and DDD conjugated effectors described in the Examples below, virtually any protein or peptide may be incorporated into a DNL® construct. However, the technique is not limiting and other methods of conjugation may be utilized.

A variety of methods are known for making fusion proteins, including nucleic acid synthesis, hybridization and/or amplification to produce a synthetic double-stranded nucleic acid encoding a fusion protein of interest. Such double-stranded nucleic acids may be inserted into expression vectors for fusion protein production by standard molecular biology techniques (see, e.g. Sambrook et al., Molecular Cloning, A laboratory manual, 2nd Ed, 1989). In such preferred embodiments, the AD and/or DDD moiety may be attached to either the N-terminal or C-terminal end of an effector protein or peptide. However, the skilled artisan will realize that the site of attachment of an AD or DDD moiety to an effector moiety may vary, depending on the chemical nature of the effector moiety and the part(s) of the effector moiety involved in its physiological activity. Site-specific attachment of a variety of effector moieties may be performed using techniques known in the art, such as the use of bivalent cross-linking reagents and/or other chemical conjugation techniques.

In various embodiments of the invention as defined in the claims, an antibody or antibody fragment may be incorporated into a DNL® complex by, for example, attaching a DDD or AD moiety to the C-terminal end of the antibody heavy chain, as described in detail below. In more preferred embodiments, the DDD or AD moiety, more preferably the AD moiety, may be attached to the C-terminal end of the antibody light chain (see, e.g., U.S. Patent Appl. Serial No. 13/901,737, filed 5/24/13.)

*Structure-Function Relationships in AD and DDD Moieties*

For different types of DNL® constructs, different AD or DDD sequences may be utilized. Exemplary DDD and AD sequences are provided below.

*DDD1*
SHIQIPPGLTELLQGYTVEVLRQQPPDLVEFAVEYFTRLREARA (SEQ ID NO: 1)
*DDD2*
CGHIQIPPGLTELLQGYTVEVLRQQPPDLVEFAVEYFTRLREARA (SEQ ID NO:2)
*AD1*
QIEYLAKQIVDNAIQQA (SEQ ID NO:3)
*AD2*
CGQIEYLAKQIVDNAIQQAGC (SEQ ID NO:4)

The skilled artisan will realize that DDD1 and DDD2 are based on the DDD sequence of the human RIIα isoform of protein kinase A. However, in alternative embodiments, the DDD and AD moieties may be based on the DDD sequence of the human RIα form of protein kinase A and a corresponding AKAP sequence, as exemplified in DDD3, DDD3C and AD3 below.

*DDD3*
SLRECELYVQKHNIQALLKDSIVQLCTARPERPMAFLREYFERLEKEEAK (SEQ ID NO:5)
*DDD3C*
MSCGGSLRECELYVQKHNIQALLKDSIVQLCTARPERPMAFLREYFERLEKEEAK (SEQ ID NO:6)
*AD3*
CGFEELAWKIAKMIWSDVFQQGC (SEQ ID NO:7)

In other alternative embodiments of the invention as defined in the claims, other sequence variants of AD and/or DDD moieties may be utilized in construction of the DNL® complexes. For example, there are only four variants of human PKA DDD sequences, corresponding to the DDD moieties of PKA RIα, RIIα, RIβ and RIIβ. The RIIα DDD sequence is the basis of DDD1 and DDD2 disclosed above. The four human PKA DDD sequences are shown below. The DDD sequence represents residues 1-44 of RIIα, 1-44 of RIIβ, 12-61 of RIα and 13-66 of RIβ. (Note that the sequence of DDD1 is modified slightly from the human PKA RIIα DDD moiety.)

*PKA RIα*
SLRECELYVQKHNIQALLKDVSIVQLCTARPERPMAFLREYFEKLEKEEAK (SEQ ID NO:8)
*PKA RIβ*
SLKGCELYVQLHGIQQVLKDCIVHLCISKPERPMKFLREHFEKLEKEENRQILA (SEQ ID NO:9)
*PKA RIIα*
SHIQIPPGLTELLQGYTVEVGQQPPDLVDFAVEYFTRLREARRQ (SEQ ID NO:10)
*PKA RIIβ*
SIEIPAGLTELLQGFTVEVLRHQPADLLEFALQHFTRLQQENER (SEQ ID NO:11)

The structure-function relationships of the AD and DDD domains have been the subject of investigation. (See, e.g., Burns-Hamuro et al., 2005, Protein Sci 14:2982-92; Carr et al., 2001, J Biol Chem 276:17332-38; Alto et al., 2003, Proc Natl Acad Sci USA 100:4445-50; Hundsrucker et al., 2006, Biochem Y 396:297-306; Stokka et al., 2006, Biochem J 400:493-99; Gold et al., 2006, Mol Cell 24:383-95; Kinderman et al., 2006, Mol Cell 24:397-408.)

For example, Kinderman et al. (2006, Mol Cell 24:397-408) examined the crystal structure of the AD-DDD binding interaction and concluded that the human DDD sequence contained a number of conserved amino acid residues that were important in either dimer formation or AKAP binding, underlined in SEQ ID NO:1 below. (See Figure 1 of Kinderman et al., 2006.) The skilled artisan will realize that in designing sequence variants of the DDD sequence, one would desirably avoid changing any of the underlined residues, while conservative amino acid substitutions might be made for residues that are less critical for dimerization and AKAP binding.

SHIQIPPGLTELLQGYTVEVLRQQPPDLVEFAVEYFTRLREARA (SEQ ID NO:1)

As discussed in more detail below, conservative amino acid substitutions have been characterized for each of the twenty common L-amino acids. Thus, based on the data of Kinderman (2006) and conservative amino acid substitutions, potential alternative DDD sequences based on SEQ ID NO:1 are shown in **Table 2.** In devising **Table 2,** only highly conservative amino acid substitutions were considered. For example, charged residues were only substituted for residues of the same charge, residues with small side chains were substituted with residues of similar size, hydroxyl side chains were only substituted with other hydroxyls, etc. Because of the unique effect of proline on amino acid secondary structure, no other residues were substituted for proline. A limited number of such potential alternative DDD moiety sequences are shown in SEQ ID NO:12 to SEQ ID NO:31 below. The skilled artisan will realize that an almost unlimited number of alternative species within the genus of DDD moieties can be constructed by standard techniques, for example using a commercial peptide synthesizer or well known site-directed mutagenesis techniques. The effect of the amino acid substitutions on AD moiety binding may also be readily determined by standard binding assays, for example as disclosed in Alto et al. (2003, Proc Natl Acad Sci USA 100:4445-50).

**Table 2. Conservative Amino Acid Substitutions in DDD1 (SEQ ID NO:1). Consensus sequence disclosed as SEQ ID NO:87.**

| S | H | I | Q | I | P | P | G | L | T | E | L | L | Q | G | Y | T | V | E | V | L | R |
|---|---|---|---|---|---|---|---|---|---|---|---|---|---|---|---|---|---|---|---|---|---|
| T | K<br>R | | N | | | | A | | S | D | | | N | A | | S | | D | | | K |

| Q | Q | P | P | D | L | V | E | F | A | V | E | Y | F | T | R | L | R | E | A | R | A |
|---|---|---|---|---|---|---|---|---|---|---|---|---|---|---|---|---|---|---|---|---|---|
| N | N | | | E | | | D | | L<br>I<br>V | | D | | | S | K | | K | D | L<br>I<br>V | K | L<br>I<br>V |

THIQIPPGLTELLQGYTVEVLRQQPPDLVEFAVEYFTRLREARA (SEQ ID NO: 12)
SKIQIPPGLTELLQGYTVEVLRQQPPDLVEFAVEYFTRLREARA (SEQ ID NO: 13)
SRIQIPPGLTELLQGYTVEVLRQQPPDLVEFAVEYFTRLREARA (SEQ ID NO: 14)
SHINIPPGLTELLQGYTVEVLRQQPPDLVEFAVEYFTRLREARA (SEQ ID NO: 15)
SHIQIPPALTELLQGYTVEVLRQQPPDLVEFAVEYFTRLREARA (SEQ ID NO: 16)
SHIQIPPGLSELLQGYTVEVLRQQPPDLVEFAVEYFTRLREARA (SEQ ID NO: 17)
SHIQIPPGLTDLLQGYTVEVLRQQPPDLVEFAVEYFTRLREARA (SEQ ID NO: 18)

SHIQIPPGLTELLNGYTVEVLRQQPPDLVEFAVEYFTRLREARA (SEQ ID NO: 19)
SHIQIPPGLTELLQAYTVEVLRQQPPDLVEFAVEYFTRLREARA (SEQ ID NO:20)
SHIQIPPGLTELLQGYSVEVLRQQPPDLVEFAVEYFTRLREARA (SEQ ID NO:21)
SHIQIPPGLTELLQGYTVDVLRQQPPDLVEFAVEYFTRLREARA (SEQ ID NO:22)
SHIQIPPGLTELLQGYTVEVLKQQPPDLVEFAVEYFTRLREARA (SEQ ID NO:23)
SHIQIPPGLTELLQGYTVEVLRNQPPDLVEFAVEYFTRLREARA (SEQ ID NO:24)
SHIQIPPGLTELLQGYTVEVLRQNPPDLVEFAVEYFTRLREARA (SEQ ID NO:25)
SHIQIPPGLTELLQGYTVEVLRQQPPELVEFAVEYFTRLREARA (SEQ ID NO:26)
SHIQIPPGLTELLQGYTVEVLRQQPPDLVDFAVEYFTRLREARA (SEQ ID NO:27)
SHIQIPPGLTELLQGYTVEVLRQQPPDLVEFLVEYFTRLREARA (SEQ ID NO:28)
SHIQIPPGLTELLQGYTVEVLRQQPPDLVEFIVEYFTRLREARA (SEQ ID NO:29)
SHIQIPPGLTELLQGYTVEVLRQQPPDLVEFVVEYFTRLREARA (SEQ ID NO:30)
SHIQIPPGLTELLQGYTVEVLRQQPPDLVEFAVDYFTRLREARA (SEQ ID NO:31)

Alto et al. (2003, Proc Natl Acad Sci USA 100:4445-50) performed a bioinformatic analysis of the AD sequence of various AKAP proteins to design an RII selective AD sequence called AKAP-IS (SEQ ID NO:3), with a binding constant for DDD of 0.4 nM. The AKAP-IS sequence was designed as a peptide antagonist of AKAP binding to PKA. Residues in the AKAP-IS sequence where substitutions tended to decrease binding to DDD are underlined in SEQ ID NO:3 below. The skilled artisan will realize that in designing sequence variants of the AD sequence, one would desirably avoid changing any of the underlined residues, while conservative amino acid substitutions might be made for residues that are less critical for DDD binding. **Table 3** shows potential conservative amino acid substitutions in the sequence of AKAP-IS (AD1, SEQ ID NO:3), similar to that shown for DDD1 (SEQ ID NO: 1) in **Table 2** above.

A limited number of such potential alternative AD moiety sequences are shown in SEQ ID NO:32 to SEQ ID NO:49 below. Again, a very large number of species within the genus of possible AD moiety sequences could be made, tested and used by the skilled artisan, based on the data of Alto et al. (2003). It is noted that Figure 2 of Alto (2003) shows an even large number of potential amino acid substitutions that may be made, while retaining binding activity to DDD moieties, based on actual binding experiments.

*AKAP-IS*

QIEYLAKQIVDNAIQQA (SEQ ID NO:3)

**Table 3. Conservative Amino Acid Substitutions in AD1 (SEQ ID NO:3). Consensus sequence disclosed as SEQ ID NO:88.**

| Q | I | E | Y | L | <u>A</u> | K | Q | <u>I</u> | <u>V</u> | D | N | <u>A</u> | <u>I</u> | Q | Q | A |
|---|---|---|---|---|---|---|---|---|---|---|---|---|---|---|---|---|
| N | L<br>V | D | F<br>T<br>S | I<br>V | | R | N | | | E | Q | | | N | N | L<br>I<br>V |

NIEYLAKQIVDNAIQQA (SEQ ID NO:32)
QLEYLAKQIVDNAIQQA (SEQ ID NO:33)
QVEYLAKQIVDNAIQQA (SEQ ID NO:34)
QIDYLAKQIVDNAIQQA (SEQ ID NO:35)
QIEFLAKQIVDNAIQQA (SEQ ID NO:36)
QIETLAKQIVDNAIQQA (SEQ ID NO:37)
QIESLAKQIVDNAIQQA (SEQ ID NO:38)
QIEYIAKQIVDNAIQQA (SEQ ID NO:39)
QIEYVAKQIVDNAIQQA (SEQ ID NO:40)
QIEYLARQIVDNAIQQA (SEQ ID NO:41)
QIEYLAKNIVDNAIQQA (SEQ ID NO:42)
QIEYLAKQIVENAIQQA (SEQ ID NO:43)
QIEYLAKQIVDQAIQQA (SEQ ID NO:44)
QIEYLAKQIVDNAINQA (SEQ ID NO:45)
QIEYLAKQIVDNAIQNA (SEQ ID NO:46)
QIEYLAKQIVDNAIQQL (SEQ ID NO:47)
QIEYLAKQIVDNAIQQI (SEQ ID NO:48)
QIEYLAKQIVDNAIQQV (SEQ ID NO:49)

Gold et al. (2006, Mol Cell 24:383-95) utilized crystallography and peptide screening to develop a SuperAKAP-IS sequence (SEQ ID NO:50), exhibiting a five order of magnitude higher selectivity for the RII isoform of PKA compared with the RI isoform. Underlined residues indicate the positions of amino acid substitutions, relative to the AKAP-IS sequence, which increased binding to the DDD moiety of RII$\alpha$. In this sequence, the N-terminal Q residue is numbered as residue number 4 and the C-terminal A residue is residue number 20. Residues where substitutions could be made to affect the affinity for RII$\alpha$ were residues 8, 11, 15, 16, 18, 19 and 20 (Gold et al., 2006). It is contemplated that in certain alternative embodiments of the invention as defined in the claims, the SuperAKAP-IS sequence may be substituted for the AKAP-IS AD moiety sequence to prepare DNL® constructs. Other alternative sequences that might be substituted for the AKAP-IS AD sequence are shown in SEQ ID NO:51-53. Substitutions relative to the AKAP-IS sequence are underlined. It is anticipated that, as with the AD2 sequence shown in SEQ ID NO:4, the AD moiety may also include the additional N-terminal residues cysteine and glycine and C-terminal residues glycine and cysteine.

*SuperAKAP-IS*
QIEYVAKQIVDYAIHQA (SEQ ID NO:50)
*Alternative AKAP sequences*

QIEYKAKQIVDHAIHQA (SEQ ID NO:51)
QIEYHAKQIVDHAIHQA (SEQ ID NO:52)
QIEYVAKQIVDHAIHQA (SEQ ID NO:53)

Figure 2 of Gold et al. disclosed additional DDD-binding sequences from a variety of AKAP proteins, shown below.

RII-Specific AKAPs

*AKAP-KL*
PLEYQAGLLVQNAIQQAI (SEQ ID NO:54)
*AKAP79*
LLIETASSLVKNAIQLSI (SEQ ID NO:55)
*AKAP-Lbc*
LIEEAASRIVDAVIEQVK (SEQ ID NO:56)

RI-Specific AKAPs

*AKAPce*
ALYQFADRFSELVISEAL (SEQ ID NO:57)
*RIAD*
LEQVANQLADQIIKEAT (SEQ ID NO:58)
*PV38*
FEELAWKIAKMIWSDVF (SEQ ID NO:59)

Dual-Specificity AKAPs

*AKAP 7*
ELVRLSKRLVENAVLKAV (SEQ ID NO:60)
*MAP2D*
TAEEVSARIVQVVTAEAV (SEQ ID NO:61)
*DAKAPI*
QIKQAAFQLISQVILEAT (SEQ ID NO:62)
*DAKAP2*
LAWKIAKMIVSDVMQQ (SEQ ID NO:63)

Stokka et al. (2006, Biochem J 400:493-99) also developed peptide competitors of AKAP binding to PKA, shown in SEQ ID NO:64-66. The peptide antagonists were designated as Ht31 (SEQ ID NO:64), RIAD (SEQ ID NO:65) and PV-38 (SEQ ID NO:66). The Ht-31 peptide exhibited a greater affinity for the RII isoform of PKA, while the RIAD and PV-38 showed higher affinity for RI.

*Ht31*
DLIEEAASRIVDAVIEQVKAAGAY (SEQ ID NO:64)
*RIAD*
LEQYANQLADQIIKEATE (SEQ ID NO:65)
*PV-38*
FEELAWKIAKMIWSDVFQQC (SEQ ID NO:66)

Hundsrucker et al. (2006, Biochem J 396:297-306) developed still other peptide competitors for AKAP binding to PKA, with a binding constant as low as 0.4 nM to the DDD of the RII form of PKA. The sequences of various AKAP antagonistic peptides are provided in Table 1 of Hundsrucker et al., reproduced in **Table 4** below. AKAPIS represents a synthetic RII subunit-binding peptide. All other peptides are derived from the RII-binding domains of the indicated AKAPs.

**Table 4. AKAP Peptide sequences**

| | Peptide Sequence |
|---|---|
| AKAPIS | QIEYLAKQIVDNAIQQA (SEQ ID NO:3) |
| AKAPIS-P | QIEYLAKQIPDNAIQQA (SEQ ID NO:67) |
| Ht31 | KGADLIEEAASRIVDAVIEQVKAAG (SEQ ID NO:68) |
| Ht31-P | KGADLIEEAASRIPDAPIEQVKAAG (SEQ ID NO:69) |
| AKAP7$\delta$-wt-pep | PEDAELVRLSKRLVENAVLKAVQQY (SEQ ID NO:70) |
| AKAP7$\delta$-L304T-pep | PEDAELVRTSKRLVENAVLKAVQQY (SEQ ID NO:71) |
| AKAP7$\delta$-L308D-pep | PEDAELVRLSKRDVENAVLKAVQQY (SEQ ID NO:72) |
| AKAP7$\delta$-P-pep | PEDAELVRLSKRLPENAVLKAVQQY (SEQ ID NO:73) |
| AKAP7$\delta$-PP-pep | PEDAELVRLSKRLPENAPLKAVQQY (SEQ ID NO:74) |
| AKAP7$\delta$-L314E-pep | PEDAELVRLSKRLVENAVEKAVQQY (SEQ ID NO:75) |
| AKAP1-pep | EEGLDRNEEIKRAAFQIISQVISEA (SEQ ID NO:76) |
| AKAP2-pep | LVDDPLEYQAGLLVQNAIQQAIAEQ (SEQ ID NO:77) |
| AKAP5-pep | QYETLLIETASSLVKNAIQLSIEQL (SEQ ID NO:78) |
| AKAP9-pep | LEKQYQEQLEEEVAKVIVSMSIAFA (SEQ ID NO:79) |
| AKAP10-pep | NTDEAQEELAWKIAKMIVSDIMQQA (SEQ ID NO:80) |
| AKAP11-pep | VNLDKKAVLAEKIVAEAIEKAEREL (SEQ ID NO:81) |
| AKAP12-pep | NGILELETKSSKLVQNIIQTAVDQF (SEQ ID NO:82) |
| AKAP14-pep | TQDKNYEDELTQVALALVEDVINYA (SEQ ID NO:83) |
| Rab32-pep | ETSAKDNINIEEAARFLVEKILVNH (SEQ ID NO:84) |

Residues that were highly conserved among the AD domains of different AKAP proteins are indicated below by underlining with reference to the AKAP IS sequence (SEQ ID NO:3). The residues are the same as observed by Alto et al. (2003), with the addition of the C-terminal alanine residue. (See FIG. 4 of Hundsrucker et al. (2006).) The sequences of peptide antagonists with particularly high affinities for the RII DDD sequence were those of AKAP-IS, AKAP76-wt-pep, AKAP7δ-L304T-pep and AKAP7δ-L308D-pep.
*AKAP-IS*
QIEYLAKQIVDNAIQQA (SEQ ID NO:3)

Carr et al. (2001, J Biol Chem 276:17332-38) examined the degree of sequence homology between different AKAP-binding DDD sequences from human and non-human proteins and identified residues in the DDD sequences that appeared to be the most highly conserved among different DDD moieties. These are indicated below by underlining with reference to the human PKA RIIα DDD sequence of SEQ ID NO:1. Residues that were particularly conserved are further indicated by italics. The residues overlap with, but are not identical to those suggested by Kinderman et al. (2006) to be important for binding to AKAP proteins. The skilled artisan will realize that in designing sequence variants of DDD, it would be most preferred to avoid changing the most conserved residues (italicized), and it would be preferred to also avoid changing the conserved residues (underlined), while conservative amino acid substitutions may be considered

for residues that are neither underlined nor italicized..
SHIQIPPGLTELLQGYTVEVLRQQPPDLVEFAVEYFTRLREARA (SEQ ID NO:1)

A modified set of conservative amino acid substitutions for the DDD1 (SEQ ID NO:1) sequence, based on the data of Carr et al. (2001) is shown in **Table** 5. Even with this reduced set of substituted sequences, there are numerous possible alternative DDD moiety sequences that may be produced, tested and used by the skilled artisan without undue experimentation. The skilled artisan could readily derive such alternative DDD amino acid sequences as disclosed above for **Table 2** and **Table 3.**

**Table 5. Conservative Amino Acid Substitutions in DDD1 (SEQ ID NO:1). Consensus sequence disclosed as SEQ ID NO:89.**

| S | H | I | Q | I | P | P | G | L | T | E | L | L | Q | G | Y | T | V | E | V | L | R |
|---|---|---|---|---|---|---|---|---|---|---|---|---|---|---|---|---|---|---|---|---|---|
| T | | | N | | | | | | S | | | | | | | | I<br>L<br>A | | | | |

| Q | Q | P | P | D | L | V | E | F | A | V | E | Y | F | T | R | L | R | E | A | R | A |
|---|---|---|---|---|---|---|---|---|---|---|---|---|---|---|---|---|---|---|---|---|---|
| N | | | | | | | | | | I<br>L<br>A | D | | | S | K | | K | | L<br>I<br>V | | L<br>I<br>V |

The skilled artisan will realize that these and other amino acid substitutions in the DDD or AD amino acid sequences may be utilized to produce alternative species within the genus of AD or DDD moieties, using techniques that are standard in the field and only routine experimentation.

**Antibody Allotypes**

Immunogenicity of therapeutic antibodies is associated with increased risk of infusion reactions and decreased duration of therapeutic response (Baert et al., 2003, N Engl J Med 348:602-08). The extent to which therapeutic antibodies induce an immune response in the host may be determined in part by the allotype of the antibody (Stickler et al., 2011, Genes and Immunity 12:213-21). Antibody allotype is related to amino acid sequence variations at specific locations in the constant region sequences of the antibody. The allotypes of IgG antibodies containing a heavy chain γ-type constant region are designated as Gm allotypes (1976, J Immunol 117:1056-59).

For the common IgG1 human antibodies, the most prevalent allotype is G1m1 (Stickler et al., 2011, Genes and Immunity 12:213-21). However, the G1m3 allotype also occurs frequently in Caucasians (*Id.*). It has been reported that G1m1 antibodies contain allotypic sequences that tend to induce an immune response when administered to non-G1m1 (nG1m1) recipients, such as G1m3 patients (*Id.*). Non-G1m1 allotype antibodies are not as immunogenic when administered to G1m1 patients *(Id.).*

The human G1m1 allotype comprises the amino acids aspartic acid at Kabat position 356 and leucine at Kabat position 358 in the CH3 sequence of the heavy chain IgG1. The nG1m1 allotype comprises the amino acids glutamic acid at Kabat position 356 and methionine at Kabat position 358. Both G1m1 and nG1m1 allotypes comprise a glutamic acid residue at Kabat position 357 and the allotypes are sometimes referred to as DEL and EEM allotypes. A non-limiting example of the heavy chain constant region sequences for G 1m 1 and nG 1m 1 allotype antibodies is shown for the exemplary antibodies rituximab (SEQ ID NO:85) and veltuzumab (SEQ ID NO:86).

*Rituximab heavy chain variable region sequence (SEQ ID NO: 85)*

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDK**KA**EPKSCDKTHTCPPCPAP
ELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPRE
PQVYTLPPSR**D**E**L**TKNQVSLTCLVKGFYPSDIAVEWESNGQPENNYKTTPPVLDSDG
SFFLYSKLTVDKSRWQQGNVFSCSVMHEALHNHYTQKSLSLSPGK

*Veltuzumab heavy chain variable region (SEQ ID NO:86*

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDK**RV**EPKSCDKTHTCPPCPAP
ELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPRE
PQVYTLPPSR**E**E**M**TKNQVSLTCLVKGFYPSDIAVEWESNGQPENNYKTTPPVLDSDG
SFFLYSKLTVDKSRWQQGNVFSCSVMHEALHNHYTQKSLSLSPGK

Jefferis and Lefranc (2009, mAbs 1:1-7) reviewed sequence variations characteristic of IgG allotypes and their effect on immunogenicity. They reported that the G1m3 allotype is characterized by an arginine residue at Kabat position 214, compared to a lysine residue at Kabat 214 in the G1m17 allotype. The nG 1m 1,2 allotype was characterized by glutamic acid at Kabat position 356, methionine at Kabat position 358 and alanine at Kabat position 431. The G1m1,2 allotype was characterized by aspartic acid at Kabat position 356, leucine at Kabat position 358 and glycine at Kabat position 431. In addition to heavy chain constant region sequence variants, Jefferis and Lefranc (2009) reported allotypic variants in the kappa light chain constant region, with the Km1 allotype characterized by valine at Kabat position 153 and leucine at Kabat position 191, the Km1,2 allotype by alanine at Kabat position 153 and leucine at Kabat position 191, and the Km3 allotypoe characterized by alanine at Kabat position 153 and valine at Kabat position 191.

With regard to therapeutic antibodies, veltuzumab and rituximab are, respectively, humanized and chimeric IgG1 antibodies against CD20, of use for therapy of a wide variety of hematological malignancies. **Table 6** compares the allotype sequences of rituximab vs. veltuzumab. As shown in **Table 6,** rituximab (G1m17,1) is a DEL allotype IgG1, with an additional sequence variation at Kabat position 214 (heavy chain CH1) of lysine in rituximab vs. arginine in veltuzumab. It has been reported that veltuzumab is less immunogenic in subjects than rituximab (*see, e.g.,* Morchhauser et al., 2009, J Clin Oncol 27:3346-53; Goldenberg et al., 2009, Blood 113:1062-70; Robak & Robak, 2011, BioDrugs 25:13-25), an effect that has been attributed to the difference between humanized and chimeric antibodies. However, the difference in allotypes between the EEM and DEL allotypes likely also accounts for the lower immunogenicity of veltuzumab.

**Table 6. Allotypes of Rituximab vs. Veltuzumab**

| | | *Heavy chain position and associated allotypes* | | | | | |
|---|---|---|---|---|---|---|---|
| | *Complete allotype* | *214* | *(allotype)* | *3561358* | *(allotype)* | *431* | *(allotype)* |
| *Rituximab* | *G1m17,1* | *K* | *17* | *OIL* | *1* | *A* | *-* |
| *Veltuzumab* | *G1m3* | R | *3* | *E/M* | *-* | A | *-* |

In order to reduce the immunogenicity of therapeutic antibodies in individuals of nG 1m 1 genotype, it is desirable to select the allotype of the antibody to correspond to the G1m3 allotype, characterized by arginine at Kabat 214, and the nG1m1,2 null-allotype, characterized by glutamic acid at Kabat position 356, methionine at Kabat position 358 and alanine at Kabat position 431. Surprisingly, it was found that repeated subcutaneous administration of G1m3 antibodies

over a long period of time did not result in a significant immune response. In alternative embodiments, the human IgG4 heavy chain in common with the G1m3 allotype has arginine at Kabat 214, glutamic acid at Kabat 356, methionine at Kabat 359 and alanine at Kabat 431. Since immunogenicity appears to relate at least in part to the residues at those locations, use of the human IgG4 heavy chain constant region sequence for therapeutic antibodies is also a preferred embodiment. Combinations of G1m3 IgG1 antibodies with IgG4 antibodies may also be of use for therapeutic administration.

## Amino Acid Substitutions

In alternative embodiments, the disclosed methods and compositions may involve production and use of proteins or peptides with one or more substituted amino acid residues. For example, the DDD and/or AD sequences used to make DNL® constructs may be modified as discussed above.

The skilled artisan will be aware that, in general, amino acid substitutions typically involve the replacement of an amino acid with another amino acid of relatively similar properties (i.e., conservative amino acid substitutions). The properties of the various amino acids and effect of amino acid substitution on protein structure and function have been the subject of extensive study and knowledge in the art.

For example, the hydropathic index of amino acids may be considered (Kyte & Doolittle, 1982, J. Mol. Biol., 157:105-132). The relative hydropathic character of the amino acid contributes to the secondary structure of the resultant protein, which in turn defines the interaction of the protein with other molecules. Each amino acid has been assigned a hydropathic index on the basis of its hydrophobicity and charge characteristics (Kyte & Doolittle, 1982), these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). In making conservative substitutions, the use of amino acids whose hydropathic indices are within $\pm$ 2 is preferred, within $\pm$ 1 are more preferred, and within $\pm$ 0.5 are even more preferred.

Amino acid substitution may also take into account the hydrophilicity of the amino acid residue (e.g., U.S. Pat. No. 4,554,101). Hydrophilicity values have been assigned to amino acid residues: arginine (+3.0); lysine (+3.0); aspartate (+3.0); glutamate (+3.0); serine (+0.3); asparagine (+0.2); glutamine (+0.2); glycine (0); threonine (-0.4); proline (-0.5 .+-.1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). Replacement of amino acids with others of similar hydrophilicity is preferred.

Other considerations include the size of the amino acid side chain. For example, it would generally not be preferred to replace an amino acid with a compact side chain, such as glycine or serine, with an amino acid with a bulky side chain, e.g., tryptophan or tyrosine. The effect of various amino acid residues on protein secondary structure is also a consideration. Through empirical study, the effect of different amino acid residues on the tendency of protein domains to adopt an alpha-helical, beta-sheet or reverse turn secondary structure has been determined and is known in the art (see, e.g., Chou & Fasman, 1974, Biochemistry, 13:222-245; 1978, Ann. Rev. Biochem., 47: 251-276; 1979, Biophys. J., 26:367-384).

Based on such considerations and extensive empirical study, tables of conservative amino acid substitutions have been constructed and are known in the art. For example: arginine and lysine; glutamate and aspartate; serine and threonine; glutamine and asparagine; and valine, leucine and isoleucine. Alternatively: Ala (A) leu, ile, val; Arg (R) gln, asn, lys; Asn (N) his, asp, lys, arg, gln; Asp (D) asn, glu; Cys (C) ala, ser; Gln (Q) glu, asn; Glu (E) gln, asp; Gly (G) ala; His (H) asn, gln, lys, arg; Ile (I) val, met, ala, phe, leu; Leu (L) val, met, ala, phe, ile; Lys (K) gln, asn, arg; Met (M) phe, ile, leu; Phe (F) leu, val, ile, ala, tyr; Pro (P) ala; Ser (S), thr; Thr (T) ser; Trp (W) phe, tyr; Tyr (Y) trp, phe, thr, ser; Val (V) ile, leu, met, phe, ala.

Other considerations for amino acid substitutions include whether or not the residue is located in the interior of a protein or is solvent exposed. For interior residues, conservative substitutions would include: Asp and Asn; Ser and Thr; Ser and Ala; Thr and Ala; Ala and Gly; Ile and Val; Val and Leu; Leu and Ile; Leu and Met; Phe and Tyr; Tyr and Trp. (See, e.g., PROWL website at rockefeller.edu) For solvent exposed residues, conservative substitutions would include: Asp and Asn; Asp and Glu; Glu and Gln; Glu and Ala; Gly and Asn; Ala and Pro; Ala and Gly; Ala and Ser; Ala and Lys; Ser and Thr; Lys and Arg; Val and Leu; Leu and Ile; Ile and Val; Phe and Tyr. (Id.) Various matrices have been constructed to assist in selection of amino acid substitutions, such as the PAM250 scoring matrix, Dayhoff matrix, Grantham matrix, McLachlan matrix, Doolittle matrix, Henikoff matrix, Miyata matrix, Fitch matrix, Jones matrix, Rao matrix, Levin matrix and Risler matrix (*Idem.*)

In determining amino acid substitutions, one may also consider the existence of intermolecular or intramolecular bonds, such as formation of ionic bonds (salt bridges) between positively charged residues (e.g., His, Arg, Lys) and negatively charged residues (e.g., Asp, Glu) or disulfide bonds between nearby cysteine residues.

Methods of substituting any amino acid for any other amino acid in an encoded protein sequence are well

known and a matter of routine experimentation for the skilled artisan, for example by the technique of site-directed mutagenesis or by synthesis and assembly of oligonucleotides encoding an amino acid substitution and splicing into an expression vector construct.

## Avimers

In certain embodiments of the invention as defined in the claims, the binding moieties described herein may comprise one or more avimer sequences. Avimers are a class of binding proteins somewhat similar to antibodies in their affinities and specificities for various target molecules. They were developed from human extracellular receptor domains by *in vitro* exon shuffling and phage display. (Silverman et al., 2005, Nat. Biotechnol. 23:1493-94; Silverman et al., 2006, Nat. Biotechnol. 24:220). The resulting multidomain proteins may comprise multiple independent binding domains, that may exhibit improved affinity (in some cases sub-nanomolar) and specificity compared with single-epitope binding proteins. (*Id*.) In various embodiments, avimers may be attached to, for example, DDD and/or AD sequences for use in the claimed methods and compositions. Additional details concerning methods of construction and use of avimers are disclosed, for example, in U.S. Patent Application Publication Nos. 20040175756, 20050048512, 20050053973, 20050089932 and 20050221384.

## Phage Display

Certain embodiments of the invention as defined in the claims may encompass binding peptides and/or peptide mimetics of various target molecules, cells or tissues. Binding peptides may be identified by any method known in the art, including but not limiting to the phage display technique. Various methods of phage display and techniques for producing diverse populations of peptides are well known in the art. For example, U.S. Pat. Nos. 5,223,409; 5,622,699 and 6,068,829 disclose methods for preparing a phage library. The phage display technique involves genetically manipulating bacteriophage so that small peptides can be expressed on their surface (Smith and Scott, 1985, Science 228:1315-1317; Smith and Scott, 1993, Meth. Enzymol. 21:228-257). In addition to peptides, larger protein domains such as single-chain antibodies may also be displayed on the surface of phage particles (Arap et al., 1998, Science 279:377-380).

Targeting amino acid sequences selective for a given organ, tissue, cell type or target molecule may be isolated by panning (Pasqualini and Ruoslahti, 1996, Nature 380:364-366; Pasqualini, 1999, The Quart. J. Nucl. Med. 43:159-162). In brief, a library of phage containing putative targeting peptides is administered to an intact organism or to isolated organs, tissues, cell types or target molecules and samples containing bound phage are collected. Phage that bind to a target may be eluted from a target organ, tissue, cell type or target molecule and then amplified by growing them in host bacteria.

The phage may be propagated in host bacteria between rounds of panning. Rather than being lysed by the phage, the bacteria may instead secrete multiple copies of phage that display a particular insert. If desired, the amplified phage may be exposed to the target organs, tissues, cell types or target molecule again and collected for additional rounds of panning. Multiple rounds of panning may be performed until a population of selective or specific binders is obtained. The amino acid sequence of the peptides may be determined by sequencing the DNA corresponding to the targeting peptide insert in the phage genome. The identified targeting peptide may then be produced as a synthetic peptide by standard protein chemistry techniques (Arap *et al*., 1998, Smith *et al*., 1985).

A subtraction protocol may be used to further reduce background phage binding. The purpose of subtraction is to remove phage from the library that bind to targets other than the target of interest. Alternatively, the phage library may be prescreened against a control cell, tissue or organ. For example, tumor-binding peptides may be identified after prescreening a library against a control normal cell line. After subtraction the library may be screened against the molecule, cell, tissue or organ of interest. Other methods of subtraction protocols are known and may be used, for example as disclosed in U.S Patent Nos. 5,840,841, 5,705,610, 5,670,312 and 5,492,807.

## Aptamers

A targeting moiety of use may be an aptamer. Methods of constructing and determining the binding characteristics of aptamers are well known in the art. For example, such techniques are described in U.S. Patent Nos. 5,582,981, 5,595,877 and 5,637,459. Methods for preparation and screening of aptamers that bind to particular targets of interest are well known, for example U.S. Pat. No. 5,475,096 and U.S. Pat. No. 5,270,163.

Aptamers may be prepared by any known method, including synthetic, recombinant, and purification methods, and may be used alone or in combination with other ligands specific for the same target. In general, a minimum of approximately 3 nucleotides, preferably at least 5 nucleotides, are necessary to effect specific binding. Aptamers of sequences shorter than 10 bases may be feasible, although aptamers of 10, 20, 30 or 40 nucleotides may be preferred.

Aptamers may be isolated, sequenced, and/or amplified or synthesized as conventional DNA or RNA molecules. Alternatively, aptamers of interest may comprise modified oligomers. Any of the hydroxyl groups ordinarily present in aptamers may be replaced by phosphonate groups, phosphate groups, protected by a standard protecting group, or activated to prepare additional linkages to other nucleotides, or may be conjugated to solid supports. One or more phosphodiester linkages may be replaced by alternative linking groups, such as P(O)O replaced by P(O)S, $P(O)NR_2$, P(O)R, P(O)OR', CO, or $CNR_2$, wherein R is H or alkyl (1-20C) and R' is alkyl (1-20C); in addition, this group may be attached to adjacent nucleotides through O or S. Not all linkages in an oligomer need to be identical.

**Affibodies and Fynomers**

Certain examples may utilize affibodies in place of antibodies. Affibodies are commercially available from Affibody AB (Solna, Sweden). Affibodies are small proteins that function as antibody mimetics and are of use in binding target molecules. Affibodies were developed by combinatorial engineering on an alpha helical protein scaffold (Nord et al., 1995, Protein Eng 8:601-8; Nord et al., 1997, Nat Biotechnol 15:772-77). The affibody design is based on a three helix bundle structure comprising the IgG binding domain of protein A (Nord et al., 1995; 1997). Affibodies with a wide range of binding affinities may be produced by randomization of thirteen amino acids involved in the Fc binding activity of the bacterial protein A (Nord et al., 1995; 1997). After randomization, the PCR amplified library was cloned into a phagemid vector for screening by phage display of the mutant proteins. The phage display library may be screened against any known antigen, using standard phage display screening techniques (e.g., Pasqualini and Ruoslahti, 1996, Nature 380:364-366; Pasqualini, 1999, Quart. J. Nucl. Med. 43:159-162), in order to identify one or more affibodies against the target antigen.

A $^{177}$Lu-labeled affibody specific for HER2/neu has been demonstrated to target HER2-expressing xenografts *in vivo* (Tolmachev et al., 2007, Cancer Res 67:2773-82). Although renal toxicity due to accumulation of the low molecular weight radiolabeled compound was initially a problem, reversible binding to albumin reduced renal accumulation, enabling radionuclide-based therapy with labeled affibody *(Id.).*

The feasibility of using radiolabeled affibodies for *in vivo* tumor imaging has been recently demonstrated (Tolmachev et al., 2011, Bioconjugate Chem 22:894-902). A maleimide-derivatized NOTA was conjugated to the anti-HER2 affibody and radiolabeled with $^{111}$In *(Id.).* Administration to mice bearing the HER2-expressing DU-145 xenograft, followed by gamma camera imaging, allowed visualization of the xenograft *(Id.).*

Fynomers can also bind to target antigens with a similar affinity and specificity to antibodies. Fynomers are based on the human Fyn SH3 domain as a scaffold for assembly of binding molecules. The Fyn SH3 domain is a fully human, 63 amino acid protein that can be produced in bacteria with high yields. Fynomers may be linked together to yield a multispecific binding protein with affinities for two or more different antigen targets. Fynomers are commercially available from COVAGEN AG (Zurich, Switzerland).

The skilled artisan will realize that affibodies or fynomers may be used as targeting molecules in the practice of the claimed methods and compositions.

**Conjugation Protocols**

The preferred conjugation protocol is based on a thiol-maleimide, a thiol-vinylsulfone, a thiol-bromoacetamide, or a thiol-iodoacetamide reaction that is facile at neutral or acidic pH. This obviates the need for higher pH conditions for conjugations as, for instance, would be necessitated when using active esters. Further details of exemplary conjugation protocols are described below in the Examples section.

**Therapeutic Treatment**

In another aspect, the instant disclosure relates to a method of treating a subject, comprising administering a therapeutically effective amount of an antibody or immunoconjugate as described herein to a subject, preferably in combination with a PARP inhibitor , microtubule inhibitor, Bruton kinase inhibitor and/or PI3K inhibitor. Diseases that may be treated with the antibodies or immunoconjugates described herein include, but are not limited to B-cell malignancies (e.g., non-Hodgkin's lymphoma, mantle cell lymphoma, multiple myeloma, Hodgkin's lymphoma, diffuse large B cell lymphoma, Burkitt lymphoma, follicular lymphoma, acute lymphocytic leukemia, chronic lymphocytic leukemia, hairy cell leukemia) using, for example an anti-CD22 antibody such as the hLL2 MAb (epratuzumab, *see* U.S. Patent No. 6,183,744), against another CD22 epitope (hRFB4) or antibodies against other B-cell antigens, such as CD19, CD20, CD21, CD22, CD23, CD37, CD40, CD40L, CD52, CD74, CD80 or HLA-DR. Other diseases include, but are not limited to, adenocarcinomas of endodermally-derived digestive system epithelia, cancers such as breast cancer and non-small cell lung cancer, and other carcinomas, sarcomas, glial tumors, myeloid leukemias, etc. In particular, antibodies against an antigen, e.g., an oncofetal antigen, produced by or associated with a malignant solid tumor or hematopoietic

neoplasm, e.g., a gastrointestinal, stomach, colon, esophageal, liver, lung, breast, pancreatic, liver, prostate, ovarian, testicular, brain, bone or lymphatic tumor, a sarcoma or a melanoma, are advantageously used. Such therapeutics can be given once or repeatedly, depending on the disease state and tolerability of the conjugate, and can also be used optionally in combination with other therapeutic modalities, such as surgery, external radiation, radioimmunotherapy, immunotherapy, chemotherapy, antisense therapy, interference RNA therapy, gene therapy, and the like. Each combination will be adapted to the tumor type, stage, patient condition and prior therapy, and other factors considered by the managing physician.

As used herein, the term "subject" refers to any animal (i.e., vertebrates and invertebrates) including, but not limited to mammals, including humans. It is not intended that the term be limited to a particular age or sex. Thus, adult and newborn subjects, as well as fetuses, whether male or female, are encompassed by the term. Doses given herein are for humans, but can be adjusted to the size of other mammals, as well as children, in accordance with weight or square meter size. Where an antibody or immunoconjugate is administered to a human subject, the person of ordinary skill will realize that the target antigen to which the antibody or immunoconjugate binds will be a human antigen.

In a preferred embodiment of the invention as defined in the claims, the immunoconjugate comprising an anti-EGP-1 (anti-Trop-2) antibodyis the hRS7 Mab whichcan be used to treat carcinomas such as carcinomas of the esophagus, pancreas, lung, stomach, colon and rectum, urinary bladder, breast, ovary, uterus, kidney and prostate, as disclosed in U.S. Patent No. 7,238,785; 7,517,964 and 8,084,583. An hRS7 antibody is a humanized antibody that comprises light chain complementarity-determining region (CDR) sequences CDR1 (KASQDVSIAVA, SEQ ID NO:90); CDR2 (SASYRYT, SEQ ID NO:91); and CDR3 (QQHYITPLT, SEQ ID NO:92) and heavy chain CDR sequences CDR1 (NYGMN, SEQ ID NO:93); CDR2 (WINTYTGEPTYTDDFKG, SEQ ID NO:94) and CDR3 (GGFGSSYWYFDV, SEQ ID NO:95).

Antibodies or immunoconjugates comprising an anti-CEACAM5 antibody (e.g., hMN-14, labretuzumab) and/or an anti-CEACAM6 antibody (e.g., hMN-3 or hMN-15) may be used to treat any of a variety of cancers that express CEACAM5 and/or CEACAM6, as disclosed in U.S. Patent Nos. 7,541,440; 7,951,369; 5,874,540; 6,676,924 and 8,267,865. Solid tumors that may be treated using anti-CEACAM5, anti-CEACAM6, or a combination of the two, include but are not limited to breast, lung, pancreatic, esophageal, medullary thyroid, ovarian, colon, rectum, urinary bladder, mouth and stomach cancers. A majority of carcinomas, including gastrointestinal, respiratory, genitourinary and breast cancers express CEACAM5 and may be treated with the subject antibodies or immunoconjugates. An hMN-14 antibody is a humanized antibody that comprises light chain variable region CDR sequences CDR1 (KASQDVGTSVA; SEQ ID NO:96), CDR2 (WTSTRHT; SEQ ID NO:97), and CDR3 (QQYSLYRS; SEQ ID NO:98), and the heavy chain variable region CDR sequences CDR1 (TYWMS; SEQ ID NO:99), CDR2 (EIHPDSSTINYAPSLKD; SEQ ID NO:100) and CDR3 (LYFGFPWFAY; SEQ ID NO:101). An hMN-3 antibody is a humanized antibody that comprises light chain variable region CDR sequences CDR1 (RSSQSIVHSNGNTYLE, SEQ ID NO:102), CDR2 (KVSNRFS, SEQ ID NO:103) and CDR3 (FQGSHVPPT, SEQ ID NO:104) and the heavy chain CDR sequences CDR1 (NYGMN, SEQ ID NO:105), CDR2 (WINTYTGEPTYADDFKG, SEQ ID NO:106) and CDR3 (KGWMDFNSSLDY, SEQ ID NO:107). An hMN-15 antibody is a humanized antibody that comprises light chain variable region CDR sequences SASSRVSYIH (SEQ ID NO:108); GTSTLAS (SEQ ID NO:109); and QQWSYNPPT (SEQ ID NO:110); and heavy chain variable region CDR sequences DYYMS (SEQ ID NO:111); FIANKANGHTTDYSPSVKG (SEQ ID NO:112); and DMGIRWNFDV (SEQ ID NO:113).

Antibodies or immunoconjugates comprising an anti-CD74 antibody (e.g., hLL1, milatuzumab, disclosed in U.S. Patent Nos. 7,074,403; 7,312,318; 7,772,373; 7,919,087 and 7,931,903) may be used to treat any of a variety of cancers that express CD74, including but not limited to renal, lung, intestinal, stomach, breast, prostate or ovarian cancer, as well as several hematological cancers, such as multiple myeloma, chronic lymphocytic leukemia, acute lymphoblastic leukemia, non-Hodgkin lymphoma, and Hodgkin lymphoma. An hLL1 antibody is a humanized antibody comprising the light chain CDR sequences CDR1 (RSSQSLVHRNGNTYLH; SEQ ID NO:114), CDR2 (TVSNRFS; SEQ ID NO:115), and CDR3 (SQSSHVPPT; SEQ ID NO:116) and the heavy chain variable region CDR sequences CDR1 (NYGVN; SEQ ID NO:117), CDR2 (WINPNTGEPTFDDDFKG; SEQ ID NO:118), and CDR3 (SRGKNEAWFAY; SEQ ID NO:119). Antibodies or immunoconjugates comprising an anti-CD22 antibody (e.g., hLL2, epratuzumab, disclosed in U.S. Patent Nos. 5,789,554; 6,183,744; 6,187,287; 6,306,393; 7,074,403 and 7,641,901, or the chimeric or humanized RFB4 antibody) may be used to treat any of a variety of cancers that express CD22, including but not limited to indolent forms of B-cell lymphomas, aggressive forms of B-cell lymphomas, chronic lymphatic leukemias, acute lymphatic leukemias, non-Hodgkin's lymphoma, Hodgkin's lymphoma, Burkitt lymphoma, follicular lymphoma or diffuse B-cell lymphoma. An hLL2 antibody is a humanized antibody comprising light chain CDR sequences CDR1 (KSSQSVLYSANHKYLA, SEQ ID NO:120), CDR2 (WASTRES, SEQ ID NO:121), and CDR3 (HQYLSSWTF, SEQ ID NO:122) and the heavy chain CDR sequences CDR1 (SYWLH, SEQ ID NO:123), CDR2 (YINPRNDYTEYNQNFKD, SEQ ID NO:124), and CDR3 (RDITTFY, SEQ ID NO:125)

Antibodies or immunoconjugates comprising anti-CSAp antibodies, such as the hMu-9 MAb, can be used to treat colorectal, as well as pancreatic and ovarian cancers as disclosed in U.S. Patent Nos. 6,962,702; 7,387,772; 7,414,121; 7,553,953; 7,641,891 and 7,670,804. In addition, antibodies or immunoconjugates comprising the hPAM4 MAb can be used to treat pancreatic cancer or other solid tumors, as disclosed in U.S. Patent Nos. 7,238,786 and

7,282,567. An hMu-9 antibody is a humanized antibody comprising light chain CDR sequences CDR1 (RSSQSIVHSNGNTYLE, SEQ ID NO:126), CDR2 (KVSNRFS, SEQ ID NO:127), and CDR3 (FQGSRVPYT, SEQ ID NO:128), and heavy chain variable CDR sequences CDR1 (EYVIT, SEQ ID NO:129), CDR2 (EIYPGSGSTSYNEKFK, SEQ ID NO:130), and CDR3 (EDL, SEQ ID NO:131). An hPAM4 antibody is a humanized antibody comprising light chain variable region CDR sequencs CDR1 (SASSSVSSSYLY, SEQ ID NO:132); CDR2 (STSNLAS, SEQ ID NO:133); and CDR3 (HQWNRYPYT, SEQ ID NO:134); and heavy chain CDR sequences CDR1 (SYVLH, SEQ ID NO:135); CDR2 (YINPYNDGTQYNEKFKG, SEQ ID NO:136)and CDR3 (GFGGSYGFAY, SEQ ID NO:137).

Antibodies or immunoconjugates comprising an anti-alpha fetoprotein (AFP) MAb, such as IMMU31, can be used to treat hepatocellular carcinoma, germ cell tumors, and other AFP-producing tumors using humanized, chimeric and human antibody forms, as disclosed in U.S. Patent No. 7,300,655. An IMMU31 antibody is a humanized antibody comprising the heavy chain CDR sequences CDR1 (SYVIH, SEQ ID NO:138), CDR2 (YIHPYNGGTKYNEKFKG, SEQ ID NO:139) and CDR3 (SGGGDPFAY, SEQ ID NO:140) and the light chain CDR1 (KASQDINKYIG, SEQ ID NO:141), CDR2 (YTSALLP, SEQ ID NO:142) and CDR3 (LQYDDLWT, SEQ ID NO:143).

Antibodies or immunoconjugates comprising an anti-HLA-DR MAb, such as hL243 (IMMU-114), can be used to treat lymphoma, leukemia, multiple myeloma, cancers of the skin, esophagus, stomach, colon, rectum, pancreas, lung, breast, ovary, bladder, endometrium, cervix, testes, kidney, liver, melanoma or other HLA-DR-producing tumors, as disclosed in U.S. Patent No. 7,612,180. An hL243 antibody is a humanized antibody comprising the heavy chain CDR sequences CDR1 (NYGMN, SEQ ID NO:144), CDR2 (WINTYTREPTYADDFKG, SEQ ID NO:145), and CDR3 (DITAVVPTGFDY, SEQ ID NO:146) and light chain CDR sequences CDR1 (RASENIYSNLA, SEQ ID NO:147), CDR2 (AASNLAD, SEQ ID NO:148), and CDR3 (QHFWTTPWA, SEQ ID NO:149).

Antibodies or immunoconjugates comprising an anti-CD20 MAb, such as veltuzumab (hA20), 1F5, obinutuzumab (GA101), or rituximab, can be used to treat lymphoma, leukemia, immune thrombocytopenic purpura, systemic lupus erythematosus, Sjögren's syndrome, Evans syndrome, arthritis, arteritis, pemphigus vulgaris, renal graft rejection, cardiac graft rejection, rheumatoid arthritis, Burkitt lymphoma, non-Hodgkin's lymphoma, follicular lymphoma, small lymphocytic lymphoma, diffuse B-cell lymphoma, marginal zone lymphoma, chronic lymphocytic leukemia, acute lymphocytic leukemia, Type I diabetes mellitus, GVHD, or multiple sclerosis, as disclosed in U.S. Patent Nos. 7,435,803 or 8,287,864. An hA20 (veltuzumab) antibody is a humanized antibody comprising the light chain CDR sequences CDRL1 (RASSSVSYIH, SEQ ID NO:150), CDRL2 (ATSNLAS, SEQ ID NO:151) and CDRL3 (QQWTSNPPT, SEQ ID NO:152) and heavy chain CDR sequences CDRH1 (SYNMH, SEQ ID NO:153), CDRH2 (AIYPGNGDTSYNQKFKG, SEQ ID NO:154) and CDRH3 (STYYGGDWYFDV, SEQ ID NO:155). Antibodies or immunoconjugates comprising an anti-CD19 MAb, such as hA19, can be used to treat B-cell related lymphomas and leukemias, such as non-Hodgkin's lymphoma, chronic lymphocytic leukemia or acute lymphoblastic leukemia. Other disease states that may be treated include autoimmune diseases, such as acute or chronic immune thrombocytopenia, dermatomyositis, Sydenham's chorea, myasthenia gravis, systemic lupus erythematosus, lupus nephritis, rheumatic fever, polyglandular syndromes, bullous pemphigoid, diabetes mellitus, Henoch-Schonlein purpura, post-streptococcal nephritis, erythema nodosurn, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitis ubiterans, Sjögren's syndrome, primary biliary cirrhosis, Hashimoto's thyroiditis, thyrotoxicosis, scleroderma, chronic active hepatitis, polymyositis/dermatomyositis, polychondritis, pemphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis, and fibrosing alveolitis, as disclosed in U.S. Patent Nos. 7,109,304, 7,462,352, 7,902,338, 8,147,831 and 8,337,840. An hA19 antibody is a humanized antibody comprising the light chain CDR sequences CDR1 KASQSVDYDGDSYLN (SEQ ID NO: 156); CDR2 DASNLVS (SEQ ID NO: 157); and CDR3 QQSTEDPWT (SEQ ID NO: 158) and the heavy chain CDR sequences CDR1 SYWMN (SEQ ID NO: 159); CDR2 QIWPGDGDTNYNGKFKG (SEQ ID NO: 160) and CDR3 RETTTVGRYYYAMDY (SEQ ID NO: 161). Antibodies or immunoconjugates comprising anti-tenascin antibodies can be used to treat hematopoietic and solid tumors, and conjugates comprising antibodies to tenascin can be used to treat solid tumors, preferably brain cancers like glioblastomas.

In a preferred embodiment of the invention as defined in the claims, the antibodies that are used in the treatment of human disease are human or humanized (CDR-grafted) versions of antibodies, although murine and chimeric versions of antibodies can be used. Same species IgG molecules as delivery agents are mostly preferred to minimize immune responses. This is particularly important when considering repeat treatments. For humans, a human or humanized IgG antibody is less likely to generate an anti-IgG immune response from patients. Antibodies such as hLL1 and hLL2 rapidly internalize after binding to internalizing antigen on target cells, which means that the drug being carried is rapidly internalized into cells as well. However, antibodies that have slower rates of internalization can also be used to effect selective therapy.

The antibodies or immunoconjugates can be used to treat autoimmune disease or immune system dysfunction (e.g., graft-versus-host disease, organ transplant rejection). Antibodies of use to treat autoimmune/immune dysfunction disease may bind to exemplary antigens including, but not limited to, BCL-1, BCL-2, BCL-6, CD1a, CD2, CD3, CD4,

CD5, CD7, CD8, CD10, CD11b, CD11c, CD13, CD14, CD15, CD16, CD19, CD20, CD21, CD22, CD23, CD25, CD33, CD34, CD38, CD40, CD40L, CD41a, CD43, CD45, CD55, CD56, CCD57, CD59, CD64, CD71, CD74, CD79a, CD79b, CD117, CD138, FMC-7 and HLA-DR. Antibodies that bind to these and other target antigens, discussed above, may be used to treat autoimmune or immune dysfunction diseases. Autoimmune diseases that may be treated with antibodies or immunoconjugates may include acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, diabetes mellitus, Henoch-Schonlein purpura, post-streptococcal nephritis, erythema nodosum, Takayasu's arteritis, ANCA-associated vasculitides, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitis obliterans, Sjogren's syndrome, primary biliary cirrhosis, Hashimoto's thyroiditis, thyrotoxicosis, scleroderma, chronic active hepatitis, polymyositis/dermatomyositis, polychondritis, bullous pemphigoid, pemphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis or fibrosing alveolitis.

In another preferred embodiment of the invention as defined in the claims, a therapeutic agent used in combination with the immunoconjugates may comprise one or more isotopes. Radioactive isotopes useful for treating diseased tissue include, but are not limited to- $^{111}$In, $^{177}$Lu, $^{212}$Bi, $^{213}$Bi, $^{211}$At, $^{62}$Cu, $^{67}$Cu, $^{90}$Y, $^{125}$I, $^{131}$I, $^{32}$P, $^{33}$P, $^{47}$Sc, $^{111}$Ag $^{67}$Ga, $^{142}$Pr, $^{153}$Sm, $^{161}$Tb, $^{166}$Dy, $^{166}$Ho, $^{186}$Re, $^{188}$Re, $^{189}$Re, $^{212}$Pb, $^{223}$Ra, $^{225}$Ac, $^{59}$Fe, $^{75}$Se, $^{77}$As, $^{89}$Sr, $^{99}$Mo, $^{105}$Rh, $^{109}$Pd, $^{143}$ Pr, $^{149}$Pm, $^{169}$Er, $^{194}$Ir, $^{198}$Au, $^{199}$Au, $^{227}$Th and $^{211}$Pb. The therapeutic radionuclide preferably has a decay-energy in the range of 20 to 6,000 keV, preferably in the ranges 60 to 200 keV for an Auger emitter, 100-2,500 keV for a beta emitter, and 4,000-6,000 keV for an alpha emitter. Maximum decay energies of useful beta-particle-emitting nuclides are preferably 20-5,000 keV, more preferably 100-4,000 keV, and most preferably 500-2,500 keV. Also preferred are radionuclides that substantially decay with Auger-emitting particles. For example, Co-58, Ga-67, Br-80m, Tc-99m, Rh-103m, Pt-109, In-111, Sb-119, I-125, Ho-161, Os-189m and Ir-192. Decay energies of useful beta-particle-emitting nuclides are preferably <1,000 keV, more preferably <100 keV, and most preferably <70 keV. Also preferred are radionuclides that substantially decay with generation of alpha-particles. Such radionuclides include, but are not limited to: Dy-152, At-211, Bi-212, Ra-223, Rn-219, Po-215, Bi-211, Ac-225, Fr-221, At-217, Bi-213, Th-227 and Fm-255. Decay energies of useful alpha-particle-emitting radionuclides are preferably 2,000-10,000 keV, more preferably 3,000-8,000 keV, and most preferably 4,000-7,000 keV. Additional potential radioisotopes of use include $^{11}$C, $^{13}$N, $^{15}$O, $^{75}$Br, $^{198}$Au, $^{224}$Ac, $^{126}$I, $^{133}$I, $^{77}$Br, $^{113m}$In, $^{95}$Ru, $^{97}$Ru, $^{103}$Ru, $^{105}$Ru, $^{107}$Hg, $^{203}$Hg, $^{121m}$Te, $^{122m}$Te, $^{125m}$Te, $^{165}$Tm, $^{167}$Tm, $^{168}$Tm, $^{197}$Pt, $^{109}$Pd, $^{105}$Rh, $^{142}$Pr, $^{143}$Pr, $^{161}$Tb, $^{166}$Ho, $^{199}$Au, $^{57}$Co, $^{58}$Co, $^{51}$Cr, $^{59}$Fe, $^{75}$Se, $^{201}$Tl, $^{225}$Ac, $^{76}$Br, $^{169}$Yb, and the like.

Radionuclides and other metals may be delivered, for example, using chelating groups attached to an antibody or immunoconjugate. Macrocyclic chelates such as NOTA, DOTA, and TETA are of use with a variety of metals and radiometals, most particularly with radionuclides of gallium, yttrium and copper, respectively. Such metal-chelate complexes can be made very stable by tailoring the ring size to the metal of interest. Other ring-type chelates, such as macrocyclic polyethers for complexing $^{223}$Ra, may be used.

Therapeutic agents of use in combination with the antibodies or immunoconjugates described herein also include, for example, chemotherapeutic drugs such as vinca alkaloids, anthracyclines, epidophyllotoxins, taxanes, antimetabolites, tyrosine kinase inhibitors, Bruton tyrosine kinase inhibitors, microtubule inhibitors, PARP inhibitors, PI3K inhibitors, alkylating agents, antibiotics, Cox-2 inhibitors, antimitotics, antiangiogenic and proapoptotic agents, particularly doxorubicin, methotrexate, taxol, other camptothecins, and others from these and other classes of anticancer agents, and the like. Other cancer chemotherapeutic drugs include nitrogen mustards, alkyl sulfonates, nitrosoureas, triazenes, folic acid analogs, pyrimidine analogs, purine analogs, platinum coordination complexes, hormones, and the like. Suitable chemotherapeutic agents are described in REMINGTON'S PHARMACEUTICAL SCIENCES, 19th Ed. (Mack Publishing Co. 1995), and in GOODMAN AND GILMAN'S THE PHARMACOLOGICAL BASIS OF THERAPEUTICS, 7th Ed. (MacMillan Publishing Co. 1985), as well as revised editions of these publications. Other suitable chemotherapeutic agents, such as experimental drugs, are known to those of skill in the art.

Exemplary drugs of use include, but are not limited to, 5-fluorouracil, afatinib, aplidin, azaribine, anastrozole, anthracyclines, axitinib, AVL-101, AVL-291, bendamustine, bleomycin, bortezomib, bosutinib, bryostatin-1, busulfan, calicheamycin, camptothecin, carboplatin, 10-hydroxycamptothecin, carmustine, celebrex, chlorambucil, cisplatin (CDDP), Cox-2 inhibitors, irinotecan (CPT-11), SN-38, carboplatin, cladribine, camptothecans, crizotinib, cyclophosphamide, cytarabine, dacarbazine, dasatinib, dinaciclib, docetaxel, dactinomycin, daunorubicin, doxorubicin, 2-pyrrolinodoxorubicine (2PDOX), pro-2PDOX, cyano-morpholino doxorubicin, doxorubicin glucuronide, epirubicin glucuronide, erlotinib, estramustine, epidophyllotoxin, erlotinib, entinostat, estrogen receptor binding agents, etoposide (VP16), etoposide glucuronide, etoposide phosphate, exemestane, fingolimod, floxuridine (FUdR), 3',5'-O-dioleoyl-FudR (FUdR-dO), fludarabine, flutamide, farnesyl-protein transferase inhibitors, flavopiridol, fostamatinib, ganetespib, GDC-0834, GS-1101, gefitinib, gemcitabine, hydroxyurea, ibrutinib, idarubicin, idelalisib, ifosfamide, imatinib, L-asparaginase, lapatinib, lenolidamide, leucovorin, LFM-A13, lomustine, mechlorethamine, melphalan, mercaptopurine, 6-mercaptopurine, methotrexate, mitoxantrone, mithramycin, mitomycin, mitotane, navelbine, neratinib, nilotinib, nitrosurea, olaparib, plicomycin, procarbazine, paclitaxel, PCI-32765, pentostatin, PSI-341, raloxifene, semustine, sorafenib, streptozocin, SU11248, sunitinib, tamoxifen, temazolomide (an aqueous form of DTIC), transplatinum, thalidomide, thioguanine, thiotepa, teniposide, topotecan, uracil mustard, vatalanib, vinorelbine, vinblastine, vincristine, vinca alkaloids and ZD1839. Such agents may be part of the conjugates described herein or may alternatively be administered in combination with the described conjugates, either prior to, simultaneously with or after the conjugate. Alternatively, one or more therapeutic naked antibodies as are known in the art may be used in combination with the described conjugates. Exemplary therapeutic naked antibodies are described above.

In preferred embodiments of the invention as defined in the claims, a therapeutic agent to be used in combination with a DNA-breaking antibody conjugate (e.g., an SN-38-ADC) is a microtubule inhibitor, such as a vinca alkaloid, a taxanes, a maytansinoid or an auristatin. Exemplary known microtubule inhibitors include paclitaxel, vincristine, vinblastine, mertansine, epothilone, docetaxel, discodermolide, combrestatin, podophyllotoxin, CI-980, phenylahistins, steganacins, curacins, 2-methoxy estradiol, E7010, methoxy benzenesuflonamides, vinorelbine, vinflunine, vindesine, dolastatins, spongistatin, rhizoxin, tasidotin, halichondrins, hemiasterlins, cryptophycin 52, MMAE and eribulin mesylate.

In an alternative preferred embodiment of the invention as defined in the claims, a therapeutic agent to be used in combination with a DNA-breaking ADC, such as an SN-38-antibody conjugate, is a PARP inhibitor, such as olaparib, talazoparib (BMN-673), rucaparib, veliparib, CEP 9722, MK 4827, BGB-290, ABT-888, AG014699, BSI-201, CEP-8983 or 3-aminobenzamide.

Alternatively, a therapeutic agent used in combination with an antibody or immunoconjugate is a Bruton kinase inhibitor, such as such as ibrutinib (PCI-32765), PCI-45292, CC-292 (AVL-292), ONO-4059, GDC-0834, LFM-A13 or RN486.

In yet another alternative, a therapeutic agent used in combination with an antibody or immunoconjugate is a PI3K inhibitor, such as idelalisib, Wortmannin, demethoxyviridin, perifosine, PX-866, IPI-145 (duvelisib), BAY 80-6946, BEZ235, RP6530, TGR1202, SF1126, INK1117, GDC-0941, BKM120, XL147, XL765, Palomid 529, GSK1059615, ZSTK474, PWT33597, IC87114, TG100-115, CAL263, PI-103, GNE477, CUDC-907, AEZS-136 or LY294002.

Therapeutic agents that may be used in the practicing og the invention as defined in the claims concert with the antibodies or immunoconjugates also may comprise toxins conjugated to targeting moieties. Toxins that may be used in this regard include ricin, abrin, ribonuclease (RNase), DNase I, Staphylococcal enterotoxin-A, pokeweed antiviral protein, gelonin, diphtheria toxin, *Pseudomonas* exotoxin, and *Pseudomonas* endotoxin. (See, e.g., Pastan. et al., Cell (1986), 47:641, and Sharkey and Goldenberg, CA Cancer J Clin. 2006 Jul-Aug;56(4):226-43.) Additional toxins suitable for use herein are known to those of skill in the art and are disclosed in U.S. 6,077,499.

Yet another class of therapeutic agent may comprise one or more immunomodulators. Immunomodulators of use may be selected from a cytokine, a stem cell growth factor, a lymphotoxin, an hematopoietic factor, a colony stimulating factor (CSF), an interferon (IFN), erythropoietin, thrombopoietin and a combination thereof. Specifically useful are lymphotoxins such as tumor necrosis factor (TNF), hematopoietic factors, such as interleukin (IL), colony stimulating factor, such as granulocyte-colony stimulating factor (G-CSF) or granulocyte macrophage-colony stimulating factor (GM-CSF), interferon, such as interferons-$\alpha$, -$\beta$, -$\gamma$ or -$\lambda$, and stem cell growth factor, such as that designated "S1 factor". Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; prostaglandin, fibroblast growth factor; prolactin; placental lactogen, OB protein; tumor necrosis factor-$\alpha$ and - $\beta$; mullerian-inhibiting substance; mouse gonadotropin-associated peptide; inhibin; activin; vascular endothelial growth factor; integrin; thrombopoietin (TPO); nerve growth factors such as NGF-$\beta$; platelet-growth factor; transforming growth factors (TGFs) such as TGF- $\alpha$ and TGF- $\beta$; insulin-like growth factor-I and -II; erythropoietin (EPO); osteoinductive factors; interferons such as interferon-$\alpha$, -$\beta$, and -$\gamma$; colony stimulating factors (CSFs) such as macrophage-CSF (M-CSF); interleukins (ILs) such as IL-1, IL,-1$\alpha$, IL-2, IL-3, IL-4, IL-5, IL-6, IL-7, IL-8, IL-9, IL-10, IL-11, IL-12; IL-13, IL-14, IL-15, IL-16, IL-17, IL-18, IL-21, IL-25, LIF, kit-ligand or FLT-3, angiostatin, thrombospondin, endostatin, tumor necrosis factor and lymphotoxin (LT). As used herein, the term cytokine includes proteins from natural sources or from recombinant cell culture and biologically active equivalents of the native sequence cytokines.

Chemokines of use include RANTES, MCAF, MIP1-alpha, MIP1-Beta and IP-10.

The person of ordinary skill will realize that the use of the subject immunoconjugates and drugs according to the present invention as defined in the claims may encompass additionally using one or more other therapeutic agents, such as a second antibody, second antibody fragment, second immunoconjugate, radionuclide, toxin, drug, chemotherapeutic agent, radiation therapy, chemokine, cytokine, immunomodulator, enzyme, hormone, oligonucleotide, RNAi or siRNA. Preferably, the therapeutic agent is a PARP inhibitor, a microtubule inhibitor, a Bruton kinase inhibitor or a PI3K inhibitor. Exemplary known PARP inhibitors include olaparib, talazoparib (BMN-673), rucaparib, veliparib, CEP 9722, MK 4827, BGB-290, ABT-888, AG014699, BSI-201, CEP-8983 or 3-aminobenzamide. Exemplary known microtubule inhibitors include, but are not limited to, vinca alkaloid, taxanes, maytansinoids, auristatins, paclitaxel, vincristine, vinblastine, mertansine, epothilone, docetaxel, discodermolide, combrestatin, podophyllotoxin, CI-980, phenylahistins, steganacins, curacins, 2-methoxy estradiol, E7010, methoxy benzenesuflonamides, vinorelbine, vinflunine, vindesine, dolastatins, spongistatin, rhizoxin, tasidotin, halichondrins, hemiasterlins, cryptophycin 52, MMAE and eribulin mesylate. Exemplary Bruton kinase inhibitors include ibrutinib (PCI-32765), PCI-45292, CC-292 (AVL-292), ONO-4059, GDC-0834, LFM-A13 or RN486. Exemplary PI3K inhibitors include idelalisib, Wortmannin, demethoxyviridin, perifosine, PX-866, IPI-145 (duvelisib), BAY 80-6946, BEZ235, RP6530, TGR1202, SF1126, INK1117, GDC-0941, BKM120, XL147, XL765, Palomid 529, GSK1059615, ZSTK474, PWT33597, IC87114, TG100-115, CAL263, PI-103, GNE477, CUDC-907, AEZS-136 or LY294002. Such additional therapeutic agents may be administered separately, in combination with, or attached to the subject antibodies or immunoconjugates.

**Formulation and Administration**

Suitable routes of administration of the antibodies, immunoconjugates and/or drugs include, without limitation, parenteral, subcutaneous, rectal, transmucosal, intestinal administration, intramuscular, intramedullary, intrathecal, direct intraventricular, intravenous, intravitreal, intraperitoneal, intranasal, or intraocular injections. The preferred routes of administration are parenteral. Alternatively, one may administer the compound in a local rather than systemic manner, for example, via injection of the compound directly into a solid tumor. Certain drugs, such as microtubule inhibitors, PARP inhibitors, Bruton kinase inhibitors or PI3K inhibitors may be designed to be administered orally.

Antibodies or immunoconjugates can be formulated according to known methods to prepare pharmaceutically useful compositions, whereby the antibody or immunoconjugate is combined in a mixture with a pharmaceutically suitable excipient. Sterile phosphate-buffered saline is one example of a pharmaceutically suitable excipient. Other suitable excipients are well-known to those in the art. See, for example, Ansel et al., PHARMACEUTICAL DOSAGE FORMS AND DRUG DELIVERY SYSTEMS, 5th Edition (Lea & Febiger 1990), and Gennaro (ed.), REMINGTON'S PHARMACEUTICAL SCIENCES, 18th Edition (Mack Publishing Company 1990), and revised editions thereof.

In a preferred embodiment, the antibody or immunoconjugate is formulated in Good's biological buffer (pH 6-7), using a buffer selected from the group consisting of N-(2-acetamido)-2-aminoethanesulfonic acid (ACES); N-(2-acetamido)iminodiacetic acid (ADA); N,N-bis(2-hydroxyethyl)-2-aminoethanesulfonic acid (BES); 4-(2-hydroxyethyl)piperazine-1-ethanesulfonic acid (HEPES); 2-(N-morpholino)ethanesulfonic acid (MES); 3-(N-morpholino)propanesulfonic acid (MOPS); 3-(N-morpholinyl)-2-hydroxypropanesulfonic acid (MOPSO); and piperazine-N,N'-bis(2-ethanesulfonic acid) [Pipes]. More preferred buffers are MES or MOPS, preferably in the concentration range of 20 to 100 mM, more preferably about 25 mM. Most preferred is 25 mM MES, pH 6.5. The formulation may further comprise 25 mM trehalose and 0.01% v/v polysorbate 80 as excipients, with the final buffer concentration modified to 22.25 mM as a result of added excipients. The preferred method of storage is as a lyophilized formulation of the conjugates, stored in the temperature range of -20 °C to 2 °C, with the most preferred storage at 2 °C to 8 °C.

The antibody or immunoconjugate can be formulated for intravenous administration via, for example, bolus injection, slow infusion or continuous infusion. Preferably, the antibody of the present invention is infused over a period of less than about 4 hours, and more preferably, over a period of less than about 3 hours. For example, the first 25-50 mg could be infused within 30 minutes, preferably even 15 min, and the remainder infused over the next 2-3 hrs. Formulations for injection can be presented in unit dosage form, e.g., in ampoules or in multi-dose containers, with an added preservative. The compositions can take such forms as suspensions, solutions or emulsions in oily or aqueous vehicles, and can contain formulatory agents such as suspending, stabilizing and/or dispersing agents. Alternatively, the active ingredient can be in powder form for constitution with a suitable vehicle, e.g., sterile pyrogen-free water, before use.

Additional pharmaceutical methods may be employed to control the duration of action of the therapeutic conjugate. Control release preparations can be prepared through the use of polymers to complex or adsorb the immunoconjugate. For example, biocompatible polymers include matrices of poly(ethylene-co-vinyl acetate) and matrices of a polyanhydride copolymer of a stearic acid dimer and sebacic acid. Sherwood et al., Bio/Technology 10: 1446 (1992). The rate of release of an antibody or immunoconjugate from such a matrix depends upon the molecular weight, the amount of antibody or immunoconjugate within the matrix, and the size of dispersed particles. Saltzman et al., Biophys. J. 55: 163 (1989); Sherwood *et al.*, *supra.* Other solid dosage forms are described in Ansel et al., PHARMACEUTICAL DOSAGE FORMS AND DRUG DELIVERY SYSTEMS, 5th Edition (Lea & Febiger 1990), and Gennaro (ed.), REMINGTON'S PHARMACEUTICAL SCIENCES, 18th Edition (Mack Publishing Company 1990), and revised editions thereof.

Generally, the dosage of an administered antibody or immunoconjugate for humans will vary depending upon such factors as the patient's age, weight, height, sex, general medical condition and previous medical history. It may be desirable to provide the recipient with a dosage of immunoconjugate that is in the range of from about 1 mg/kg to 24 mg/kg as a single intravenous infusion, although a lower or higher dosage also may be administered as circumstances dictate. A dosage of 1-20 mg/kg for a 70 kg patient, for example, is 70-1,400 mg, or 41-824 $mg/m^2$ for a 1.7-m patient. The dosage may be repeated as needed, for example, once per week for 4-10 weeks, once per week for 8 weeks, or

once per week for 4 weeks. It may also be given less frequently, such as every other week for several months, or monthly or quarterly for many months, as needed in a maintenance therapy. Preferred dosages may include, but are not limited to, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, and 18 mg/kg. The dosage is preferably administered multiple times, once or twice a week, or as infrequently as once every 3 or 4 weeks. A minimum dosage schedule of 4 weeks, more preferably 8 weeks, more preferably 16 weeks or longer may be used. The schedule of administration may comprise administration once or twice a week, on a cycle selected from the group consisting of: (i) weekly; (ii) every other week; (iii) one week of therapy followed by two, three or four weeks off; (iv) two weeks of therapy followed by one, two, three or four weeks off; (v) three weeks of therapy followed by one, two, three, four or five week off; (vi) four weeks of therapy followed by one, two, three, four or five week off; (vii) five weeks of therapy followed by one, two, three, four or five week off; (viii) monthly and (ix) every 3 weeks. The cycle may be repeated 2, 4, 6, 8, 10, 12, 16 or 20 times or more.

Alternatively, an antibody or immunoconjugate may be administered as one dosage every 2 or 3 weeks, repeated for a total of at least 3 dosages. Or, twice per week for 4-6 weeks. If the dosage is lowered to approximately 200-300 $mg/m^2$ (340 mg per dosage for a 1.7-m patient, or 4.9 mg/kg for a 70 kg patient), it may be administered once or even twice weekly for 4 to 10 weeks. Alternatively, the dosage schedule may be decreased, namely every 2 or 3 weeks for 2-3 months. It has been determined, however, that even higher doses, such as 12 mg/kg once weekly or once every 2-3 weeks can be administered by slow i.v. infusion, for repeated dosing cycles. The dosing schedule can optionally be repeated at other intervals and dosage may be given through various parenteral routes, with appropriate adjustment of the dose and schedule.

Examples of cancers which can be treated in accordance with the invention as defined in the claims include, but are not limited to, carcinoma, lymphoma, glioblastoma, melanoma, sarcoma, and leukemia, myeloma, or lymphoid malignancies. More particular examples of such cancers are noted below and include: squamous cell cancer (e.g., epithelial squamous cell cancer), Ewing sarcoma, Wilms tumor, astrocytomas, glioblastomas, lung cancer including small-cell lung cancer, non-small cell lung cancer, adenocarcinoma of the lung and squamous carcinoma of the lung, cancer of the peritoneum, gastric or stomach cancer including gastrointestinal cancer, pancreatic cancer, glioblastoma multiforme, cervical cancer, ovarian cancer, liver cancer, bladder cancer, hepatoma, hepatocellular carcinoma, neuroendocrine tumors, medullary thyroid cancer, differentiated thyroid carcinoma, breast cancer, ovarian cancer, colon cancer, rectal cancer, endometrial cancer or uterine carcinoma, salivary gland carcinoma, kidney or renal cancer, prostate cancer, vulvar cancer, anal carcinoma, penile carcinoma, as well as head-and-neck cancer. The term "cancer" includes primary malignant cells or tumors (e.g., those whose cells have not migrated to sites in the subject's body other than the site of the original malignancy or tumor) and secondary malignant cells or tumors (e.g., those arising from metastasis, the migration of malignant cells or tumor cells to secondary sites that are different from the site of the original tumor).

Other examples of cancers or malignancies include, but are not limited to: Acute Childhood Lymphoblastic Leukemia, Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Lymphoma, Adult Lymphocytic Leukemia, Adult Non-Hodgkin's Lymphoma, Adult Primary Liver Cancer, Adult Soft Tissue Sarcoma, AIDS-Related Lymphoma, AIDS-Related Malignancies, Anal Cancer, Astrocytoma, Bile Duct Cancer, Bladder Cancer, Bone Cancer, Brain Stem Glioma, Brain Tumors, Breast Cancer, Cancer of the Renal Pelvis and Ureter, Central Nervous System (Primary) Lymphoma, Central Nervous System Lymphoma, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Childhood Cerebellar Astrocytoma, Childhood Cerebral Astrocytoma, Childhood Extracranial Germ Cell Tumors, Childhood Hodgkin's Disease, Childhood Hodgkin's Lymphoma, Childhood Hypothalamic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumors, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-Cell Lymphoma, Endocrine Pancreas Islet Cell Carcinoma, Endometrial Cancer, Ependymoma, Epithelial Cancer, Esophageal Cancer, Ewing's Sarcoma and Related Tumors, Exocrine Pancreatic Cancer, Extracranial Germ Cell Tumor, Extragonadal Germ Cell Tumor, Extrahepatic Bile Duct Cancer, Eye Cancer, Female Breast Cancer, Gaucher's Disease, Gallbladder Cancer, Gastric Cancer, Gastrointestinal Carcinoid Tumor, Gastrointestinal Tumors, Germ Cell Tumors, Gestational Trophoblastic Tumor, Hairy Cell Leukemia, Head and Neck Cancer, Hepatocellular Cancer, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lymphoproliferative Disorders, Macroglobulinemia, Male Breast Cancer, Malignant Mesothelioma, Malignant Thymoma, Medulloblastoma, Melanoma, Mesothelioma, Metastatic Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative

Disorders, Nasal Cavity and Paranasal Sinus Cancer, Nasopharyngeal Cancer, Neuroblastoma, Non-Hodgkin's Lymphoma, Nonmelanoma Skin Cancer, Non-Small Cell Lung Cancer, Occult Primary Metastatic Squamous Neck Cancer, Oropharyngeal Cancer, Osteo-/Malignant Fibrous Sarcoma, Osteosarcoma/Malignant Fibrous Histiocytoma, Osteosarcoma/Malignant Fibrous Histiocytoma of Bone, Ovarian Epithelial Cancer, Ovarian Germ Cell Tumor, Ovarian Low Malignant Potential Tumor, Pancreatic Cancer, Paraproteinemias, Polycythemia vera, Parathyroid Cancer, Penile Cancer, Pheochromocytoma, Pituitary Tumor, Primary Central Nervous System Lymphoma, Primary Liver Cancer, Prostate Cancer, Rectal Cancer, Renal Cell Cancer, Renal Pelvis and Ureter Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumors, T-Cell Lymphoma, Testicular Cancer, Thymoma, Thyroid Cancer, Transitional Cell Cancer of the Renal Pelvis and Ureter, Transitional Renal Pelvis and Ureter Cancer, Trophoblastic Tumors, Ureter and Renal Pelvis Cell Cancer, Urethral Cancer, Uterine Cancer, Uterine Sarcoma, Vaginal Cancer, Visual Pathway and Hypothalamic Glioma, Vulvar Cancer, Waldenström's macroglobulinemia, Wilms' tumor, and any other hyperproliferative disease, besides neoplasia, located in an organ system listed above.

The invention as defined in the claims may be used to treat malignant or premalignant conditions and to prevent progression to a neoplastic or malignant state, including but not limited to those disorders described above. Such uses are indicated in conditions known or suspected of preceding progression to neoplasia or cancer, in particular, where non-neoplastic cell growth consisting of hyperplasia, metaplasia, or most particularly, dysplasia has occurred (for review of such abnormal growth conditions, see Robbins and Angell, Basic Pathology, 2d Ed., W. B. Saunders Co., Philadelphia, pp. 68-79 (1976)).

Dysplasia is frequently a forerunner of cancer, and is found mainly in the epithelia. It is the most disorderly form of non-neoplastic cell growth, involving a loss in individual cell uniformity and in the architectural orientation of cells. Dysplasia characteristically occurs where there exists chronic irritation or inflammation. Dysplastic disorders which can be treated include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis hemimelia, dysplasia epiphysialis multiplex, dysplasia epiphysialis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia, hidrotic ectodermal dysplasia, hypohidrotic ectodermal dysplasia, lymphopenic thymic dysplasia, mammary dysplasia, mandibulofacial dysplasia, metaphysial dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, opthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

Additional pre-neoplastic disorders which can be treated include, but are not limited to, benign dysproliferative disorders (e.g., benign tumors, fibrocystic conditions, tissue hypertrophy, intestinal polyps or adenomas, and esophageal dysplasia), leukoplakia, keratoses, Bowen's disease, Farmer's Skin, solar cheilitis, and solar keratosis.

In preferred embodiments, the invention as defined in the claims is used to inhibit growth, progression, and/or metastasis of cancers, in particular those listed above.

Additional hyperproliferative diseases, disorders, and/or conditions include, but are not limited to, progression, and/or metastases of malignancies and related disorders such as leukemia (including acute leukemias; e.g., acute lymphocytic leukemia, acute myelocytic leukemia [including myeloblastic, promyelocytic, myelomonocytic, monocytic, and erythroleukemia]) and chronic leukemias (e.g., chronic myelocytic [granulocytic] leukemia and chronic lymphocytic leukemia), polycythemia vera, lymphomas (e.g., Hodgkin's disease and non-Hodgkin's disease), multiple myeloma, Waldenström's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, emangioblastoma, acoustic neuroma, oligodendroglioma, meningioma, melanoma, neuroblastoma, and retinoblastoma.

Autoimmune diseases that may be treated with antibodies or immunoconjugates may include acute and chronic immune thrombocytopenias, dermatomyositis, Sydenham's chorea, myasthenia gravis, systemic lupus erythematosus,

lupus nephritis, rheumatic fever, polyglandular syndromes, bullous pemphigoid, diabetes mellitus, Henoch-Schonlein purpura, post-streptococcal nephritis, erythema nodosum, Takayasu's arteritis, ANCA-associated vasculitides, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitis obliterans, Sjogren's syndrome, primary biliary cirrhosis, Hashimoto's thyroiditis, thyrotoxicosis, scleroderma, chronic active hepatitis, polymyositis/dermatomyositis, polychondritis, bullous pemphigoid, pemphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis or fibrosing alveolitis.

Kits

The present disclosure also concerns kits containing components suitable for treating diseased tissue in a patient. Exemplary kits may contain at least one antibody or immunoconjugate as described herein. A kit may also include a drug selected from microtubule inhibitors, PARP inhibitors, Bruton kinase inhibitors and/or PI3K inhibitors. If the composition containing components for administration is not formulated for delivery via the alimentary canal, such as by oral delivery, a device capable of delivering the kit components through some other route may be included. One type of device, for applications such as parenteral delivery, is a syringe that is used to inject the composition into the body of a subject. Inhalation devices may also be used.

The kit components may be packaged together or separated into two or more containers. In some embodiments, the containers may be vials that contain sterile, lyophilized formulations of a composition that are suitable for reconstitution. A kit may also contain one or more buffers suitable for reconstitution and/or dilution of other reagents. Other containers that may be used include, but are not limited to, a pouch, tray, box, tube, or the like. Kit components may be packaged and maintained sterilely within the containers. Another component that can be included is instructions to a person using a kit for its use.

**EXAMPLES**

**Example 1. Combination therapy with ADC IMMU-132 and microtubule inhibitors or PARP inhibitors**

In current clinical trials (ClinicalTrials.gov, NCT01631552), triple-negative breast cancer (TNBC) patients treated with IMMU-132, which is composed of the active metabolite of irinotecan, SN-38, conjugated to an anti-Trop-2 antibody, shows manageable toxicity and very encouraging responses in relapsed/refractory cases.

Synthetic lethality is a concept in which a cell harboring one out of two possible gene or protein defects is viable, while a cell containing both defects is nonviable. *BRCA1/2* mutations are linked to deficiencies in DNA repair and are associated with TNBC. Other repair mechanisms involve poly(adenosine diphosphoribose) polymerase (PARP), which can be used by cancer cells to overcome loss of *BRACA1/2.* Treatment of TNBC cells with either IMMU-132 or paclitaxel results in cleavage and deactivation of PARP, whereas the small molecule olaparib directly inhibits PARP. Therefore, the rationale of combining IMMU-132 with either paclitaxel or olaparib to effectively knock-out PARP activity was investigated in TNBC xenografts to ascertain if these combinations will result in synthetic lethality.

The purpose of this study was to determine whether combining an antibody-drug conjugate that induces DNA strand breaks, such as sacituzumab govitecan (also known as IMMU-132, an anti-Trop-2 hRS7-CL2A-SN-38), with microtubule inhibitors (e.g., paclitaxel or eribulin mesylate) or poly(adenosine diphosphoribose) polymerase (PARP) inhibitors (e.g., olaparib) in cancer (e.g., nude mice bearing TNBC xenografts) improves anti-tumor effects. The person of ordinary skill will realize that the unexpected superior effects of the combination of antibody-SN-38 conjugates with PARP or microtubule inhibitors are not limited to the specific exemplary antibody, drug, PARP inhibitor or microtubule inhibitor, but rather are characteristic of the classes of antibodies against tumor-associated antigens (TAAs), drugs that induce DNA strand breaks, PARP inhibitors and microtubule inhibitors.

*Experimental Procedures*

In a non-limiting example, mice bearing human TNBC (triple negative breast cancer) xenografts (MDA-MB-468 or HCC1806; ~0.3 $cm^3$) were treated with the maximum tolerated dose of paclitaxel (15 mg/kg weekly x 5 wks) and IMMU-132 at either 10 mg/kg or 12.5 mg/kg on days 1, 8, 22, and 29. Mice bearing HCC1806 tumors (~0.28 $cm^3$) were treated for 2 cycles with IMMU-132 (12.5 mg/kg) and 0.5 mg/kg of eribulin mesylate (equivalent to human dose of 1.4 $mg/m^2$) weekly for 2 weeks on a 21-day cycle. Studies examining PARP inhibition used mice bearing MDA-MB-468 tumors (~0.32 $cm^3$) treated with olaparib (50 mg/kg, qdx5d, x 4 wks; 33% of human dose equaling 800 mg daily) and IMMU-132 (10 mg/kg, twice weekly x 4 wks). Olaparib was administered as i.p. injections daily for 5 days in a row with two day's rest before repeating (qdx5). This was done for four weeks. IMMU-132 was administered i.p. twice weekly for

four weeks. Control animals received the non-tumor targeting anti-CD20 ADC hA20-CL2A-SN-38, either alone or in combination with olaparib. The primary endpoint was the median survival time (MST), defined as the time for tumors to progress to 1.0 $cm^3$.

In alternative embodiments, assay for synergistic effects may be determined by *in vitro* assay. A clonogenic assay may be used to determine survival fraction of cells (Ibrahim et al., 2012, Cancer Discovery 2:1036-47). Briefly, 350-800 cells are plated in 6-well flat bottom cell culture plates in duplicates. Twenty-four hours after plating, cells are washed and fresh medium is added in the presence or absence of increasing doses of ADC and/or PARP or microtubule inhibitor (e.g., olaparib) alone and in combination. Media containing the drug and/or is refreshed on day 4. Colonies are fixed and stained after 7 days of treatment with 1.5 ml of 6.0 % glutaraldehyde and 0.5 % crystal violet and colonies are counted by standard procedures. The surviving fraction (SF) of cells is calculated as follows:

$$SF = \frac{\text{Number of colonies formed after treatment}}{\text{Number of cells seeded x Plating Efficiency}}$$

$$\text{Plating Efficiency} = \frac{\text{Number of colonies formed in control}}{\text{Number of cells seeded}}$$

where

The interaction between ADC and PARP or microtubule inhibitor is assessed using the multiple drug effects analysis method of Chou and Talalay (1984, Adv Enzyme Regul 22:27-55). This method quantitatively describes the interaction between two or more drugs, with values less than 1 indicating synergistic interactions, values greater than 1 indicating antagonistic interactions, and values equal to 1 indicating additive interactions.

*Results*

Mice with MDA-MB-468 tumors given the combination of IMMU-132 and paclitaxel exhibited superior anti-tumor effects (**FIG. 1A-1C**), with >11-fold tumor shrinkage, in comparison to 1.4-fold shrinkage in the IMMU-132 group alone (P=0.0003; area under the curve, AUC) or 11.4-fold increase in tumor size in mice treated with paclitaxel alone (P<0.0001; AUC).

In MDA-MB-468, the combination of 200 µg IMMU-132 plus paclitaxel has superior anti-tumor effects in terms of area under the curve (AUC) when compared to all the other groups (**Table 7**, *P*<0.0013). Lowering the amount of IMMU-132 administered with paclitaxel to 100 µg likewise results in significant anti-tumor effects as compared to mice treated with paclitaxel alone, IMMU-132 alone (100 µg), or untreated animals (**Table 8**, P<0.0328). No further comparisons between growth curves can be made with paclitaxel or untreated control groups since each began to lose mice due to disease progression (i.e., TV>1.0 $cm^3$) as of therapy day 49.

**Table 7. Area under the curve comparisons between IMMU-132 (200 µg) *plus* Paclitaxel treated MDA-MB-468 tumor-bearing mice and all other treatment groups.**

| Treatments | | Time of Comparison | Tumor Volumes ($cm^3$) on that day (mean ± s.d.) | *P*-Value (AUC) |
|---|---|---|---|---|
| **IMMU-132 (200 µg) *plus* Paclitaxel *versus*** | **IMMU-132 (200 µg)** | **Up to therapy Day 98** | **0.162 ± 0.144 vs. 0.621 ± 0.324** | **0.0003** |
| | **IMMU-132 (100 µg)** | **Up to therapy Day 70** | **0.050 ± 0.062 vs. 0.634 ± 0.335** | **0.0002** |
| | **Paclitaxel** | **Up to therapy Day 49** | **0.025 ± 0.041 vs. 0.705 ± 0.206** | **<0.0001** |
| | **IMMU-132 (100 µg) + Paclitaxel** | **Up to therapy Day 112** | **0.202 ± 0.191 vs. 0.496 ± 0.286** | **0.0013** |
| | **Untreated** | **Up to therapy Day 49** | **0.025 ± 0.041 vs. 0.663 ± 0.349** | **<0.0001** |

| Table 8. Area under the curve comparisons between IMMU-132 (100 μg) *plus* Paclitaxel treated MDA-MB-468 tumor-bearing mice and all other treatment groups. | | | | |
|---|---|---|---|---|
| **Treatments** | | **Time of Comparison** | **Tumor Volumes ($cm^3$) on that day (mean ± s.d.)** | **P-Value (AUC)** |
| **IMMU-132 (100 μg) *plus* Paclitaxel *versus*** | **IMMU-132 (200 μg)** | **Up to therapy Day 98** | **0.663 ± 0.349 vs. 0.621 ± 0.324** | **0.9539** |
| | **IMMU-132 (100 μg)** | **Up to therapy Day 70** | **0.311 ± 0.196 vs. 0.634 ± 0.335** | **0.0328** |
| | **Paclitaxel** | **Up to therapy Day 49** | **0.211 ± 0.155 vs. 0.705 ± 0.206** | **<0.0001** |
| | **Untreated** | **Up to therapy Day 49** | **0.211 ± 0.155 vs. 0.663 ± 0.349** | **0.0001** |

In the rapidly-progressing HCC1806 xenografts (**FIG. 2A-2B**), the combination of IMMU-132 plus paclitaxel proved to have a superior anti-tumor effect when compared to IMMU-132 monotherapy ($P$=0.0195, $AUC_{17days}$). This is a very aggressive tumor with a median survival time (MST) of only 10 days post-therapy initiation for the untreated control animals (18 days post-tumor cell inoculation). In terms of survival, the combination, which reached its MST of 38 days, provided a significant survival benefit when compared to all other therapies ($P$<0.017; log-rank). It should be noted that this was achieved at a low dose of only 0.25 mg which would be the human equivalent dose of only 1 mg/kg.

Mice treated with the combination of IMMU-132 plus eribulin mesylate (FIG. 3A-3E) exhibited a significantly greater anti-tumor response than all other monotherapy groups ($P$<0.0432; paired *t*-test). This resulted in a significant survival benefit for the combination (MST=23 days) when compared to eribulin or IMMU-132 monotherapy (MST=18 and 14 days, respectively; $P$<0.0044; log-rank).

Likewise, combining IMMU-132 therapy with olaparib was superior to single agent therapy in mice bearing MDA-MB-468 tumors (P<0.0032; AUC) (**FIG. 4**). Results are summarized in **Table 9.** All the IMMU-132 combination treatments were well-tolerated.

| Table 9. Area under the curve comparisons between IMMU-132 *plus* Olaparib treated MDA-MB-468 tumor-bearing mice and all other treatment groups. | | | | |
|---|---|---|---|---|
| **Treatments** | | **Time of Comparison** | **Tumor Volumes ($cm^3$) on that day (mean ± s.d.)** | ***P*-Value (AUC)** |
| **IMMU-132 *plus* Olaparib *versus*** | **IMMU-132 Alone** | **Up to therapy Day 49** | **0.030 ± 0.038 vs. 0.088 ± 0.069** | **0.0023** |
| | **hA20-SN-38 *plus* Olaparib** | **Up to therapy Day 49** | **0.030 ± 0.038 vs. 0.652 ± 0.306** | **<0.0001** |
| | **hA20-SN-38 Alone** | **Up to therapy Day 42** | **0.045 ± 0.045 vs. 0.654 ± 0.285** | **0.0002** |
| | **Olaparib Alone** | **Up to therapy Day 28** | **0.083 ± 0.050 vs. 0.649 ± 0.267** | **<0.0001** |
| | **Saline** | **Up to therapy Day 28** | **0.083 ± 0.050 vs. 0.697 ± 0.352** | **<0.0001** |

Drug-antibody ratio (DAR) determination. Five clinical lots of IMMU-132 were evaluated by hydrophobic interaction HPLC (HIC-HPLC), which resolved three peaks representing species with DARs of 6, 7 and 8, with the greatest fraction comprising a DAR=8 (not shown). IMMU-132 was produced consistently by this manufacturing process, with an overall DAR ($PAR_{AVE}$) of 7.60 ± 0.03 among the five clinical lots. HIC-HPLC results were confirmed by liquid chromatography-mass spectrometry (LC-MS). The analysis showed that >99% of the 8 available sulfhydryl groups were coupled with the CL2A linker, either with or without SN-38 (not shown). There were no unsubstituted (or N-ethylmaleimide capped) heavy or light chains detected. Thus, the difference in DAR among the species results from SN-38 liberation from the linker during manufacturing and not from a lower initial substitution ratio. Once prepared and lyophilized, IMMU-132 has been stable for several years.

Effect of DAR on pharmacokinetics and anti-tumor efficacy in mice. Mice bearing Trop-$2^+$ human gastric carcinoma xenografts (NCI-N87) were given 2 treatments 7 days apart, each with equal protein (0.5 mg) doses of IMMU-132 having DARs of 6.89, 3.28, or 1.64. Animals treated with the ADCs having a DAR of 6.89 had a significantly improved median survival time (MST) compared to mice given ADCs with either 3.38 or 1.64 DARs (MST=39 days vs. 25 and 21 days, respectively; *P*<0.0014) (not shown). There was no difference between groups treated with the 3.28 or 1.64 DAR conjugates and the saline control group.

To further elucidate the importance of a higher DAR, mice bearing NCI-N87 gastric tumors were administered 0.5 mg IMMU-132 with a DAR of 6.89 twice weekly for two weeks (not shown). Another group received twice the protein (1 mg) dose of an IMMU-132 conjugate with a DAR of 3.28. Although both groups received the same total amount of SN-38 (36 μg) with each dosing scheme, those treated with the 6.89 DAR conjugate inhibited tumor growth significantly more than tumor-bearing animals treated with the 3.28 DAR conjugate (*P*=0.0227; AUC) (not shown). Additionally, treatment with the lower DAR was not significantly different than the untreated controls. Collectively, these studies indicate that a lower DAR reduces efficacy.

An examination of the pharmacokinetic behavior of conjugates prepared at these different ratios was performed in non-tumor-bearing mice given 0.2 mg of each conjugate, unconjugated hRS7 IgG, or hRS7 IgG that was reduced and then capped with N-ethylmaleimide. Serum was taken at 5 intervals from 0.5 to 168 h and assayed by ELISA for hRS7 IgG. There was no significant difference in the clearance of these conjugates compared to the unconjugated IgG (not shown). Thus, the substitution level did not affect the pharmacokinetics of the conjugates, and equally important, the reduction of the interchain disulfide bonds did not appear to destabilize the antibody.

Mechanism of action of IMMU-132 in TNBC. The apoptotic pathway utilized by IMMU-132 was examined in the TNBC cell line, MDA-MB-468, and in the HER$2^+$ SK-BR-3 cell line, in order to confirm that the ADC functions on the basis of its incorporated SN-38. Cells were exposed to 1 μM SN-38, the SN-38-equivalent of IMMU-132, or protein equivalent of hRS7. Cells were harvested and Western blots were performed. SN-38 alone and IMMU-132 mediated >2-fold up-regulation of $p21^{WAF1/Cip1}$ within 24 h in MDA-MB-468, and by 48 h, the amount of $p21^{WAF1/Cip1}$ in these cells began to decrease (31% and 43% with SN-38 or IMMU-132, respectively) (not shown). Interestingly, in the HER$2^+$ SK-BR-3 tumor line, neither SN-38 nor IMMU-132 mediated the up-regulation of $p21^{WAF1/Cip1}$ above constitutive levels in the first 24 h, but as seen in MDA-MB-468 cells after 48-h exposure to SN-38 or IMMU-132, the amount of $p21^{WAF1/Cip1}$ decreased >57% (not shown). Both SN-38 and IMMU-132 resulted in cleavage of pro-caspase-3 into its active fragments within 24 h, but with the greater degree of active fragments observed after exposure for 48 h. Of note, in both cell lines, IMMU-132 mediated a greater degree of pro-caspase-3 cleavage, with the highest level observed after 48 h when compared to cells exposed to SN-38 (not shown). Finally, SN-38 and IMMU-132 both mediated poly ADP ribose polymerase (PARP) cleavage, starting at 24 h, with near complete cleavage after 48 h (not shown). Taken together, these results confirm that IMMU-132 has a mechanism of action similar to that of free SN-38 when administered *in vitro.*

Delivery of SN-38 by IMMU-132 vs. irinotecan in a human tumor xenograft model. Constitutive products derived from irinotecan or IMMU-132 were determined in the serum and tumors of mice implanted s.c. with a human pancreatic cancer xenograft (Capan-1) administered irinotecan (773 μg; SN-38 equivalents = 448 μg) and IMMU-132 (1.0 mg; SN-38 equivalents = 16 μg). Following administration, at 5 intervals 3 animals from each group were euthanized with serum extracted for the products of interest.

Irinotecan cleared very rapidly from serum, with conversion to SN-38 and SN-38G seen within 5 min (not shown). None of the products was detected at 24 h. The AUCs over a 6-h period were 21.0, 2.5, and 2.8 μg/mL·h for irinotecan, SN-38, and SN-38G, respectively (SN-38 conversion in mice = [2.5 + 2.8)/21 = 25.2%]). Animals given IMMU-132 had much lower concentrations of free SN-38 in the serum, but it was detected through 48 h (not shown). Free SN-38G was detected only at 1 and 6 h, and was 3- to 7-times lower than free SN-38 (not shown).

In the Capan-1 tumors excised from irinotecan-treated animals, irinotecan levels were high over 6 h, but undetectable a 24 h ($AUC_{5min\text{-}6\,h}$ = 48.4 μg/g·h). SN-38 was much lower and detected only through 2 h (i.e., $AUC_{5min\text{-}2\,h}$ = 0.4 μg/g·h), with SN-38G values almost 3-fold higher (AUC = 1.1 μg/g·h) (not shown). Tumors taken from animals given IMMU-132 did not have any detectable free SN-38 or SN-38G, but instead, all SN-38 in the tumor was bound to IMMU-132. Importantly, since no SN-38G was detected in the tumors, this suggests SN-38 bound to IMMU-132 was not glucuronidated. The AUC for SN-38 bound to IMMU-132 in these tumors was 54.3 μg/g·h, which is 135-fold higher than the amount of SN-38 in the tumors of animals treated with irinotecan over the 2-h period that SN-38 could be detected, even though mice given irinotecan received 28-fold more SN-38 equivalents than administered with IMMU-132 (i.e., 448 vs 16 μg SN-38 equivalents, respectively)

*Conclusions*

IMMU-132 is a humanized anti-Trop-2 antibody conjugated with 7.6 molecules of SN-38, the active metabolite of irinotecan, a topoisomerase I inhibitor. Clinically, IMMU-132 has shown manageable toxicity and encouraging responses in patients with relapsed/refractory TNBC (ClinicalTrials.gov, NCT01631552). IMMU-132 therapy alone demonstrated significant anti-tumor effects in human TNBC xenografts at a human equivalent dose that is 5-fold less than that being used clinically (i.e., 10 mg/kg). Since preclinical studies indicate IMMU-132 can be combined with two different microtubule-inhibitors or a PARP-inhibitor with significantly enhanced anti-tumor activity, these data support the use of IMMU-132 and other antibody-drug conjugates (ADCs) that cause DNA breaks, in combination with microtubule inhibitors and/or PARP inhibitors in general, as well as other chemotherapeutic agents that target cell division through microtubule inhibition or DNA-repair mechanisms. A preferred ADC class is represented by anti-Trop-2 antibody conjugates in patients with Trop-2 positive cancers, including but not limited to TNBC, metastatic colon cancer, SCLC and NSCLC, since this is a target that is expressed in high amounts in a large number of cancers, and is localized on the cell surface and cytoplasmically in the cancer cells. However, other cancer antigen targets can also be used for the ADC in this application if they bear a drug that also causes DNA breaks, such as via targeting topoisomerase I, like SN-38.

Synergy was achieved when IMMU-132 was combined with PARP-inhibitors (e.g., olaparib) in TNBC tumor lines that had BRCA1/2 defects, as well as wild-type expression, including one with only a PTEN defect. This suggests that IMMU-132 may synergize with any tumor that has any kind of disruption in DNA homologous recombination pathways. Combined with olaparib, IMMU-132 therapy achieved significant anti-tumor effects above that observed with monotherapy with each, resulting in a significant survival benefit. IMMU-132 combined with microtubule inhibitors, (e.g., paclitaxel or eribulin mesylate) also enhanced efficacy significantly compared to monotherapy with each agent.

Overall, these data evidence the unexpected significant advantage of combination therapy with an antibody-drug conjugate (ADC) that targets cancer cells and induces DNA strand breaks, such as IMMU-132, and microtubule inhibitors or PARP inhibitors. Targeting the PARP DNA repair pathway in *BRCA1/2* mutant TNBC tumors by combining IMMU-132 therapy with either paclitaxel or olaparib achieved synthetic lethality in this disease model with no observable toxicity. In an exemplary embodiment, the combination of IMMU-132 and a PARP or microtubule inhibitor is of use to treat Trop-2 positive cancers, such as TNBC. These data provide the rationale for use of IMMU-132 in combination with other chemotherapeutics that likewise target DNA-repair mechanisms in patients with TNBC or similar tumors.

**Example 2. Combination therapy with IMMU-114 (anti-HLA-DR) plus Bruton kinase inhibitor in chronic lymphocytic leukemia (CLL)**

*Experimental Design.*

Human chronic B cell leukemia cells (JVM-3) were plated in a 96-well plate. Cells were then incubated with various doses of the Bruton kinase inhibitor ibrutinib ($1\times10^{-5}$ to $3.9\times10^{-9}$M) plus a constant amount of IMMU-114 (0.25, 0.5, 0.75, or 1 nM) for 96 h. Cell viability was assessed by MTS assay and dose/response curves were generated based on percent growth inhibition relative to untreated control cells. $IC_{50}$-values were determined using Prism Graph-Pad. Data were normalized and isobolograms generated to demonstrate the effect of IMMU-114 on ibrutinib in JVM-3 cells.

*Results*

An example of how IMMU-114 shifted the $IC_{50}$ of ibrutinib is shown in **FIG. 5A**. Cells incubated with ibrutinib alone resulted in an $IC_{50}$ of $2.2\times10^{-6}$ M. However, when 0.5 nM of IMMU-114 is combined with ibrutinib, the $IC_{50}$-value drops 2-fold to $1.08\times10^{-6}$M. It should be noted that IMMU-114 at 0.5 nM only results in an approximate 22% inhibition of growth. Likewise, 0.75 nM IMMU-114 shifted ibrutinib $IC_{50}$-value downward 5.7-fold.

These data, along with the results from two other assays, were normalized to produce an isobologram in order to ascertain whether this interaction was synergistic, additive, or antagonistic (**FIG. 6A**). As shown, when the JVM-3 cells were incubated with ibrutinib combined with IMMU-114, an additive effect is evident. These data demonstrate that while each agent alone is capable of inhibiting the growth of CLL in vitro, when combined an additive effect is achieved which supports combining IMMU-114 and Bruton kinase therapies in patients with CLL.

**Example 3. Combination therapy with IMMU-114 (anti-HLA-DR) plus phosphatidylinositide 3-kinase (PI3K) inhibitor in chronic lymphocytic leukemia (CLL)**

*Experimental Design.*

Human chronic leukemia B cells (JVM-3) were plated in a 96-well plate. Cells were then incubated with various doses of a PI3K inhibitor idelalisib ($1\times10^{-5}$ to $3.9\times10^{-9}$M) plus a constant amount of IMMU-114 (0.25, 0.5, 0.75, or 1 nM) for 96 h. Cell viability was assessed by MTS assay and dose/response curves were generated based on percent growth inhibition relative to untreated control cells. $IC_{50}$-values were determined using Prism Graph-Pad. Data were normalized and isobolograms generated to demonstrate the effect of IMMU-114 on ibrutinib in JVM-3 cells.

*Results*

As with ibrutinib, IMMU-114 shifted the $IC_{50}$ of idelalisib in JVM-3 cells (**FIG. 5B**). Cells incubated with idelalisib alone resulted in an $IC_{50}$ of $1.51\times10^{-6}$M. However, when 0.5 nM of IMMU-114 is combined with idelalisib, the $IC_{50}$-value drops 2-fold to $0.77\times10^{-6}$M (IMMU-114 at 0.5 nM only results in an approximate 26% inhibition of growth). Likewise, 1 nM IMMU-114 shifted ibrutinib $IC_{50}$-value downward >18-fold.

These data, along with the results from one other assay, were normalized to produce an isobologram (**FIG. 6B**). As shown, an additive effect was achieved when JVM-3 cells were incubated with idelalisib combined with IMMU-114. These data demonstrate the utility of combining IMMU-114 with a PI3K inhibitor to treat CLL clinically.

**Example 4. Treatment of relapsed chronic lymphocytic leukemia with IMMU-114 and ibrutinib**

A 65-year-old woman with a history of CLL, as defined by the International Workshop on Chronic Lymphocytic Leukemia and World Health Organization classifications, presents with relapsed disease after prior therapies with fludarabine, dexamethasone, and rituximab, as well as a regimen of CVP. She now has fever and night sweats associated with generalized lymph node enlargement, a reduced hemoglobin and platelet production, as well as a rapidly rising leukocyte count. Her LDH is elevated and the beta-2-microglobulin is almost twice normal. The patient is given therapy with anti-HLA-DR IMMU-114 (IgG4 hL243) at 200 mg administered subcutaneously twice weekly. The antibody is given in combination with the Bruton kinase inhibitor ibrutinib, administered orally at a dosage of 420 mg daily. After 6 weeks, evaluation of the patient's hematological and lab values indicate that she has shown a partial response to the combination therapy.

**Example 5. Treatment of relapsed/refractory non-Hodgkin's lymphoma (NHL) with IMMU-114 and idelalisib**

Seventeen patients with follicular NHL who have not had a response to rituximab and an alkylating agent, or have had a relapse within 6 months after receipt of those therapies receive 200 mg IMMU-114, injected s.c. twice weekly, in combination with the PI3K inhibitor idelalisib, administered orally at 150 mg twice daily, until the disease progresses or the patient withdraws from the study. Responses are assessed by CT scans, with other evaluations including adverse events, B-cell blood levels, serum IMMU-114 levels and human anti-IMMU-114 (HAHA) titers.

Only occasional, mild to moderate transient injection reactions are seen and no other safety issues except neutropenia up to Grade 3 (but reversible after interrupting therapy until reduced to Grade 1) are observed. Transient B-cell depletion (up to about 25%) is observed. The objective response rate (partial responses plus complete responses plus complete responses unconfirmed) is 47% (8/17) with a complete response/complete response unconfirmed rate of 24% (4/17). Four of the eight objective responses continue for 30 weeks or more. All serum samples evaluated for human anti-IMMU-114 antibody (HAHA) are negative.

**Example 6. Conjugation of bifunctional SN-38 products to mildly reduced antibodies**

The anti-CEACAM5 humanized MAb, hMN-14 (also known as labetuzumab), the anti-CD22 humanized MAb, hLL2 (also known as epratuzumab), the anti-CD20 humanized MAb, hA20 (also known as veltuzumab), the anti-EGP-1 humanized MAb, hRS7, and anti-mucin humanized MAb, hPAM4 (also known as clivatuzumab), were used in these studies. Each antibody was reduced with dithiothreitol (DTT), used in a 50-to-70-fold molar excess, in 40 mM PBS, pH 7.4, containing 5.4 mM EDTA, at 37 °C (bath) for 45 min. The reduced product was purified by size-exclusion chromatography and/or diafiltration, and was buffer-exchanged into a suitable buffer at pH 6.5. The thiol content was determined by Ellman's assay, and was in the 6.5-to-8.5 SH/IgG range. Alternatively, the antibodies were reduced with Tris (2-carboxyethyl) phosphine (TCEP) in phosphate buffer at pH in the range of 5-7, followed by *in situ* conjugation. The reduced MAb was reacted with ~ 10-to-15-fold molar excess of CL2A-SN-38 (prepared as disclosed in U.S. Patent No. 7,999,083) using DMSO at 7-15 % v/v as co-solvent, and incubating for 20 min at ambient temperature. The conjugate was purified by centrifuged SEC, passage through a hydrophobic column, and finally by ultrafiltration-diafiltration. The product was assayed for SN-38 by absorbance at 366 nm and correlating with standard values, while the protein concentration was deduced from absorbance at 280 nm, corrected for spillover of SN-38 absorbance at this wavelength. This way, the SN-38/MAb substitution ratios were determined. The purified conjugates were stored as lyophilized formulations in glass vials, capped under vacuum and stored in a -20 °C freezer. SN-38 molar substitution ratios (MSR) obtained for some of these conjugates, which were typically in the 5-to-7 range, are shown in **Table 10**. The person of ordinary skill will realize that the conjugation method may be applied to any antibody of use in the disclosed methods.

**Table 10: SN-38/MAb Molar substitution ratios (MSR) in some conjugates**

| MAb | Conjugate | MSR |
|---|---|---|
| hMN-14 | hMN-14-CL2A-SN-38 | 6.1 |
| hRS7 | hRS7-CL2A-SN-38 | 5.8 |
| hA20 | hA20-CL2A-SN-38 | 5.8 |
| hLL2 | hLL2-CL2A-SN-38 | 5.7 |
| hPAM4 | hPAM4-CL2A-SN-38 | 5.9 |

**Example 7. *In vivo* therapeutic efficacies in preclinical models of human pancreatic or colon carcinoma**

Immune-compromised athymic nude mice (female), bearing subcutaneous human pancreatic or colon tumor xenografts were treated with either specific CL2A-SN-38 conjugate or control conjugate or were left untreated. The therapeutic efficacies of the specific conjugates were observed. **FIG. 7** shows a Capan 1 pancreatic tumor model, wherein specific CL2A-SN-38 conjugates of hRS7 (anti-EGP-1), hPAM4 (anti-mucin), and hMN-14 (anti-CEACAM5) antibodies showed better efficacies than control hA20-CL2A-SN-38 conjugate (anti-CD20) and untreated control. Similarly in a BXPC3 model of human pancreatic cancer, the specific hRS7-CL2A-SN-38 showed better therapeutic efficacy than control treatments (**FIG. 8**). Likewise, in an aggressive LS174T model of human colon carcinoma, treatment with specific hMN-14-CL2A-SN-38 was more efficacious than non-treatment (**FIG. 9**).

**Example 8. *In vivo* therapy of lung metastases of GW-39 human colonic tumors in nude mice using hMN-14-[CL1-SN-38] and hMN-14-[CL2-SN-38]**

A lung metastatic model of colonic carcinoma was established in nude mice by i.v. injection of GW-39 human colonic tumor suspension, and therapy was initiated 14 days later. Specific anti-CEACAM5 antibody conjugates, hMN14-CL1-SN-38 and hMN14-CL2-SN-38, as well as nontargeting anti-CD22 MAb control conjugates, hLL2-CL1-SN-38 and hLL2-CL2-SN-38 and equidose mixtures of hMN14 and SN-38 were injected at a dose schedule of q4d$\times$8, using different doses. **FIG. 10** (MSR = SN-38/antibody molar substitution ratio) shows selective therapeutic effects due to hMN-14 conjugates. At equivalent dosages of 250 $\mu$g, the mice treated with hMN14-CL1-SN-38 or hMN14-CL2-SN-38 showed a median survival of greater than 107 days. Mice treated with the control conjugated antibodies hLL2-CL1-SN-38 and hLL2-CL2-SN-38, which do not specifically target lung cancer cells, showed median survival of 56 and 77 days, while mice treated with unconjugated hMN14 IgG and free SN-38 showed a median survival of 45 days, comparable to the untreated saline control of 43.5 days. A significant and surprising increase in effectiveness of the conjugated, cancer cell targeted antibody-SN-38 conjugate, which was substantially more effective than unconjugated antibody and free chemotherapeutic agent alone, was clearly seen. The dose-responsiveness of therapeutic effect of conjugated antibody was also observed. These results demonstrate the clear superiority of the SN-38-antibody conjugates compared to the combined effect of both unconjugated antibody and free SN-38 in the same *in vivo* human lung cancer system.

**Example 9. Use of Humanized Anti-Trop-2 IgG-SN-38 Conjugate for Effective Treatment of Diverse Epithelial Cancers**

*Summary*

The purpose of this study was to evaluate the efficacy of an SN-38-anti-Trop-2 antibody-drug conjugate (ADC) against several human solid tumor types, and to assess its tolerability in mice and monkeys, the latter with tissue cross-reactivity to hRS7 similar to humans. Two SN-38 derivatives, CL2-SN-38 and CL2A-SN-38, were conjugated to the anti-Trop-2-humanized antibody, hRS7. The immunoconjugates were characterized *in vitro* for stability, binding, and cytotoxicity. Efficacy was tested in five different human solid tumor-xenograft models that expressed Trop-2 antigen. Toxicity was assessed in mice and in Cynomolgus monkeys.

The hRS7 conjugates of the two SN-38 derivatives were equivalent in drug substitution (~6), cell binding ($K_d$ ~ 1.2 nmol/L), cytotoxicity ($IC_{50}$ ~ 2.2 nmol/L), and serum stability *in vitro* ($t/_{1/2}$ ~ 20 hours). Exposure of cells to the ADC demonstrated signaling pathways leading to PARP cleavage, but differences *versus* free SN-38 in p53 and p21 upregulation were noted. Significant antitumor effects were produced by hRS7-SN-38 at nontoxic doses in mice bearing Calu-3 ($P \leq 0.05$), Capan-1 ($P < 0.018$), BxPC-3 ($P < 0.005$), and COLO 205 tumors ($P < 0.033$) when compared to nontargeting control ADCs. Mice tolerated a dose of 2 $\times$ 12 mg/kg (SN-38 equivalents) with only short-lived elevations in ALT and

AST liver enzyme levels. Cynomolgus monkeys infused with 2 × 0.96 mg/kg exhibited only transient decreases in blood counts, although, importantly, the values did not fall below normal ranges.

We conclude that the anti-Trop-2 hRS7-CL2A-SN-38 ADC provided significant and specific antitumor effects against a range of human solid tumor types. It was well tolerated in monkeys, with tissue Trop-2 expression similar to humans. (Cardillo et al., 2011, Clin Cancer Res 17:3157-69.)

Successful irinotecan treatment of patients with solid tumors has been limited due in large part to the low conversion rate of the CPT-11 prodrug into the active SN-38 metabolite. Others have examined nontargeted forms of SN-38 as a means to bypass the need for this conversion and to deliver SN-38 passively to tumors. We conjugated SN-38 covalently to a humanized anti-Trop-2 antibody, hRS7. This antibody-drug conjugate has specific antitumor effects in a range of s.c. human cancer xenograft models, including non-small cell lung carcinoma, pancreatic, colorectal, and squamous cell lung carcinomas, all at nontoxic doses (e.g., ≤3.2 mg/kg cumulative SN-38 equivalent dose).

Trop-2 is widely expressed in many epithelial cancers, but also some normal tissues, and therefore a dose escalation study in Cynomolgus monkeys was performed to assess the clinical safety of this conjugate. Monkeys tolerated 24 mg SN-38 equivalents/kg with only minor, reversible, toxicities. Given its tumor-targeting and safety profile, hRS7-SN-38 may provide an improvement in the management of solid tumors responsive to irinotecan.

*Introduction*

Human trophoblast cell-surface antigen (Trop-2), also known as GA733-1 (gastric antigen 733-1), EGP-1 (epithelial glycoprotein-1), and TACSTD2 (tumor-associated calcium signal transducer), is expressed in a variety of human carcinomas and has prognostic significance in some, being associated with more aggressive disease (see, e.g., Alberti et al., 1992, Hybridoma 11:539-45; Stein et al., 1993, Int J Cancer 55:938-46; Stein et al., 1994, Int J Cancer Suppl. 8:98-102). Studies of the functional role of Trop-2 in a mouse pancreatic cancer cell line transfected with murine Trop-2 revealed increased proliferation in low serum conditions, migration, and anchorage-independent growth *in vitro,* and enhanced growth rate with evidence of increased Ki-67 expression *in vivo* and a higher likelihood to metastasize (Cubas et al., 2010, Mol Cancer 9:253).

Trop-2 antigen's distribution in many epithelial cancers makes it an attractive therapeutic target. Stein and colleagues (1993, Int J Cancer 55:938-46) characterized an antibody, designated RS7-3G11 (RS7), that bound to EGP-1, which was present in a number of solid tumors, but the antigen was also expressed in some normal tissues, usually in a lower intensity, or in restricted regions. Targeting and therapeutic efficacies were documented in a number of human tumor xenografts using radiolabeled RS7 (Shih et al., 1995, Cancer Res 55:5857s-63s; Stein et al., 1997, Cancer 80:2636-41; Govindan et al., 2004, Breast Cancer Res Treat 84: 173-82), but this internalizing antibody did not show therapeutic activity in unconjugated form (Shih et al., 1995, Cancer Res 55:5857s-63s). However, *in vitro* it has demonstrated antibody-dependent cellular cytotoxicity (ADCC) activity against Trop-2 positive carcinomas.

We reported the preparation of antibody-drug conjugates (ADC) using an anti-CEACAM5 (CD66e) IgG coupled to several derivatives of SN-38, a topoisomerase-I inhibitor that is the active component of irinotecan, or CPT-11 (Moon et al., 2008, J Med Chem 51:6916-26; Govindan et al., 2009, Clin Cancer Res 15:6052-61). The derivatives varied in their *in vitro* serum stability properties, and *in vivo* studies found one form (designated CL2) to be more effective in preventing or arresting the growth of human colonic and pancreatic cancer xenografts than other linkages with more or less stability.

Importantly, these effects occurred at nontoxic doses, with initial testing failing to determine a dose-limiting toxicity (Govindan et al., 2009, Clin Cancer Res 15:6052-61). These results were encouraging, but also surprising, because the CEACAM5 antibody does not internalize, a property thought to be critical to the success of an ADC. We speculated that the therapeutic activity of the anti-CEACAM5-SN-38 conjugate might be related to the slow release of SN-38 within the tumor after the antibody localized. Because irinotecan performs best when cells are exposed during the S-phase of their growth cycle, a sustained release is expected to improve responses. Indeed, SN-38 coupled to nontargeting, plasma extending agents, such as polyethylene glycol (PEG) or micelles, has shown improved efficacy over irinotecan or SN-38 alone (e.g., Koizumi et al., 2006, Cancer Res 66:10048-56), lending additional support to this mechanism.

Given the RS7 antibody's broad reactivity with epithelial cancers and its internalization ability, we hypothesized that an RS7-SN-38 conjugate could benefit not only from the sustained release of the drug, but also from direct intracellular delivery. Therefore, we prepared and tested the efficacy of SN-38 conjugates using a humanized version of the murine RS7 antibody (hRS7), wherein the SN-38 was attached to the antibody using an improved CL2A linker, which improved the quality of the conjugate and its *in vivo* efficacy without altering its *in vitro* stability. This new derivative (designated CL2A) is a preferred agent for SN-38 coupling to antibodies.

Herein, we show the efficacy of the hRS7-SN-38 conjugate in several epithelial cancer cell lines implanted in nude mice at nontoxic dosages, with other studies revealing that substantially higher doses could be tolerated. More importantly, toxicity studies in monkeys that also express Trop-2 in similar tissues as humans showed that hRS7-SN-38 was tolerated at appreciably higher amounts than the therapeutically effective dose in mice.

*Materials and Methods*

Cell lines, antibodies, and chemotherapeutics. All human cancer cell lines used in this study were purchased from the American Type Culture Collection. These include Calu-3 (non-small cell lung carcinoma), SK-MES-1 (squamous cell lung carcinoma), COLO 205 (colonic adenocarcinoma), Capan-1 and BxPC-3 (pancreatic adenocarcinomas), and PC-3 (prostatic adenocarcinomas). Humanized RS7 IgG and control humanized anti-CD20 (hA20 IgG, veltuzumab) and anti-CD22 (hLL2 IgG, epratuzumab) antibodies were prepared at Immunomedics, Inc. Irinotecan (20 mg/mL) was obtained from Hospira, Inc.

SN-38 immunoconjugates and *in vitro* aspects. Synthesis of CL2-SN-38 has been described previously (Moon et al., 2008, J Med Chem 51:6916-26). Its conjugation to hRS7 IgG and serum stability were performed as described (Moon et al., 2008, J Med Chem 51:6916-26; Govindan et al., 2009, Clin Cancer Res 15:6052-61). Preparations of CL2A-SN-38 (M.W. 1480) and its hRS7 conjugate, and stability, binding, and cytotoxicity studies, were conducted as described previously (Moon et al., 2008, J Med Chem 51:6916-26). Cell lysates were prepared and immunoblotting for $p21^{Waf1/Cip}$, p53, and PARP (poly-ADP-ribose polymerase) was performed.

*In vivo* therapeutic studies. For all animal studies, the doses of SN-38 immunoconjugates and irinotecan are shown in SN-38 equivalents. Based on a mean SN-38/IgG substitution ratio of 6, a dose of 500 μg ADC to a 20-g mouse (25 mg/kg) contains 0.4 mg/kg of SN-38. Irinotecan doses are likewise shown as SN-38 equivalents (i.e., 40 mg irinotecan/kg is equivalent to 24 mg/kg of SN-38). NCr female athymic nude (*nu*/*nu*) mice, 4 to 8 weeks old, and male Swiss-Webster mice, 10 weeks old, were purchased from Taconic Farms. Tolerability studies were performed in Cynomolgus monkeys (*Macaca fascicularis*; 2.5-4 kg male and female) by SNBL USA, Ltd. Animals were implanted subcutaneously with different human cancer cell lines. Tumor volume (TV) was determined by measurements in 2 dimensions using calipers, with volumes defined as: $L \times w^2/2$, where L is the longest dimension of the tumor and w is the shortest. Tumors ranged in size between 0.10 and 0.47 $cm^3$ when therapy began. Treatment regimens, dosages, and number of animals in each experiment are described in the Results. The lyophilized hRS7-CL2A-SN-38 and control ADC were reconstituted and diluted as required in sterile saline. All reagents were administered intraperitoneally (0.1 mL), except irinotecan, which was administered intravenously. The dosing regimen was influenced by our prior investigations, where the ADC was given every 4 days or twice weekly for varying lengths of time (Moon et al., 2008, J Med Chem 51:6916-26; Govindan et al., 2009, Clin Cancer Res 15:6052-61). This dosing frequency reflected a consideration of the conjugate's serum half-life *in vitro,* to allow a more continuous exposure to the ADC.

Statistics. Growth curves were determined as percent change in initial TV over time. Statistical analysis of tumor growth was based on area under the curve (AUC). Profiles of individual tumor growth were obtained through linear-curve modeling. An *f*-test was employed to determine equality of variance between groups before statistical analysis of growth curves. A 2-tailed *t*-test was used to assess statistical significance between the various treatment groups and controls, except for the saline control, where a 1-tailed *t*-test was used (significance at $P \leq 0.05$). Statistical comparisons of AUC were performed only up to the time that the first animal within a group was euthanized due to progression.

Pharmacokinetics and biodistribution. $^{111}$In-radiolabeled hRS7-CL2A-SN-38 and hRS7 IgG were injected into nude mice bearing s.c. SK-MES-1 tumors (~0.3 $cm^3$). One group was injected intravenously with 20 μCi (250-μg protein) of $^{111}$In-hRS7-CL2A-SN-38, whereas another group received 20 μCi (250-μg protein) of $^{111}$In-hRS7 IgG. At various timepoints mice (5 per timepoint) were anesthetized, bled via intracardiac puncture, and then euthanized. Tumors and various tissues were removed, weighed, and counted by y scintillation to determine the percentage injected dose per gram tissue (% ID/g). A third group was injected with 250 μg of unlabeled hRS7-CL2A-SN-38 3 days before the administration of $^{111}$In-hRS7-CL2A-SN-38 and likewise necropsied. A 2-tailed *t*-test was used to compare hRS7-CL2A-SN-38 and hRS7 IgG uptake after determining equality of variance using the *f*-test. Pharmacokinetic analysis on blood clearance was performed using WinNonLin software (Parsight Corp.).

Tolerability in Swiss-Webster mice and Cynomolgus monkeys. Briefly, mice were sorted into 4 groups each to receive 2-mL i.p. injections of either a sodium acetate buffer control or 3 different doses of hRS7-CL2A-SN-38 (4, 8, or 12 mg/kg of SN-38) on days 0 and 3 followed by blood and serum collection, as described in Results. Cynomolgus monkeys (3 male and 3 female; 2.5-4.0 kg) were administered 2 different doses of hRS7-CL2A-SN-38. Dosages, times, and number of monkeys bled for evaluation of possible hematologic toxicities and serum chemistries are described in the Results.

*Results*

Stability and potency of hRS7-CL2A-SN-38. Two different linkages were used to conjugate SN-38 to hRS7 IgG. The first is termed CL2-SN-38 and has been described previously (Moon et al., 2008, J Med Chem 51:6916-26;

Govindan et al., 2009, Clin Cancer Res 15:6052-61). A change was made to the synthesis of the CL2 linker in that the phenylalanine moiety was removed. This change simplified the synthesis, but did not affect the conjugation outcome (e.g., both CL2-SN-38 and CL2A-SN-38 incorporated ~6 SN-38 per IgG molecule). Side-by-side comparisons found no significant differences in serum stability, antigen binding, or *in vitro* cytotoxicity (not shown).

To confirm that the change in the SN-38 linker from CL2 to CL2A did not impact *in vivo* potency, hRS7-CL2A and hRS7-CL2-SN-38 were compared in mice bearing COLO 205 or Capan-1 tumors (not shown), using 0.4 mg or 0.2 mg/kg SN-38 twice weekly × 4 weeks, respectively, and with starting tumors of 0.25 $cm^3$ size in both studies. Both the hRS7-CL2A and CL2-SN-38 conjugates significantly inhibited tumor growth compared to untreated ($AUC_{14days}$ *P* < 0.002 vs. saline in COLO 205 model; $AUC_{21days}$ *P* < 0.001 vs. saline in Capan-1 model), and a nontargeting anti-CD20 control ADC, hA20-CL2A-SN-38 ($AUC_{14days}$ *P* < 0.003 in COLO-205 model; $AUC_{35days}$: *P* < 0.002 in Capan-1 model). At the end of the study (day 140) in the Capan-1 model, 50% of the mice treated with hRS7-CL2A-SN-38 and 40% of the hRS7-CL2-SN-38 mice were tumor-free, whereas only 20% of the hA20-ADC-treated animals had no visible sign of disease.

Mechanism of action. *In vitro* cytotoxicity studies demonstrated that hRS7-CL2A-SN-38 had $IC_{50}$ values in the nmol/L range against several different solid tumor lines (**Table 11**). The $IC_{50}$ with free SN-38 was lower than the conjugate in all cell lines. Although there was no correlation between Trop-2 expression and sensitivity to hRS7-CL2A-SN-38, the $IC_{50}$ ratio of the ADC versus free SN-38 was lower in the higher Trop-2-expressing cells, most likely reflecting the enhanced ability to internalize the drug when more antigen is present.

**Table 11. Expression of Trop-2 and *in vitro* cytotoxicity of SN-38 and hRS7-SN-38 in sereval solid tumor lines**

| | Trop-2 expression *via* FACS | | Cytotoxicity results | | | | |
|---|---|---|---|---|---|---|---|
| Cell line | Median fluorescence (background) | Percent positive | SN-38 | 95% CI | hRS7-SN-38[a] | 95% CI | ADC/free SN-38 ratio |
| | | | $IC_{50}$ (nmol/L) | $IC_{50}$ (nmol/L) | $IC_{50}$ (nmol/L) | $IC_{50}$ (nmol/L) | |
| Calu-3 | 282.2 (4.7) | 99.6% | 7.19 | 5.77-8.95 | 9.97 | 8.12-12.25 | 1.39 |
| COLO 205 | 141.5 (4.5) | 99.5% | 1.02 | 0.66-1.57 | 1.95 | 1.26-3.01 | 1.91 |
| Capan-1 | 100.0 (5.0) | 94.2% | 3.50 | 2.17-5.65 | 6.99 | 5.02-9.72 | 2.00 |
| PC-3 | 1146.2 (5.5) | 73.6% | 1.86 | 1.16-2.99 4.24 | | 2.99-6.01 2.28 | |
| SK-MES-1 | 44.0 (3.5) | 91.2% | 8.61 | 6.30-11.76 | 23.14 | 17.98-29.78 | 2.69 |
| BxPC-3 | 26.4 (3.1) | 98.3% | 1.44 | 1.04-2.00 4.03 | | 3.25-4.98 2.80 | |

[a]$IC_{50}$-value is shown as SN-38 equivalents of hRS7-SN-38

SN-38 is known to activate several signaling pathways in cells, leading to apoptosis. Our initial studies examined the expression of 2 proteins involved in early signaling events ($p21^{Waf1/Cip1}$ and p53) and 1 late apoptotic event [cleavage of poly-ADP-ribose polymerase (PARP)] *in vitro* (not shown). In BxPC-3, SN-38 led to a 20-fold increase in $p21^{Waf1/Cip1}$ expression, whereas hRS7-CL2A-SN-38 resulted in only a 10-fold increase, a finding consistent with the higher activity with free SN-38 in this cell line (**Table 11**). However, hRS7-CL2A-SN-38 increased $p21^{Waf1/Cip1}$ expression in Calu-3 more than 2-fold over free SN-38 (not shown).

A greater disparity between hRS7-CL2A-SN-38- and free SN-38-mediated signaling events was observed in p53 expression. In both BxPC-3 and Calu-3, upregulation of p53 with free SN-38 was not evident until 48 hours, whereas hRS7-CL2A-SN-38 upregulated p53 within 24 hours (not shown). In addition, p53 expression in cells exposed to the ADC was higher in both cell lines compared to SN-38 (not shown). Interestingly, although hRS7 IgG had no appreciable effect on $p21^{Waf1/Cip1}$ expression, it did induce the upregulation of p53 in both BxPC-3 and Calu-3, but only after a 48-hour exposure. In terms of later apoptotic events, cleavage of PARP was evident in both cell lines when incubated with either SN-38 or the conjugate (not shown). The presence of the cleaved PARP was higher at 24 hours in BxPC-3, which correlates with high expression of p21 and its lower $IC_{50}$. The higher degree of cleavage with free SN-38 over the ADC

was consistent with the cytotoxicity findings.

Efficacy of hRS7-SN-38. Because Trop-2 is widely expressed in several human carcinomas, studies were performed in several different human cancer models, which started with an evaluation of the hRS7-CL2-SN-38 linkage, but later, conjugates with the CL2A-linkage were used. Calu-3-bearing nude mice given 0.04 mg SN-38/kg of the hRS7-CL2-SN-38 every 4 days × 4 had a significantly improved response compared to animals administered the equivalent amount of hLL2-CL2-SN-38 (TV = 0.14 ± 0.22 $cm^3$ vs. 0.80 ± 0.91 $cm^3$, respectively; $AUC_{42days}$ $P < 0.026$; **FIG. 11A**). A dose-response was observed when the dose was increased to 0.4 mg/kg SN-38. At this higher dose level, all mice given the specific hRS7 conjugate were "cured" within 28 days, and remained tumor-free until the end of the study on day 147, whereas tumors regrew in animals treated with the irrelevant ADC (specific vs. irrelevant $AUC_{98days}$: $P = 0.05$). In mice receiving the mixture of hRS7 IgG and SN-38, tumors progressed >4.5-fold by day 56 (TV = 1.10 ± 0.88 $cm^3$; $AUC_{56days}$ $P < 0.006$ vs. hRS7-CL2-SN-38).

Efficacy also was examined in human colonic (COLO 205) and pancreatic (Capan-1) tumor xenografts. In COLO 205 tumor-bearing animals, (**FIG. 11B**), hRS7-CL2-SN-38 (0.4 mg/kg, q4dx8) prevented tumor growth over the 28-day treatment period with significantly smaller tumors compared to control anti-CD20 ADC (hA20-CL2-SN-38), or hRS7 IgG (TV = 0.16 ± 0.09 $cm^3$, 1.19 ± 0.59 $cm^3$, and 1.77 ± 0.93 $cm^3$, respectively; $AUC_{28days}$ $P < 0.016$). The MTD of irinotecan (24 mg SN-38/kg, q2dx5) was as effective as hRS7-CL2-SN-38, because mouse serum can more efficiently convert irinotecan to SN-38 than human serum, but the SN-38 dose in irinotecan (2,400 μg cumulative) was 37.5-fold greater than with the conjugate (64 μg total).

Animals bearing Capan-1 showed no significant response to irinotecan alone when given at an SN-38-dose equivalent to the hRS7-CL2-SN-38 conjugate (e.g., on day 35, average tumor size was 0.04 ± 0.05 $cm^3$ in animals given 0.4 mg SN-38/kg hRS7-SN-38 vs. 1.78 ± 0.62 $cm^3$ in irinotecan-treated animals given 0.4 mg/kg SN-38; $AUC_{day35}$ $P < 0.001$; **FIG. 11C**). When the irinotecan dose was increased 10-fold to 4 mg/kg SN-38, the response improved, but still was not as significant as the conjugate at the 0.4 mg/kg SN-38 dose level (TV = 0.17 ± 0.18 $cm^3$ vs. 1.69 ± 0.47 $cm^3$, $AUC_{day49}$ $P < 0.001$). An equal dose of nontargeting hA20-CL2-SN-38 also had a significant antitumor effect as compared to irinotecan-treated animals, but the specific hRS7 conjugate was significantly better than the irrelevant ADC (TV = 0.17 ± 0.18 $cm^3$ vs. 0.80 ± 0.68 $cm^3$, $AUC_{day49}$ $P < 0.018$).

Studies with the hRS7-CL2A-SN-38 ADC were then extended to 2 other models of human epithelial cancers. In mice bearing BxPC-3 human pancreatic tumors (**FIG. 11D**), hRS7-CL2A-SN-38 again significantly inhibited tumor growth in comparison to control mice treated with saline or an equivalent amount of nontargeting hA20-CL2A-SN-38 (TV = 0.24 ± 0.11 $cm^3$ vs. 1.17 ± 0.45 $cm^3$ and 1.05 ± 0.73 $cm^3$, respectively; $AUC_{day21}$ $P < 0.001$), or irinotecan given at a 10-fold higher SN-38 equivalent dose (TV = 0.27 ± 0.18 $cm^3$ vs. 0.90 ± 0.62 $cm^3$, respectively; $AUC_{day25}$ $P < 0.004$). Interestingly, in mice bearing SK-MES-1 human squamous cell lung tumors treated with 0.4 mg/kg of the ADC (**FIG. 11E**), tumor growth inhibition was superior to saline or unconjugated hRS7 IgG (TV = 0.36 ± 0.25 $cm^3$ vs. 1.02 ± 0.70 $cm^3$ and 1.30 ± 1.08 $cm^3$, respectively; $AUC_{28days}$, $P < 0.043$), but nontargeting hA20-CL2A-SN-38 or the MTD of irinotecan provided the same antitumor effects as the specific hRS7-SN-38 conjugate. In all murine studies, the hRS7-SN-38 ADC was well tolerated in terms of body weight loss (not shown).

Biodistribution of hRS7-CL2A-SN-38. The biodistributions of hRS7-CL2A-SN-38 or unconjugated hRS7 IgG were compared in mice bearing SK-MES-1 human squamous cell lung carcinoma xenografts (not shown), using the respective $^{111}$In-labeled substrates. A pharmacokinetic analysis was performed to determine the clearance of hRS7-CL2A-SN-38 relative to unconjugated hRS7 (not shown). The ADC cleared faster than the equivalent amount of unconjugated hRS7, with the ADC exhibiting ~40% shorter half-life and mean residence time. Nonetheless, this had a minimal impact on tumor uptake (not shown). Although there were significant differences at the 24- and 48-hour timepoints, by 72 hours (peak uptake) the amounts of both agents in the tumor were similar. Among the normal tissues, hepatic and splenic differences were the most striking (not shown). At 24 hours postinjection, there was >2-fold more hRS7-CL2A-SN-38 in the liver than hRS7 IgG. Conversely, in the spleen there was 3-fold more parental hRS7 IgG present at peak uptake (48-hour timepoint) than hRS7-CL2A-SN-38. Uptake and clearance in the rest of the tissues generally reflected differences in the blood concentration.

Because twice-weekly doses were given for therapy, tumor uptake in a group of animals that first received a predose of 0.2 mg/kg (250 μg protein) of the hRS7 ADC 3 days before the injection of the $^{111}$In-labeled antibody was examined. Tumor uptake of $^{111}$In-hRS7-CL2A-SN-38 in predosed mice was substantially reduced at every timepoint in comparison to animals that did not receive the predose (e.g., at 72 hours, predosed tumor uptake was 12.5% ± 3.8% ID/g vs. 25.4% ± 8.1% ID/g in animals not given the predose; $P = 0.0123$). Predosing had no appreciable impact on blood clearance or tissue uptake (not shown). These studies suggest that in some tumor models, tumor accretion of the specific antibody can be reduced by the preceding dose(s), which likely explains why the specificity of a therapeutic response could be diminished with increasing ADC doses and why further dose escalation is not indicated.

Tolerability of hRS7-CL2A-SN-38 in Swiss-Webster mice and Cynomolgus monkeys. Swiss-Webster mice tolerated 2 doses over 3 days, each of 4, 8, and 12 mg SN-38/kg of the hRS7-CL2A-SN-38, with minimal transient weight loss (not shown). No hematopoietic toxicity occurred and serum chemistries only revealed elevated aspartate transaminase (AST) and alanine transaminase (not shown). Seven days after treatment, AST rose above normal levels (>298 U/L) in all 3 treatment groups (not shown), with the largest proportion of mice being in the 2 × 8 mg/kg group. However, by 15 days posttreatment, most animals were within the normal range. ALT levels were also above the normal range (>77 U/L) within 7 days of treatment (not shown) and with evidence of normalization by Day 15. Livers from all these mice did not show histologic evidence of tissue damage (not shown). In terms of renal function, only glucose and chloride levels were somewhat elevated in the treated groups. At 2 × 8 mg/kg, 5 of 7 mice had slightly elevated glucose levels (range of 273-320 mg/dL, upper end of normal 263 mg/dL) that returned to normal by 15 days postinjection. Similarly, chloride levels were slightly elevated, ranging from 116 to 127 mmol/L (upper end of normal range 115 mmol/L) in the 2 highest dosage groups (57% in the 2 × 8 mg/kg group and 100% of the mice in the 2 × 12 mg/kg group), and remained elevated out to 15 days postinjection. This also could be indicative of gastrointestinal toxicity, because most chloride is obtained through absorption by the gut; however, at termination, there was no histologic evidence of tissue damage in any organ system examined (not shown).

Because mice do not express Trop-2 bound by hRS7, a more suitable model was required to determine the potential of the hRS7 conjugate for clinical use. Immunohistology studies revealed binding in multiple tissues in both humans and Cynomolgus monkeys (breast, eye, gastrointestinal tract, kidney, lung, ovary, fallopian tube, pancreas, parathyroid, prostate, salivary gland, skin, thymus, thyroid, tonsil, ureter, urinary bladder, and uterus; not shown). Based on this cross-reactivity, a tolerability study was performed in monkeys.

The group receiving 2 × 0.96 mg SN-38/kg of hRS7-CL2A-SN-38 had no significant clinical events following the infusion and through the termination of the study. Weight loss did not exceed 7.3% and returned to acclimation weights by day 15. Transient decreases were noted in most of the blood count data (not shown), but values did not fall below normal ranges. No abnormal values were found in the serum chemistries. Histopathology of the animals necropsied on day 11 (8 days after last injection) showed microscopic changes in hematopoietic organs (thymus, mandibular and mesenteric lymph nodes, spleen, and bone marrow), gastrointestinal organs (stomach, duodenum, jejunum, ileum, cecum, colon, and rectum), female reproductive organs (ovary, uterus, and vagina), and at the injection site. These changes ranged from minimal to moderate and were fully reversed at the end of the recovery period (day 32) in all tissues, except in the thymus and gastrointestinal tract, which were trending towards full recovery at this later timepoint.

At the 2 × 1.92 mg SN-38/kg dose level of the conjugate, there was 1 death arising from gastrointestinal complications and bone marrow suppression, and other animals within this group showed similar, but more severe adverse events than the 2 × 0.96 mg/kg group. These data indicate that dose-limiting toxicities were identical to that of irinotecan; namely, intestinal and hematologic. Thus, the MTD for hRS7-CL2A-SN-38 lies between 2 × 0.96 and 1.92 mg SN-38/kg, which represents a human equivalent dose of 2 × 0.3 to 0.6 mg/kg SN-38.

*Discussion*

Trop-2 is a protein expressed on many epithelial tumors, including lung, breast, colorectal, pancreas, prostate, and ovarian cancers, making it a potentially important target for delivering cytotoxic agents. The RS7 antibody internalizes when bound to Trop-2 (Shih et al., 1995, Cancer Res 55:5857s-63s), which enables direct intracellular delivery of cytotoxics.

Conjugation of chemotherapeutic drugs to antibodies has been explored for over 30 years. Because a substantial portion of an ADC is not processed by the tumor, but by normal tissues, there is a risk that these agents will be too toxic to normal organ systems before reaching the therapeutic level in tumors. As with any therapeutic, the therapeutic window is a key factor determining the potential of an ADC, and thus rather than examining "ultratoxic" drugs, we chose SN-38 as the drug component of the Trop-2-targeted ADC.

SN-38 is a potent topoisomerase-I inhibitor, with $IC_{50}$ values in the nanomolar range in several cell lines. It is the active form of the prodrug, irinotecan, that is used for the treatment of colorectal cancer, and which also has activity in lung, breast, and brain cancers. We reasoned that a directly targeted SN-38, in the form of an ADC, would be a significantly improved therapeutic over CPT-11, by overcoming the latter's low and patient-variable bioconversion to active SN-38.

The Phe-Lys peptide inserted in the original CL2 derivative allowed for possible cleavage via cathepsin B. In an effort to simplify the synthetic process, in CL2A, phenylalanine was eliminated, and thus the cathepsin B cleavage site was removed. Interestingly, this product had a better-defined chromatographic profile compared to the broad profile obtained with CL2 (not shown), but more importantly, this change had no negative impact on the conjugate's binding, stability, or potency in side-by-side testing. These data suggest that SN-38 in CL2 was released from the conjugate primarily by the cleavage at the pH-sensitive benzyl carbonate bond to SN-38's lactone ring and not the cathepsin B cleavage site.

*In vitro* cytotoxicity of hRS7 ADC against a range of solid tumor cell lines consistently had $IC_{50}$ values in the nmol/L range. However, cells exposed to free SN-38 demonstrated a lower $IC_{50}$ value compared to the ADC. This disparity between free and conjugated SN-38 was also reported for ENZ-2208 (Sapra et al., 2008, Clin Cancer Res

14:1888-96) and NK012 (Koizumi et al., 2006, Cancer Res 66:10048-56). ENZ-2208 utilizes a branched PEG to link about 3.5 to 4 molecules of SN-38 per PEG, whereas NK012 is a micelle nanoparticle containing 20% SN-38 by weight. With our ADC, this disparity (i.e., ratio of potency with free vs. conjugated SN-38) decreased as the Trop-2 expression levels increased in the tumor cells, suggesting an advantage to targeted delivery of the drug. In terms of *in vitro* serum stability, both the CL2- and CL2A-SN-38 forms of hRS7-SN-38 yielded a $t/_{1/2}$ of ~20 hours, which is in contrast to the short $t/_{1/2}$ of 12.3 minutes reported for ENZ-2208 (Zhao et al., 2008, Bioconjug Chem 19:849-59), but similar to the 57% release of SN-38 from NK012 under physiological conditions after 24 hours (Koizumi et al., 2006, Cancer Res 66:10048-56).

Treatment of tumor-bearing mice with hRS7-SN-38 (either with CL2-SN-38 or CL2A-SN-38) significantly inhibited tumor growth in 5 different tumor models. In 4 of them, tumor regressions were observed, and in the case of Calu-3, all mice receiving the highest dose of hRS7-SN-38 were tumor-free at the conclusion of study. Unlike in humans, irinotecan is very efficiently converted to SN-38 by a plasma esterase in mice, with a greater than 50% conversion rate, and yielding higher efficacy in mice than in humans. When irinotecan was administered at 10-fold higher or equivalent SN-38 levels, hRS7-SN-38 was significantly better in controlling tumor growth. Only when irinotecan was administered at its MTD of 24 mg/kg q2dx5 (37.5-fold more SN-38) did it equal the effectiveness of hRS7-SN-38. In patients, we would expect this advantage to favor hRS7-CL2A-SN-38 even more, because the bioconversion of irinotecan would be substantially lower.

We also showed in some antigen-expressing cell lines, such as SK-MES-1, that using an antigen-binding ADC does not guarantee better therapeutic responses than a nonbinding, irrelevant conjugate. This is not an unusual or unexpected finding. Indeed, the nonbinding SN-38 conjugates mentioned earlier enhance therapeutic activity when compared to irinotecan, and so an irrelevant IgG-SN-38 conjugate is expected to have some activity. This is related to the fact that tumors have immature, leaky vessels that allow the passage of macromolecules better than normal tissues. With our conjugate, 50% of the SN-38 will be released in ~ 13 hours when the pH is lowered to a level mimicking lysosomal levels (e.g., pH 5.3 at 37°C; data not shown), whereas at the neutral pH of serum, the release rate is reduced nearly 2-fold. If an irrelevant conjugate enters an acidic tumor microenvironment, it is expected to release some SN-38 locally. Other factors, such as tumor physiology and innate sensitivities to the drug, will also play a role in defining this "baseline" activity. However, a specific conjugate with a longer residence time should have enhanced potency over this baseline response as long as there is ample antigen to capture the specific antibody. Biodistribution studies in the SK-MES-1 model also showed that if tumor antigen becomes saturated as a consequence of successive dosing, tumor uptake of the specific conjugate is reduced, which yields therapeutic results similar to that found with an irrelevant conjugate.

Although it is challenging to make direct comparisons between our ADC and the published reports of other SN-38 delivery agents, some general observations can be made. In our therapy studies, the highest individual dose was 0.4 mg/kg of SN-38. In the Calu-3 model, only 4 injections were given for a total cumulative dose of 1.6 mg/kg SN-38 or 32 $\mu$g SN-38 in a 20 g mouse. Multiple studies with ENZ-2208 were done using its MTD of 10 mg/kg $\times$ 5, and preclinical studies with NK012 involved its MTD of 30 mg/kg $\times$ 3. Thus, significant antitumor effects were obtained with hRS7-SN-38 at 30-fold and 55-fold less SN-38 equivalents than the reported doses in ENZ-2208 and NK012, respectively. Even with 10-fold less hRS7 ADC (0.04 mg/kg), significant antitumor effects were observed, whereas lower doses of ENZ-2208 were not presented, and when the NK012 dose was lowered 4-fold to 7.5 mg/kg, efficacy was lost (Koizumi et al., 2006, Cancer Res 66:10048-56). Normal mice showed no acute toxicity with a cumulative dose over 1 week of 24 mg/kg SN-38 (1,500 mg/kg of the conjugate), indicating that the MTD was higher. Thus, tumor-bearing animals were effectively treated with 7.5- to 15-fold lower amounts of SN-38 equivalents.

As a topoisomerase-I inhibitor, SN-38 induces significant damage to a cell's DNA, with upregulation of p53 and $p21^{WAF1/Cip1}$ resulting in caspase activation and cleavage of PARP. When we exposed BxPC-3 and Calu-3 cells to our ADC, both p53 and $p21^{WAF1/Cip1}$ were upregulated above basal levels. In addition, PARP cleavage was also evident in both cell lines, confirming an apoptotic event in these cells. Of interest was the higher upregulation of $p21^{WAF1/Cip1}$ in BxPC-3 and Calu-3 relative to p53 by both free SN-38 and our hRS7-SN-38. This may be indicative of the mutational status of p53 in these 2 cell lines and the use of a p53-independent pathway for $p21^{WAF1/Cip1}$-mediated apoptosis.

An interesting observation was the early upregulation of p53 in both BxPC-3 and Calu-3 at 24 hours mediated by the hRS7-ADC relative to free SN-38. Even the naked hRS7 IgG could upregulate p53 in these cell lines, although only after a 48-hour exposure. Trop-2 overexpression and cross-linking by antibodies has been linked to several MAPK-related signaling events, as well as intracellular calcium release. While binding of hRS7 was not sufficient to induce apoptosis in BxPC-3 and Calu-3, as evidenced by the lack of PARP cleavage, it may be enough to prime a cell, such that the inclusion of SN-38 conjugated to hRS7 may lead to a greater effect on tumor growth inhibition. Studies are currently underway to understand which pathways are involved with hRS7-delivery of SN-38 and how they may differ from free SN-38, and what effect p53 status may play in this signaling.

Biodistribution studies revealed the hRS7-CL2A-SN-38 had similar tumor uptake as the parental hRS7 IgG, but cleared substantially faster with 2-fold higher hepatic uptake, which may be due to the hydrophobicity of SN-38. With

the ADC being cleared through the liver, hepatic and gastrointestinal toxicities were expected to be dose limiting. Although mice had evidence of increased hepatic transaminases, gastrointestinal toxicity was mild at best, with only transient loss in weight and no abnormalities noted upon histopathologic examination. Interestingly, no hematological toxicity was noted. However, monkeys showed an identical toxicity profile as expected for irinotecan, with gastrointestinal and hematological toxicity being dose-limiting.

Because Trop-2 recognized by hRS7 is not expressed in mice, it was critically important to perform toxicity studies in monkeys that have a similar tissue expression of Trop-2 as humans. Monkeys tolerated 0.96 mg/kg/dose (~12 $mg/m^2$) with mild and reversible toxicity, which extrapolates to a human dose of ~0.3 mg/kg/dose (~11 $mg/m^2$). In a Phase I clinical trial of NK012, patients with solid tumors tolerated 28 $mg/m^2$ of SN-38 every 3 weeks with Grade 4 neutropenia as dose-limiting toxicity (Hamaguchi et al., 2010, Clin Cancer Res 16:5058-66). Similarly, Phase I clinical trials with ENZ-2208 revealed dose-limiting febrile neutropenia, with a recommendation to administer 10 $mg/m^2$ every 3 weeks or 16 $mg/m^2$ if patients were administered G-CSF. Because monkeys tolerated a cumulative human equivalent dose of 22 $mg/m^2$, it is possible that even though hRS7 binds to a number of normal tissues, the MTD for a single treatment of the hRS7 ADC could be similar to that of the other nontargeting SN-38 agents. Indeed, the specificity of the anti-Trop-2 antibody did not appear to play a role in defining the DLT, because the toxicity profile was similar to that of irinotecan. More importantly, if antitumor activity can be achieved in humans as in mice that responded with human equivalent dose of just at 0.03 mg SN-38 equivalents/kg/dose, then significant antitumor responses could be realized clinically.

In conclusion, toxicology studies in monkeys, combined with *in vivo* human cancer xenograft models in mice, have indicated that this ADC targeting Trop-2 is an effective therapeutic in several tumors of different epithelial origin.

**Example 10. Efficacy of Anti-Trop-2 Antibody Conjugated to a Prodrug Form of 2-Pyrrolinodoxorubicin (2-PDox)**

A prodrug form of 2-PDox (referred to as pro-2-PDox) was prepared and conjugated to antibodies as disclosed in U.S. Patent Application 14/175,089. Unless otherwise stated below, the number of drug moieties per antibody molecule was in the range of about 6.5 to about 7.5.

*In vitro* cell-binding studies - Retention of antibody binding was confirmed by cell binding assays comparing binding of the conjugated to the unconjugated antibody (Chari, 2008, Acc Chem Res 41:98-107). The potency of the conjugate was tested in a 4-day MTS assay using appropriate target cells. The anti-Trop-2 ADC (hRS7-pro-2-PDox) exhibited $IC_{50}$ values of 0.35-1.09 nM in gastric (NCI-N87), pancreatic (Capan-1), and breast (MDA-MB-468) human cancer cell lines, with free drug exhibiting 0.02-0.07 nM potency in the same cell lines. In additional studies, hRS7-pro-2-PDox was observed to be cytotoxic to MDA-MB-468, AG S, NCI-N87 and Capan-1 solid tumor cell lines (not shown).

No significant difference in binding of the antibody moiety to NCI-N87 gastric carcinoma cells was observed between unconjugated hRS7 and pro-2-PDox-hRS7 conjugated to 6 molecules of pro-2-PDox per antibody (not shown). It is concluded that conjugation of pro-2-PDox to antibodies does not affect antibody-antigen binding activity.

Serum stability - Serum stability of anti-Trop-2 ADC (hRS7-pro-2-PDox) was determined by incubation in human serum at a concentration of 0.2 mg/mL at 37°C. The incubate was analyzed by HPLC using butyl hydrophobic interaction chromatography (HIC). The analysis showed that there was no release of free drug from the conjugate, suggesting high serum stability of the conjugate. When the same experiment was repeated with hRS7-doxorubicin conjugate, containing the same cleavable linker as hRS7-pro-2-PDox, and where the free drug was independently verified to be released with a half-life of 96 h, clear formation of a peak corresponding to free doxorubicin was seen on HIC HPLC.

Surprisingly, it was determined that the pro-2-PDox conjugate was held tightly to the antibody because it cross-linked the peptide chains of the antibody together. The cross-linking stabilizes the attachment of the drug to the antibody so that the drug is only released intracellularly after the antibody is metabolized. The cross-linking assists in minimizing toxicity, for example cardiotoxicity, that would result from release of free drug in circulation. Previous use of 2-PDox peptide conjugates failed because the drug cross-linked the peptide to other proteins or peptides *in vivo*. With the present anti-Trop-2 ADC, the pro-2-PDox is attached to interchain disulfide thiol groups while in the prodrug form. The prodrug protection is rapidly removed *in vivo* soon after injection and the resulting 2-PDox portion of the conjugate cross-links the peptide chains of the antibody, forming intramolecular cross-linking within the antibody molecule. This both stabilizes the ADC and prevents cross-linking to other molecules in circulation.

*In vivo* preclinical studies - Tumor size was determined by caliper measurements of length (L) and width (W) with tumor volume calculated as $(L \times W^2)/2$. Tumors were measured and mice weighed twice a week. Mice were euthanized if their tumors reached >1 $cm^3$ in size, lost greater than 15% of their starting body weight, or otherwise became moribund. Statistical analysis for the tumor growth data was based on area under the curve (AUC) and survival time. Profiles of individual tumor growth were obtained through linear curve modeling. An *f*-test was employed to determine equality of variance between groups prior to statistical analysis of growth curves. A two-tailed t-test was used to assess statistical significance between all the various treatment groups and non-specific controls. For the saline control analysis a one-tailed t-test was used to assess significance. Survival studies were analyzed using Kaplan-Meier plots (log-rank

analysis), using the Prism GraphPad Software (v4.03) software package (Advanced Graphics Software, Inc.; Encinitas, CA). All doses in preclinical experiments are expressed in antibody amounts. In terms of drug, 100 μg of antibody (5 mg/kg) in a 20-g mouse, for example, carries 1.4 μg-2.8 μg (0.14-0.17 mg/kg) of pro-2-PDox equivalent dose when using an ADC with 3-6 drugs/IgG.

A single i.v. dose of ≥ 300 μg [~ 10 μg of pro-2-PDox] of the anti-Trop-2 ADC was lethal, but 4 doses of 45 μg given in 2 weeks were tolerated by all animals. Using this dosing regimen, we examined the therapeutic effect of anti-Trop-2 hRS7-pro-2-PDox in 2 human tumor xenograft models, Capan-1 (pancreatic cancer) and NCI-N87 (gastric cancer). Therapy began 7 days after tumor transplantation in nude mice. In the established, 7-day-old, Capan-1 model, 100% of established tumors quickly regressed, with no evidence of re-growth (not shown). This result was reproduced in a repeat experiment (not shown). The anti-Trop-2 conjugate of pro-2-PDox was much more effective than the same drug conjugated to an antibody (hMN-14) against CEACAM5, which is also expressed in pancreatic cancer, or an antibody against CD20 (hA20), which is not. All treatments were superior to the saline control.

Similar results were observed in the established NCI-N87 model (not shown), where a 2nd course of therapy, administered after day 70, was safely tolerated and led to further regressions of residual tumor (not shown). The internalizing hRS7-SN-38 conjugate, targeting Trop-2, provided better therapeutic responses than a conjugate of a poorly internalizing anti-CEACAM5 antibody, hMN-14 (not shown). A non-targeted anti-CD20 ADC, hA20-pro-2-PDox, was ineffective, indicating selective therapeutic efficacy (not shown). Data from a breast cancer xenograft (MDA-MB-468) and a second pancreatic cancer xenograft (not shown) showed the same pattern, with the anti-Trop-2 ADC significantly more efficacious compared to non-targeting ADC or saline control. In both cases, administration of anti-Trop-2 ADC produced a clear inhibition of tumor growth to the end of the study.

PK and toxicity of hRS7-pro-2-PDox with substitutions of 6.8 or 3.7 drug/IgG - Antibody-drug conjugates (ADCs) carrying as much as 8 ultratoxic drugs/MAb are known to clear faster than unmodified MAb and to increase off-target toxicity, a finding that has led to the current trends to use drug substitutions of ≤ 4 (Hamblett et al., 2004, Clin Cancer Res 10:7063-70). ADCs were prepared and evaluated with mean drug/MAb substitution ratios (MSRs) of ~6:1 and ~3:1. Groups of normal mice (n = 5) were administered, i.v., single doses of unmodified hRS7 or hRS7-pro-2-PDox with drug substitution of 6.8 or 3.7 (same protein dose), and serum samples were collected at 30 min, 4 h, 24 h, 72 h, and 168 h post-injection. These were analyzed by ELISA for antibody concentration. There were no significant differences in serum concentrations at various times, indicating that these showed similar clearance from the blood. The PK parameters (Cmax, AUC, etc.) were also similar. ADCs with either higher or lower drug substitution had similar tolerability in nude mice, when the administered at the same dose of conjugated drug.

Therapeutic Efficacy at Minimum Effective Dose (MED) - Anti-Trop-2 ADC (hRS7-pro-2-PDox), was evaluated in nude mice bearing NCI-N87 human gastric cancer xenografts by administering a single bolus protein dose of 9 mg/kg, 6.75 mg/kg, 4.5 mg/kg, 2.25 mg/kg, or 1 mg/kg. The therapy was started when the mean tumor volume (mTV) was 0.256 $cm^3$. On day 21, mTV in the saline control group (non-treatment group) was 0.801 ± 0.181 $cm^3$ which was significantly larger than that in mice treated with 9, 6.75, 4.5, or 2.25 mg/kg dose with mTV of 0.211 ± 0.042 $cm^3$, 0.239 ± 0.0.054 $cm^3$, 0.264 ± 0.087 $cm^3$, and 0.567 ± 0.179 $cm^3$, respectively (P<0.0047, one tailed t-test). From these, the minimum effective dose was estimated to be 2.25 mg/kg, while 9 mg/kg represented MTD.

### Example 11. Anti-Trop-2 ADC Comprising hRS7 and Paclitaxel

A new antibody-drug conjugate (ADC) was made by conjugating paclitaxel (TAXOL®) to the hRS7 anti-human Trop-2 antibody (hRS7-paclitaxel). The final product had a mean drug to antibody substitution ratio of 2.2. This ADC was tested *in vitro* using two different Trop-2-postive cell lines as targets: BxPC-3 (human pancreatic adenocarcinoma) and MDA-MB-468 (human triple negative breast carcinoma). One day prior to adding the ADC, cells were harvested from tissue culture and plated into 96-well plates at 2000 cells per well. The next day cells were exposed to free paclitaxel ($6.1 \times 10^{-11}$ to $4 \times 10^{-6}$ M) or the drug-equivalent of hRS7-paclitaxel. For comparison, hRS7-SN-38 and free SN-38 were also tested at a range of $3.84 \times 10^{-12}$ to $2.5 \times 10^{-7}$ M. Plates were incubated at 37°C for 96 h. After this incubation period, an MTS substrate was added to all of the plates and read for color development at half-hour intervals until untreated control wells had an $OD_{492nm}$ reading of approximately 1.0. Growth inhibition was measured as a percent of growth relative to untreated cells using Microsoft Excel and Prism software (non-linear regression to generate sigmoidal dose response curves which yield $IC_{50}$-values).

The hRS7-paclitaxel ADC exhibited cytotoxic activity in the MDA-MB-468 breast cell line (not shown), with an $IC_{50}$-value approximately 4.5-fold higher than hRS7-SN-38. The free paclitaxel was much more potent than the free SN-38 (not shown). While the $IC_{50}$ for free SN-38 was $1.54 \times 10^{-9}$ M, the $IC_{50}$ for free paclitaxel was less than $6.1 \times 10^{-11}$ M. Similar results were obtained for the BxPC-3 pancreatic cell line (not shown) in which the hRS7-paclitaxel ADC had an $IC_{50}$-value approximately 2.8-fold higher than the hRS7-SN-38 ADC. These results show the efficacy of anti-Trop-2 conjugated paclitaxel *in vitro,* with $IC_{50}$-values in the nanomolar range, similar to the hRS7-SN-38 ADC.

**Example 12. Cytotoxicity of Anti-Trop-2 ADC (MAB650-SN-38)**

A novel anti-Trop-2 ADC was made with SN-38 and MAB650, yielding a mean drug to antibody substitution ratio of 6.89. Cytotoxicity assays were performed to compare the MAB650-SN-38 and hRS7-SN-38 ADCs using two different human pancreatic adenocarcinoma cell lines (BxPC-3 and Capan-1) and a human triple negative breast carcinoma cell line (MDA-MB-468) as targets.

One day prior to adding the ADCs, cells were harvested from tissue culture and plated into 96-well plates. The next day cells were exposed to hRS7-SN-38, MAB650-SN-38, and free SN-38 at a drug range of $3.84\times10^{-12}$ to $2.5\times10^{-7}$ M. Unconjugated MAB650 was used as a control at protein equivalent doses as the MAB650-SN-38. Plates were incubated at 37°C for 96 h. After this incubation period, an MTS substrate was added to all of the plates and read for color development at half-hour intervals until an $OD_{492nm}$ of approximately 1.0 was reached for the untreated cells. Growth inhibition was measured as a percent of growth relative to untreated cells using Microsoft Excel and Prism software (non-linear regression to generate sigmoidal dose response curves which yield $IC_{50}$-values.

hRS7-SN-38 and MAB650-SN-38 had similar growth-inhibitory effects (not shown) with $IC_{50}$-values in the low nM range which is typical for SN-38-ADCs in these cell lines. In the human Capan-1 pancreatic adenocarcinoma cell line (not shown), the hRS7-SN-38 ADC showed an $IC_{50}$ of 3.5 nM, compared to 4.1 nM for the MAB650-SN-38 ADC and 1.0 nM for free SN-38. In the human BxPC-3 pancreatic adenocarcinoma cell line (not shown), the hRS7-SN-38 ADC showed an $IC_{50}$ of 2.6 nM, compared to 3.0 nM for the MAB650-SN-38 ADC and 1.0 nM for free SN-38. In the human NCI-N87 gastric adenocarcinoma cell line (not shown), the hRS7-SN-38 ADC showed an $IC_{50}$ of 3.6 nM, compared to 4.1 nM for the MAB650-SN-38 ADC and 4.3 nM for free SN-38.

In summary, in these *in vitro* assays, the SN-38 conjugates of two anti-Trop-2 antibodies, hRS7 and MAB650, showed equal efficacies against several tumor cell lines, which was similar to that of free SN-38. Because the targeting function of the anti-Trop-2 antibodies would be a much more significant factor *in vivo* than *in vitro,* the data support that anti-Trop-2-SN-38 ADCs as a class would be highly efficacious *in vivo,* as demonstrated in the Examples above for hRS7-SN-38.

**Example 13. Cytotoxicity of Anti-Trop-2 ADC (162-46.2-SN-38)**

A novel anti-Trop-2 ADC was made with SN-38 and 162-46.2, yielding a drug to antibody substitution ratio of 6.14. Cytotoxicity assays were performed to compare the 162-46.2-SN-38 and hRS7-SN-38 ADCs using two different Trop-2-postive cell lines as targets, the BxPC-3 human pancreatic adenocarcinoma and the MDA-MB-468 human triple negative breast carcinoma.

One day prior to adding the ADC, cells were harvested from tissue culture and plated into 96-well plates at 2000 cells per well. The next day cells were exposed to hRS7-SN-38, 162-46.2-SN-38, or free SN-38 at a drug range of $3.84 \times 10^{-12}$ to $2.5 \times 10^{-7}$ M. Unconjugated 162-46.2 and hRS7 were used as controls at the same protein equivalent doses as the 162-46.2-SN-38 and hRS7-SN-38, respectively. Plates were incubated at 37°C for 96 h. After this incubation period, an MTS substrate was added to all of the plates and read for color development at half-hour intervals until untreated control wells had an $OD_{492nm}$ reading of approximately 1.0. Growth inhibition was measured as a percent of growth relative to untreated cells using Microsoft Excel and Prism software (non-linear regression to generate sigmoidal dose response curves which yield $IC_{50}$-values).

The 162-46.2-SN-38 ADC had a similar $IC_{50}$-values when compared to hRS7-SN-38 (not shown). When tested against the BxPC-3 human pancreatic adenocarcinoma cell line (not shown), hRS7-SN-38 had an $IC_{50}$ of 5.8 nM, compared to 10.6 nM for 162-46.2-SN-38 and 1.6 nM for free SN-38. When tested against the MDA-MB-468 human breast adenocarcinoma cell line (not shown), hRS7-SN-38 had an $IC_{50}$ of 3.9 nM, compared to 6.1 nM for 162-46.2-SN-38 and 0.8 nM for free SN-38. The free antibodies alone showed little cytotoxicity to either Trop-2 positive cancer cell line.

In summary, comparing the efficacies *in vitro* of three different anti-Trop-2 antibodies conjugated to the same cytotoxic drug, all three ADCs exhibited equivalent cytotoxic effects against a variety of Trop-2 positive cancer cell lines. These data support that the class of anti-Trop-2 antibodies, incorporated into drug-conjugated ADCs, are effective anti-cancer therapeutic agents for Trop-2 expressing solid tumors.

**Example 14. Clinical Trials With IMMU-132 Anti-Trop-2 ADC Comprising hRS7 Antibody Conjugated to SN-38**

*Summary*

The present Example reports results from a phase I clinical trial and ongoing phase II extension with IMMU-132, an ADC of the internalizing, humanized, hRS7 anti-Trop-2 antibody conjugated by a pH-sensitive linker to SN-38 (mean drug-antibody ratio = 7.6). Trop-2 is a type I transmembrane, calcium-transducing, protein expressed at high density (~$1 \times 10^5$), frequency, and specificity by many human carcinomas, with limited normal tissue expression. Preclinical studies in nude mice bearing Capan-1 human pancreatic tumor xenografts have revealed IMMU-132 is capable of delivering as much as 120-fold more SN-38 to tumor than derived from a maximally tolerated irinotecan therapy.

The present Example reports the initial Phase I trial of 25 patients (pts) who had failed multiple prior therapies (some including topoisomerase-I/II inhibiting drugs), and the ongoing Phase II extension now reporting on 69 pts, including in colorectal (CRC), small-cell and non-small cell lung (SCLC, NSCLC, respectively), triple-negative breast (TNBC), pancreatic (PDC), esophageal, and other cancers.

As discussed in detail below, Trop-2 was not detected in serum, but was strongly expressed ($\geq 2^+$) in most archived tumors. In a 3+3 trial design, IMMU-132 was given on days 1 and 8 in repeated 21-day cycles, starting at 8 mg/kg/dose, then 12 and 18 mg/kg before dose-limiting neutropenia. To optimize cumulative treatment with minimal delays, phase II is focusing on 8 and 10 mg/kg (n=30 and 14, respectively). In 49 pts reporting related AE at this time, neutropenia ≥G3 occurred in 28% (4% G4). Most common non-hematological toxicities initially in these pts have been fatigue (55%;≥G3 = 9%), nausea (53%;≥G3=0%), diarrhea (47%;≥G3 = 9%), alopecia (40%), and vomiting (32%;≥G3 = 2%). Homozygous UGT1A1 *28/*28 was found in 6 pts, 2 of whom had more severe hematological and GI toxicities. In the Phase I and the expansion phases, there are now 48 pts (excluding PDC) who are assessable by RECIST/CT for best response. Seven (15%) of the patients had a partial response (PR), including patients with CRC (N = 1), TNBC (N = 2), SCLC (N = 2), NSCLC (N = 1), and esophageal cancers (N = 1), and another 27 pts (56%) had stable disease (SD), for a total of 38 pts (79%) with disease response; 8 of 13 CT-assessable PDC pts (62%) had SD, with a median time to progression (TTP) of 12.7 wks compared to 8.0 weeks in their last prior therapy. The TTP for the remaining 48 pts is 12.6+ wks *(range 6. 0 to 51.4 wks).* Plasma CEA and CA19-9 correlated with responses. No anti-hRS7 or anti-SN-38 antibodies were detected despite dosing over months. The conjugate cleared from the serum within 3 days, consistent with *in vivo* animal studies where 50% of the SN-38 was released daily, with >95% of the SN-38 in the serum being bound to the IgG in a non-glucoronidated form, and at concentrations as much as 100-fold higher than SN-38 reported in patients given irinotecan. These results show that the hRS7-SN-38-containing ADC is therapeutically active in metastatic solid cancers, with manageable diarrhea and neutropenia.

*Pharmacokinetics*

Two ELISA methods were used to measure the clearance of the IgG (capture with anti-hRS7 idiotype antibody) and the intact conjugate (capture with anti-SN-38 IgG/probe with anti-hRS7 idiotype antibody). SN-38 was measured by HPLC. Total IMMU-132 fraction (intact conjugate) cleared more quickly than the IgG (not shown), reflecting known gradual release of SN-38 from the conjugate. HPLC determination of SN-38 (Unbound and TOTAL) showed >95% the SN-38 in the serum was bound to the IgG. Low concentrations of SN-38G suggest SN-38 bound to the IgG is protected from glucoronidation. Comparison of ELISA for conjugate and SN-38 HPLC revealed both overlap, suggesting the ELISA is a surrogate for monitoring SN-38 clearance.

A summary of the dosing regiment and patient poll is provided in **Table 12.**

**Table 12. Clinical Trial Parameters**

| | |
|---|---|
| **Dosing regimen** | Once weekly for 2 weeks administered every 21 days for up to 8 cycles. In the initial enrollment, the planned dose was delayed and reduced if ≥ G2 treatment-related toxicity; protocol was amended to dose delay and reduction only in the event of ≥ G3 toxicity. |
| **Dose level cohorts** | 8, 12, 18 mg/kg; later reduced to an intermediate dose level of 10 mg/kg. |
| **Cohort size** | Standard Phase I [3+3] design; expansion includes 15 patients in select cancers. |
| **DLT** | G4 ANC ≥ 7 d; ≥G3 febrile neutropenia of any duration; G4 Pit ≥ 5 d; G4 Hgb; Grade 4 N/V/D any duration/G3 N/V/D for > 48 h; G3 infusion-related reactions; related ≥G3 non-hematological toxicity. |
| **Maximum Acceptable Dose (MAD)** | Maximum dose where ≥2/6 patients tolerate 1st 21-d cycle w/o delay or reduction or ≥ G3 toxicity. |
| **Patients** | Metastatic colorectal, pancreas, gastric, esophageal, lung (NSCLC, SCLC), triple-negative breast (TNBC), prostate, ovarian, renal, urinary bladder, head/neck, hepatocellular. Refractory/relapsed after standard treatment regimens for metastatic cancer. Prior irinotecan-containing therapy NOT required for enrollment. No bulky lesion > 5 cm. Must be 4 weeks beyond any major surgery, and 2 weeks beyond radiation or chemotherapy regimen. Gilbert's disease or known CNS metastatic disease are excluded. |

*Clinical Trial Status*

A total of 69 patients (including 25 patients in Phase I) with diverse metastatic cancers having a median of 3 prior therapies were reported. Eight patients had clinical progression and withdrew before CT assessment. Thirteen CT-assessable pancreatic cancer patients were separately reported. The median TTP (time to progression) in PDC patients was 11.9 wks (range 2 to 21.4 wks) compared to median 8 wks TTP for the preceding last therapy.

A total of 48 patients with diverse cancers had at least 1 CT-assessment from which Best Response (not shown) and Time to Progression (TTP; not shown) were determined. To summarize the Best Response data, of 8 assessable patients with TNBC (triple-negative breast cancer), there were 2 PR (partial response), 4 SD (stable disease) and 2 PD (progressive disease) for a total response [PR + SD] of 6/8 (75%). For SCLC (small cell lung cancer), of 4 assessable patients there were 2 PR, 0 SD and 2 PD for a total response of 2/4 (50%). For CRC (colorectal cancer), of 18 assessable patients there were 1 PR, 11 SD and 6 PD for a total response of 12/18 (67%). For esophageal cancer, of 4 assessable patients there were 1 PR, 2 SD and 1 PD for a total response of 3/4 (75%). For NSCLC (non-small cell lung cancer), of 5 assessable patients there were 1 PR, 3 SD and 1 PD for a total response of 4/5 (80%). Over all patients treated, of 48 assessable patients there were 7 PR, 27 SD and 14 PD for a total response of 34/48 (71%). These results demonstrate that the anti-TROP-2 ADC (hRS7-SN-38) showed significant clinical efficacy against a wide range of solid tumors in human patients.

The reported side effects of therapy (adverse events) are summarized in **Table 13.** As apparent from the data of **Table 13,** the therapeutic efficacy of hRS7-SN-38 was achieved at dosages of ADC showing an acceptably low level of adverse side effects.

**Table 13.**

| **Related Adverse Events Listing for IMMU-132-01 Criteria: Total ≥ 10% or ≥ Grade 3** | | | |
|---|---|---|---|
| | N = 47 patients | | |
| | TOTAL | Grade 3 | Grade 4 |
| **Fatigue** | 55% | 4 (9%) | 0 |
| **Nausea** | 53% | 0 | 0 |
| **Diarrhea** | 47% | 4 (9%) | 0 |
| **Neutropenia** | 43% | 11 (24%) | 2 (4%) |
| **Alopecia** | 40% | -- | -- |
| **Vomiting** | 32% | 1 (2%) | 0 |
| **Anemia** | 13% | 2 (4%) | 0 |
| **Dysgeusia** | 15% | 0 | 0 |
| **Pyrexia** | 13% | 0 | 0 |
| **Abdominal pain** | 11% | 0 | 0 |
| **Hypokalemia** | 11% | 1 (2%) | 0 |
| **WBC Decrease** | 6% | 1 (2%) | 0 |
| **Febrile Neutropenia** | 6% | 1 (2%) | 2 (4%) |
| **Deep vein thrombosis** | 2% | 1 (2%) | 0 |
| **Grading by CTCAE v 4.0** | | | |

Exemplary partial responses to the anti-Trop-2 ADC were confirmed by CT data (not shown). As an exemplary PR in CRC, a 62 year-old woman first diagnosed with CRC underwent a primary hemicolectomy. Four months later, she had a hepatic resection for liver metastases and received 7 mos of treatment with FOLFOX and 1 mo 5FU. She presented with multiple lesions primarily in the liver (3+ Trop-2 by immunohistology), entering the hRS7-SN-38 trial at a starting dose of 8 mg/kg about 1 year after initial diagnosis. On her first CT assessment, a PR was achieved, with a 37% reduction in target lesions (not shown). The patient continued treatment, achieving a maximum reduction of 65% decrease after 10 months of treatment (not shown) with decrease in CEA from 781 ng/mL to 26.5 ng/mL), before progressing 3 months

later.

As an exemplary PR in NSCLC, a 65 year-old male was diagnosed with stage IIIB NSCLC (sq. cell). Initial treatment of caboplatin/etoposide (3 mo) in concert with 7000 cGy XRT resulted in a response lasting 10 mo. He was then started on Tarceva maintenance therapy, which he continued until he was considered for IMMU-132 trial, in addition to undergoing a lumbar laminectomy. He received first dose of IMMU-132 after 5 months of Tarceva, presenting at the time with a 5.6 cm lesion in the right lung with abundant pleural effusion. He had just completed his $6^{th}$ dose two months later when the first CT showed the primary target lesion reduced to 3.2 cm (not shown).

As an exemplary PR in SCLC, a 65 year-old woman was diagnosed with poorly differentiated SCLC. After receiving carboplatin/etoposide (Topo-II inhibitor) that ended after 2 months with no response, followed with topotecan (Topo-I inhibitor) that ended after 2 months, also with no response, she received local XRT (3000 cGy) that ended 1 month later. However, by the following month progression had continued. The patient started with IMMU-132 the next month (12 mg/kg; reduced to 6.8 mg/kg; Trop-2 expression 3+), and after two months of IMMU-132, a 38% reduction in target lesions, including a substantial reduction in the main lung lesion occurred (not shown). The patient progressed 3 months later after receiving 12 doses.

These results are significant in that they demonstrate that the anti-Trop-2 ADC was efficacious, even in patients who had failed or progressed after multiple previous therapies.

In conclusion, at the dosages used, the primary toxicity was a manageable neutropenia, with few Grade 3 toxicities. IMMU-132 showed evidence of activity (PR and durable SD) in relapsed/refractory patients with triple-negative breast cancer, small cell lung cancer, non-small cell lung cancer, colorectal cancer and esophageal cancer, including patients with a previous history of relapsing on topoisomerase-I inhibitor therapy. These results show efficacy of the anti-Trop-2 ADC in a wide range of cancers that are resistant to existing therapies.

**Example 15. Anti-CD22 (Epratuzumab) Conjugated-SN-38 for the Therapy of Hematologic Malgnancies**

*Abstract*

We previously found that slowly internalizing antibodies conjugated with SN-38 could be used successfully when prepared with a linker that allows approximately 50% of the IgG-bound SN-38 to dissociate in serum every 24 hours. In this study, the efficacy of SN-38 conjugates prepared with epratuzumab (rapidly internalizing) and veltuzumab (slowly internalizing), humanized anti-CD22 and anti-CD20 IgG, respectively, was examined for the treatment of B-cell malignancies. Both antibody-drug conjugates had similar nanomolar activity against a variety of human lymphoma/leukemia cell lines, but slow release of SN-38 compromised potency discrimination *in vitro* even against an irrelevant conjugate. When SN-38 was stably linked to the anti-CD22 conjugate, its potency was reduced 40- to 55-fold. Therefore, further studies were conducted only with the less stable, slowly dissociating linker. *In vivo,* similar antitumor activity was found between CD22 and CD20 antibody-drug conjugate in mice-bearing Ramos xenografts, even though Ramos expressed 15-fold more CD20 than CD22, suggesting that the internalization of the epratuzumab-SN-38 conjugate (Emab-SN-38) enhanced its activity. Emab-SN-38 was more efficacious than a nonbinding, irrelevant IgG-SN-38 conjugate *in vivo,* eliminating a majority of well-established Ramos xenografts at nontoxic doses. *In vitro* and *in vivo* studies showed that Emab-SN-38 could be combined with unconjugated veltuzumab for a more effective treatment. Thus, Emab-SN-38 is active in lymphoma and leukemia at doses well below toxic levels and therefore represents a new promising agent with therapeutic potential alone or combined with anti-CD20 antibody therapy. (Sharkey et al., 2011, Mol Cancer Ther 11:224-34.)

*Introduction*

A significant effort has focused on the biologic therapy of leukemia and lymphoma, where unconjugated antibodies (e.g., rituximab, alemtuzumab, ofatumumab), radioimmunoconjugates ($^{90}$Y-ibritumomab tiuxetan, $^{131}$I-tositumomab), and a drug conjugate (gemtuzumab ozogamicin) received U.S. Food and Drug Administration (FDA) approval. Another antibody-drug conjugate (ADC), brentuximab vedotin (SGN-35; anti-CD30-auristatin E), recently received accelerated approval by the FDA for Hodgkin lymphoma and anaplastic large-cell lymphomas. There are also a number of other ADCs in preclinical and clinical development that target CD19, CD22, CD37, CD74, and CD79b.

Antibodies against all of these targets are logical choices for carriers of drugs, because they are internalizing. Internalization and specificity of CD22 have made it a particularly important target for leukemia and lymphomas, with at least 3 different anti-CD22 conjugates in clinical investigation, including CMC-544 (acid-labile-conjugated calicheamicin), an anti-CD22-maytansine conjugate (stably linked MCC-DM1), and CAT-3888 (formally BL22; a Pseudomonas exotoxin single-chain fusion protein). The active agent in all of these conjugates has subnanomolar potency (i.e., so called ultra-toxics).

We recently developed methods to conjugate antibodies with SN-38, a topoisomerase I inhibitor with low

nanomolar potency that is derived from the prodrug, irinotecan (Govindan et al., 2009, Clin Cancer Res 15:6052-62; Moon et al., 2008, J Med Chem 51:6916-26). Four SN-38 linkage chemistries were examined initially using conjugates prepared with a slowly internalizing anti-CEACAM5 antibody (Govindan et al., 2009, Clin Cancer Res 15:6052-62; Moon et al., 2008, J Med Chem 51:6916-26). The conjugates retained CEACAM5 binding but differed in the dissociation rate of SN-38 in human serum, with half-lives varying from approximately 10 to 67 hours (Govindan et al., 2009, Clin Cancer Res 15:6052-62). Ultimately, the linker designated CL2, with intermediate stability (~ 50% dissociated in 24-35 hours), was selected for further development. CL2 was modified recently, eliminating the phenylalanine in the cathepsin B-cleavable dipeptide to simplify and improve manufacturing yields. The new derivative, designated CL2A, retains the pH-sensitive carbonate linkage to the SN-38, but it is no longer selectively cleaved by cathepsin B. Nevertheless, it has identical serum stability and improved *in vivo* activity compared to the original CL2 linker (Cardillo et al., 2011, Clin Cancer Res 17:3157-69). Because significant efficacy without toxicity was found with the slowly internalizing anti-CEACAM5-SN-38, we postulated that its activity was aided by the slow release of SN-38 from the antibody after it localized in a tumor. Thus, the main objective in this report was to evaluate the therapeutic prospects of conjugates prepared using the CL2A linker with two antibodies that are highly specific for B-cell cancers but differ in their antigen expression and internalization properties.

Epratuzumab (Emab) is a rapidly internalizing (e.g., ≥50% within 1 hour), humanized anti-CD22 IgG1 that has been evaluated extensively in lymphoma and leukemia in an unconjugated or conjugated form. Veltuzumab (Vmab) is a humanized anti-CD20 antibody that is also being studied clinically but internalizes slowly (e.g., ~10% in 1 hour). CD20 is usually expressed at much higher levels than CD22 in non-Hodgkin lymphoma, whereas CD22 is preferentially expressed in acute lymphoblastic leukemia (ALL) but not in multiple myeloma. Both antibodies are effective in patients as unconjugated agents, but only veltuzumab is active in murine xenograft models (Stein et al., 2004, Clin Cancer Res 10:2868-76). On the basis of previous studies that showed $^{90}$Y-Emab combined with unconjugated veltuzumab had enhanced efficacy in NHL models (Mattes et al., 2008, Clin Cancer Res 14:6154-60), we also examined the Emab-SN-38 + Vmab combination, as this could provide additional benefit without competing for the same target antigen or having additional toxicity.

*Materials and Methods*

Cell lines. Ramos, Raji, Daudi (Burkitt lymphomas), and JeKo-1 (mantle cell lymphoma) were purchased from American Type Culture Collection. REH, RS4;11, MN-60, and 697 (ALL) were purchased from Deutsche Sammlung von Mikroorganismen und Zellkulturen. WSU-FSCCL (follicular NHL) was the gift of Dr. Mitchell R. Smith (Fox Chase Cancer Center, Philadelphia, PA). All cell lines were cultured in a humidified $CO_2$ incubator (5%) at 37°C in recommended supplemented media containing 10 to 20% fetal calf serum and were checked periodically for Mycoplasma.

Antibodies and conjugation methods. Epratuzumab and veltuzumab are humanized anti-CD22 and anti-CD20 IgG1 monoclonal antibodies, respectively. Labetuzumab (Lmab), a humanized anti-CEACAM5 IgG1, and RS7, a humanized anti-Trop-2 antibody (both from Immunomedics, Inc.), were used as nonbinding, irrelevant controls. Herein, Emab-SN-38, Vmab-SN-38, and Lmab-SN-38 refer to conjugates prepared using the CL2A linker that was described above. *In vitro* studies in human serum showed that approximately 50% of the active SN-38 moiety is released from the IgG each day (Cardillo et al., 2011, Clin Cancer Res 17:3157-69). Another linker, designated CL2E, is stable in human serum over 14 days, but it contains a cathepsin B cleavage site to facilitate the release of SN-38 when processed in lysosomes. The method to prepare CL2E and the structures of the CL2A and CL2E linkers are given in the Examples above. The conjugates contained approximately 6 SN-38 units per IgG (e.g., 1.0 mg of the IgG-SN-38 conjugate contains ~16 μg of SN-38).

*In vitro* cell binding and cytotoxicity. Flow cytometry was carried out using the unconjugated specific and irrelevant antibodies incubated for 1 hour at 4°C, with binding revealed using fluorescein isothiocyanate (FITC)-Fcγ fragment-specific goat anti-human IgG (Jackson ImmunoResearch), also incubated for 1 hour at 4°C. Median fluorescence was determined on a FACSCALIBUR® flow cytometer (Becton Dickinson) using a CellQuest software package.

Cytotoxicity was determined using the MTS dye reduction assay (Promega). Dose-response curves [with/without goat anti-human Fcγ F(ab')$_2$; Jackson ImmunoResearch] were generated from the mean of triplicate determinations, and $IC_{50}$-values were calculated using PRISM® GraphPad software (v5), with statistical comparisons using an F test on the best fit curves for the data. Significance was set at $P < 0.05$.

Immunoblotting. After 24- or 48-hour exposure to the test agents, markers of early (p21 expression) and late (PARP cleavage) apoptosis were revealed by Western blotting.

*In vivo* studies. The subcutaneous Ramos model was initiated by implanting $1 \times 10^7$ cells (0.2 mL) from culture (>95% viability) into 4- to 6-week-old female nude mice (Taconic). Three weeks from implantation, animals with tumors ranging from 0.4 to 0.8 cm$^3$ (measured by caliper, L × W × D) were segregated into groups of animals, each with the same range of tumor sizes. Tumor size and body weights were measured at least once weekly, with animals removed from the study when tumors grew to 3.0 cm$^3$ or if they experienced 20% or greater body weight loss. The intravenous

WSU-FSCCL and 697 models were initiated by intravenous injection of $2.5 \times 10^6$ and $1 \times 10^7$ cells, respectively, in female severe combined immunodeficient (SCID) mice (Taconic). Treatment began 5 days after administration of the WSU-FSCCL cells and 7 days after the 697 inoculation. Animals were observed daily, using hind leg paralysis or other signs of morbidity as surrogate survival endpoints. All treatments were given intraperitoneally in $\leq$0.2 mL. The specific dosages and frequency are given in the Results section. Because mice convert irinotecan to SN-38 efficiently, irinotecan dosing was adjusted on the basis of SN-38 equivalents; SN-38 mole equivalents are based on 1.6% of ADC mass and 60% of irinotecan mass.

Efficacy was expressed in a Kaplan-Meier curve, using time to progression (TTP) as surrogate survival endpoints as indicated above. Statistical analysis was conducted by a log-rank test using PRISM® GraphPad software (significance, $P < 0.05$).

*Results*

Antigen expression and cytotoxicity *in vitro.* All cell lines were highly susceptible to SN-38, with $EC_{50}$ values ranging from 0.13 nmol/L for Daudi to 2.28 nmol/L for RS4;11 (**Table** 14). Except for 697 and RS4;11, the Emab-SN-38 anti-CD22 conjugate was 2- to 7-fold less effective than SN-38. This is a common finding with our targeted, as well as other nontargeted, SN-38 conjugates. Despite differences in antigen expression, the Emab-SN-38 and Vmab-SN-38 had similar potencies as the nonbinding, Lmab-SN-38 anti-CEACAM5 conjugate, which was likely due to dissociation of approximately 90% of SN-38 during the 4-day MTS assay. Other *in vitro* procedures using shorter exposure times were also ineffective in discriminating differences in the potencies of conjugates. For example, Annexin V staining after a 1-day exposure failed to find differences between untreated and treated cells (not shown). Upregulation of p21 and PARP cleavage was also examined as early and late markers of apoptosis, respectively. Ramos did not express p21. However, PARP cleavage was detected, but only after a 48-hour exposure, being more strongly expressed in SN-38-treated cells (not shown). The WSU-FSCCL cell line expressed p21, but neither p21 upregulation nor PARP cleavage was evident until 48 hours after Emab-SN-38 exposure. However, both were observed after a 24-hour exposure with free SN-38 (not shown). While the enhanced intensity and earlier activation of apoptotic events with free SN-38 are consistent with its lower $EC_{50}$ over the IgG-conjugated form, the results indicated that an exposure period of at least 48 hours would be required, but at this time, approximately 75% of the SN-38 would be released from the conjugate.

**Table 14.** Expression of CD20 and CD22 by FACScan and *in vitro* cytotoxicity by MTS assay of SN-38 and specific Emab anti-CD22-SN-38, Vmab anti-CD20-SN-38, and Lmab anti-CEACAM5-SN-38 conjugates against several hematopoietic tumor cell lines

| | CD20 expression | CD22 expression | $EC_{50}$ values-[a] | | | | | | | |
|---|---|---|---|---|---|---|---|---|---|---|
| Cell line | Median fluorescence (background) | Median fluorescence (background) | SN-38,nmol/L | 95%CI | Emab-SN-38, nmol/L | 95% CI | Vmab-SN-38, nmol/L | 95% CI | Lmab-SN-38, nmol/L | 95% CI |
| NBL:Burkitt | | | | | | | | | | |
| Raji | 422.2 (6.8) | 45.9 (6.8) | 1.42 | 0.8-2.4 | 2.10 | 1.2-3.8 | ND | - | ND | - |
| | | | | | 4.61 | 2.2-9.5 | 4.88 | 2.7-9.0 | 3.73 | 1.8-7.6 |
| | CD20 expression | CD22 expression | $EC_{50}$ values[a] | | | | | | | |
| Cell line | Median fluorescence (background) | Median fluorescence (background) | SN-38,nmol/L | 95% CI | Emab-SN-38, nmol/L | 95% CI | Vmab-SN-38, nmol/L | 95% CI | Lmab-SN-38, nmol/L | 95% CI |
| Ramos | 620.4(4.1) | 40.8 (4.1) | 0.40 | 0.2-0.7 | 2.92 | 1.6-5.4 | ND | - | ND | - |
| | | | | | 9.84 | 4.5-21.6 | 13.56 | 4.9-37.2 | 8.08 | 2.9-22.2 |
| Daudi | 815.1 (5.9) | 145.0 (5.9) | 0.13 | 0.1-0.2 | 0.52 | 0.4-0.7 | ND | - | ND | - |
| NHL:follicular | | | | | | | | | | |
| WSU-FSCCL | 97.4 (4.9) | 7.7 (4.9) | 0.50 | 0.3-1.0 | 0.68 | 0.4-1.1 | ND | - | ND | - |
| | | | | | 1.05 | 0.8-1.4 | 0.83 | 0.6-1.1 | 1.17 | 0.8-1.7 |
| NHL:mantle cell | | | | | | | | | | |
| Jeko-1 | 604.6 (6.5) | 11.2 (6.5) | ND | - | 2.25 | 1.3-3.8 | 1.98 | 1.1-3.5 | 2.27 | 1.3-3.9 |
| ALL:B cell | | | | | | | | | | |
| REH | 12.3 (4.1) | 22.9 (4.1) | 0.47 | 0.3-0.9 | 1.22 | 0.8-1.9 | ND | - | ND | - |
| 697 | 6.9 (4.2) | 16.0 (4.2) | 2.23 | 1.3-3.9 | 2.67 | 1.7-3.7 | ND | - | ND | - |
| RS4;11 | 3.7 (4.1) | 23.3 (4.1) | 2.28 | 1.1-4.9 | 1.68 | 1.0-3.0 | ND | - | ND | - |
| MN-60 | 21.5 (5.8) | 10.3 (5.8) | 1.23 | 0.6-2.1 | 3.65 | 2.2-6.2 | ND | - | ND | - |

Abbreviations: CI, confidence interval; ND, not determined. [a]$EC_{50}$ expressed as mole equivalents of SN-38 in Emab-SN-38.

We again examined PARP cleavage and p21 expression, this time in cells treated with Emab-SN-38 + Vmab. Confirming the earlier study in Ramos, PARP cleavage first occurs only after a 48-hour exposure to the conjugate, with expression unchanged in the presence of a cross-linking antibody (not shown). Exposure to veltuzumab for more than 48 hours had no effect on PARP cleavage, but cleavage was strong within 24 hours when a cross-linking antibody was added (not shown). However, when veltuzumab alone (no cross-linker) was combined with Emab-SN-38, PARP cleavage occurred after a 24-hour exposure (not shown), indicating veltuzumab could induce a more rapid onset of apoptosis, even in the absence of cross-linking. The only notable difference in the WSU-FSCCL cell line was that the combination greatly enhanced p21 expression at 48 hours (not shown), again suggesting an acceleration of apoptosis induction when veltuzumab is combined with the Emab-SN-38 conjugate. The delay in apoptosis induction in WSU-FSCCL as compared with Ramos is likely explained by the lower expression of CD22 and CD20.

Ultratoxic agents often use linkers that are highly stable in serum, as their premature release would increase toxicity, but these conjugates must be internalized for the drug to be delivered optimally. Because epratuzumab internalizes rapidly, we examined whether it might benefit from a more stably linked SN-38, comparing in vitro cytotoxicity of the CL2A-linked Emab-SN-38 conjugate with the serum-stable CL2E-SN-38 conjugate. Both conjugates had a similar binding affinity (not shown), but the more stable Emab-CL2E-SN-38 was approximately 40- to 55-times less potent than the CL2A conjugate in 3 cell lines (not shown). While specificity was lacking with the CL2A conjugates, the Emab-CL2E-SN-38 consistently was approximately two times more potent than the nonbinding Lmab-anti-CEACAM5-CL2E-SN-38 conjugate (not shown). We concluded that it was unlikely that the more stably linked conjugate would be appropriate for a slowly internalizing veltuzumab conjugate and therefore continued our investigation only with CL2A-linked SN-38 conjugates.

Because of limitations of the *in vitro* assays, efficacy was assessed in xenograft models. As indicated in **Table 14,** all of the lymphoma cell lines have much higher expression of CD20 than CD22. Daudi had the highest expression of CD22 and CD20, but it is very sensitive *in vivo* to unconjugated veltuzumab and *in vitro* testing revealed the highest sensitivity to SN-38 **(Table 14).** These properties would likely make it difficult to assess differences in activity attributed to the SN-38 conjugate versus the unconjugated antibody, particularly when unconjugated epratuzumab is not an effective therapeutic in animals. Because Ramos had been used previously to show an advantage for combining $^{90}$Y-Emab with veltuzumab (Mattes et al., 2008, Clin Cancer Res 14:6154-60), we elected to start with a comparison of the Emab-SN-38 and Vmab-SN-38 conjugates in the Ramos human Burkitt cell line. Despite flow cytometry showing a 15-fold higher expression of CD20 over CD22, immunohistology of Ramos xenografts showed abundant CD22 and CD20, with CD22 seemingly expressed more uniformly than CD20 (not shown).

Ramos xenografts in untreated animals progressed rapidly, reaching the 3.0-cm$^3$ termination size from their starting size of 0.4 cm$^3$ within 6 days (not shown), and as reported previously, neither veltuzumab nor epratuzumab appreciably affected the progression of well-established Ramos xenografts (Sharkey et al., 2009, J Nucl Med 50:444-53). Consistent with previous findings using other SN-38 conjugates, none of the animals treated with a 4-week, twice-weekly, 0.5 mg/dose treatment regimen had appreciable weight loss. Both conjugates were highly effective in controlling tumor growth, with 80% or more of the animals having no evidence of tumor by the end of the 4-week treatment **(FIG. 12A-12D).** The 0.25-mg Vmab-SN-38 dose was better at controlling growth over the first 4 weeks, but at 0.5 mg, similar early growth control was observed for both conjugates. Thus, despite a 15-fold higher expression of CD20 than CD22, Emab-SN-38 compared favorably with Vmab-SN-38. Therefore, the remaining studies focused on Emab-SN-38 alone or in combination with unconjugated veltuzumab.

Emab-SN-38 dose-response and specificity. A dose-response relationship was seen for the specific Emab-SN-38 and irrelevant Lmab-SN-38 conjugates, but Emab-SN-38 had significantly better growth control at 2 of the 3 levels tested, and with a strong trend favoring the specific conjugate at the intermediate dose **(FIG. 13A-13C).** Again, 0.25 mg of Emab-SN-38 ablated a majority of the tumors; here, 7 of 10 animals were tumor-free at the end of the 12-week monitoring period, with no change in body weight. Animals given irinotecan alone (6.5 μg/dose; approximately the same SN-38 equivalents as 0.25 mg of conjugate) had a median survival of 1.9 weeks, with 3 of 11 animals tumor-free at the end of the study, which was not significantly different from the 3.45-week median survival for the irrelevant Lmab-SN-38 conjugate (P = 0.452; **FIG. 13C).**

In the 697-disseminated leukemia model, the median survival of saline-treated animals was just 17 days from tumor inoculation. Animals given unconjugated epratuzumab plus irinotecan (same mole equivalents of SN-38 as 0.5 mg of the conjugate) had the same median survival, whereas animals given 0.5 mg of Emab-SN-38 twice weekly starting 7 days from tumor inoculation survived to 24.5 days, significantly longer than untreated animals (P < 0.0001) or for unconjugated epratuzumab given with irinotecan (P = 0.016). However, Emab-SN-38 was not significantly better than the irrelevant conjugate (median survival = 22 days; P = 0.304), most likely reflecting the low expression of CD22 in this cell line.

Emab-SN-38 combined with unconjugated Vmab anti-CD20. We previously reported improved responses when $^{90}$Y-Emab was combined with unconjugated veltuzumab in the subcutaneous Ramos model (Mattes et al., 2008, Clin Cancer Res 14:6154-60) and thus this possibility was examined with Emab-SN-38. In a pilot study, 5 animals bearing

subcutaneous Ramos tumors averaging approximately 0.3 $cm^3$ were given veltuzumab (0.1 mg), 0.1 mg of Emab-SN-38, or Emab-SN-38 + Vmab (all agents given twice weekly for 4 weeks). The median TTP to 2.0 $cm^3$ was 22, 14, and more than 77 days, respectively (veltuzumab vs. Emab-SN-38 alone, $P = 0.59$; Emab-SN-38 + Vmab vs. Emab-SN-38, $P = 0.0145$), providing an initial indication that the combination of veltuzumab with Emab-SN-38 improved the overall therapeutic response. In a follow-up study that also used a twice-weekly, 4-week treatment regimen, 6 of 11 animals given 0.1 mg of Emab-SN-38 plus 0.1 mg of veltuzumab had no evidence of tumors 16 weeks from the start of treatment, whereas the median survival for animals receiving veltuzumab alone or with 0.1 mg of the control Lmab-SN-38 was 1.9 and 3.3 weeks, respectively, with 3 of 11 animals being tumor-free at 16 weeks in each of these groups (not shown). Despite the longer median TTP and more survivors, no significant differences were found between the groups. Thus, in the Ramos model, which has abundant CD20 and moderate levels of CD22, the Emab-SN-38 conjugate given at nontoxic dose levels was not significantly better than unconjugated anti-CD20 therapy, but the addition of Emab-SN-38 to unconjugated anti-CD20 therapy appeared to improve the response without toxicity. It is important to emphasize that the SN-38 conjugates are given at levels far less than their maximum tolerated dose, and therefore these results should not be interpreted that the unconjugated anti-CD20 therapy is equal to that of the Emab-SN-38 conjugate.

Two additional studies were conducted in an intravenous implanted model using the WSU-FSCCL follicular NHL cell line that has a low expression of CD20 and CD22 (not shown). The median survival time for saline-treated animals was 40 to 42 days from tumor implantation. Irinotecan alone (not shown), given at a dose containing the same SN-38 equivalents as 0.3 mg of the ADC, increased the median survival (49 vs. 40 days, respectively; $P = 0.042$), but 14 of 15 animals succumbed to disease progression on day 49, the same day the final 4 of 15 animals in the saline group were eliminated (not shown). Despite its relatively low CD20 expression, veltuzumab alone (35 $\mu$g twice weekly $\times$ 4 weeks) was effective in this model. The median survival increased to 91 days in the first study, with 2 cures (day 161), and to 77 days in the second, but with no survivors after 89 days (veltuzumab alone vs. saline-treated, $P < 0.001$ in both studies). Unconjugated epratuzumab (0.3 mg/dose) combined with irinotecan and veltuzumab had the same median survival as veltuzumab alone, suggesting that neither epratuzumab nor irinotecan contributed to the net response.

As expected because of the low CD22 expression by WSU-FSCCL, Emab-SN-38 alone was not as effective as in Ramos. At the 0.15-mg dose, no significant benefit over the saline group was seen, but at 0.3 mg, the median survival increased to 63 days, providing a significant improvement compared with the saline-treated animals ($P = 0.006$). The second study, using 0.3 mg of Emab-SN-38, confirmed an enhanced survival compared with the saline group (75 vs. 40 days; $P < 0.0001$). The specificity of this response was not apparent in the first study, where the median survival of the irrelevant Lmab-SN-38 conjugate and Emab-SN-38 were not different at either 0.15- or 0.3-mg dose levels (42 vs. 49 days and 63 vs. 63 days for the Emab-SN-38 vs. anti-CEACAM5-SN-38 conjugates at the 2 doses levels, respectively). However, in the second study, the 0.3-mg dose of Emab-SN-38 provided a significantly improved survival over the irrelevant conjugate (75 vs. 49 days; $P < 0.0001$). Again, the difficulty in showing specificity in this model is most likely related to low CD22 expression.

Combining the specific Emab-SN-38 with veltuzumab substantially increases survival, with evidence of more robust responses than the control Lmab-SN-38. For example, in the first study, animals treated with veltuzumab plus 0.15 or 0.3 mg of the control conjugate had a median survival of 98 and 91 days, respectively, which was similar to that of veltuzumab alone (91 days; not shown). However, veltuzumab plus 0.15 mg of the specific Emab-SN-38 conjugate increased the median survival to 140 days. While this improvement was not significantly higher than veltuzumab alone ($P = 0.257$), when the Emab-SN-38 dose was increased to 0.3 mg with veltuzumab, 6 of 10 animals remained alive at the end of the study, providing a significant survival advantage over the control conjugate plus veltuzumab ($P = 0.0002$). In a second study, the median survival of veltuzumab alone was shorter than in the first (77 vs. 91 days), yet the median survival for the control conjugate with veltuzumab was again 91 days, which now yielded a significant survival advantage over veltuzumab alone ($P < 0.0001$). Combining the specific Emab-SN-38 conjugate with veltuzumab extended the median survival to 126 days, which was significantly longer than the median survival of 75 and 77 days for Emab-SN-38 and veltuzumab alone, respectively (P < 0.0001 for each). However, in this study, it did not quite meet the requirements for a statistical improvement over the combination with control anti-CEACAM5-SN-38 conjugate ($P = 0.078$).

*Discussion*

Over the past 10 years, ADCs have made substantial gains in cancer therapy, yet there also have been some setbacks. The gains occurred largely when investigators chose to examine agents that were too toxic to be used alone, but when coupled to an antibody, these so-called ultratoxics produced substantially improved responses in preclinical testing. The recent approval of brentuximab vedotin, an auristatin conjugate, in Hodgkin lymphoma and the clinical success with trastuzumab-DM1 anti-HER2-maytansine conjugate as a single agent in breast cancer refractive to unconjugated trastuzumab suggest that these ADCs bearing ultratoxic agents are becoming accepted treatment modalities. However, conjugates prepared with agents that are themselves potent in the picomolar range can have an increased risk for toxicity, as the recent decision to withdraw gemtuzumab ozogamicin, the anti-CD33-calicheamicin conjugate,

from the market suggests (Ravandi, 2011, J Clin Oncol 29:349-51). Thus, the success of an ADC may depend on identifying appropriate chemistries to bind the drug and antibody together, as well as defining a suitable target that is sufficiently expressed to allow an adequate and selective delivery of the cytotoxic agent.

We developed a linker for coupling SN-38 to IgG that allows SN-38 to be released slowly from the conjugate in serum (about 50% per day). With this linker, an antibody that is slowly internalized could be an effective therapeutic, perhaps because the conjugate localized to a tumor releases a sufficient amount of drug locally, even without being internalized. The CL2A linker also was used recently with an antibody to Trop-2 that was reported to be internalized rapidly (Cardillo et al., 2011, Clin Cancer Res 17:3157-69.). Thus, it appears that the slow release mechanism is beneficial for internalizing and noninternalizing antibodies.

In this report, we expanded our assessment of the CL2A linker by comparing SN-38 conjugates prepared with epratuzumab, a rapidly internalizing anti-CD22 IgG, and veltuzumab, a slowly internalizing anti-CD20 IgG, for the treatment of B-cell malignancies, and we have now found combinations with DNA-breaking agents, such as microtubule inhibitors and PARP inhibitors that show synergistic anti-tumor effects. Prior studies with the murine parent of epratuzumab had indicated that most of the antibody internalizes within 1 hour and 50% of CD22 is reexpressed on the cell surface within 5 hours (Shih et al., 1994, Int J Cancer 56:538-45). This internalization and reexpression process would permit intracellular delivery that might compensate for lower surface expression of CD22. Because many of the B-cell malignancies express much more CD20 than CD22, a conjugate targeting CD20 might deliver more moles of drug by releasing its toxic payload after being localized in the tumor.

*In vitro* cytotoxicity studies could not discriminate the potency of the specific conjugates or even an irrelevant conjugate because of the release of SN-38 from the conjugate into the media. Indeed, SN-38 alone was somewhat more potent than the conjugates, which may reflect its accelerated ability to enter the cell and engage topoisomerase I. Because other studies revealed that the conjugates required a 48-hour exposure before early signs of apoptosis could be seen, we concluded that in vitro testing would not be able to discriminate the potency of these 2 conjugates and therefore resorted to *in vivo* studies.

In xenograft models, both conjugates had similar antitumor activity against Ramos tumors, which flow cytometry had indicated expressed nearly 15-fold more CD20 than CD22. This lent support to selecting the Emab anti-CD22-SN-38 conjugate especially because it could be combined with unconjugated Vmab anti-CD20 therapy without concern that either agent would interfere with the binding of the other agent. Indeed, if an anti-CD20-SN-38 conjugate were used, the total IgG protein dose given likely would be below a level typically needed for effective unconjugated anti-CD20 antibody treatments, as the dose-limiting toxicity would be driven by the SN-38 content. Adding more unlabeled anti-CD20 to an anti-CD20-SN-38 conjugate would risk reducing the conjugate's uptake and potentially diminishing its efficacy. However, as we showed previously in combination studies using radiolabeled epratuzumab with unconjugated veltuzumab, benefit can be derived from both agents given at their maximum effective and safe dosages. *In vitro* studies showed veltuzumab, even in the absence of cross-linking that is used to enhance signaling, accelerated apoptotic events initiated with Emab-SN-38. Thus, as long as the Emab-SN-38 conjugate was as effective as the anti-CD20 conjugate, selecting the Emab-SN-38 conjugate is a logical choice because it allows for a more effective combination therapy, even in tumors where one or both of the antigens are low in expression.

Because most ADCs using ultratoxic drugs are stably linked, we also tested a serum-stable, but intracellularly cleavable, anti-CD22-SN-38 conjugate, but determined it was 40-to 55-fold less potent than the CL2A linker. Others have examined a variety of ultratoxic drugs conjugated to anti-CD20 or anti-CD22 antibodies, finding that internalizing conjugates are generally more active, but also observing that even slowly internalizing antibodies could be effective if the released drug penetrated the cell membrane. While the CL2A-type linker may be appropriate for SN-38, it may not be optimal for a more toxic agent, where even a small, sustained release in the serum would increase toxicity and compromise the therapeutic window.

Emab-SN-38 was active at a cumulative dose of 0.6 mg in mice bearing Ramos (75 $\mu$g twice weekly for 4 weeks), which extrapolates to a human dose of just 2.5 mg/kg. Thus, Emab-SN-38 should have an ample therapeutic window in patients. Furthermore, an effective and safe dose of the anti-Trop-2-SN-38 conjugate was combined with a maximum tolerated dose of a $^{90}$Y-labeled antibody without an appreciable increase in toxicity but with improved efficacy (Sharkey et al., 2011, Mol Cancer Ther 10:1072-81). Thus, the safety and efficacy profile of these SN-38 antibody conjugates are very favorable for other combination therapies.

Even though irinotecan is not used routinely for the treatment of hematopoietic cancers, SN-38 was as potent in lymphoma and leukemia cell lines as in solid tumors (Cardillo et al., 2011, Clin Cancer Res 17:3157-69.). In the WSU-FSCCL cell line, the specific and irrelevant IgG conjugates were significantly better than irinotecan, whereas in Ramos, the median TTP with the irrelevant conjugate was longer but not significantly better than irinotecan. These results are consistent with other studies that have shown that a nonspecific IgG is an excellent carrier for drugs and more potent *in vivo* than free drug or conjugates prepared with albumin or polyethylene glycol (PEG)-Fc. While the PEG-SN-38 conjugate had significant antitumor effects, it was given at its maximum tolerated amounts, ranging from 10 to 30 mg/kg SN-38 equivalents (Sapra et al., 2009, Haematologica 94:1456-9). In contrast, the maximum cumulative dose of SN-38

given over 4 weeks to animals bearing Ramos was only 1.6 mg/kg (i.e., dosing of 0.25 mg of Emab-SN-38 given twice weekly over 4 weeks) and this was nontoxic.

The specific therapeutic activity of Emab-SN-38 appeared to improve in cell lines with higher CD22 expression. For example, in Ramos, specific therapeutic effects of Emab-SN-38 alone were recorded at 2 of the 3 different dose levels examined, and a sizeable number of tumors were completely ablated. In contrast, in WSU-FSCCL that had about 2.5-fold lower expression of CD22, Emab-SN-38 improved survival significantly compared with the irrelevant anti-CEACAM5-SN-38 conjugate in 1 of 2 studies. However, it is important to emphasize that when used in combination with unconjugated anti-CD20 therapy, Emab-SN-38 amplifies the therapeutic response. Thus, the combination of these two treatments could augment the response even in situations where CD22 is not highly expressed.

In conclusion, using the less-stable CL2A-SN-28 linker, Emab anti-CD22-SN-38 conjugate was equally active at nontoxic doses *in vivo* as a similar anti-CD20-SN-38 conjugate, despite the fact that CD20 expression was more than a log-fold higher than CD22. Therapeutic responses benefited by the combination of Emab-SN-38 with unconjugated Vmab anti-CD20 therapy, even when CD22 expression was low, suggesting that the combination therapy could improve responses in a number of B-cell malignancies when both antigens are present The current studies suggest that this combination is very potent in diverse lymphoma and leukemia preclinical models, yet appears to have less host toxicity. Also, combinations of this ADC with microtubule and PARP inhibitors can be synergistic in inhibiting tumor growth and extending survival of the tumor-bearing host.This result is surprising, in that it has been previously reported that the PARP inhibitor iniparib failed to sensitize cancer cells to combination therapy with standard anti-cancer agents, such as cisplatin, gemcitabine or paclitaxel (Bao et al., 2015, Oncotarget [Epub ahead of print, September 22, 2015])

**Example 16. Anti-CD74 (Milatuzumab) SN-38 Conjugates for Treatment of CD74+ Human Cancers**

*Abstract*

CD74 is an attractive target for antibody-drug conjugates (ADC), because it internalizes and recycles after antibody binding. CD74 mostly is associated with hematological cancers, but is expressed also in solid cancers. Therefore, the utility of ADCs prepared with the humanized anti-CD74 antibody, milatuzumab, for the therapy CD74-expressing solid tumors was examined. Milatuzumab-doxorubicin and two milatuzumab-SN-38 conjugates were prepared with cleavable linkers (CL2A and CL2E), differing in their stability in serum and how they release SN-38 in the lysosome. CD74 expression was determined by flow cytometry and immunohistology. *In vitro* cytotoxicity and *in vivo* therapeutic studies were performed in the human cancer cell lines A-375 (melanoma), HuH-7 and Hep-G2 (hepatoma), Capan-1 (pancreatic), and NCI-N87 (gastric), and Raji Burkitt lymphoma. The milatuzumab-SN-38 ADC was compared to SN-38 ADCs prepared with anti-Trop-2 and anti-CEACAM6 antibodies in xenografts expressing their target antigens.

Milatuzumab-doxorubicin was most effective in the lymphoma model, while in A-375 and Capan-1, only the milatuzumab-CL2A-SN-38 showed a therapeutic benefit. Despite much lower surface expression of CD74 than Trop-2 or CEACAM6, milatuzumab-CL2A-SN-38 had similar efficacy in Capan-1 as anti-Trop-2 CL2A-SN-38, but in NCI-N87, the anti-CEACAM6 and anti-Trop-2 conjugates were superior. Studies in 2 hepatoma cell lines at a single dose level showed significant benefit over saline-treated animals, but not against an irrelevant IgG conjugate. CD74 is a suitable target for ADCs in some solid tumor xenografts, with efficacy largely influenced by uniformity of CD74 expression, and with CL2A-linked SN-38 conjugates providing the best therapeutic responses.

*Introduction*

CD74, referred to as invariant chain or Ii, is a type II transmembrane glycoprotein that associates with HLA-DR and inhibits the binding of antigenic peptides to the class II antigen presentation structure. It serves as a chaperone molecule, directing the invariant chain complexes to endosomes and lysosomes, an accessory molecule in the maturation of B cells, using a pathway mediated by NF-kB, and in T-cell responses via interactions with CD44 (Naujokas et al., 1993, Cell 74:257-68), and it is a receptor for the pro-inflammatory cytokine, macrophage migration inhibitory factor (Leng et al., 2003, J Exp Med 197:1467-76), which is involved in activating cell proliferation and survival pathways.

In normal human tissues, CD74 is primarily expressed in B cells, monocytes, macrophages, dendritic cells, Langerhans cells, subsets of activated T cells, and thymic epithelium (not shown), and it is expressed in over 90% of B-cell tumors (Burton et al., 2004, Clin Cancer Res 10:6606-11; Stein et al., 2004, Blood 104:3705-11). Early studies had conflicting data on whether CD74 is present on the membrane, in part because the antibodies to the invariant chain were specific for the cytoplasmic portion of the molecule, but also because there are relatively few copies on the surface, and its half-life on the cell surface is very short. Approximately 80% of the CD74 on the cell surface is associated with the MHC II antigen HLA-DR (Roche et al., 1993, PNAS USA 90:8581-85). Using the murine anti-CD74 antibody, LL1, the Raji Burkitt lymphoma cell line was estimated to have $4.8 \times 10^4$ copies/cell, but because of rapid intracellular transit, $\sim 8 \times 10^6$ antibody molecules were internalized and catabolized per day (Hansen et al., 1996, Biochem J320:293-300).

Thus, CD74 internalization is highly dynamic, with the antibody being moved quickly from the surface and unloaded inside the cell, followed by CD74 re-expression on the surface. Fab' internalization occurs just as rapidly as IgG binding, indicating that bivalent binding is not required. Later studies with a CDR-grafted version of murine LL1, milatuzumab (hLL1), found that the antibody could alter B-cell proliferation, migration, and adhesion molecule expression (Stein et al., 2004, Blood 104:3705-11; Qu et al., 2002, Proc Am Assoc Cancer Res 43:255; Frolich et al., 2012, Arthritis Res Ther 14:R54), but the exceptional internalization properties of the anti-CD74 antibody made it an efficient carrier for the intracellular delivery of cancer therapeutics (e.g., Griffiths et al., 2003, Clin Cancer Res 9:6567-71). Based on preclinical efficacy and toxicology results, Phase I clinical trials with milatuzumab-doxorubicin in multiple myeloma (Kaufman et al., 2008, ASH Annual Meeting Abstracts, 112:3697), as well as non-Hodgkin lymphoma and chronic lymphocytic leukemia, have been initiated.

Interestingly, CD74 also is expressed in non-hematopoietic cancers, such as gastric, renal, urinary bladder, non-small cell lung cancers, certain sarcomas, and glioblastoma (e.g., Gold et al., 2010, Int J Clin Exp Pathol 4:1-12), and therefore it may be a therapeutic target for solid tumors expressing this antigen. Since a milatuzumab-doxorubicin conjugate was highly active in models of hematological cancers, it was a logical choice for this assessment. However, we recently developed procedures for coupling the highly potent topoisomerase I inhibitor, SN-38, to antibodies. SN-38 is the active form of irinotecan, whose pharmacology and metabolism are well known. These conjugates have nanomolar potency in solid tumor cell lines, and were found to be active with antibodies that were not actively internalized. Prior studies indicated a preference for a linker (CL2A) that allowed SN-38 to dissociate from the conjugate in serum with a half-life of ~1 day, rather than other linkers that were either more or less stable in serum. However, given milatuzumab's exceptional internalization capability, a new linker that is highly stable in serum, but can release SN-38 when taken into the lysosome, was developed.

The current investigation examines the prospects for using these three milatuzumab anti-CD74 conjugates, one with doxorubicin, and two SN-38 conjugates, for effective therapy primarily against solid tumors.

*Materials and Methods*

Human tumor cell lines. Raji Burkitt lymphoma, A-375 (melanoma), Capan-1 (pancreatic adenocarcinoma), NCI-N87 (gastric carcinoma), Hep-G2 hepatoma and MC/CAR myeloma cell lines were purchased from American Tissue Culture Collection (Manassas, VA). HuH-7 hepatoma cell line was purchased from Japan Health Science Research Resources Bank (Osaka, Japan). All cell lines were cultured in a humidified $CO_2$ incubator (5%) at 37 °C in recommended media containing 10% to 20% fetal-calf serum and supplements. Cells were passaged <50 times and checked regularly for mycoplasma.

Antibodies and conjugation methods. Milatuzumab (anti-CD74 MAb), epratuzumab (anti-CD22), veltuzumab (anti-CD20), labetuzumab (anti-CEACAM5), hMN15 (anti-CEACAM6), and hRS7 (anti-Trop-2) are humanized $IgG_1$ monoclonal antibodies. CL2A and CL2E linkers and their SN-38 derivatives were prepared and conjugated to antibodies as described in the Examples above. The milatuzumab-doxorubicin conjugates were prepared as previously described (Griffiths et al., 2003, Clin Cancer Res 9:6567-71). All conjugates were prepared by disulfide reduction of the IgG, followed by reaction with the corresponding maleimide derivatives of these linkers. Spectrophotometric analyses estimated the drug:IgG molar substitution ratio was 5-7 (1.0 mg of the protein contains ~16 μg of SN-38 or 25 μg of doxorubicin equivalent).

*In vitro* cell binding and cytotoxicity. Assays to compare cell binding of the unconjugated and conjugated milatuzumab to antigen-positive cells and cytotoxicity testing used the MTS dye reduction method (Promega, Madison, WI).

Flow cytometry and immunohistology. Flow cytometry was performed in a manner that provided an assessment of only membrane-bound or membrane and cytoplasmic antigen. Immunohistology was performed on formalin-fixed, paraffin-embedded sections of subcutaneous tumor xenografts, staining without antigen retrieval methods, using antibodies at 10 μg/mL that were revealed with an anti-human IgG conjugate.

*In vivo* studies. Female nude mice (4-8 weeks old) or female SCID mice (7 weeks old) were purchased from Taconic (Germantown, NY) and used after a 1-week quarantine. All agents, including saline controls, were administered intraperitoneally twice-weekly for 4 weeks. Specific doses are given in Results. Toxicity was assessed by weekly weight measurements. For the Raji Burkitt lymphoma model, SCID mice were injected intravenously with $2.5 \times 10^6$ Raji cells in 0.1 mL media. Five days later, animals received a single intravenous injection (0.1 mL) of the conjugate or saline (N = 10/group). Mice were observed daily for signs of distress and paralysis, and were euthanized when either hind-limb paralysis developed, >15% loss of initial weight, or if otherwise moribund (surrogate survival endpoints).

Subcutaneous tumors were measure by caliper in two dimensions, and the tumor volume (TV) calculated as $L \times w^2/2$, where $L$ is the longest diameter and w is the shortest. Measurements were made at least once weekly, with animals terminated when tumors grew to 1.0 $cm^3$ (i.e., surrogate survival end-point). The A-375 melanoma cell line ($6 \times 10^6$ cells in 0.2 mL) was implanted in nude mice and therapy was initiated when tumors averaged $0.23 \pm 0.06$ $cm^3$ (N = 8/group). Capan-1 was implanted subcutaneously in nude mice using a combination of tumor suspension from

serially-passaged tumors (0.3 mL of a 15% w/v tumor suspension) combined with $8\times10^6$ cells from tissue culture. Treatments were initiated when TV averaged $0.27 \pm 0.05$ $cm^3$ (N = 10/group). NCI-N87 gastric tumor xenografts were initiated by injecting 0.2 mL of a 1:1 (v/v) mixture of matrigel and $1\times10^7$ cells from terminal culture subcutaneously. Therapy was started when the TV averaged $0.249 \pm 0.045$ $cm^3$ (N = 7/group). The same procedure was followed for developing the Hep-G2 and HuH-7 hepatoma xenografts in nude mice. Therapy was started when Hep-G2 averaged $0.364 \pm 0.062$ $cm^3$ (N = 5/group) and HuH-7 averaged $0.298 \pm 0.055$ $cm^3$ (N = 5/group).

Efficacy is expressed in Kaplan-Meier survival curves, using the surrogate end-points mentioned above for determining the median survival times. Analysis was performed by a log-rank (Mantel-Cox) test using Prism GraphPad software (LaJolla, CA), with significance at $P$ <0.05.

*Results*

CD74 expression in human tumor cell lines and xenografts. Six cell lines derived from 4 different solid tumor types were identified as CD74-positive based primarily on the analysis of permeabilized cells (**Table 15**), since the MFI of membrane-only CD74 in the solid tumor cell lines very often was <2-fold higher than the background MFI (except A-375 melanoma cell line). Surface CD74 expression in Raji was >5-fold higher than the solid tumor cell lines, but total CD74 in permeabilized Raji cells was similar to most of the solid tumor cell lines.

**Table 15.** CD74 expression by flow cytometry expressed as mean fluorescent intensity (MFI) of milatuzumab-positive gated cells.

| Cell line | | Surface | | Surface and cytoplasmic | |
|---|---|---|---|---|---|
| | | **hLL1 (bkgd)[a]** | **MFI Ratio hLL1: bkgd** | **hLL1 (bkgd)[b]** | **MFI Ratio hLL1: bkgd** |
| Pane CA[c] | Capan-1 | 22 (12) | 1.8 | 248 (5) | 49.6 |
| Gastric | Hs746T | 17 (8) | 2.1 | 144 (5) | 28.8 |
| | NCI-N87 | 5 (4) | 1.3 | 220 (6) | 36.7 |
| Melanoma | A-375 | 16 (3) | 5.3 | 185 (6) | 30.8 |
| Hepatoma | Hep-G2 | 9 (6) | 1.5 | 156 (5) | 31.2 |
| | HuH-7 | 8 (5) | 1.6 | 114 (4) | 28.5 |
| Lymphoma | Raji | 59 (3) | 19.6 | 143 (5) | 28.6 |

ND, not done

[a]Background MFI of cells incubated with GAH-FITC only.

Immunohistology showed Raji subcutaneous xenografts had a largely uniform and intense staining, with prominent cell surface labeling (not shown). The Hep-G2 hepatoma cell line had the most uniform uptake of the solid tumors, with moderately strong, but predominantly cytoplasmic, staining (not shown), followed by the A-375 melanoma cell line that had somewhat less uniform staining with more intense, yet mostly cytoplasmic, expression (not shown). The Capan-1 pancreatic (not shown) and NCI-N87 (not shown) gastric carcinoma cell lines had moderate (Capan-1) to intense (NCI-N87) CD74 staining, but it was not uniformly distributed. The HuH-7 hepatoma cell line (not shown) had the least uniform and the weakest staining.

Immunoreactivity of the conjugates. $K_d$ values for unconjugated milatuzumab, milatuzumab-CL2A- and CL2E-SN-38 conjugates were not significantly different, averaging 0.77 nM, 0.59 nM, and 0.80 nM, respectively. $K_d$ values for the unconjugated and doxorubicin-conjugated milatuzumab measured in the MC/CAR multiple myeloma cell line were $0.5 \pm 0.02$ nM and $0.8 \pm 0.2$ nM, respectively (Sapra et al., 2008, Clin Cancer Res 14:1888-96).

*In vitro* drug release and serum stabilities of conjugates. The release mechanisms of SN-38 from the mercaptoethanol-capped CL2A and CL2E linkers were determined in an environment partially simulating lysosomal conditions, namely, low pH (pH 5.0), and in the presence or absence of cathepsin B. The CL2E-SN-38 substrate was inert at pH 5 in the absence of the enzyme (not shown), but in the presence of cathepsin B, cleavage at the Phe-Lys site proceeded quickly, with a half-life of 34 min (not shown). The formation of active SN-38 requires intramolecular cyclization of the carbamate bond at the $10^{th}$ position of SN-38, which occurred more slowly, with a half-life of 10.7 h (not shown).

As expected, cathepsin B had no effect on the release of active SN-38 in the CL2A linker. However, CL2A has a cleavable benzyl carbonate bond, releasing active SN-38 at a rate similar to the CL2E linker at pH 5.0, with a half-life

of ~ 10.2 h (not shown). The milatuzumab-doxorubicin conjugate, which has a pH-sensitive acylhydrazone bond, had a half-life of 7 to 8 h at pH 5.0 (not shown).

While all of these linkers release the drug at relatively similar rates under lysosomally-relevant conditions, they have very different stabilities in serum. Milatuzumab-CL2A-SN-38 released 50% of free SN-38 in 21.55 ± 0.17 h (not shown), consistent with other CL2A-SN-38 conjugates. The CL2E-SN-38 conjugate, however, was highly inert, with a half-life extrapolated to -2100 h. The milatuzumab-doxorubicin conjugate released 50% of the doxorubicin in 98 h, which was similar to 2 other antibody-doxorubicin conjugates (not shown).

Cytotoxicity. A significant issue related to the evaluation of these conjugates was the relative potency of free doxorubicin and SN-38 in hematopoietic and solid tumor cell lines. Our group previously reported that SN-38 was active in several B-cell lymphoma and acute leukemia cell lines, with potencies ranging from 0.13 to 2.28 nM (Sharkey et al., 2011, Mol Cancer Ther 11 :224-34). SN-38 potency in 4 of the solid tumor cell lines that were later used for *in vivo* therapy studies ranged from 2.0 to 6 nM (not shown). Doxorubicin had a mixed response, with 3-4 nM potency in the Raji lymphoma and the A-375 melanoma cell lines, but it was nearly 10 times less potent against Capan-1, NCI-N87, and Hep G2 cell lines. Other studies comparing the potency of SN-38 to doxorubicin found: LS174T colon cancer, 18 vs. 18 (nM potency of SN-38 vs. doxorubicin, respectively); MDA-MB-231 breast cancer, 2 vs. 2 nM; SK-OV-4 ovarian cancer, 18 vs. 90 nM; Calu-3 lung adenocarcinoma, 32 vs. 582 nM; Capan-2 pancreatic cancer, 37 vs. 221 nM; and NCI-H466 small cell lung cancer, 0.1 vs. 2 nM. Thus, SN-38 was 5- to 20-fold more potent than doxorubicin in 4 of these 6 cell lines, with similar potency in LS174T and MDA-MB-231. Collectively, these data indicate that doxorubicin is less effective against solid tumors than SN-38, while SN-38 appears to be equally effective in solid and hematopoietic tumors.

As expected, the 3 conjugate forms were often some order of magnitude less potent than the free drug *in vitro,* since both drugs are expected to be transported readily into the cells, while drug conjugates require antibody binding to transport drug inside the cell (not shown). The CL2A-linked SN-38 conjugate is an exception, since more than 90% of the SN-38 is released from the conjugate into the media over the 4-day assay period (Cardillo et al., 2011, Clin Cancer Res 17:3157-69; Sharkey et al., 2011, Mol Cancer Ther 11:224-34). Thus, even if the conjugate was internalized rapidly, it would be difficult to discern differences between the free drug and the CL2A-linked drug.

The stable CL2E-linked SN-38 performed comparatively well in the Raji cell line, compared to free SN-38, but it had substantially (7- to 16-fold) lower potency in the 4 solid tumor cell lines, suggesting the relatively low surface expression of CD74 may be playing a role in minimizing drug transport in these solid tumors. The milatuzumab-doxorubicin conjugate had substantial differences in its potency when compared to the free doxorubicin in all cell lines, which was of similar magnitude as the CL2E-SN-38 conjugates to free SN-38 in the solid tumor cell lines.

In the 6 additional cell lines mentioned above, the milatuzumab-CL2A-SN-38 conjugate was 9- to 60-times more potent than the milatuzumab-doxorubicin conjugate (not shown), but again, this result was influenced largely by the fact that the CL2A-linked conjugate releases most of its SN-38 into the media over the 4-day incubation period, whereas the doxorubicin conjugate would at most release 50% of its drug over this same time. The CL2E-linked milatuzumab was not examined in these other cell lines.

*In vivo* therapy of human tumor xenografts. Previous *in vivo* studies with the milatuzumab-doxorubicin or SN-38 conjugates prepared with various antibodies had indicated they were efficacious at doses far lower than their maximum tolerated dose (Griffiths et al., 2003, Clin Cancer Res 9:6567-71; Sapra et al., 2005, Clin Cancer Res 11:5257-64; Govindan et al., 2009, Clin Cancer Res 15:6052-61; Cardillo et al., 2011, Clin Cancer Res 17:3157-69; Sharkey et al., 2011, Mol Cancer Ther 11:224-34), and thus *in vivo* testing focused on comparing similar, but fixed, amounts of each conjugate at levels that were well-tolerated.

Initial studies first examined the doxorubicin and SN-38 conjugates in a disseminated Raji model of lymphoma in order to gauge how the milatuzumab-doxorubicin conjugate compared to the 2 SN-38 conjugates (not shown). All specific conjugates were significantly better than non-targeting labetuzumab-SN-38 conjugate or saline-treated animals, which had a median survival of only 20 days (P <0.0001). Despite *in vitro* studies indicating as much as an 8-fold advantage for the SN-38 conjugates in Raji, the best survival was seen with the milatuzumab-doxorubicin conjugates, where all animals given a single 17.5 mg/kg (350 μg) dose and 7/10 animals given 2.0 mg/kg (40 μg) were alive at the conclusion of the study (day 112) (e.g., 17.5 mg/kg dose milatuzumab-doxorubicin vs. milatuzumab-CL2A-SN-38, P = 0.0012). Survival was significantly lower for the more stable CL2E-SN-38 conjugates (P< 0.0001 and *P* = 0.0197, 17.5 and 2.0 mg/kg doses for the CL2A vs. CL2E, respectively), even though *in vitro* studies suggested that both conjugates would release active SN-38 at similar rates when internalized.

Five solid tumor cell lines were examined, starting with the A-375 melanoma cell line, since it had the best *in vitro* response to both doxorubicin and SN-38. A-375 xenografts grew rapidly, with saline-treated control animals having a median survival of only 10.5 days (not shown). A 12.5 mg/kg (0.25 mg per animal) twice-weekly dose of the milatuzumab-CL2A-SN-38 conjugate extended survival to 28 days (*P* = 0.0006), which was significantly better than the control epratuzumab-CL2A-SN-38 conjugate having a median survival of 17.5 days (*P* = 0.0089), with the latter not being significantly different from the saline-treated animals (*P* = 0.1967). The milatuzumab-CL2A conjugate provided significantly longer survival than the milatuzumab-CL2E-SN-38 conjugate (*P* = 0.0014), which had the same median survival of 14 days as

its control epratuzumab-CL2E-SN-38 conjugate. Despite giving a 2-fold higher dose of the milatuzumab-doxorubicin than the SN-38 conjugates, the median survival was no better than the saline-treated animals (10.5 days).

As with the A-375 melanoma model, in Capan-1, only the CL2A-linked SN-38 conjugate was effective, with a median survival of 35 days, significantly different from untreated animals (*P* <0.036) (not shown), even at a lower dose (5 mg/kg; 100 μg per animal) (*P*<0.02). Neither the milatuzumab-CL2E nor the non-targeting epratuzumab-CL2A-SN-38 conjugates, or a 2-fold higher dose of the milatuzumab-doxorubicin conjugate, provided any survival advantage (*P* = 0.44 vs. saline). It is noteworthy that in the same study with animals given the same dose of the internalizing anti-Trop-2 CL2A-SN-38 conjugate (hRS7-SN-38; IMMU-132), the median survival was equal to milatuzumab-CL2A-SN-38 (not shown). The hRS7-CL2A-SN-38 conjugate had been identified previously as an ADC of interest for treating a variety of solid tumors (Cardillo et al., 2011, Clin Cancer Res 17:3157-69). The MFI for surface-binding hRS7 on Capan-1 was 237 (not shown), compared to 22 for milatuzumab (see **Table 15).** Thus, despite having a substantially lower surface antigen expression, the milatuzumab-CL2A-SN-38 conjugate performed as well as the hRS7-CL2A-SN-38 conjugate in this model.

With the milatuzumab-doxorubicin conjugate having inferior therapeutic results in 2 of the solid tumor xenografts, the focus shifted to compare the milatuzumab-SN-38 conjugates to SN-38 conjugates prepared with other humanized antibodies against Trop-2 (hRS7) or CEACAM6 (hMN-15), which are more highly expressed on the surface of many solid tumors (Blumenthal et al., 2007, BMC Cancer 7:2; Stein et al., 1993, Int J Cancer 55:938-46). Three additional xenograft models were examined.

In the gastric tumor model, NCI-N87, animals given 17.5 mg/kg/dose (350 μg) of milatuzumab-CL2A-SN-38 provided some improvement in survival, but it failed to meet statistical significance compared to the saline-treated animals (31 vs. 14 days; *P* = 0.0760) or to the non-binding veltuzumab anti-CD20-CL2A-SN39 conjugate (21 days; *P* = 0.3128) (not shown). However, the hRS7- and hMN-15-CL2A conjugates significantly improved the median survival to 66 and 63 days, respectively (*P* = 0.0001). The MFI for surface-expressed Trop-2 and CEACAM6 were 795 and 1123, respectively, much higher than CD74 that was just 5 (see **Table 15).** Immunohistology showed a relatively intense cytoplasmic expression of CD74 in the xenograft of this cell line, but importantly it was scattered, appearing only in defined pockets within the tumor (not shown). CEACAM6 and Trop-2 were more uniformly expressed than CD74 (not shown), with CEACAM6 being more intensely present both cytoplasmically and on the membrane, and Trop-2 primarily found on the membrane. Thus, the improved survival with the anti-CEACAM6 and anti-Trop-2 conjugates most likely reflects both higher antigen density and more uniform expression in NCI-N87.

In the Hep-G2 hepatoma cell line (not shown), immunohistology showed a very uniform expression with moderate cytoplasmic staining of CD74, and flow cytometry indicated a relatively low surface expression (MFI = 9). The MFI with hMN-15 was 175 and immunohistology showed a fairly uniform membrane and cytoplasmic expression of CEACAM6, with isolated pockets of very intense membrane staining (not shown). A study in animals bearing Hep-G2 xenografts found the milatuzumab-CL2A-SN-38 extended survival to 45 days compared to 21 days in the saline-treated group (P = 0.0048), while the hMN-15-CL2A-SN-38 conjugate improved survival to 35 days. There was a trend favoring the milatuzumab conjugate over hMN-15-CL2A-SN-38, but it did not achieve statistical significance (46 vs. 35 days; *P* = 0.0802). However, the non-binding veltuzumab-CL2A-SN-38 conjugate provided a similar survival advantage as the milatuzumab conjugate. We previously observed therapeutic results with non-binding conjugates could be similar to the specific CL2A-linked conjugate, particularly at higher protein doses, but titration of the specific and control conjugates usually revealed selectively. Thus, neither of the specific conjugates provided a selective therapeutic advantage at these doses in this cell line.

Another study using the HuH-7 hepatoma cell line (not shown), which had similar surface expression, but slightly lower cytoplasmic levels as Hep-G2 (see **Table 15),** found the hMN-15-SN-38 conjugate providing a longer (35 vs. 18 days), albeit not significantly different, survival advantage than the milatuzumab-CL2A conjugate (*P* = 0.2944). While both the hMN-15 and milatuzumab conjugates were significantly better than the saline-treated animals (*P* = 0.008 and 0.009, respectively), again, neither conjugate was significantly different from the non-targeted veltuzumab-SN-38 conjugate at this dose level (*P* = 0.4602 and 0.9033, respectively). CEACAM6 surface expression was relatively low in this cell line (MFI = 81), and immunohistology showed that both CD74 (not shown) and CEACAM6 (not shown) were very faint and highly scattered.

*Discussion*

The antibody-drug conjugate (ADC) approach for tumor-selective chemotherapy is an area of considerable current interest (e.g., Govindan et al., 2012, Expert Opin Biol Ther 12:873-90; Sapra et al., 2011, Expert Opin Biol Ther 20:1131-49. The recent clinical successes (Pro et al., 2012, Expert Opin Biol Ther 12:1415-21; LoRusso et al., 2011, Clin Cancer Res 17:437-47) have occurred in a large part with the adoption of supertoxic drugs in place of the conventional chemotherapeutic agents that had been used previously. However, target selection, the antibody, and the drug linker are all factors that influence optimal performance of an ADC. For example, in the case of trastuzumab-DM1, HER2 is

abundant on tumors expressing this antigen, the antibody is internalized, and the antibody itself has anti-tumor activity, all of which could combine to enhance therapeutic outcome. In stark contrast, CD74 is expressed at a much lower level on the surface of cells, but its unique internalization and surface re-expression properties has allowed a milatuzumab anti-CD74 ADC to be effective in hematopoietic cancer xenograft models even with a moderately toxic drug, such as doxorubicin (Griffiths et al., 2003, Clin Cancer Res 9:6567-71; Sapra et al., 2005, Clin Cancer Res 11:5257-64). Although doxorubicin is used more frequently in hematopoietic cancers, while SN-38 and other camptothecins are administered to patients with solid tumors, we decided to assess the utility of doxorubicin and SN-38 conjugates of milatuzumab in solid tumors. The milatuzumab-doxorubicin conjugate was effective in xenograft models of various hematological cancers, leading to its clinical testing (NCT01101594 and NCT01585688), while several SN-38 conjugates were effective in solid and hematological tumor models, leading to 2 new SN-38 conjugates being pursued in Phase I clinical trials of colorectal and diverse epithelial cancers (NCT01270698 and NCT01631552).

*In vitro,* unconjugated doxorubicin and SN-38 had similar potency as doxorubicin against the Raji lymphoma cell line, but SN-38 was more potent in a number of different solid tumor cell lines. Interestingly, *in vivo,* the milatuzumab-doxorubicin conjugate provided the best response in Raji as compared to the milatuzumab-SN-38 conjugates. However, in Capan-1 and A-375, milatuzumab-doxorubicin was less effective than the CL2A-linked SN-38 milatuzumab conjugate, even though *in vitro* testing had indicated that A-375 was equally sensitive to free doxorubicin as to free SN-38. Two other cell lines, MDA-MB-231 breast cancer and LS174T colon cancer, also had similar potency with free doxorubicin as SN-38 *in vitro,* but since *in vitro* testing indicated SN-38 was equally effective in solid and hematological cancers, and with SN-38 having a 5- to 20-fold higher potency than doxorubicin in most solid tumor cell lines evaluated, we decided to focus on the 2 milatuzumab-SN-38 conjugates for solid tumor therapy. However, to better gauge the utility of the milatuzumab-SN-38 conjugates, we included a comparative assessment to SN-38 ADCs prepared with antibodies against other antigens that are present in a variety of solid tumors.

We previously had investigated therapeutic responses with the internalizing hRS7 anti-Trop-2 CL2A-linked SN-38 conjugate in the Capan-1 cell line (Cardillo et al., 2011, Clin Cancer Res 17:3157-69) , and therefore the efficacy of milatuzumab and hRS7 SN-38 conjugates were compared. In this study, both conjugates significantly improved survival compared to control antibodies, with the CL2A-linked SN-38 conjugates of each being superior to the CL2E-linked conjugates. Since flow cytometry had indicated Trop-2 expression was higher than CD74 in Capan-1, this result suggested that the transport capabilities of CD74, which were known to be exceptional, were more efficient than Trop-2. However, it is well known that antigen accessibility (i.e., membrane vs. cytoplasm, physiological and "binding-site" barriers) and distribution among cells within a tumor are critical factors influencing every form of targeted therapy, particularly those that depend on adequate intracellular delivery of a product to individual cells (Thurber et al., 2008, Adv Drug Del Rev 60: 1421-34). In situations where the antigen is not uniformly expressed in all cells within the tumor, having a targeted agent that slowly releases its payload after localizing in the tumor, such as the CL2A-linked conjugates, would allow the drug to diffuse to nontargeted bystander cells, thereby enhancing its efficacy range. Indeed, high antigen expression could potentially impede tumor penetration as per the binding-site barrier effect, but the extracellular release mechanism could provide a mechanism for the drug to diffuse within the tumor. This mechanism also is thought to aid the efficacy of other conjugates that we have examined using poorly internalizing antibodies, such as anti-CEACAM5 and the anti-CEACAM6 used herein. Conjugates based on milatuzumab rely more heavily on the antibody's direct interaction with the tumor cell, taking advantage of CD74's rapid internalization and re-expression that can compensate for its lower abundance on the surface of cells. However, this advantage would be mitigated when CD74 is highly scattered within the tumor, and without a mechanism to retain the conjugate within the tumor, the benefit of the drug's slow release from the conjugate would be lost. A previous review of human gastrointestinal tumors by our group suggests that they often have a high level of expression with good uniformity (Gold et al., 2010, Int J Clin Exp Pathol 4:1-12).

## Example 17. Use of hRS7-SN-38 (IMMU-132) to treat therapy-refractive metastatic breast cancer

The patient was a 57-year-old woman with stage IV, triple-negative, breast cancer (ER/PR negative, HER-neu negative), originally diagnosed in 2005. She underwent a lumpectomy of her left breast in 2005, followed by Dose-Dense ACT in adjuvant setting in September 2005. She then received radiation therapy, which was completed in November. Local recurrence of the disease was identified when the patient palpated a lump in the contralateral (right) breast in early 2012, and was then treated with CMF (cyclophosphamide, methotrexate, 5-fluorouracil) chemotherapy. Her disease recurred in the same year, with metastatic lesions in the skin of the chest wall. She then received a carboplatin + TAXOL® chemotherapy regimen, during which thrombocytopenia resulted. Her disease progressed and she was started on weekly doxorubicin, which was continued for 6 doses. The skin disease also was progressing. An FDG-PET scan on 09/26/12 showed progression of disease on the chest wall and enlarged, solid, axillary nodes. The patient was given oxycodone for pain control.

She was given IXEMPRA® from October 2012 until February 2013 (every 2 weeks for 4 months), when the chest wall lesion opened up and bled. She was then put on XELODA®, which was not tolerated well due to neuropathy

in her hands and feet, as well as constipation. The skin lesions were progressive and then she was enrolled in the IMMU-132 trial after giving informed consent. The patient also had a medical history of hyperthyroidism and visual disturbances, with high risk of CNS disease (however, brain MRI was negative for CNS disease). At the time of enrollment to this trial, her cutaneous lesions (target) in the right breast measured 4.4 cm and 2.0 cm in the largest diameter. She had another non-target lesion in the right breast and one enlarged lymph node each in the right and left axilla.

The first IMMU-132 infusion (12 mg/kg) was started on March 12, 2013, which was tolerated well. Her second infusion was delayed due to Grade 3 absolute neutrophil count (ANC) reduction (0.9) on the scheduled day of infusion, one week later. After a week delay and after receiving NEULASTA®, her second IMMU-132 was administered, with a 25% dose reduction at 9 mg/kg. Thereafter she has been receiving IMMU-132 on schedule as per protocol, once weekly for 2 weeks, then one week off. Her first response assessment on May 17, 2013, after 3 therapy cycles, showed a 43% decrease in the sum of the long diameter of the target lesions, constituting a partial response by RECIST criteria. She is continuing treatment at the 9 mg/kg dose level. Her overall health and clinical symptoms improved considerably since she started treatment with IMMU-132.

**Example 18. Use of hRS7-SN-38 (IMMU-132) to treat refractory, metastatic, non-small cell lung cancer**

This is a 60-year-old man diagnosed with non-small cell lung cancer. The patient is given chemotherapy regimens of carboplatin, bevacizumab for 6 months and shows a response, and then after progressing, receives further courses of chemotherapy with carboplatin, etoposide, TAXOTERE®, gemcitabine over the next 2 years, with occasional responses lasting no more than 2 months. The patient then presents with a left mediastinal mass measuring 6.5 x 4 cm and pleural effusion.

After signing informed consent, the patient is given IMMU-132 at a dose of 18 mg/kg every other week. During the first two injections, brief periods of neutropenia and diarrhea are experienced, with 4 bowel movements within 4 hours, but these resolve or respond to symptomatic medications within 2 days. After a total of 6 infusions of IMMU-132, CT evaluation of the index lesion shows a 22% reduction, just below a partial response but definite tumor shrinkage. The patient continues with this therapy for another two months, when a partial response of 45% tumor shrinkage of the sum of the diameters of the index lesion is noted by CT, thus constituting a partial response by RECIST criteria.

**Example 19. Use of hRS7-SN-38 (IMMU-132) plus olaparib to treat refractory, metastatic, small-cell lung cancer**

This is a 65-year-old woman with a diagnosis of small-cell lung cancer, involving her left lung, mediastinal lymph nodes, and MRI evidence of a metastasis to the left parietal brain lobe. Prior chemotherapy includes carboplatin, etoposide, and topotecan, but with no response noted. Radiation therapy also fails to control her disease. She is then given combination therapy with IMMU-132 plus olaparib on a 21-day cycle. Olaparib is administered at 200 mg twice a day on days 1-10 of the cycle. IMMU-132 is administered at 8 mg/kg on days 1 and 8 of the cycle. After 3 cycles, there is a 31% shrinkage of sum of the longest diameters of the lung and lymph node tumors by CT, while the putative brain metastasis is no longer detected. This constitutes a PR by RECIST 1. 1, because the shrinkage is confirmed 4 weeks later (35% shrinkage of the sum of all target lesions. She continues her IMMU-132 dosing every 3 weeks for another 3 months, and continues to show objective and subjective improvement of her condition.

**Example 20. Therapy of a gastric cancer patient with Stage IV metastatic disease with hRS7-SN-38 (IMMU-132) plus paclitaxel**

This patient is a 60-year-old male with a history of smoking and periods of excessive alcohol intake over a 40-year-period. He experiences weight loss, eating discomfort and pain not relieved by antacids, frequent abdominal pain, lower back pain, and most recently palpable nodes in both axilla. He seeks medical advice, and after a workup is shown to have an adenocarcinoma, including some squamous features, at the gastro-esophageal junction, based on biopsy via a gastroscope. Radiological studies (CT and FDG-PET) also reveal metastatic disease in the right and left axilla, mediastinal region, lumbar spine, and liver (2 tumors in the right lobe and 1 in the left, all measuring between 2 and 4 cm in diameter). His gastric tumor is resected and he is then put on a course of chemotherapy with epirubicin, cisplatin, and 5-fluorouracil. After 4 months and a rest period of 6 weeks, he is switched to docetaxel chemotherapy, which also fails to control his disease, based on progression confirmed by CT measurements of the metastatic tumors and some general deterioration.

The patient is then given combination therapy with IMMU-132 (hRS7-SN-38) and paclitaxel on a 21 day cycle. Paclitaxel is administered at a dosage of 175 $mg/m^2$ on days 1, 7 and 14 of the cycle. IMMU-132 is administered at 10 mg/kg on days 1 and 8 of the cycle. After 3 cycles CT studies are done to assess status of his disease. The infusions are tolerated well, with some mild nausea and diarrhea, and also with Grade 3 neutropenia, controlled with symptomatic medications and with G-CSF (Neulasta®) for the neutropenia. The CT studies reveal that the sum of his index metastatic

lesions has decreased by 28%, so he continues on this therapy for another 5 cycles. Follow-up CT studies show that the disease remains about 35% reduced by RECIST criteria from his baseline measurements prior to combination therapy, and his general condition also appears to have improved, with the patient regaining an optimistic attitude toward his disease being under control.

**Example 21. Therapy of advanced colon cancer patient refractory to prior chemoimmunotherapy, using only IMMU-130 (labetuzumab-SN-38)**

The patient is a 50-year-old man with a history of stage-IV metastatic colonic cancer, first diagnosed in 2008 and given a colectomy and partial hepatectomy for the primary and metastatic colonic cancers, respectively. He then received chemotherapy, as indicated **FIG. 14,** which included irinotecan, oxaliplatin, FOLFIRINOX (5-fluoruracil, leucovorin, irinotecan, oxaliplatin), and bevacizumab, as well as bevacizumab combined with 5-fluorouracil/leucovorin, for almost 2 years. Thereafter, he was given courses of cetuximab, either alone or combined with FOLFIRI (leucovorin, 5-flurouracil, irinotecan) chemotherapy during the next year or more. In 2009, he received radiofrequency ablation therapy to his liver metastasis while under chemo-immunotherapy, and in late 2010 he underwent a wedge resection of his lung metastases, which was repeated a few months later, in early 2011. Despite having chemo-immunotherapy in 2011, new lung metastases appeared at the end of 2011, and in 2012, both lung and liver metastases were visualized. His baseline plasma carcinoembryonic antigen (CEA) titer was 12.5 ng/mL just before undergoing the antibody-drug therapy with IMMU-130. The index lesions chosen by the radiologist for measuring tumor size change by computed tomography were the mid-lobe of the right lung and the liver metastases, both totaling 91 mm as the sum of their longest diameters at the baseline prior to IMMU-130 (anti-CEACAM5-SN-38) therapy.

This patient received doses of 16 mg/kg of IMMU-130 by slow IV infusion every other week for a total of 17 treatment doses. The patient tolerated the therapy well, having only a grade 1 nausea, diarrhea and fatigue after the first treatment, which occurred after treatments 4 and 5, but not therafter, because he received medication for these side-effects. After treatment 3, he did show alopecia (grade 2), which was present during the subsequent therapy. The nausea, diarrhea, and occasional vomiting lasted only 2-3 days, and his fatigue after the first infusion lasted 2 weeks. Otherwise, the patient tolerated the therapy well. Because of the long duration of receiving this humanized (CDR-grafted) antibody conjugated with SN-38, his blood was measured for anti-labetuzumab antibody, and none was detected, even after 16 doses.

The first computed tomography (CT) measurements were made after 4 treatments, and showed a 28.6% change from the sum of the measurements made at baseline, prior to this therapy, in the index lesions. After 8 treatments, this reduction became 40.6%, thus constituting a partial remission according to RECIST criteria. This response was maintained for another 2 months, when his CT measurements indicated that the index lesions were 31.9% less than the baseline measurements, but somewhat higher than the previous decrease of 40.6% measured. Thus, based on careful CT measurements of the index lesions in the lung and liver, this patient, who had failed prior chemotherapy and immunotherapy, including irinotecan (parent molecule of SN-38), showed an objective response to the active metabolite of irintotecan (or camptotechin), SN-38, when targeted via the anti-CEACAM5 humanized antibody, labetuzumab (hMN-14). It was surprising that although irinotecan (CPT-11) acts by releasing SN-38 *in vivo,* the SN-38 conjugated anti-CEACAM5 antibody proved effective in a colorectal cancer patient by inducing a partial response after the patient earlier failed to respond to his last irinotecan-containing therapy. The patient's plasma CEA titer reduction also corroborated the CT findings: it fell from the baseline level of 12.6 ng/mL to 2.1 ng/mL after the third therapy dose, and was between 1.7 and 3.6 ng/mL between doses 8 and 12. The normal plasma titer of CEA is usually considered to be between 2.5 and 5.0 ng/mL, so this therapy effected a normalization of his CEA titer in the blood.

**Example 22. Therapy of a patient with advanced colonic cancer with IMMU-130 plus eribulin mesylate**

This patient is a 75-year-old woman initially diagnosed with metastatic colonic cancer (Stage IV). She has a right partial hemicolectomy and resection of her small intestine and then receives FOLFOX, FOLFOX + bevacizumab, FOLFIRI + ramucirumab, and FOLFIRI + cetuximab therapies for a year and a half, when she shows progression of disease, with spread of disease to the posterior cul-de-sac, omentum, with ascites in her pelvis and a pleural effusion on the right side of her chest cavity. Her baseline plasma CEA titer just before this therapy is 15 ng/mL. She is given combination therapy with eribulin mesylate (1.4 mg/m$^2$) and 10 mg/kg IMMU-130 (anti-CEACAM5-SN-38), both administered on days 1 and 8 of a 21 day cycle, which is tolerated very well, without any major hematological or non-hematological toxicities. Within 2 months of therapy, her plasma CEA titer shrinks modestly to 1.3 ng/mL, but at the 8-week evaluation she shows a 21% shrinkage of the index tumor lesions, which increases to a 27% shrinkage at 13 weeks. Surprisingly, the patient's ascites and pleural effusion both decrease (with the latter disappearing) at this time, thus improving the patient's overall status remarkably. The patient continues her investigational therapy.

**Example 23. Gastric cancer patient with Stage IV metastatic disease treated with IMMU-130 plus rucaparib**

The patient is a 52-year-old male who sought medical attention because of gastric discomfort and pain related to eating for about 6 years, and with weight loss during the past 12 months. Palpation of the stomach area reveals a firm lump which is then gastroscoped, revealing an ulcerous mass at the lower part of his stomach. This is biopsied and diagnosed as a gastric adenocarcinoma. Laboratory testing reveals no specific abnormal changes, except that liver function tests, LDH, and plasma CEA are elevated, the latter being 10.2 ng/mL. The patient then undergoes a total-body PET scan, which discloses, in addition to the gastric tumor, metastatic disease in the left axilla and in the right lobe of the liver (2 small metastases). The patient has his gastric tumor resected, and then has baseline CT measurements of his metastatic tumors. Four weeks after surgery, he receives 3 courses of combination chemotherapy consisting of a regimen of cisplatin and 5-fluorouracil (CF), but does not tolerate this well, so is switched to treatment with docetaxel. It appears that the disease is stabilized for about 4 months, based on CT scans, but then the patient's complaints of further weight loss, abdominal pain, loss of appetite, and extreme fatigue cause repeated CT studies, which show increase in size of the metastases by a sum of 20% and a suspicious lesion at the site of the original gastric resection.

The patient is then given combination therapy with IMMU-130 (anti-CEACAM5-SN-38, 8 mg/kg) and the PARP inhibitor rucaparib (12 mg/m$^2$) on a weekly schedule. He tolerates this well, but after 3 weeks shows a grade 3 neutropenia and grade 1 diarrhea. His fourth infusion is postponed by one week, and then the weekly infusions are reinstituted, with no evidence of diarrhea or neutropenia for the next 4 injections. The patient then undergoes a CT study to measure his metastatic tumor sizes and to view the original area of gastric resection. The radiologist measures, according to RECIST criteria, a decrease of the sum of the metastatic lesions, compared to baseline prior to combination therapy, of 23%. There does not seem to be any clear lesion in the area of the original gastric resection. The patient's plasma CEA titer at this time is 7.2 ng/mL, which is much reduced from the baseline value of 14.5 ng/mL. The patient continues on weekly combination therapy at the same dosages, and after a total of 13 infusions, his CT studies show that one liver metastasis has disappeared and the sum of all metastatic lesions is decreased by 41%, constituting a partial response by RECIST. The patient's general condition improves and he resumes his usual activities while continuing to receive a maintenance therapy of 8 mg/kg IMMU-130 every third week for another 4 injections. At the last measurement of blood CEA, the value is 4.8 ng/mL, which is within the normal range for a smoker, which is the case for this patient. HAHA serum measurements do not disclose an anti-antibody or anti-SN-38 antibodies, so the therapy appears not to be immunogenic.

**Example 24. Therapy of relapsed triple-negative metastatic breast cancer with IMMU-130 plus veliparib**

A 58-year-old woman with triple-negative metastatic breast cancer formerly treated with bevacizumab plus paclitaxel, without response, presents with metastases to several ribs, lumbar vertebrae, a solitary lesion measuring 3 cm in diameter in her left lung, with considerable bone pain and fatigue. She is given combination therapy with the anti-CEACAM5 IMMU-130 (hMN-14-SN-48) plus veliparib. IMMU-130 is administered at 12 mg/kg on days 1 and 8 of a 21-day cycle, while veliparib is administered once daily at 60 mg. Except for transient grade 2 neutropenia and some initial diarrhea, she tolerates the therapy well, which is then repeated, after a rest of 2 months, for another course. Radiological examination indicates that she has partial response by RECIST criteria, because the sum of the diameters of the index lesions decrease by 39%. Her general condition, including bone pain, also improves, and she returns to almost the same level of activity as prior to her illness.

**Example 25. Therapy of relapsed, generally refractive, metastatic colonic carcinoma with hMN-15-SN-38 plus paclitaxel**

A 46-year-old woman has Stage IV metastatic colonic cancer, with a prior history of resection of the primary lesion that also had synchronous liver metastases to both lobes of the liver, as well as a single focus of spread to the right lung; these metastases measured, by CT, between 2 and 5 cm in diameter. She undergoes various courses of chemotherapy over a period of 3 years, including 5-fluorouracil, leucovorin, irinotecan, oxaliplatin, cetuximab, and bevacizumab. On two occasions, there is evidence of stabilization of disease or a short-term response, but no reduction of 30% or more of her measured lesions. Her plasma CEA titer at baseline prior to combination therapy is 46 ng/mL, and her total index lesions measure a sum of 92 mm.

Combination therapy with hMN-15-SN-38 and paclitaxel is instituted on a 21-day cycle. hMN-15-SN-38 is administered at 12 mg/kg on days 1 and 8 and paclitaxel at a dosage of 135 mg/m$^2$ on days 1, 7 and 14 of the cycle. This cycle is repeated 3 times, with only transient neutropenia and gastrointestinal side effects (nausea, vomiting, diarrhea). Surprisingly, despite failing to respond to FOLFIRI therapy (which includes irinotecan, or CPT-11), the patient shows a partial response by RECIST criteria after completing her therapy. She is then placed on a maintenance schedule of this therapy once every month for the next 6 months. Followup scans show that her disease remains under control as a partial response (PR), and the patient is generally in good condition with a 90% Karnofsky performance status.

**Example 26. Therapy of relapsed metastatic ovarian cancer with IMMU-130 plus olaparib**

A 66-year-old woman with FIGO stage IV ovarian cancer positive for *BRCA1* mutatation undergoes primary surgery and postoperative paclitaxel and carboplatin (TC). After a 20-month platinum-free interval, an elevated CA125 level and recurrence in the peritoneum is confirmed by CT. Following retreatment with TC, a hypersensitivity reaction occurs to the carboplatin, which is changed to nedaplatin. A complete response is confirmed by CT. After an 8-month PFI, an elevated serum CA125 level and recurrence in the peritoneum and liver are confirmed.

She is then given combination therapy with anti-CEACAM5 IMMU-130 (hMN-14-SN-38) plus oliparib. IMMU-130 is administered at 10 mg/kg on days 1 and 8 of a 21-day cycle, while oliparib is administered at 200 mg twice a day on days 1-10 of the cycle. Except for transient grade 2 neutropenia and some initial diarrhea, she tolerates the therapy well, which is then repeated, after a rest of 2 months, for another course. Radiological examination indicates that she has partial response by RECIST criteria, because the sum of the diameters of the index lesions decrease by 45%. Her general condition also improves, and she returns to almost the same level of activity as prior to her illness.

**Example 27. Colonic cancer patient with Stage IV metastatic disease treated with anti-CSAp-SN-38 conjugate plus paclitaxel**

This patient presents with colonic cancer metastases to the left lobe of the liver and to both lungs, after having a resection of a 9-cm sigmoid colon adenocarinoma, followed by chemo-immunotherapy with FOLIFIRI and cetuximab for 6 months, and then FOLFOX followed by bevacizumab for an additional period of about 9 months. Ten months after the initial resection and then commencement of therapy, the stable disease thought to be present shows progression by the lesions growing and a new metastasis appearing in the left adrenal gland. Her plasma CEA at this time is 52 ng/mL, and her general condition appears to have deteriorated, with abdominal pains, fatigue, and borderline anemia, suggesting possibly internal bleeding.

She is then given combination therapy with an SN-38 conjugate of hMu-9 (anti-CSAp) antibody and paclitaxel on a 21 day cycle. The antibody or immunoconjugate is administered on days 1 and 8 of the cycle at a dose of 12 mg/kg, and paclitaxel is administered at a dosage of 175 mg/$m^2$ on days 1, 7 and 14 of the cycle, which is repeated for additional treatment cycles, measuring her blood counts every week and receiving atropine medication to control gastrointestinal reactions. Grade 2 alopecia is noted after the first treatment cycle, but only a Grade 1 neutropenia. After 3 treatment cycles, her plasma CEA titer is reduced to 19 ng/ml, and at this time her CT measurements show a decrease of the index lesions in the liver and lungs by 24.1%. After an additional 3 courses of therapy, she shows a CT reduction of the index lesions of 31.4%, and a decrease in the size of the adrenal mass by about 40%. This patient is considered to be responding to combination therapy, and continues on this therapy. Her general condition appears to be improved, with less fatigue, no abdominal pain or discomfort, and generally more energy.

**Example 28. Treatment of metastatic pancreatic cancer with anti-MUC5ac-SN-38 immunoconjugate plus rucaparib**

This 44-year-old patient has a history of metastatic pancreatic carcinoma, with inoperable pancreas ductal adenocarcinoma in the pancreas head, and showing metastases to left and right lobes of the liver, the former measuring 3 × 4 cm and the latter measuring 2 × 3 cm. The patient is given a course of gemcitabine but shows no objective response. Four weeks later, he is given combination therapy with hPAM4-SN-38 i.v. at a dose of 8 mg/kg twice-weekly for 2 weeks, with one week off, plus rucaparib (12 mg/$m^2$) once a week. This is repeated for another 2 cycles. CT studies are done one week later and show a total reduction in tumor mass (all sites) of 32% (partial response), alongside a drop in his blood CA19-9 titer from 220 at baseline to 75 at the time of radiological evaluation. The patient shows only grade 1 nausea and vomiting after each treatment and a grade 2 neutropenia at the end of the last treatment cycle, which resolves 4 weeks later. No premedication for preventing infusion reactions is given.

**Example 29. Use of anti-CD74 hLL1-SN-38 plus oliparib to treat therapy-refractive metastatic colonic cancer (mCRC)**

The patient is a 67-year-old man who presents with metastatic colon cancer. Following transverse colectomy shortly after diagnosis, the patient then receives 4 cycles of FOLFOX chemotherapy in a neoadjuvant setting prior to partial hepatectomy for removal of metastatic lesions in the left lobe of the liver. This is followed by an adjuvant FOLFOX regimen for a total of 10 cycles of FOLFOX.

CT shows metastases to liver. His target lesion is a 3.0-cm tumor in the left lobe of the liver. Non-target lesions included several hypo-attenuated masses in the liver. Baseline CEA is 685 ng/mL.

The patient is then treated with SN-38 conjugated anti-CD74 milatuzumab (hLL1-SN38) in combination with

oliparib. hLL1-SN-38 (10 mg/kg) is given on days 1 and 8 of a 21-day cycle, while oliparib is administered at 200 mg twice a day on days 1-10 of the cycle. The patient experiences nausea (Grade 2) and fatigue (Grade 2) during the first cycle and continues the treatment without major adverse events. The first response assessment done (after 3 cycles) shows shrinkage of the target lesion by 26% by computed tomography (CT) and his CEA level decreases to 245 ng/mL. In the second response assessment (after 8 cycles), the target lesion has shrunk by 35%. His overall health and clinical symptoms are considerably improved.

**Example 30. Treatment of relapsed chronic lymphocytic leukemia with IMMU-114-SN-38 and paclitaxel**

A 67-year-old man with a history of CLL, as defined by the International Workshop on Chronic Lymphocytic Leukemia and World Health Organization classifications, presents with relapsed disease after prior therapies with fludarabine, dexamethasone, and rituximab, as well as a regimen of CVP. He now has fever and night sweats associated with generalized lymph node enlargement, a reduced hemoglobin and platelet production, as well as a rapidly rising leukocyte count. His LDH is elevated and the beta-2-microglobulin is almost twice normal. The patient is given therapy with anti-HLA-DR IMMU-114-SN-38 (IgG4 hL243-SN-38) conjugate in combination with paclitaxel on a 21-day cycle. The IMMU-114-SN-38 is administered at 8 mg/kg on days 1 and 8 and paclitaxel is administered at a dosage of 175 mg/$m^2$ on days 1, 7 and 14 of the cycle, then the cycle is repeated. After 4 cycles, evaluation shows that the patient's hematological parameters improve and his circulating CLL cells appear to be decreasing in number. The therapy is resumed for another 3 cycles, after which his hematological and lab values indicate that he has a partial response.

**Example 31. Treatment of follicular lymphoma patient with hA19-SN-38, rucaparib and paclitaxel**

A 60-year-old male presents with abdominal pain and the presence of a palpable mass. The patient has CT and FDG-PET studies confirming the presence of the mass with pathologic adenopathies in the mediastinum, axillary, and neck nodes. Lab tests are unremarkable except for elevated LDH and beta-2-microglobulin. Bone marrow biopsy discloses several paratrabecular and perivascular lymphoid aggregates. These are lymphocytic with expression of CD20, CD19, and CD10 by immunostaining. The final diagnosis is grade-2 follicular lymphoma, stage IVA, with a FLIPI score of 4. The longest diameter of the largest involved node is 7 cm. The patient is given combination therapy with a humanized anti-CD19 monoclonal antibody IgG (hA19) conjugated with SN-38, plus rucaparib and paclitaxel, on a 21-day cycle. The ADC is given at 6 mg/kg on days 7 and 14, rucaparib is administered at 10 mg/$m^2$ on days 1, 8 and 15, and paclitaxel is administered at 125 mg/$m^2$ on days 1, 7 and 14 of the cycle. After 5 cycles, bone marrow and imaging (CT) evaluations show a partial response, where the measurable lesions decrease by about 60% and the bone marrow is much less infiltrated. Also, LDH and beta-2-microglobulin titers also decrease.

**Example 32. Treatment of relapsed precursor B-cell ALL with hA20-SN-38 plus olaparib**

This 51-year-old woman has been under therapy for precursor, Philadelphia chromosome-negative, B-cell ALL, which shows the ALL cells stain for CD19, CD20, CD 10, CD38, and CD45. More than 20% of the marrow and blood lymphoblasts express CD19 and CD20. The patient has received prior therapy with clofarabine and cytarabine, resulting in considerable hematological toxicity, but no response. A course of high-dose cytarabine (ara-C) was also started, but could not be tolerated by the patient. She is given hA20-SN-38 (veltuzumab-SN-38) and oliparib on a 21-day cycle,with doses of hA20-SN-38 by infusion of 6 mg/kg on days 7 and 14 and oliparib at 200 mg twice a day on days 1-10 of the cycle.

After 3 cycles, surprisingly, she shows improvement in her blood and marrow counts, sufficient for a partial response to be determined. After a rest of 2 months because of neutropenia (Grade 3), therapy resumes for another 4 courses. At this time, she is much improved and is under consideration for maintenance therapy to try to bring her to a stage where she could be a candidate for stem-cell transplantation.

**Example 33. Treatment of lymphoma with Anti-CD22-SN-38 plus paclitaxel**

The patient is a 62 year-old male with relapsed diffuse large B-cell lymphoma (DLBCL). After 6 courses of R-CHOP chemoimmunotherapy, he now presents with extensive lymph node spread in the mediastinum, axillary, and inguinal lymph nodes. He is given anti-CD22 epratuzumab-SN-38 plus paclitaxel on a 21-day cycle. The ADC is administered at a dose of 12 mg/kg on days 1 and 7 and paclitaxel is administered at 175 mg/$m^2$ on days 1, 7 and 14 of the cycle. After 3 cycles, the patient is evaluated by CT imaging, and his total tumor bulk is measured and shows a decrease of 35% (partial response), which appears to be maintained over the next 3 months. Side effects are only thrombocytopenia and grade 1 nausea and vomiting after therapy, which resolve within 2 weeks. No pretherapy for reducing infusion reactions is given.

**Example 34. Frontline therapy of follicular lymphoma using veltuzumab-SN-38 and rucaparib**

The patient is a 41-year-old woman presenting with low-grade follicular lymphoma, with measurable bilateral cervical and axillary lymph nodes (2-3 cm each), mediastinal mass of 4 cm diameter, and an enlarged spleen. She is given veltuzumab-SN-38 (anti-CD20-SN-38) in combination with rucaparib, with ADC administered at 10 mg/kg on day 1 and rucaparib at 12 mg/m$^2$ on days 1, 8 and 15. After 4 cycles, her tumor measurements by CT show a reduction of 80%. She is then given 2 additional courses of therapy, and CT measurements indicate that a complete response is achieved. This is confirmed by FDG-PET imaging.

**Example 35. Production and use of pro-2-pyrrolinodoxorubicin (P2PDox)**

Pro-2-pyrrolinodoxorubicin (P2PDox) was synthesized and conjugated to antibodies as described in U.S. Patent No. 8,877,202. Exemplaray P2PDox ADCs are disclosed in Table 16 below.

**Table 16. Exemplary P2PDox-Antibody Conjugates**

| | Conjugate | Lot | % Protein recover | P2PDo x/IgG | HPLC | |
|---|---|---|---|---|---|---|
| | | | | | Aggr. | Free drug |
| 1 | hIMMU-31-P2PDox | II22-138 | 75.0% | 7.39 | 1.9% | 0.26% |
| 2 | hA20-P2PDox | II22-135 | 85.7% | 6.79 | <2% | <0.1% |
| 3 | hLL1-P2PDox | II22-145 | 88.6% | 7.10 | 2.8% | 0.2% |
| 4 | hRS7-P2PDox | II22-142 | 80.1% | 7.17 | 1.8% | 0.12% |
| 5 | hMN15-P2PDox | II22-180 | 74.9% | 6.87 | 1.1% | 0.46% |
| 6 | hMN-14-P2PDox | II22-183 | 80.2% | 6.78 | 2.1% | 0.53% |

Conjugates were also prepared for hPAM4-P2PDox, hLL2-P2PDox and RFB4-P2PDox, with similar protein recovery and purity (not shown).

*In vitro* cell-binding studies - Retention of antibody binding was confirmed by cell binding assays comparing binding of the conjugate to unconjugated antibody (Chari, 2008, Acc Chem Res 41:98-107). The potency of the conjugate was tested in a 4-day MTS assay using appropriate target cells. The hRS7-P2PDox conjugate exhibited $IC_{50}$ values of 0.35-1.09 nM in gastric (NCI-N87), pancreatic (Capan-1), and breast (MDA-MB-468) human cancer cell lines, with free drug exhibiting 0.02-0.07 nM potency in the same cell lines.

Serum stability - Serum stability of prototypical P2PDox conjugate, hRS7-P2PDox, was determined by incubating in human serum at a concentration of 0.2 mg/mL at 37 $^0$C. The incubate was analyzed by HPLC using butyl hydrophobic interaction chromatography (HIC) column in which there was good retention time separation between the peak due to free drug and that due to conjugate or higher molecular weight species. This analysis showed that there was no release of free drug from the conjugate, suggesting high serum stability of the conjugate. When the same experiment was repeated with hRS7-doxorubicin conjugate, containing the same cleavable linker as hRS7-P2PDox, and where the free drug was independently verified to be released with a half-life of 96 h, clear formation of free drug peak, namely doxorubicin peak, was seen on HIC HPLC.

Surprisingly, it was determined that the P2PDox conjugate was held tightly to the antibody because it cross-linked the peptide chains of the antibody together. The cross-linking stabilizes the attachment of the drug to the antibody so that the drug is only released intracellularly after the antibody is metabolized. The cross-linking assists in minimizing toxicity, for example cardiotoxicity, that would result from release of free drug in circulation. Previous use of 2-PDox peptide conjugates failed because the drug cross-linked the peptide to other proteins or peptides *in vivo.* With the present conjugates, the P2PDox is attached to interchain disulfide thiol groups while in the prodrug form. The prodrug protection is rapidly removed *in vivo* soon after injection and the resulting 2-PDox portion of the conjugate crosslinks the peptide chains of the antibody, forming intramolecular cross-linking within the antibody molecule. This both stabilizes the ADC and prevents cross-linking to other molecules in circulation.

**EXAMPLE 34. *In vivo* studies**

General - Tumor size was determined by caliper measurements of length (L) and width (W) with tumor volume calculated as $(L \times W^2)/2$. Tumors were measured and mice weighed twice a week. Mice were euthanized if their tumors reached >1 cm$^3$ in size, lost greater than 15% of their starting body weight, or otherwise became moribund. Statistical

analysis for the tumor growth data was based on area under the curve (AUC) and survival time. Profiles of individual tumor growth were obtained through linear curve modeling. An *f*-test was employed to determine equality of variance between groups prior to statistical analysis of growth curves. A two-tailed t-test was used to assess statistical significance between all the various treatment groups and non-specific controls. For the saline control analysis a one-tailed t-test was used to assess significance. Survival studies were analyzed using Kaplan-Meier plots (log-rank analysis), using the Prism GraphPad Software (v4.03) software package (Advanced Graphics Software, Inc.; Encinitas, CA). All doses in preclinical experiments are expressed in antibody amounts. In terms of drug, 100 μg of antibody (5 mg/kg) in a 20-g mouse, for example, carries 1.4 μg-2.8 μg (0.14-0.17 mg/kg) of P2PDox equivalent dose when using an ADC with 3-6 drugs/IgG.

A single i.v. dose of ≥ 300 μg [~ 10 μg of P2PDox] of the conjugate was lethal, but 4 doses of 45 μg given in 2 weeks were tolerated by all animals. Using this dosing regimen, we examined the therapeutic effect of hRS7-P2PDox in 2 human tumor xenograft models, Capan-1 (pancreatic cancer) and NCI-N87 (gastric cancer). Therapy began 7 days after tumor transplantation in nude mice. In the established, 7-day-old, Capan-1 model, 100% of established tumors quickly regressed, with no evidence of re-growth (not shown). This result was reproduced in a repeat experiment (not shown). Similar findings were made in the established NCI-N87 model (not shown), where a 2$^{nd}$ course of therapy, administered after day 70, was safely tolerated and led to further regressions of residual tumor (not shown). The internalizing hRS7-SN-38 conjugate, targeting Trop-2, provided better therapeutic responses than a conjugate of a poorly internalizing anti-CEACAM5 antibody, hMN-14 (not shown). A non-targeted anti-CD20 ADC, hA20-P2PDox, was ineffective, indicating selective therapeutic efficacy (not shown). Data from a breast cancer xenograft (MDA-MB-468) and a second pancreatic cancer xenograft (not shown) reiterate the same trend of the conjugate's specific and significant antitumor effects.

PK and toxicity of hRS7-P2PDox with substitutions of 6.8 or 3.7 drug/IgG - Antibody-drug conjugates (ADCs) carrying as much as 8 ultratoxic drugs/MAb are known to clear faster than unmodified MAb and to increase off-target toxicity, a finding that has led to the current trends to use drug substitutions of ≤ 4 (Hamblett et al., 2004, Clin Cancer Res 10:7063-70). Conjugates were prepared and evaluated with mean drug/MAb substitution ratios (MSRs) of ~6:1 and ~3:1. Groups of normal mice (n = 5) were administered, i.v., single doses of unmodified hRS7 or hRS7-P2PDox with drug substitution of 6.8 or 3.7 (same protein dose), and serum samples were collected at 30 min, 4 h, 24 h, 72 h, and 168 h postinjection. These were analyzed by ELISA for antibody concentration (not shown). There were no significant differences in serum concentrations at various times, indicating that these cleared similarly. The PK parameters (Cmax, AUC, etc.) were similar. Conjugates with either higher or lower drug substitution had similar tolerability in nude mice, when the administered at the same dose of conjugated drug.

Therapeutic Efficacy at Minimum Effective Dose (MED) - Anti-TROP-2 antibody conjugate, hRS7-P2PDox, was evaluated in nude mice bearing NCI-N87 human gastric cancer xenografts by administering a single bolus protein dose of 9 mg/kg, 6.75 mg/kg, 4.5 mg/kg, 2.25 mg/kg, or 1 mg/kg. The therapy was started when the mean tumor volume (mTV) was 0.256 $cm^3$. On day 21, mTV in the saline control group (non-treatment group) was 0.801 ± 0.181 $cm^3$ which was significantly larger than that in mice treated with 9, 6.75, 4.5, or 2.25 mg/kg dose with mTV of 0.211 ± 0.042 $cm^3$, 0.239 ± 0.0.054 $cm^3$, 0.264 ± 0.087 $cm^3$, and 0.567 ± 0.179 $cm^3$, respectively (P<0.0047, one tailed t-test). From these, the minimum effective dose was judged to be 2.25 mg/kg, while 9 mg/kg represented MTD.

MTD of Antibody-P2PDox - An MTD study comparing 2-PDox and P2PDox conjugates of prototype antibody, hLL1, in mice demonstrated that the P2PDox conjugate was much more potent (not shown). The MTD of a single i.v. injection was between 100 and 300 μg. The MTD of multiple injections, at a schedule of every four days for a total of four injections (q4dx4) was then determined, using protein doses between 25 μg to 150 μg per injection. At these doses, a cumulative dose of between 100 and 600 μg was given to the animals. **Table 17** below summarizes the various groups.

**Table 17. Dosage and Schedule for MTD of antibody-P2PDox**

| **12 Female Athymic Nude Mice** | | | |
|---|---|---|---|
| **Group** | **N** | **Treatment** | **Total Amount** |
| **1** | **3** | 25 μg i.v. q4dx4 | 100 μg |
| **2** | **3** | 50 μg i.v. q4dx4 | 200 μg |
| **3** | **3** | 100 μg i.v. q4dx4 | 400 μg |
| **4** | **3** | 150 μg i.v. q4dx4 | 600 μg |

Only those mice treated with 25 μg P2PDox-ADC continue to show no signs of toxicity (not shown). This is a cumulative dose of 100 μg which was also the dose tolerated when administered as a single injection (not shown).

Therefore, the MTD for multiple injections of a P2PDox-ADC in mice is 25 μg q4dx4 from this experiment. A more detailed analysis of data and repetition of the experiment established the MTD for fractionated dosing to be 45 μg of protein dose of the conjugate, administered every 4 days for 2 weeks (45 μg, q4dx4 schedule).

Binding Studies - No significant difference in binding of the antibody moiety to NCI-N87 gastric carcinoma cells was observed between unconjugated hRS7 and P2PDox-hRS7 conjugated to 6 molecules of P2PDox per antibody (not shown). The lack of effect of conjugation on antibody binding to target antigen was confirmed for P2PDox-hMN-15 (anti-CEACAM6), P2PDox-hLL2 (anti-CD22) and P2PDox-hMN-24 (anti-CEACAM5) conjugates. It is concluded that conjugation of P2PDox to antibodies does not affect antibody-antigen binding activity.

Cytotoxicity Studies - The cytotoxicity of P2PDox-mAb conjugates to target cells was examined. hRS7-P2PDox and hMN-15-P2PDox were cytotoxic to MDA-MB-468, AG S, NCI-N87 and Capan-1 solid tumor cell lines (not shown). hMN-14-P2PDox was cytotoxic to Capan-1, BxPC-3 and AsPC-1 human pancreatic tumor lines and AGS, NCI-N87 and LS147T human gastric and colonic tumor lines (not shown). hLL2-P2PDOx was cytotoxic to Daudi, Raji, Ramos and JVM-3 hematopoietic tumor lines (not shown). $IC_{50}$ values for the conjugates were in the nanomolar concentration range (not shown).

## EXAMPLE 35. Further *in vivo* studies

Further *in vivo* efficacy studies were performed in nude mice implanted with NCI-N87 human gastric cancer xenografts (not shown). One treatment cycle with 4 × 45 μg of hRS7-P2PDox rapidly regressed all tumors (not shown). A second treatment cycle was initiated about 2 months after the end of the first cycle, resulting in complete regression of all but one of the hRS7-P2PDox treated animals. The hA20, hLL1 and hMN-14 conjugates had little effect on tumor progression (not shown). Administration of P2PDox-hMN-15 resulted in a delayed regression of gastric cancer, which was less effective than the hRS7 conjugate.

The effect of varying dosage schedule on anti-tumor efficacy was examined (not shown). The experiment began 9 days after tumor implantation when mean tumor volume for all groups was 0.383 $cm^3$, and ended on day 93 (84 days after initiation of therapy). In this study, a single dose of 180 μg, two weekly doses of 90 μg, and q4dx4 of 45 μg all resulted in significantly enhanced survival (not shown). For the saline control, 0 of 9 mice survived (not shown). For mice receiving 45 μg q4dx4 of hRS7-P2PDox, 8 of 9 mice were alive at day 94 (not shown). For mice receiving 90 μg weekly x 2 of hRS7-P2PDox, 9 of 9 mice were alive at day 94 (not shown). For mice receiving a single dose of 180 μg of hRS7-P2PDox, 8 of 9 mice were alive at day 94 (not shown). At the same dosage schedule, the control hA20 conjugate had no effect on survival (not shown). A toxicity study showed that the three dosage schedules of hRS7-P2PDox resulted in similarly low levels of toxicity (not shown).

The hRS7-P2PDox conjugate was also effective in Capan-1 pancreatic cancer (not shown) and was more effective at inhibiting tumor growth than a hRS7-SN-38 conjugate (not shown). The hPAM4-P2PDox conjugate was also more effective at inhibiting growth of Capan-1 human pancreatic cancer than an hPAM4-SN-38 conjugate (not shown). At 63 days after Capan-1 tumor injection (with therapy starting at 1 days post-innoculation), 0 of 10 mice were alive in the saline control, 10 of 10 mice were alive in mice treated twice weekly x 2 weeks with 45 μg of hPAM4-P2PDox, 2 of 10 mice were alive in mice treated twice weekly x 2 weeks with 45 μg of hA20-P2PDox, 0 of 10 mice were alive in mice treated twice weekly x 4 weeks with 250 μg of hPAM4-SN-38, and 0 of 10 mice were alive in mice treated twice weekly x 4 weeks with 250 μg of h20-SN-38.

hRS7-P2PDox was substantially more effective than hRS7-SN-38 at inhibiting growth of PxPC-3 pancreatic cancer (not shown) and was slightly more effective than hRS7-SN-38 at inhibiting growth of MDA-MB-468 breast cancer (not shown).

The effect of different single doses of hRS7-P2PDox on growth of NCI-N87 gastric carcinoma xenografts was examined. Using a single dose, the maximum effect on tumor growth was observed at 90 μg or higher (not shown). A single dose of 45 μg was the minimum required to see a significant survival benefit compared to saline control (not shown).

The ADCC activity of various hRS7-ADC conjugates was determined in comparison to hRS7 IgG (not shown). PBMCs were purified from blood purchased from the Blood Center of New Jersey. A Trop-2-positive human pancreatic adenocarcinoma cell line (BxPC-3) was used as the target cell line with an effector to target ratio of 100:1. ADCC mediated by hRS7 IgG was compared to hRS7-Pro-2-PDox, hRS7-CL2A-SN-38, and the reduced and capped hRS7-NEM. All were used at 33.3 nM. Overall activity was low, but significant (not shown). There was 8.5% specific lysis for the hRS7 IgG which was not significantly different from hRS7-Pro-2-PDox. Both were significantly better than hLL2 control and hRS7-NEM and hRS7-SN-38 ($P<0.02$, two-tailed t-test). There was no difference between hRS7-NEM and hRS7-SN-38.

## Example 36. Treatment of non-Hodgkin's lymphoma (NHL) with anti-CD22 P2PDox-epratuzumab plus oliparib

A P2PDox-epratuzumab ADC is prepared as described above. Seventeen patients with previously untreated

or relapsed NHL receive 4 doses of 70, 100 or 150 mg P2PDox-epratuzumab injected i.v. on days 1 and 14 of a 28 day cycle. Oliparib at 200 mg a day is administered on days 1-7 and 14-21 of the cycle. Responses are assessed by CT scans, with other evaluations including adverse event, B-cell blood levels, serum epratuzumab levels and human anti-epratuzumab (HAHA) titers.

Only occasional, mild to moderate transient injection reactions are seen and no other safety issues except neutropenia up to Grade 3 (but reversible after interrupting therapy until reduced to Grade 1) are observed. Transient B-cell depletion (up to about 25%) is observed at all dosage levels of P2PDox-epratuzumab in combination with oliparib. The objective response rate (partial responses plus complete responses plus complete responses unconfirmed) is 47% (8/17) with a complete response/complete response unconfirmed rate of 24% (4/17). Four of the eight objective responses continue for 30 weeks or more. Objective responses are observed at all dose levels of P2PDox-epratuzumab plus oliparib. All serum samples evaluated for human anti- epratuzumab antibody (HAHA) are negative.

**Example 37. Treatment of triple negative breast cancer with P2PDox-hRS7 plus paclitaxel**

Anti-Trop-2 P2PDox-hRS7 ADC is prepared as described in above. Patients with triple-negative breast cancer who had failed at least two standard therapies receive 70 mg P2PDox-hRS7 injected i.v. on day 1 of a 21 day cycle, with paclitaxel administered at 175 $mg/m^2$ on days 1, 7 and 14 of the cycle. After 3 cycles, objective responses are observed, with an average decrease in tumor volume of 35%. All serum samples evaluated for human anti-hRS7 antibody (HAHA) are negative.

**Example 38. Treatment of metastatic colon cancer with P2PDox-hMN-14 plus olaparib**

A 52-year old man with metastatic colon cancer (3-5 cm diameters) to his left and right liver lobes, as well as a 5 cm metastasis to his right lung, and an elevated blood CEA value of 130 ng/mL, is treated with the combination of anti-CEACAM5 hMN-14-P2PDox plus olaparib. A 150 mg dose of hMN-14-P2PDox is administered on days 1 and 14 of a 28 day cycle. Oliparib is administered at 200 mg twice a day on days 1-10 of the cycle. Upon CT evaluation after 2 cycles, a 25% reduction of the total mean diameters of the 3 target lesions is measured, thus constituting a good stable disease response by RECIST1.1 criteria. At the same time, his blood CEA titer is reduced to 30 ng/mL. Repeated courses of therapy continue as his neutropenia normalizes.

**Example 39. Immunoconjugate storage**

The ADC conjugates are were purified and buffer-exchanged with 2-(N-morpholino)ethanesulfonic acid (MES), pH 6.5, and further formulated with trehalose (25 mM final concentration) and polysorbate 80 (0.01% v/v final concentration), with the final buffer concentration becoming 22.25 mM as a result of excipient addition. The formulated conjugates are lyophilized and stored in sealed vials, with storage at 2 °C - 8 °C. The lyophilized immunoconjugates are stable under the storage conditions and maintain their physiological activities.

## Claims

1. An immunoconjugate that binds to Trop-2 for use in a method of treating Trop-2 expressing cancer, wherein the method comprises:

    a) administering to a human subject with the cancer the immunoconjugate; and
    b) administering to the subject at least one therapeutic agent selected from the group consisting of a PARP inhibitor and a microtubule inhibitor

    wherein the immunoconjugate comprises an anti-Trop-2 antibody conjugated to SN-38.

2. At least one therapeutic agent selected from the group consisting of a PARP inhibitor and a microtubule inhibitor, for use in a method of treating Trop-2 expressing cancer, wherein the method comprises:

    a) administering to a human subject with the cancer an immunoconjugate that binds to Trop-2; and
    b) administering to the subject the at least one therapeutic agent

    wherein the immunoconjugate comprises an anti-Trop-2 antibody conjugated to SN-38.

3. The immunoconjugate for use of claim 1 and the at least one therapeutic agent for use of claim 2, wherein the combination of antibody or immunoconjugate and therapeutic agent has a synergistic effect in inhibiting tumor growth.

4. The immunoconjugate for use of claim 1 and the at least one therapeutic agent for use of claim 2, wherein the anti-Trop-2 antibody is hRS7.

5. The immunoconjugate for use of claim 1 and the at least one therapeutic agent for use of claim 2, wherein the PARP inhibitor is selected from the group consisting of olaparib, talazoparib (BMN-673), rucaparib, veliparib, CEP 9722, MK 4827, BGB-290, ABT-888, AG014699, BSI-201, CEP-8983 and 3-aminobenzamide; or
the microtubule inhibitor is selected from the group consisting of a vinca alkaloid, a taxane, a maytansinoid, an auristatin, vincristine, vinblastine, paclitaxel, mertansine, demecolcine, nocodazole, epothilone, docetaxel, disodermolide, colchicine, combrestatin, podophyllotoxin, CI-980, phenylahistins, steganacins, curacins, 2-methoxy estradiol, E7010, methoxy benzenesuflonamides, vinorelbine, vinflunine, vindesine, dolastatins, spongistatin, rhizoxin, tasidotin, halichondrins, hemiasterlins, cryptophycin 52, MMAE and eribulin mesylate.

6. The immunoconjugate for use of claim 5 and the at least one therapeutic agent for use of claim 5, wherein the PARP inhibitor is olaparib; or
the microtubule inhibitor is paclitaxel or eribulin mesylate.

7. The immunoconjugate for use of claim 1 and the at least one therapeutic agent for use of claim 2, wherein the cancer is a solid tumor and the treatment results in a reduction in tumor size of at least 15%, at least 20%, at least 30%, or at least 40%.

8. The immunoconjugate for use of claim 1 and the at least one therapeutic agent for use of claim 2, wherein the cancer is metastatic and the method further comprises reducing in size or eliminating the metastases.

9. The immunoconjugate for use of claim 1 and the at least one therapeutic agent for use of claim 2, wherein the cancer is refractory to other therapies but responds to the combination of antibody-drug conjugate and therapeutic agent.

10. The immunoconjugate for use of claim 9 and the at least one therapeutic agent for use of claim 9, wherein the patient has failed to respond to therapy with irinotecan, prior to treatment with the antibody-drug conjugate.

11. The immunoconjugate for use of claim 1 and the at least one therapeutic agent for use of claim 2, wherein the method further comprises administering to the subject one or more additional therapeutic modalities selected from the group consisting of unconjugated antibodies, radiolabeled antibodies, drug-conjugated antibodies, toxin-conjugated antibodies, gene therapy, chemotherapy, therapeutic peptides, cytokine therapy, oligonucleotides, localized radiation therapy, surgery and interference RNA therapy.

12. The immunoconjugate for use of claim 11 and the at least one therapeutic agent for use of claim 11, wherein the therapeutic modality comprises treatment with an agent selected from the group consisting of 5-fluorouracil, afatinib, aplidin, azaribine, anastrozole, anthracyclines, axitinib, AVL-101, AVL-291, bendamustine, bleomycin, bortezomib, bosutinib, bryostatin-1, busulfan, calicheamycin, camptothecin, carboplatin, 10-hydroxycamptothecin, carmustine, celebrex, chlorambucil, cisplatin (CDDP), Cox-2 inhibitors, irinotecan (CPT-11), SN-38, carboplatin, cladribine, camptothecans, cyclophosphamide, crizotinib, cytarabine, dacarbazine, dasatinib, dinaciclib, docetaxel, dactinomycin, daunorubicin, doxorubicin, 2-pyrrolinodoxorubicine (2P-DOX), cyano-morpholino doxorubicin, doxorubicin glucuronide, epirubicin glucuronide, erlotinib, estramustine, epidophyllotoxin, erlotinib, entinostat, estrogen receptor binding agents, etoposide (VP16), etoposide glucuronide, etoposide phosphate, exemestane, fingolimod, flavopiridol, floxuridine (FUdR), 3',5'-O-dioleoyl-FudR (FUdR-dO), fludarabine, flutamide, farnesyl-protein transferase inhibitors, fostamatinib, ganetespib, GDC-0834, GS-1101, gefitinib, gemcitabine, hydroxyurea, ibrutinib, idarubicin, idelalisib, ifosfamide, imatinib, L-asparaginase, lapatinib, lenolidamide, leucovorin, LFM-A13, lomustine, mechlorethamine, melphalan, mercaptopurine, 6-mercaptopurine, methotrexate, mitoxantrone, mithramycin, mitomycin, mitotane, navelbine, neratinib, nilotinib, nitrosurea, olaparib, plicomycin, procarbazine, paclitaxel, PCI-32765, pentostatin, PSI-341, raloxifene, semustine, sorafenib, streptozocin, SU11248, sunitinib, tamoxifen, temazolomide (an aqueous form of DTIC), transplatinum, thalidomide, thioguanine, thiotepa, teniposide, topotecan, uracil mustard, vatalanib, vinorelbine, vinblastine, vincristine, vinca alkaloids and ZD1839.

13. The immunoconjugate for use of claim 1 and the at least one therapeutic agent for use of claim 2, wherein the cancer is selected from the group consisting of triple-negative breast cancer, breast cancer, ovarian cancer, endometrial

cancer, urothelial cancer, uterine cancer, non-small-cell lung cancer, small-cell lung cancer, stomach cancer, urinary bladder cancer, renal cancer, prostate cancer and colorectal cancer.

## Patentansprüche

**1.** Trop-2-bindendes Immunkonjugat zur Verwendung in einem Verfahren zum Behandeln von Trop-2 exprimierendem Krebs, wobei das Verfahren umfasst:

a) Verabreichen des Immunkonjugates an ein menschliches Lebewesen mit dem Krebs; und
b) Verabreichen an das Lebewesen wenigstens eines therapeutischen Agens, das ausgewählt ist aus der Gruppe bestehend aus einem PARP-Inhibitor und einem Mikrotubulus-Inhibitor,

wobei das Immunkonjugat einen an SN-38 konjuguierten anti-Trop-2-Antikörper umfasst.

**2.** Wenigstens ein therapeutisches Agens, das ausgewählt ist aus der Gruppe bestehend aus einem PARP-Inhibitor und einem Mikrotubulus-Inhibitor, zur Verwendung in einem Verfahren zum Behandeln von Trop-2 exprimierendem Krebs, wobei das Verfahren umfasst:

a) Verabreichen des Trop-2 bindenden Immunkonjugates an ein menschliches Lebewesen mit dem Krebs; und
b) Verabreichen des wenigstens einen therapeutischen Agens an das Lebewesen,

wobei das Immunkonjugat einen an SN-38 konjuguierten anti-Trop-2-Antikörper umfasst.

**3.** Immunkonjugat zur Verwendung nach Anspruch 1 und das wenigstens eine therapeutische Agens zur Verwendung nach Anspruch 2, wobei die Kombination von Antikörper oder Immunkonjugat und therapeutischem Agens eine synergistische Wirkung bezüglich Inhibieren von Tumorwachstum aufweist.

**4.** Immunkonjugat zur Verwendung nach Anspruch 1 und das wenigstens eine therapeutische Agens zur Verwendung nach Anspruch 2, wobei der anti-Trop-2-Antikörper hRS7 ist.

**5.** Immunkonjugat zur Verwendung nach Anspruch 1 und das wenigstens eine therapeutische Agens zur Verwendung nach Anspruch 2, wobei der PARP-Inhibitor ausgewählt ist aus der Gruppe bestehend aus Olaparib, Talazoparib (BMN-673), Rucaparib, Veliparib, CEP 9722, MK 4827, BGB-290, ABT-888, AG014699, BSI-201, CEP-8983 und 3-Aminobenzamid; oder
der Mikrotubulus-Inhibitor ausgewählt ist aus der Gruppe bestehend aus einem Vincaalkaloid, einem Taxan, einem Maytansinoid, einem Auristatin, Vincristin, Vinblastin, Paclitaxel, Mertansin, Demecolcin, Nocodazol, Epothilon, Docetaxel, Disodermolid, Colchicin, Combrestatin, Podophyllotoxin, CI-980, Phenylahistinen, Steganacinen, Curacinen, 2-Methoxyestradiol, E7010, Methoxybenzensuflonamiden, Vinorelbin, Vinflunin, Vindesin, Dolastatinen, Spongistatin, Rhizoxin, Tasidotin, Halichondrinen, Hemiasterlinen, Cryptophycin 52, MMAE und Eribulin-Mesylat.

**6.** Immunkonjugat zur Verwendung nach Anspruch 5 und das wenigstens eine therapeutische Agens zur Verwendung nach Anspruch 5, wobei der PARP-Inhibitor Olaparib ist; oder
der Mikrotubulus-Inhibitor Paclitaxel oder Eribulin-Mesylat ist.

**7.** Immunkonjugat zur Verwendung nach Anspruch 1 und das wenigstens eine therapeutische Agens zur Verwendung nach Anspruch 2, wobei der Krebs ein solider Tumor ist und die Behandlung zu einer Verringerung der Tumorgröße von wenigstens 15 %, wenigstens 20 %, wenigstens 30 % oder wenigstens 40 % führt.

**8.** Immunkonjugat zur Verwendung nach Anspruch 1 und das wenigstens eine therapeutische Agens zur Verwendung nach Anspruch 2, wobei der Krebs metastatisch ist und das Verfahren weiter Verringern der Größe oder Eliminieren der Metastasen umfasst.

**9.** Immunkonjugat zur Verwendung nach Anspruch 1 und das wenigstens eine therapeutische Agens zur Verwendung nach Anspruch 2, wobei der Krebs refraktär ist gegenüber anderen Therapien, aber auf die Kombination von Antikörper-Droge-Immunkonjugat und therapeutischem Agens anspricht.

**10.** Immunkonjugat zur Verwendung nach Anspruch 9 und das wenigstens eine therapeutische Agens zur Verwendung

nach Anspruch 9, wobei der Patient vor der Behandlung mit dem Antikörper-Droge-Immunkonjugat nicht auf eine Therapie mit Irinotecan angesprochen hat.

**11.** Immunkonjugat zur Verwendung nach Anspruch 1 und das wenigstens eine therapeutische Agens zur Verwendung nach Anspruch 2, wobei das Verfahren weiter umfasst Verabreichen an das Lebewesen einer oder mehrerer zusätzlicher Therapiemodalitäten, die ausgewählt sind aus der Gruppe bestehend aus nicht-konjugierten Antikörpern, radiomarkierten Antikörpern, mit einer Droge konjugierten Antikörpern, mit einem Toxin konjugierten Antikörpern, Gentherapie, Chemotherapie, therapeutischen Peptiden, Zytokintherapie, Oligonukleotiden, örtlicher Strahlentherapie, Chirurgie und Therapie mit interferierender RNA.

**12.** Immunkonjugat zur Verwendung nach Anspruch 11 und das wenigstens eine therapeutische Agens zur Verwendung nach Anspruch 11, wobei die therapeutische Modalität Behandlung mit einem Agens umfasst, das ausgewählt ist aus der Gruppe bestehend aus 5-Fluorouracil, Afatinib, Aplidin, Azaribin, Anastrozol, Anthracyclinen, Axitinib, AVL-101, AVL-291, Bendamustin, Bleomycin, Bortezomib, Bosutinib, Bryostatin-1, Busulfan, Calicheamycin, Camptothecin, Carboplatin, 10-Hydroxycamptothecin, Carmustin, Celebrex, Chlorambucil, Cisplatin (CDDP), Cox-2-Inhibitoren, Irinotecan (CPT-11), SN-38, Carboplatin, Cladribin, Camptothecanen, Cyclophosphamid, Crizotinib, Cytarabin, Dacarbazin, Dasatinib, Dinaciclib, Docetaxel, Dactinomycin, Daunorubicin, Doxorubicin, 2-Pyrrolinodoxorubicin (2P-DOX), Cyano-Morpholindoxorubicin, Doxorubicin-Glucuronid, Epirubicin-Glucuronid, Erlotinib, Estramustin, Epidophyllotoxin, Erlotinib, Entinostat, Estrogen-Rezeptor-bindenden Agenzien, Etoposid (VP16), Etoposid-Glucuronid, Etoposid-Phosphat, Exemestan, Fingolimod, Flavopiridol, Floxuridin (FUdR), 3',5'-O-Dioleoyl-FudR (FUdR-dO), Fludarabin, Flutamid, Farnesyl-Protein-Transferase-Inhibitoren, Fostamatinib, Ganetespib, GDC-0834, GS-1101, Gefitinib, Gemcitabin, Hydroxyharnstoff, Ibrutinib, Idarubicin, Idelalisib, Ifosfamid, Imatinib, L-Asparaginase, Lapatinib, Lenolidamid, Leucovorin, LFM-A13, Lomustin, Mechlorethamin, Melphalan, Mercaptopurin, 6-Mercaptopurin, Methotrexat, Mitoxantron, Mithramycin, Mitomycin, Mitotan, Navelbin, Neratinib, Nilotinib, Nitrosoharnstoff, Olaparib, Plicomycin, Procarbazin, Paclitaxel, PCI-32765, Pentostatin, PSI-341, Raloxifen, Semustin, Sorafenib, Streptozocin, SU11248, Sunitinib, Tamoxifen, Temazolomid (eine wässrige Form von DTIC), Transplatin, Thalidomid, Thioguanin, Thiotepa, Teniposid, Topotecan, Uracil-Senfgas, Vatalanib, Vinorelbin, Vinblastin, Vincristin, Vincaalkaloiden und ZD1839.

**13.** Immunkonjugat zur Verwendung nach Anspruch 1 und das wenigstens eine therapeutische Agens zur Verwendung nach Anspruch 2, wobei der Krebs ausgewählt ist aus der Gruppe bestehend aus dreifach-negativem Brustkrebs, Brustkrebs, Ovarialkarzinom, Endometriumkarzinom, Harnwegsepithelkrebs, Uteruskarzinom, nicht-kleinzelligem Lungenkarzinom, kleinzelligem Lungenkarzinom, Magenkrebs, Harnblasenkrebs, Nierenkrebs, Prostatakrebs und kolorektalem Karzinom.

## Revendications

**1.** Immunoconjugué qui se lie à Trop-2 pour une utilisation dans une méthode de traitement du cancer exprimant Trop-2

a) dans lequel la méthode comprend :
b) l'administration de l'immunoconjugué à un sujet humain atteint du cancer ; et
c) l'administration au sujet d'au moins un agent thérapeutique choisi dans le groupe constitué par un inhibiteur de PARP et un inhibiteur de microtubules
a) dans lequel l'immunoconjugué comprend un anticorps anti-Trop-2 conjugué au SN-38.

**2.** Au moins un agent thérapeutique choisi dans le groupe constitué par un inhibiteur de PARP et un inhibiteur de microtubules, pour une utilisation dans une méthode de traitement du cancer exprimant Trop-2, dans laquelle la méthode comprend :

a) l'administration à un sujet humain atteint du cancer d'un immunoconjugué qui se lie à Trop-2 ; et
b) l'administration au sujet d'au moins un agent thérapeutique, l'immunoconjugué comprenant un anticorps anti-Trop-2 conjugué au SN-38.

**3.** Immunoconjugué pour l'utilisation selon la revendication 1 et au moins un agent thérapeutique pour l'utilisation selon la revendication 2, dans lesquelles la combinaison de l'anticorps ou de l'immunoconjugué et de l'agent thérapeutique a un effet synergique dans l'inhibition de la croissance tumorale.

**4.** Immunoconjugué pour l'utilisation selon la revendication 1 et au moins un agent thérapeutique pour l'utilisation selon la revendication 2, dans lesquelles l'anticorps anti-Trop-2 est hRS7.

**5.** Immunoconjugué pour l'utilisation selon la revendication 1 et au moins un agent thérapeutique pour l'utilisation selon la revendication 2, dans lesquelles l'inhibiteur de PARP est choisi dans le groupe constitué par l'olaparib, le talazoparib, le talazoparib, le talazoparib, le (BMN-673), le rucaparib, le veliparib, le CEP 9722, le MK 4827, le BGB-290, le ABT-888, le AG014699, le BSI-201, le CEP-8983 et le 3-aminobenzamide ; ou
l'inhibiteur de microtubules est choisi dans le groupe constitué par un vinca-alcaloïde, un taxane, un maytansinoïde, une auristatine, la vincristine, la vinblastine, le paclitaxel, la mertansine, la démécolcine, le nocodazole, l'épothilone, le docétaxel, le disodermolide, la colchicine, la combrestatine, la podophyllotoxine, le CI-980, les phénylahistines, les stéganacines, les curacines, le 2-méthoxy estradiol, le E7010, les méthoxy benzènesuflonamides, la vinorelbine, la vinflunine, la vindesine, les dolastatines, la spongistatine, le rhizoxine, la tasidotine, les halichondrines, les hémiastérolines, la cryptophycine 52, le MMAE et le mésylate d'éribuline.

**6.** Immunoconjugué pour l'utilisation selon la revendication 5 et au moins un agent thérapeutique pour l'utilisation selon la revendication 5, dans laquelle l'inhibiteur de PARP est l'olaparib ; ou
l'inhibiteur de microtubules est le paclitaxel ou le mésylate d'éribuline.

**7.** Immunoconjugué pour l'utilisation selon la revendication 1 et au moins un agent thérapeutique pour l'utilisation selon la revendication 2, dans lesquelles le cancer est une tumeur solide et le traitement résulte en une réduction de la taille de la tumeur d'au moins 15%, au moins 20%, au moins 30%, ou au moins 40%.

**8.** Immunoconjugué pour l'utilisation selon la revendication 1 et au moins un agent thérapeutique pour l'utilisation selon la revendication 2, dans lesquelles le cancer est métastatique et la méthode comprend en outre la réduction de la taille ou l'élimination des métastases.

**9.** Immunoconjugué pour l'utilisation selon la revendication 1 et au moins un agent thérapeutique pour l'utilisation selon la revendication 2, dans lesquelles le cancer est réfractaire à d'autres thérapies mais répond à la combinaison du conjugué anticorps-drogue et de l'agent thérapeutique.

**10.** Immunoconjugué pour l'utilisation selon la revendication 9 et au moins un agent thérapeutique pour l'utilisation selon la revendication 9, dans laquelle le patient n'a pas répondu à la thérapie avec l'irinotécan, avant le traitement avec le conjugué anticorps-drogue.

**11.** Immunoconjugué pour l'utilisation selon la revendication 1 et au moins un agent thérapeutique pour l'utilisation selon la revendication 2, dans lesquelles la méthode comprend en outre l'administration au sujet d'une ou plusieurs modalités thérapeutiques supplémentaires choisies dans le groupe constitué par les anticorps non conjugués, les anticorps radiomarqués, les anticorps conjugués à un médicament, les anticorps conjugués à une toxine, la thérapie génique, la chimiothérapie, les peptides thérapeutiques, la thérapie par cytokines, les oligonucléotides, la radiothérapie localisée, la chirurgie et la thérapie par ARN interférence.

**12.** Immunoconjugué pour l'utilisation selon la revendication 11 et au moins un agent thérapeutique pour l'utilisation selon la revendication 11, dans laquelle la modalité thérapeutique comprend le traitement avec un agent choisi dans le groupe constitué par le 5-fluorouracile, l'afatinib, l'aplidine, l'azaribine, l'anastrozole, les anthracyclines, l'axitinib, l'AVL-101, l'AVL-291, la bendamustine, la bléomycine, le bortézomib, le bosutinib, la bryostatine-1, le busulfan, la calichéamycine, la camptothécine, le carboplatine, la 10-hydroxycamptothécine, la carmustine, le celebrex, le chlorambucil, cisplatine (CDDP), les inhibiteurs de Cox-2, l'irinotécan (CPT-11), le SN-38, le carboplatine, la cladribine, le camptothécans, le cyclophosphamide, le crizotinib, la cytarabine, la dacarbazine, le dasatinib, le dinaciclib, le docetaxel, la dactinomycine, la daunorubicine, la doxorubicine, la 2-pyrrolinodoxorubicine (2P-DOX), la cyanomorpholino doxorubicine, le glucuronide de doxorubicine, le glucuronide d'épirubicine, l' erlotinib, l'estramustine, l'épidophyllotoxine, l'erlotinib, l'entinostat, les agents de liaison aux récepteurs des oestrogènes, l'étoposide (VP16), le glucuronide d'étoposide, le phosphate d'étoposide, l'exémestane, le fingolimod, le flavopiridol, la floxuridine (FUdR), le 3',5'-0-dioléoyl-FudR (FUdR-d0), la fludarabine, le flutamide, les inhibiteurs de la farnesyl-protéine transférase, le fostamatinib, le ganetespib, le GDC-0834, le GS-1101, le géfitinib, la gemcitabine, l'hydroxyurée, l'ibrutinib, l'idarubicine, l'idelalisib, l'ifosfamide, l'imatinib, la L-asparaginase, le lapatinib, le lenolidamide, la leucovorine, le LFM-A13, la lomustine, la méchloréthamine, la melphalan, la mercaptopurine, la 6-mercaptopurine, le méthotrexate, la mitoxantrone, la mithramycine, la mitomycine, le mitotane, la navelbine, le neratinib, le nilotinib, la nitrosurée, l'olaparib, la plicomycine, la procarbazine, la paclitaxel, le PCI-32765, la pentostatine, le PSI-341, le raloxifène, la

semustine, le sorafenib, la streptozocine, le SU11248, le sunitinib, le tamoxifène, la témazolomide (une forme aqueuse de DTIC), le transplatine, le thalidomide, la thioguanine, le thiotépa, le téniposide, le topotécan, la moutarde à l'uracile, le vatalanib, la vinorelbine, la vinblastine, la vincristine, les vinca-alcaloïdes et le ZD1839.

**13.** Immunoconjugué pour l'utilisation selon la revendication 1 et au moins un agent thérapeutique pour l'utilisation selon la revendication 2, dans lequel le cancer est choisi dans le groupe constitué par le cancer du sein triple négatif, le cancer du sein, le cancer de l'ovaire, le cancer de l'endomètre, le cancer urothélial, le cancer de l'utérus, le cancer du poumon non à petites cellules, le cancer du poumon à petites cellules, le cancer de l'estomac, le cancer de la vessie urinaire, le cancer du rein, le cancer de la prostate et le cancer colorectal.

FIG. 1A

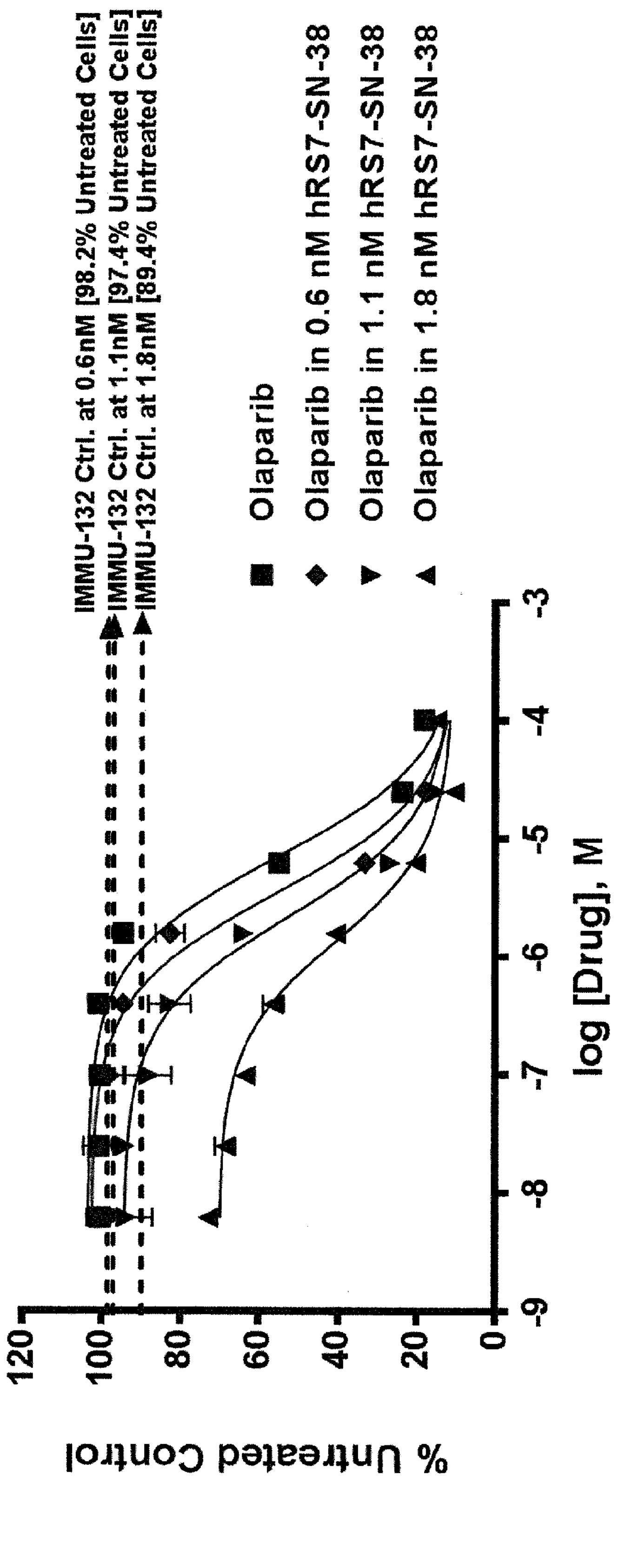

IMMU-132 Ctrl. at 0.6nM [98.2% Untreated Cells]
IMMU-132 Ctrl. at 1.1nM [97.4% Untreated Cells]
IMMU-132 Ctrl. at 1.8nM [89.4% Untreated Cells]
Olaparib
Olaparib in 0.6 nM hRS7-SN-38
Olaparib in 1.1 nM hRS7-SN-38
Olaparib in 1.8 nM hRS7-SN-38
% Untreated Control
120
100
80
60
40
20
0
-9
-8
-7
-6
-5
-4
-3
log [Drug], M

*FIG. 1B*

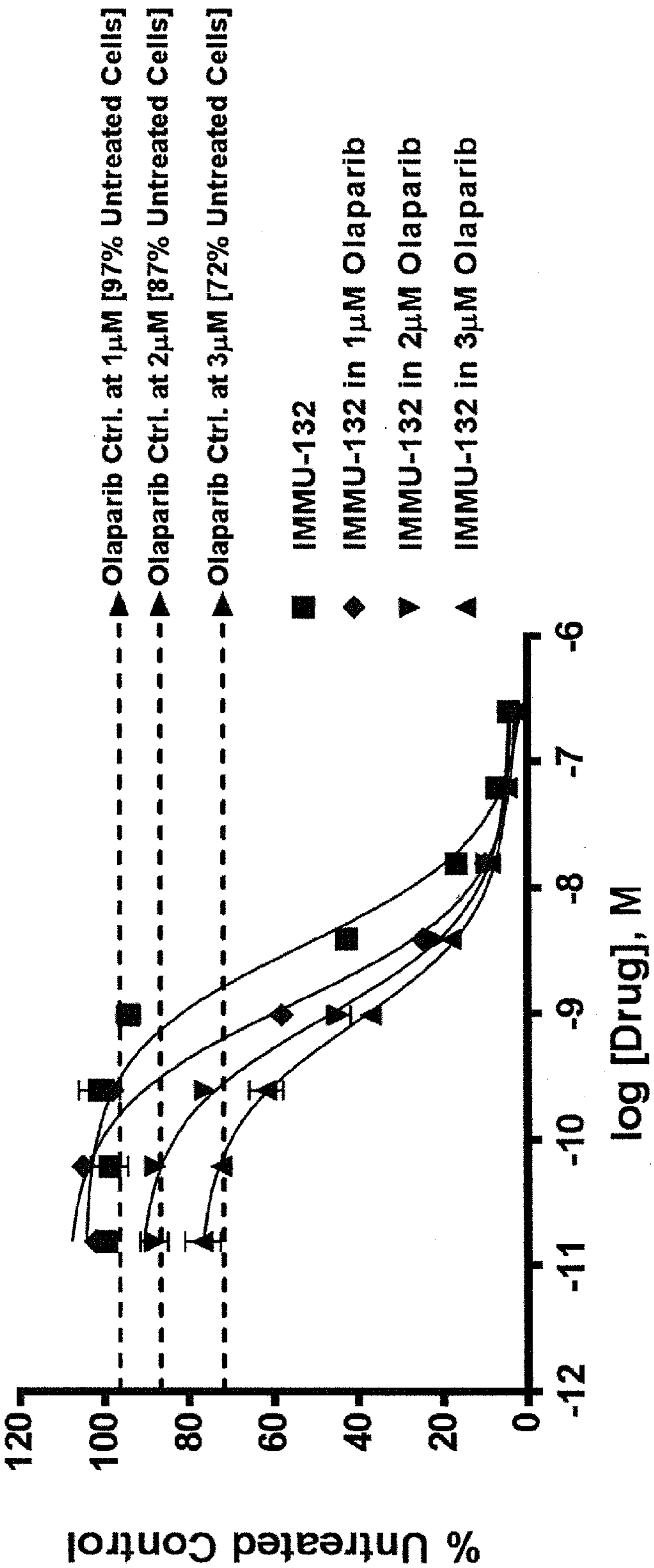
Olaparib Ctrl. at 1μM [97% Untreated Cells]
Olaparib Ctrl. at 2μM [87% Untreated Cells]
Olaparib Ctrl. at 3μM [72% Untreated Cells]
IMMU-132
IMMU-132 in 1μM Olaparib
IMMU-132 in 2μM Olaparib
IMMU-132 in 3μM Olaparib
% Untreated Control
120
100
80
60
40
20
0
-12
-11
-10
-9
-8
-7
-6
log [Drug], M

*FIG. 1C*

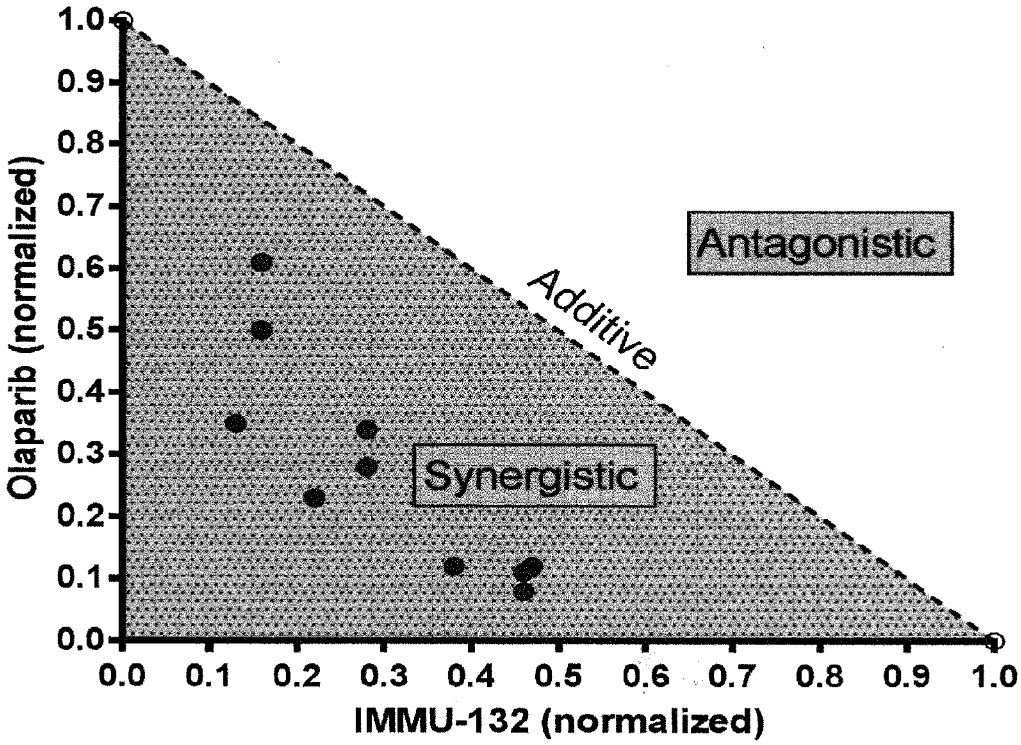


-⊖· $IC_{50}$-value of single agents

● $IC_{50}$ of IMMU-132 in constant Olaparib (1, 2, or 3 μM)

● $IC_{50}$ of Olaparib in constant IMMU-132 (0.6, 1.1, or 1.8 nM)

*FIG. 2A*

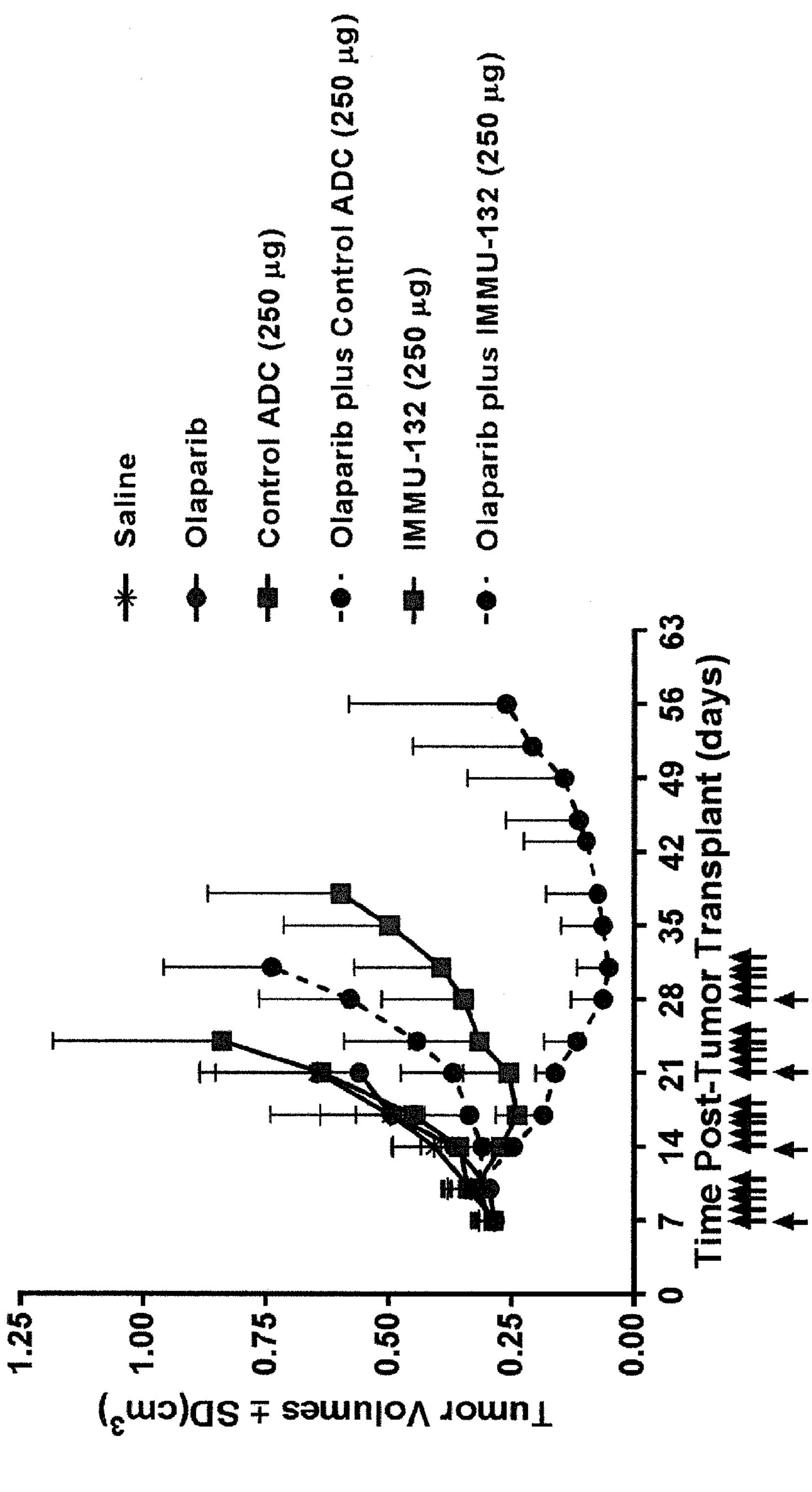
Saline
Olaparib
Control ADC (250 µg)
Olaparib plus Control ADC (250 µg)
IMMU-132 (250 µg)
Olaparib plus IMMU-132 (250 µg)
Tumor Volumes ± SD(cm³)
1.25
1.00
0.75
0.50
0.25
0.00
0
7
14
21
28
35
42
49
56
63
Time Post-Tumor Transplant (days)

*FIG. 2B*

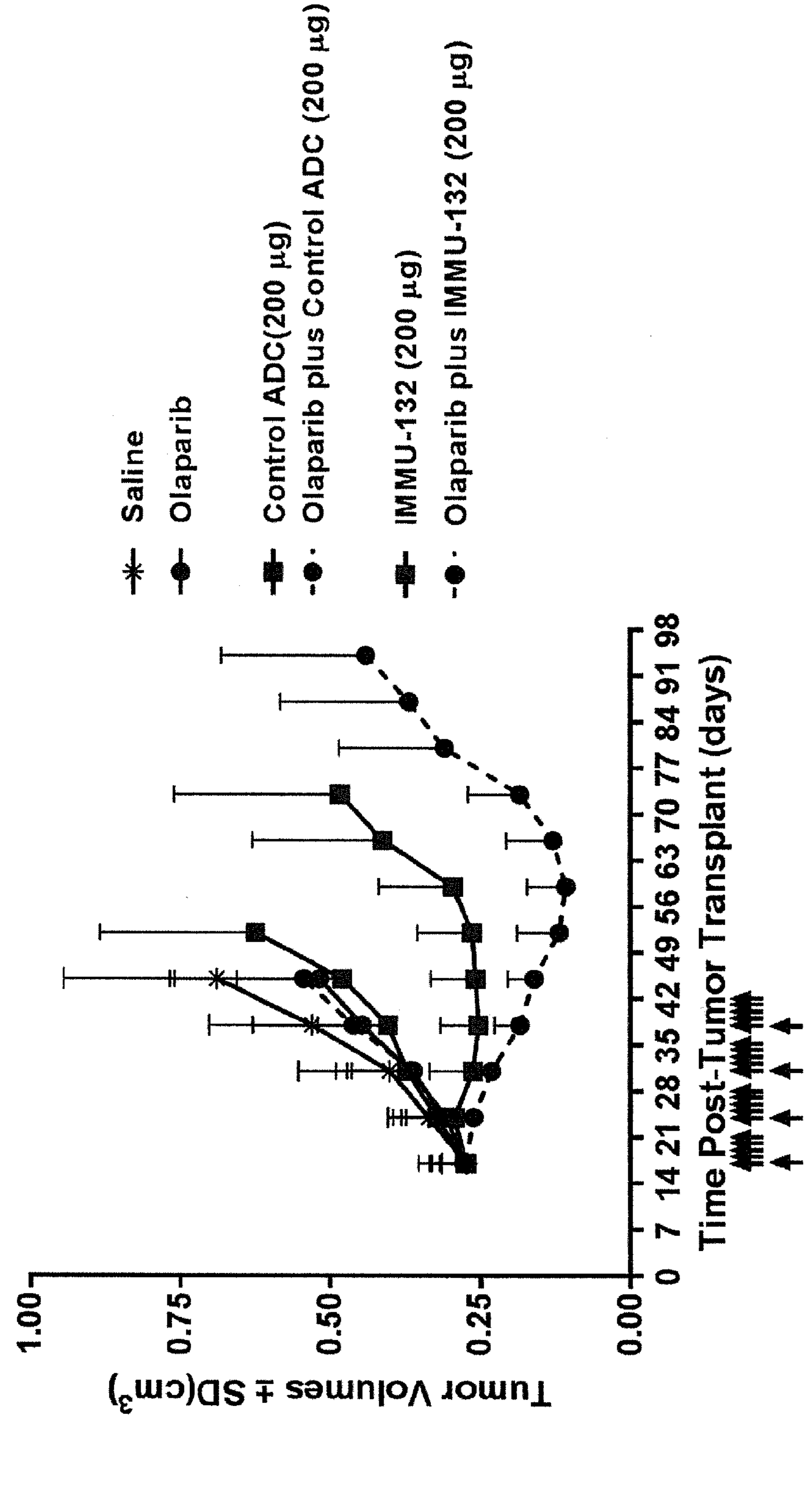
Saline
Olaparib
Control ADC(200 μg)
Olaparib plus Control ADC (200 μg)
IMMU-132 (200 μg)
Olaparib plus IMMU-132 (200 μg)
Tumor Volumes ± SD(cm3)
1.00
0.75
0.50
0.25
0.00
0 7 14 21 28 35 42 49 56 63 70 77 84 91 98
Time Post-Tumor Transplant (days)

FIG. 3A

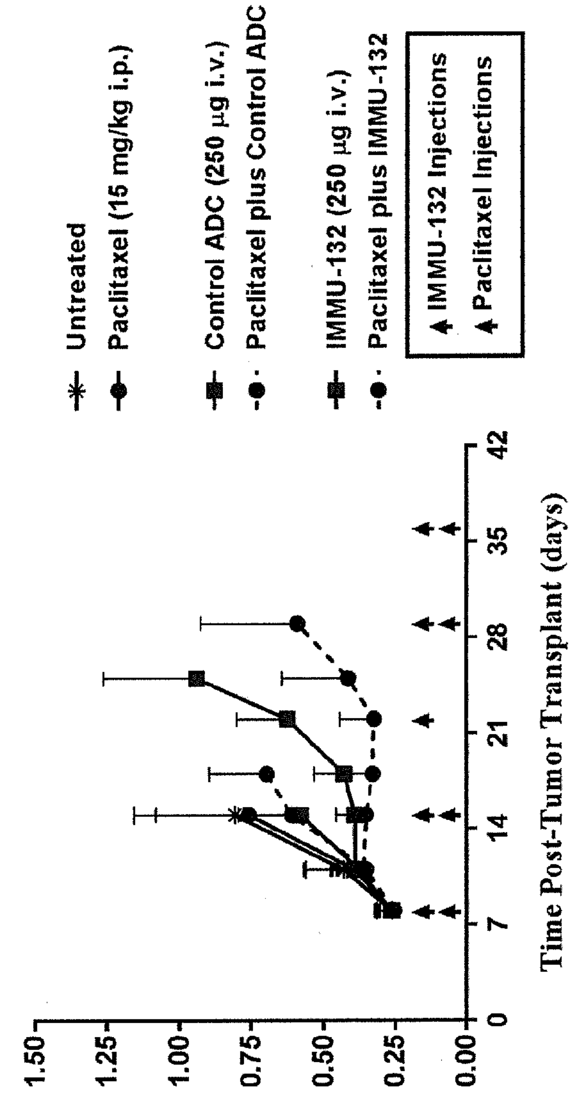

Mean Tumor Volumes ± s.d.(cm3)
1.50
1.25
1.00
0.75
0.50
0.25
0.00
0
7
14
21
28
35
42
Time Post-Tumor Transplant (days)
Untreated
Paclitaxel (15 mg/kg i.p.)
Control ADC (250 µg i.v.)
Paclitaxel plus Control ADC
IMMU-132 (250 µg i.v.)
Paclitaxel plus IMMU-132
IMMU-132 Injections
Paclitaxel Injections

*FIG. 3B*

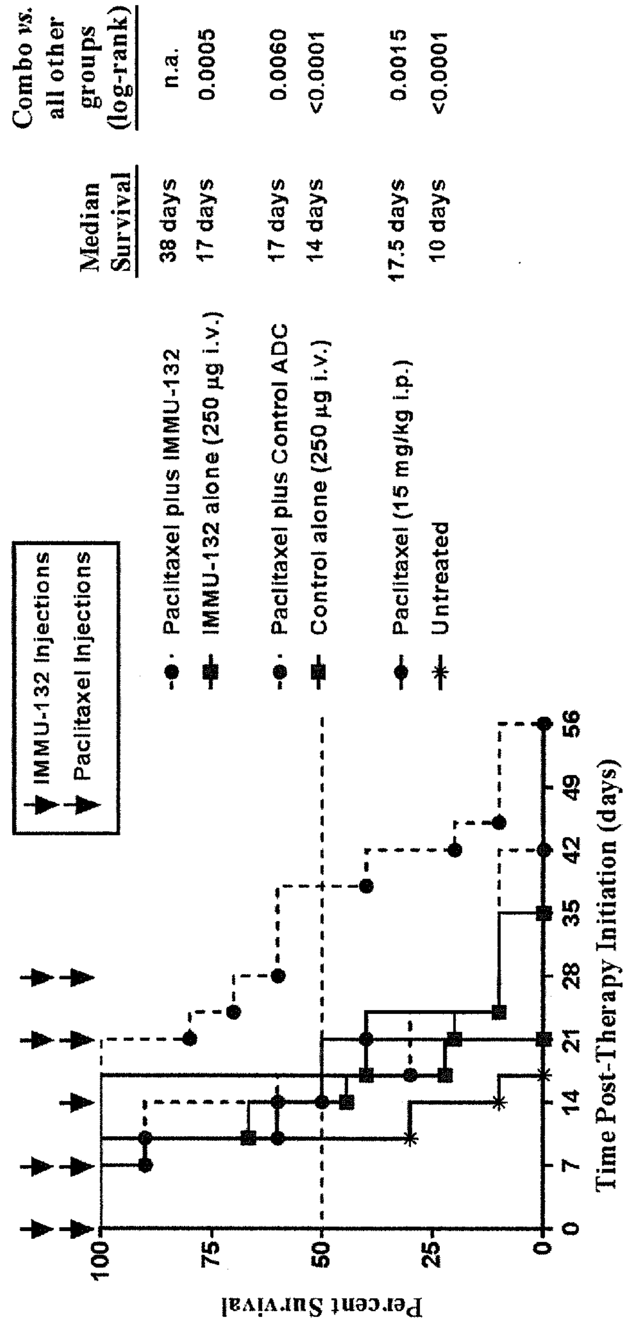

IMMU-132 Injections
Paclitaxel Injections
Percent Survival
100
75
50
25
0
0
7
14
21
28
35
42
49
56
Time Post-Therapy Initiation (days)
Median Survival
Combo vs. all other groups (log-rank)
Paclitaxel plus IMMU-132
38 days
n.a.
IMMU-132 alone (250 µg i.v.)
17 days
0.0005
Paclitaxel plus Control ADC
17 days
0.0060
Control alone (250 µg i.v.)
14 days
<0.0001
Paclitaxel (15 mg/kg i.p.)
17.5 days
0.0015
Untreated
10 days
<0.0001

*FIG. 3C*

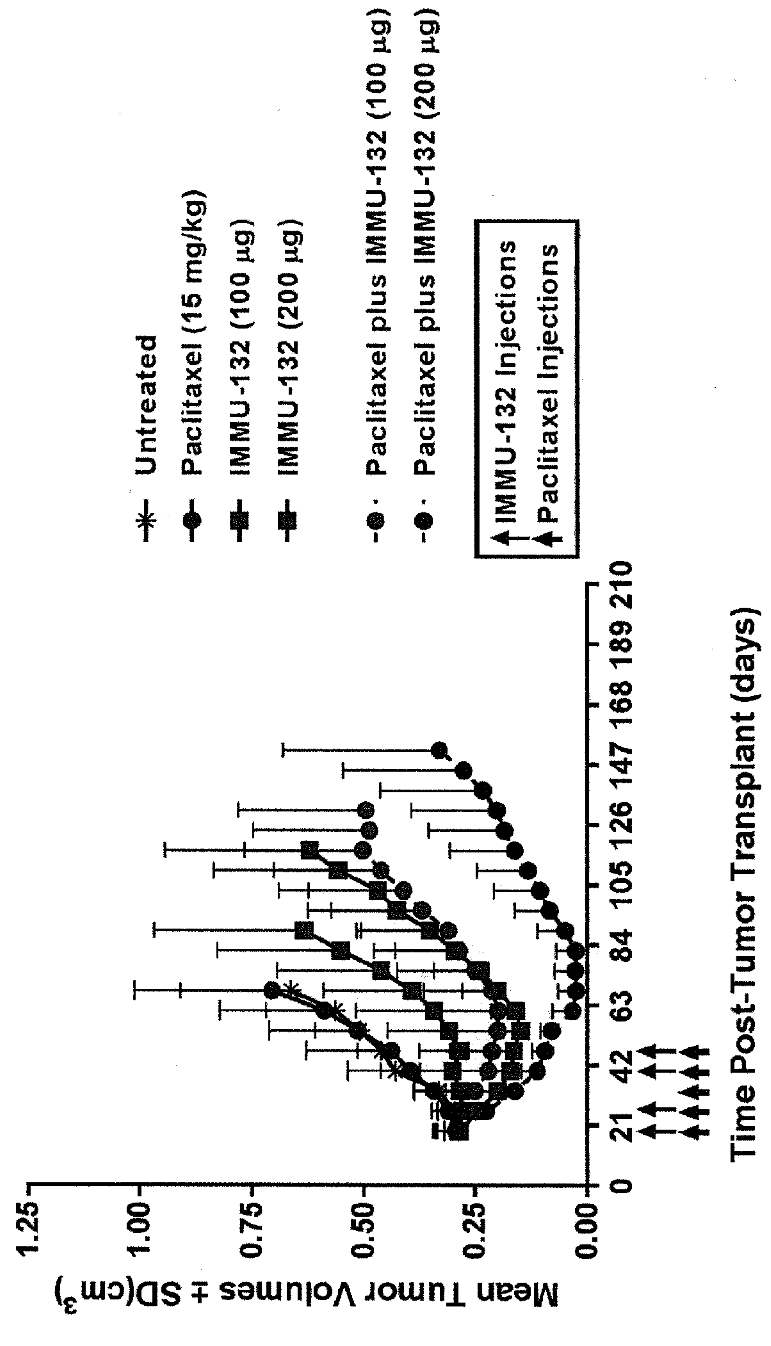
Mean Tumor Volumes ± SD(cm³)
1.25
1.00
0.75
0.50
0.25
0.00
0
21
42
63
84
105
126
147
168
189
210
Time Post-Tumor Transplant (days)
Untreated
Paclitaxel (15 mg/kg)
IMMU-132 (100 µg)
IMMU-132 (200 µg)
Paclitaxel plus IMMU-132 (100 µg)
Paclitaxel plus IMMU-132 (200 µg)
IMMU-132 Injections
Paclitaxel Injections

FIG. 3D

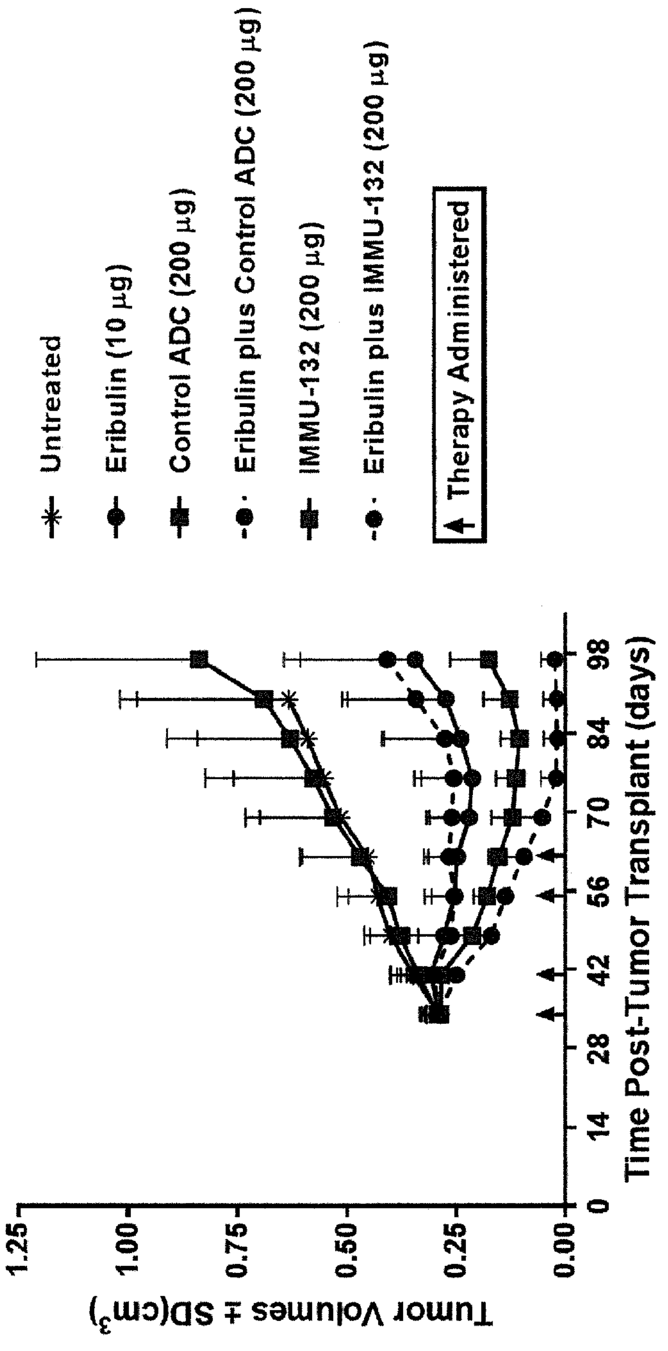
Tumor Volumes ± SD(cm$^3$)
1.25
1.00
0.75
0.50
0.25
0.00
0
14
28
42
56
70
84
98
Time Post-Tumor Transplant (days)
Untreated
Eribulin (10 μg)
Control ADC (200 μg)
Eribulin plus Control ADC (200 μg)
IMMU-132 (200 μg)
Eribulin plus IMMU-132 (200 μg)
Therapy Administered

*FIG. 3E*

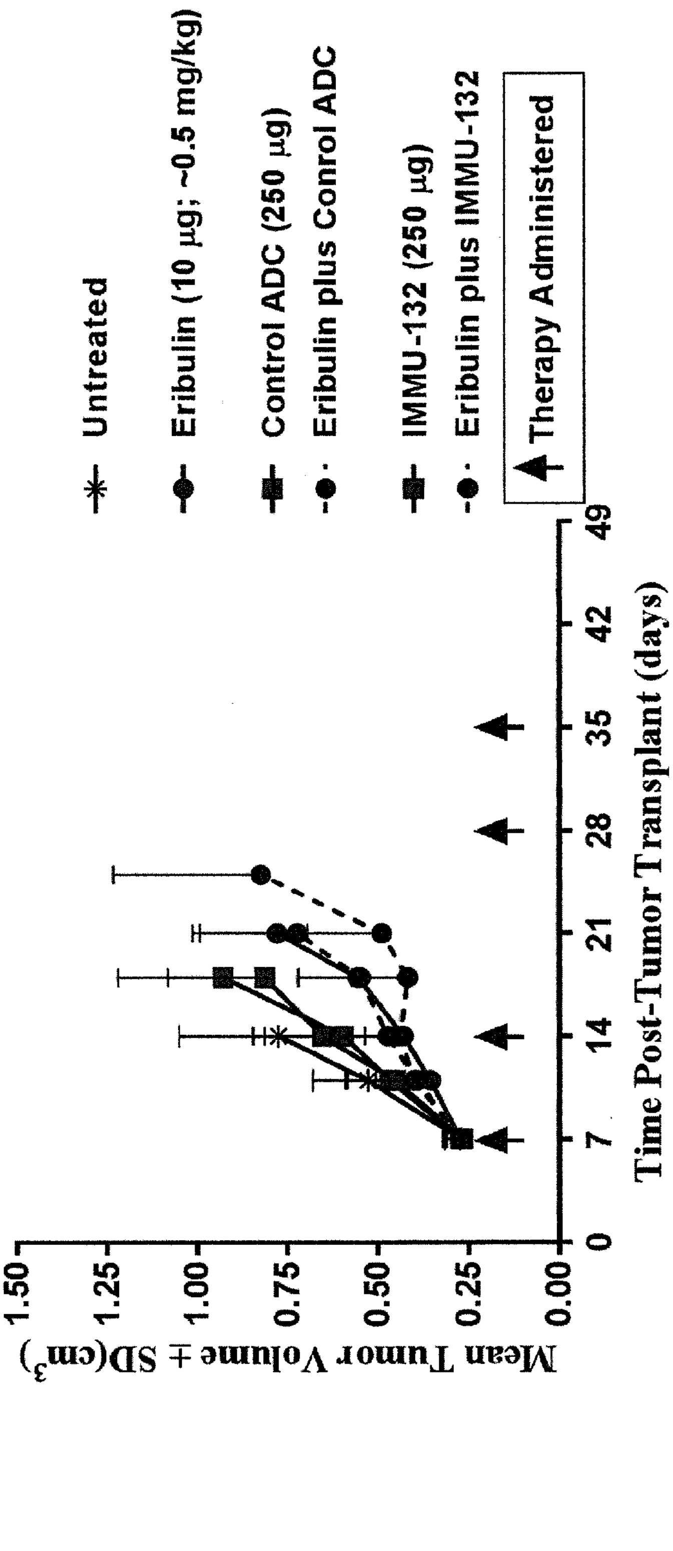
Mean Tumor Volume ± SD(cm³)
0.00
0.25
0.50
0.75
1.00
1.25
1.50
0
7
14
21
28
35
42
49
Time Post-Tumor Transplant (days)
Untreated
Eribulin (10 μg; ~0.5 mg/kg)
Control ADC (250 μg)
Eribulin plus Conrol ADC
IMMU-132 (250 μg)
Eribulin plus IMMU-132
Therapy Administered

FIG. 3F

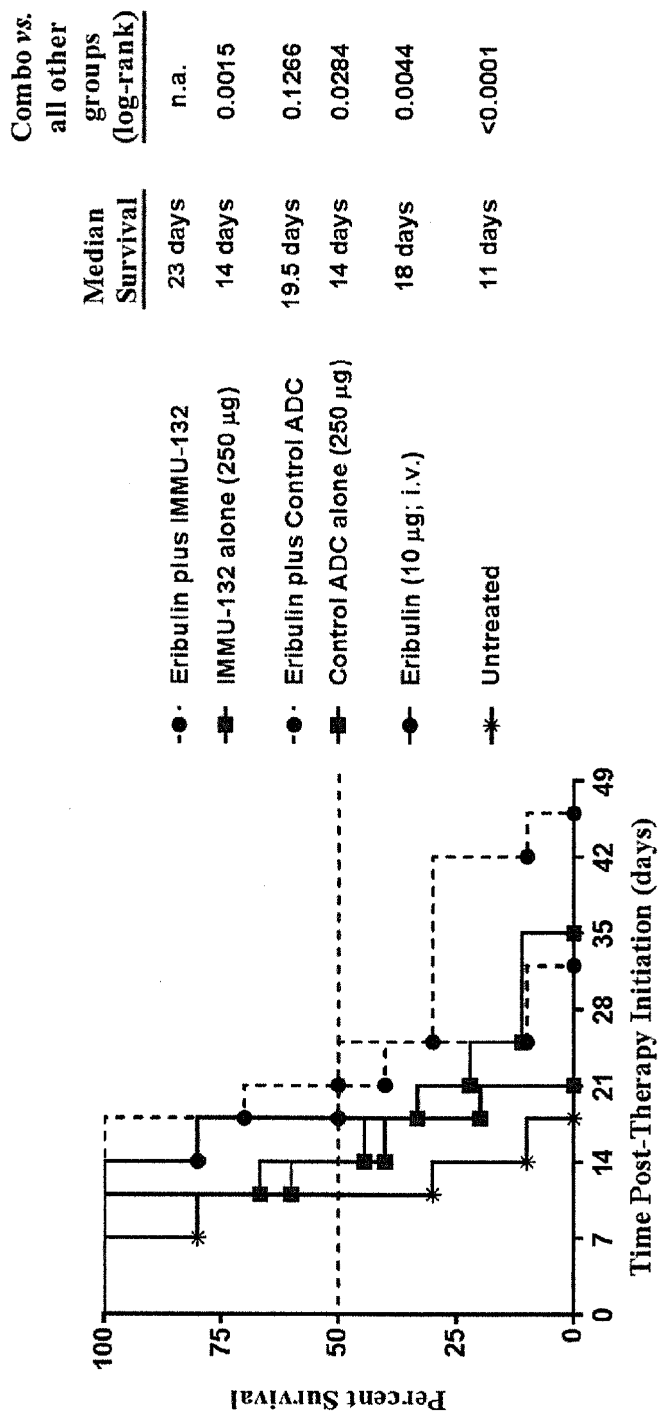
Eribulin plus IMMU-132
IMMU-132 alone (250 μg)
Eribulin plus Control ADC
Control ADC alone (250 μg)
Eribulin (10 μg; i.v.)
Untreated
Median Survival
23 days
14 days
19.5 days
14 days
18 days
11 days
Combo vs. all other groups (log-rank)
n.a.
0.0015
0.1266
0.0284
0.0044
<0.0001
Percent Survival
100
75
50
25
0
0
7
14
21
28
35
42
49
Time Post-Therapy Initiation (days)

*FIG. 4*

| Cell Line | Single Agent $IC_{50}$ (nM) | | Dose/Response + Constant Amount ($IC_{10}$, $IC_{20}$, $IC_{30}$) | | CI[c] |
|---|---|---|---|---|---|
| | IMMU-132 | Olaparib | IMMU-132 + Olaparib | Olaparib + IMMU-132 | |
| MDA-MB-468 (*PTEN* defective) | 3.87 | 8,696 | 1.46[a] | 4,389[b] | 0.8820 |
| | | | 0.84[a] | 2,442[b] | 0.4979 |
| | | | 0.51[a] | 709[b] | 0.2133 |
| MDA-MB-231 (*BRCA1/2* w.t.) | 5.19 | 28,803 | 1.89[a] | 16,679[b] | 0.9432 |
| | | | 0.51[a] | 5,695[b] | 0.2960 |
| | | | 0.19[a] | 3,382[b] | 0.1540 |
| HCC1806 (*BRCA1/2* defective) | 1.89 | 5,883 | 0.87[a] | 4,112[b] | 1.1593 |
| | | | 0.67[a] | 3,980[b] | 1.0310 |
| | | | 0.45[a] | 1,450[b] | 0.4846 |
| HCC38 (*BRCA1/2* defective) | 3.08 | 7,954 | 0.95[a] | 3,534[b] | 0.7528 |
| | | | 0.76[a] | 593[b] | 0.3213 |
| | | | 0.61[a] | 157[b] | 0.2178 |

*FIG. 5A*

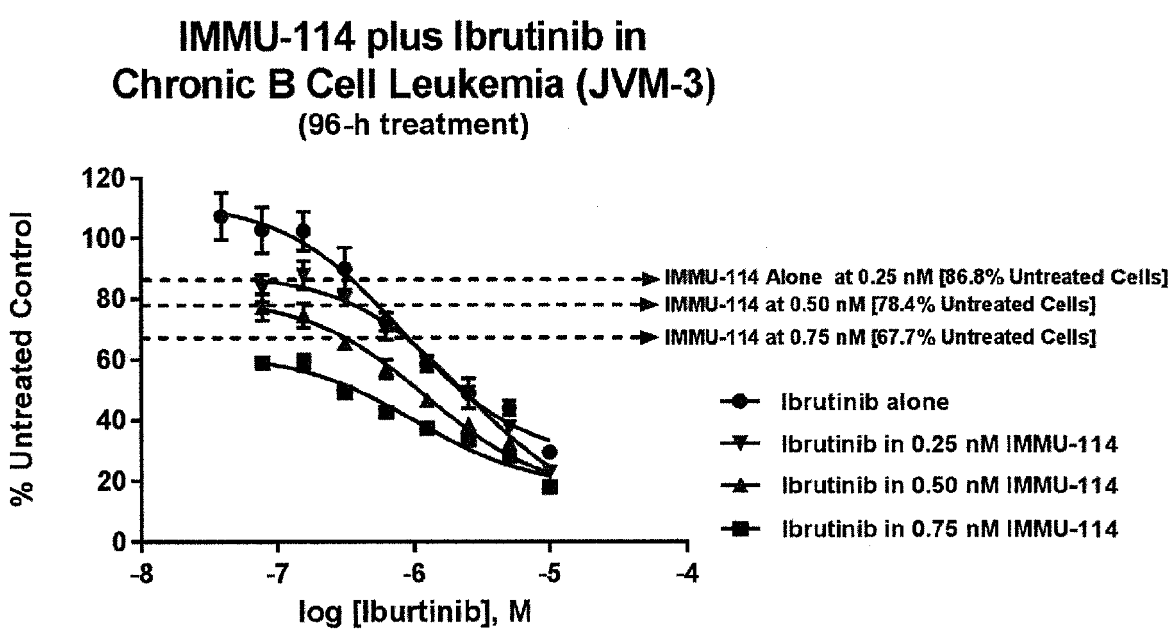


| | $IC_{50}$(M) | $R^2$ |
|---|---|---|
| Ibrutinib | $2.20x10^{-6}$ | 0.96 |
| Ibrutinib in 0.75 nM IMMU-114 | $0.38x10^{-6}$ | 0.96 |
| Ibrutinib in 0.50 nM IMMU-114 | $1.08x10^{-6}$ | 0.97 |
| Ibrutinib in 0.25 nM IMMU-114 | $2.26x10^{-6}$ | 0.98 |

*FIG. 5B*

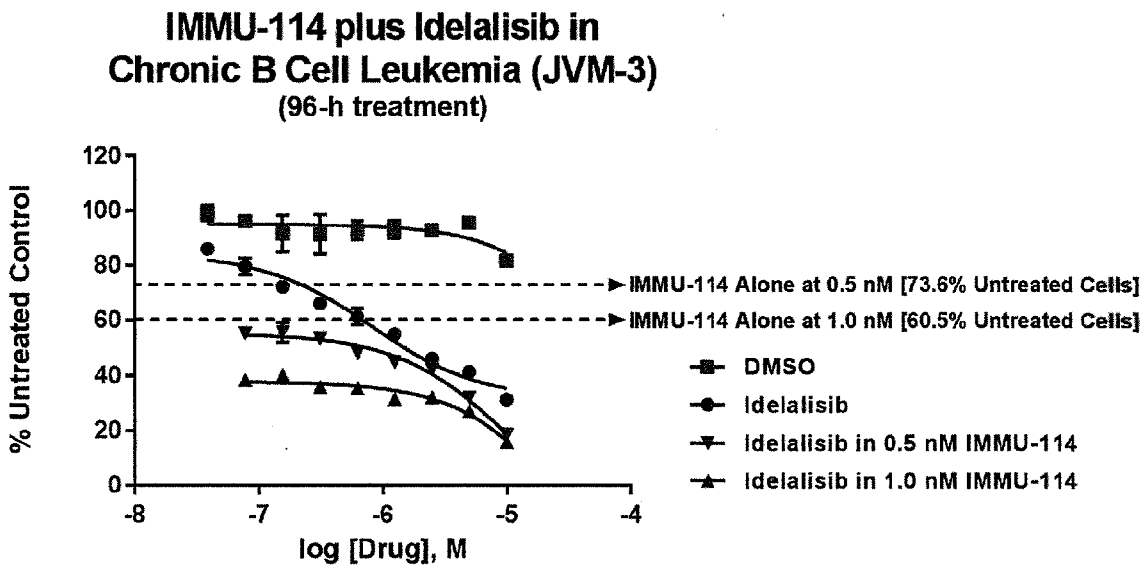


| | $IC_{50}$(M) | $R^2$ |
|---|---|---|
| Idelalisib | $1.51 \times 10^{-6}$ | 0.96 |
| Idelalisib in 1.0 nM IMMU-114 | $<0.08 \times 10^{-6}$ | 0.92 |
| Idelalisib in 0.5 nM IMMU-114 | $0.77 \times 10^{-6}$ | 0.97 |

FIG. 6A

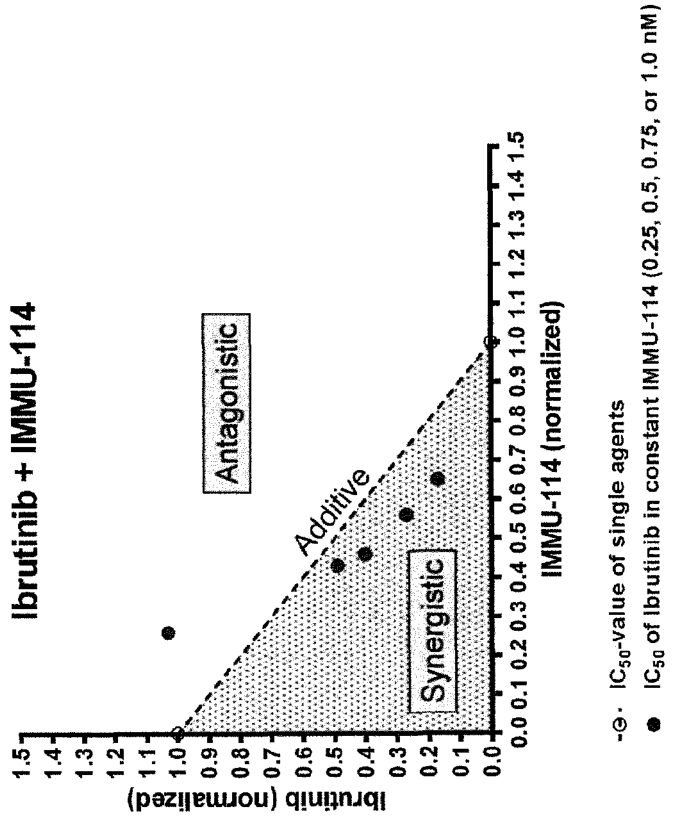
Ibrutinib + IMMU-114
Antagonistic
Additive
Synergistic
Ibrutinib (normalized)
IMMU-114 (normalized)
1.5 1.4 1.3 1.2 1.1 1.0 0.9 0.8 0.7 0.6 0.5 0.4 0.3 0.2 0.1 0.0
0.0 0.1 0.2 0.3 0.4 0.5 0.6 0.7 0.8 0.9 1.0 1.1 1.2 1.3 1.4 1.5
$IC_{50}$-value of single agents
$IC_{50}$ of Ibrutinib in constant IMMU-114 (0.25, 0.5, 0.75, or 1.0 nM)

FIG. 6B

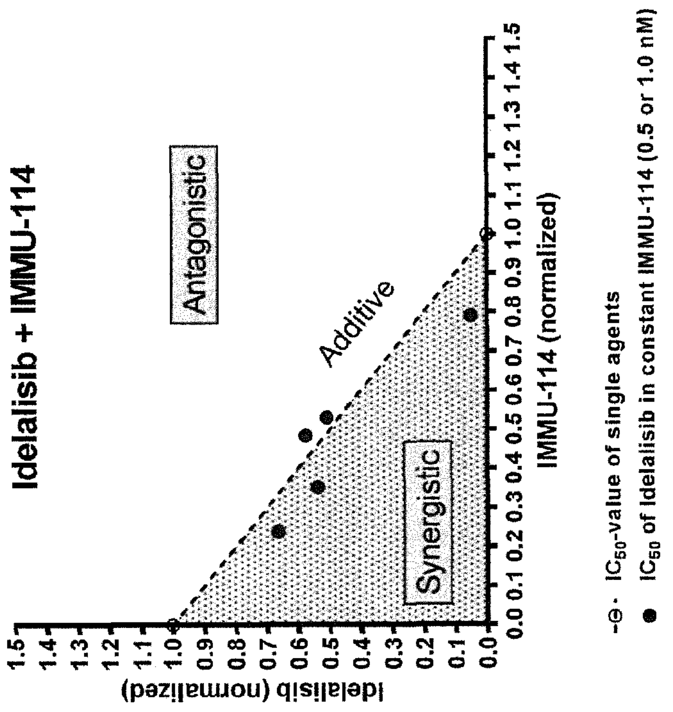
Idelalisib + IMMU-114
Antagonistic
Additive
Synergistic
Idelalisib (normalized)
IMMU-114 (normalized)
1.5 1.4 1.3 1.2 1.1 1.0 0.9 0.8 0.7 0.6 0.5 0.4 0.3 0.2 0.1 0.0
0.0 0.1 0.2 0.3 0.4 0.5 0.6 0.7 0.8 0.9 1.0 1.1 1.2 1.3 1.4 1.5
$IC_{50}$-value of single agents
$IC_{50}$ of Idelalisib in constant IMMU-114 (0.5 or 1.0 nM)

*FIG. 7*

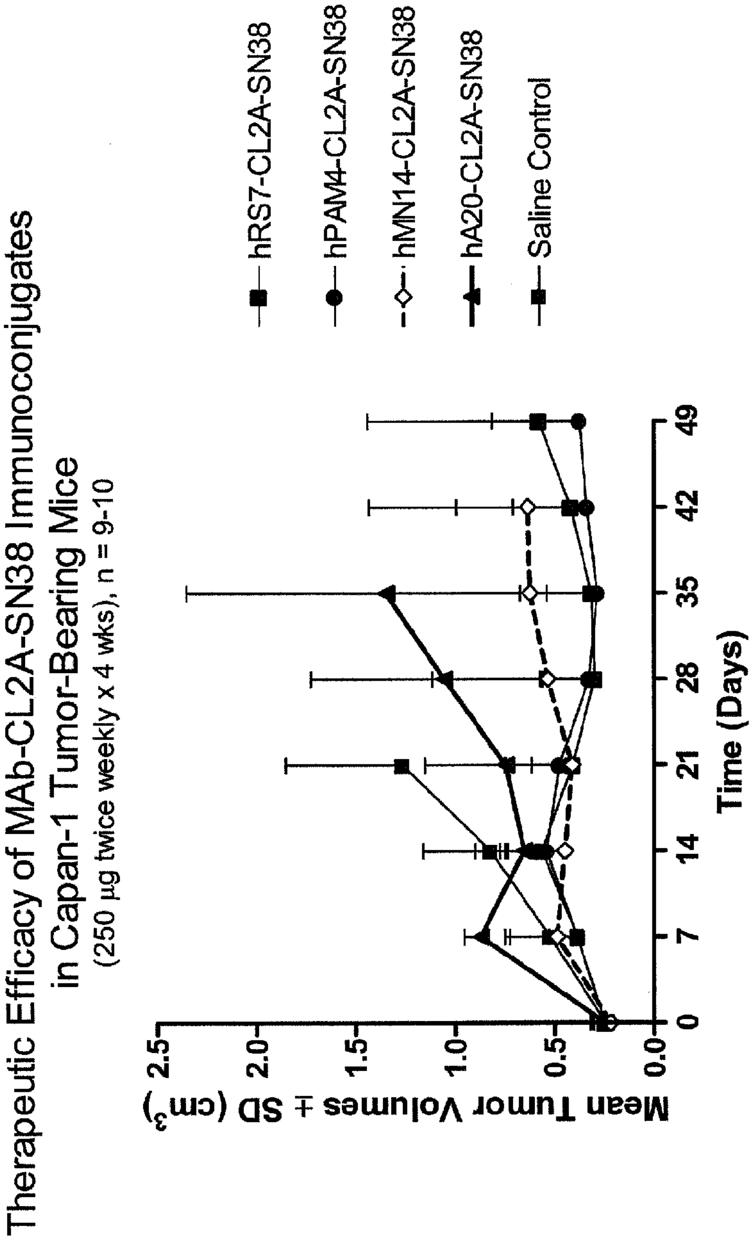
Therapeutic Efficacy of MAb-CL2A-SN38 Immunoconjugates
in Capan-1 Tumor-Bearing Mice
(250 µg twice weekly x 4 wks), n = 9-10
Mean Tumor Volumes ± SD (cm3)
2.5
2.0
1.5
1.0
0.5
0.0
0
7
14
21
28
35
42
49
Time (Days)
hRS7-CL2A-SN38
hPAM4-CL2A-SN38
hMN14-CL2A-SN38
hA20-CL2A-SN38
Saline Control

*FIG. 8*

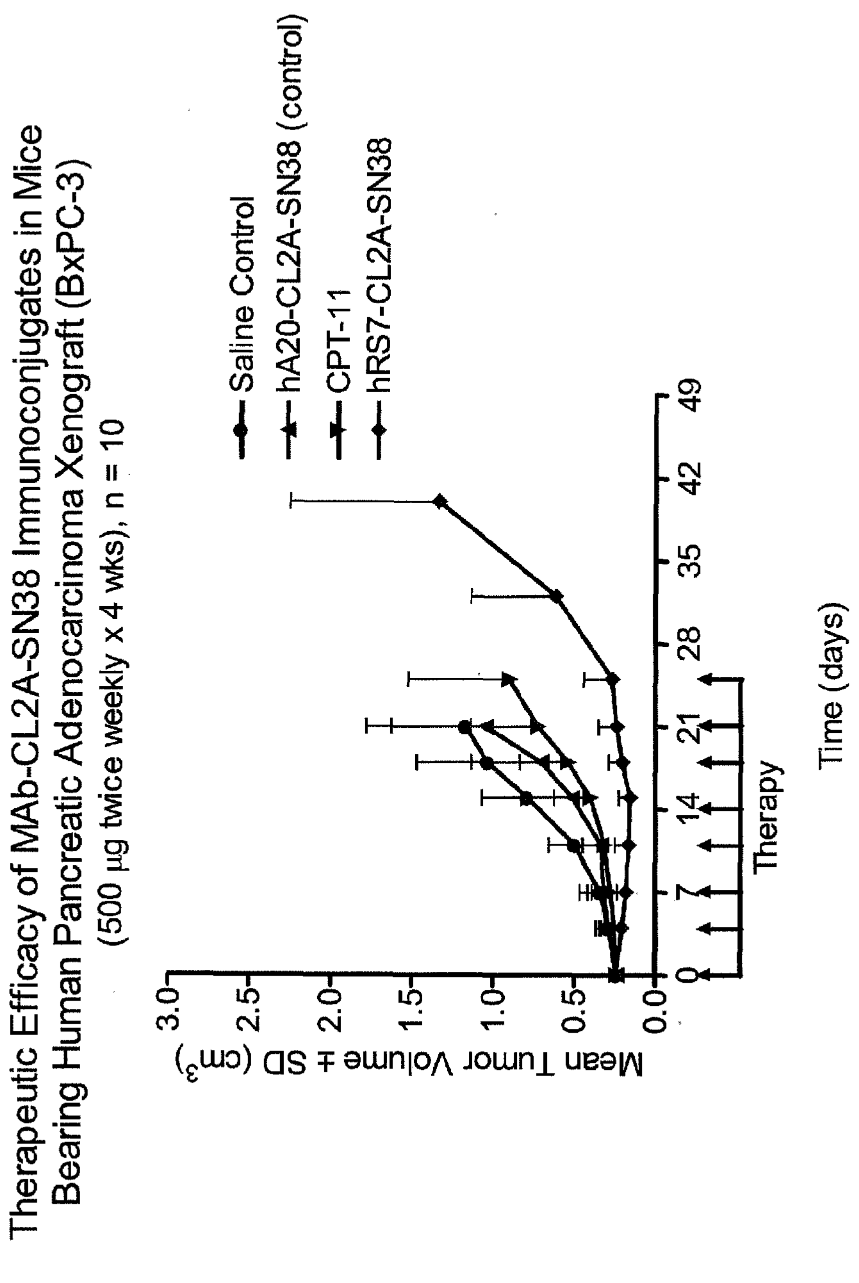
Therapeutic Efficacy of MAb-CL2A-SN38 Immunoconjugates in Mice
Bearing Human Pancreatic Adenocarcinoma Xenograft (BxPC-3)
(500 µg twice weekly x 4 wks), n = 10
Mean Tumor Volume ± SD ($cm^3$)
3.0
2.5
2.0
1.5
1.0
0.5
0.0
0
7
14
21
28
35
42
49
Therapy
Time (days)
Saline Control
hA20-CL2A-SN38 (control)
CPT-11
hRS7-CL2A-SN38

*FIG. 9*

Therapeutic Efficacy of hMN-14-CL2A-SN38 in Experimental Human Colorectal Cancer (LS 174T)
(500 mg i.p. Twice a wk x 2 wks), n = 10

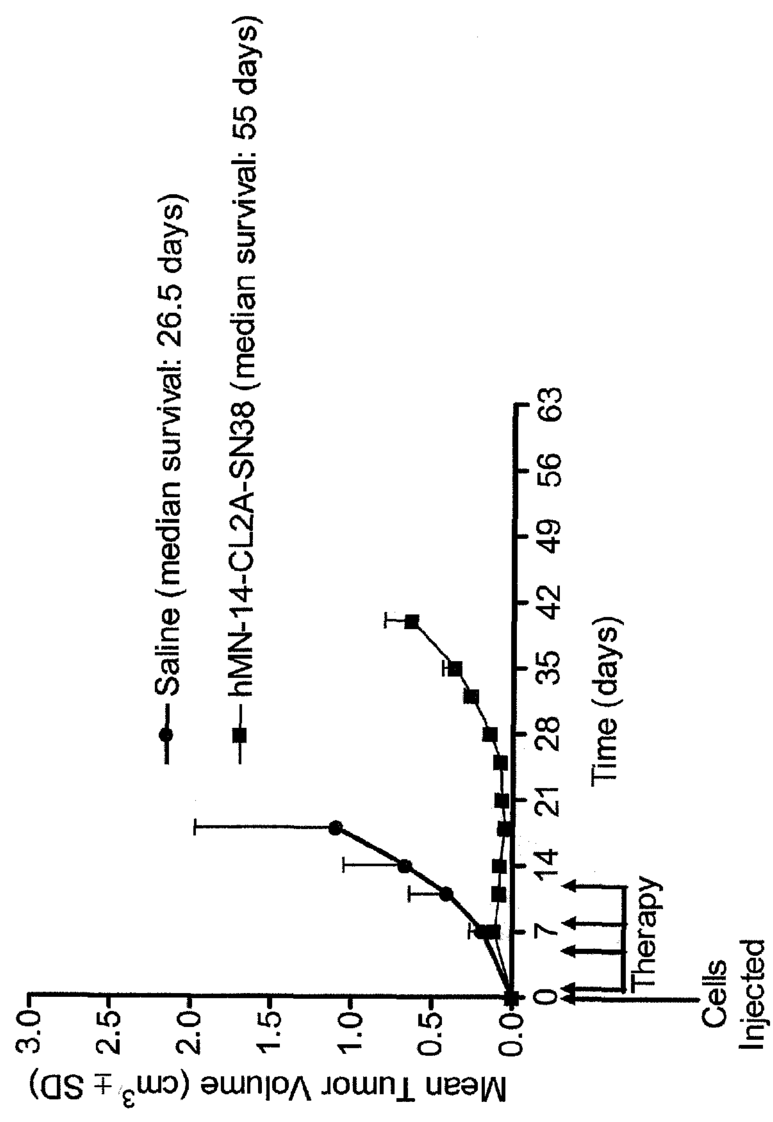

*FIG. 10*

Survival Curves of hMN14-CL-SN38 Treated Mice
Bearing GW-39 Lung Metastatic Disease
(i.p. q4dx8)

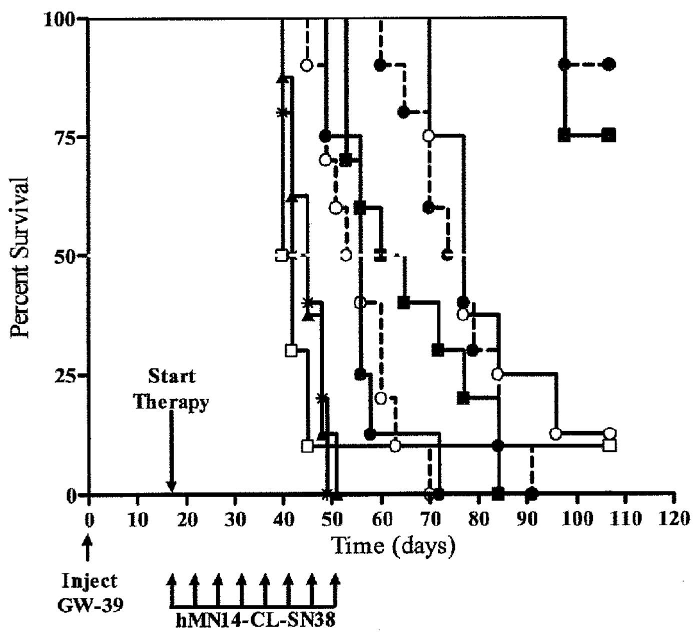


| | | Median Survival | Survivors/ Total |
|---|---|---|---|
| MSR=7.7 | 250 µg hMN14-CL1-SN38 | >107 days | 6/8 |
| | 100 µg hMN14-CL1-SN38 | 62.5 days | 0/10 |
| | 50 µg hMN14-CL1-SN38 | 41 days | 1/10 |
| MSR=6.8 | 250 µg hMN14-CL2-SN38 | >107 days | 9/10 |
| | 100 µg hMN14-CL2-SN38 | 75.5 days | 0/10 |
| | 50 µg hMN14-CL2-SN38 | 54.5 days | 0/10 |
| | | 40 days | 0/7 |
| MSR=7.4 | 250 µg hLL2-CL1-SN38 | 56 days | 0/8 |
| MSR=6.6 | 250 µg hLL2-CL2-SN38 | 77 days | 1/8 |
| | 250 µg hMN14 IgG + free SN38 | 45 days | 0/8 |
| | 100 µL Saline | 43.5 days | 0/10 |

*FIG. 11A*

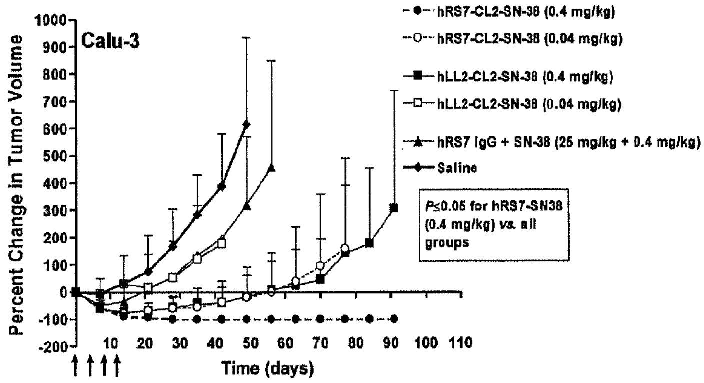


*FIG. 11B*

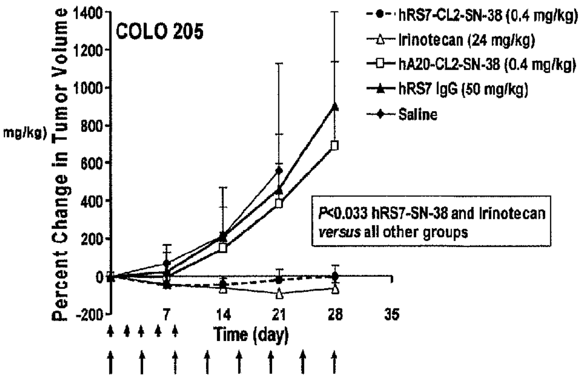


*FIG. 11C*

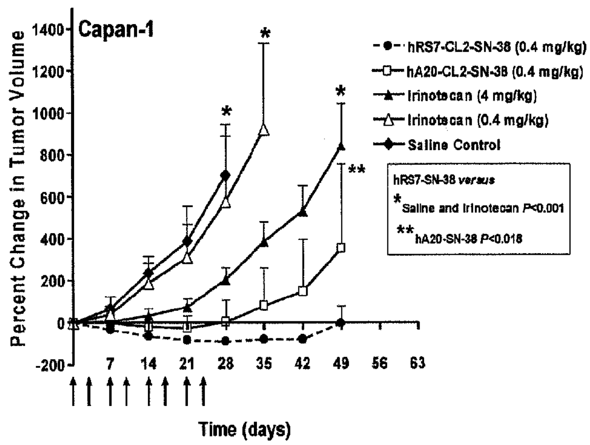


*FIG. 11D*

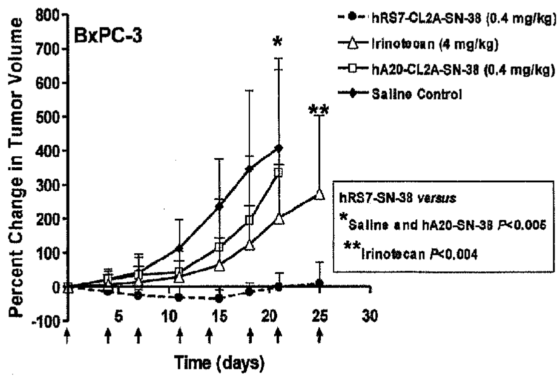


*FIG. 11E*

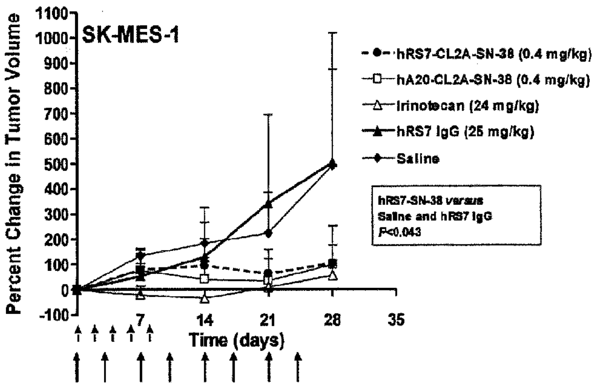

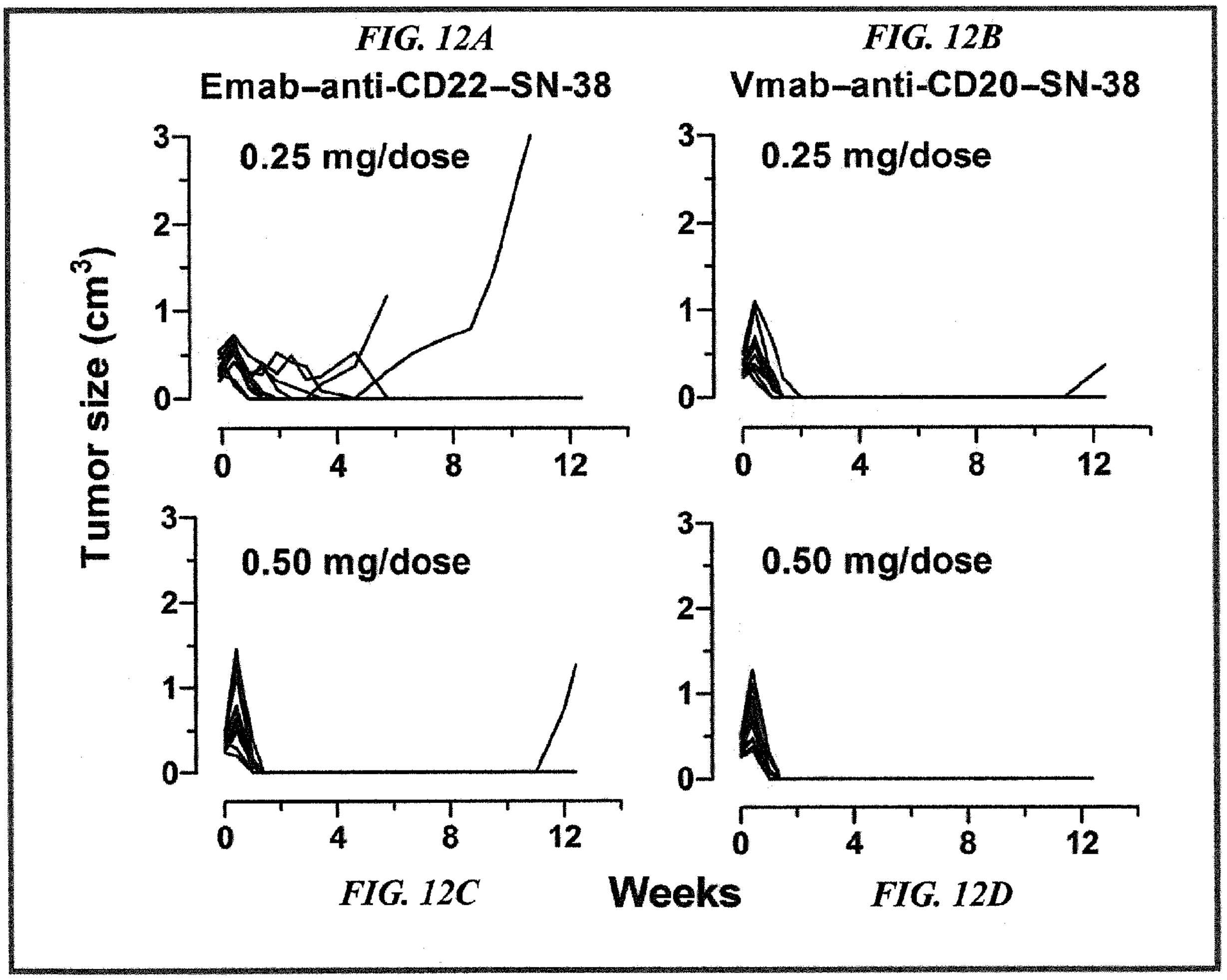
FIG. 12A
Emab–anti-CD22–SN-38
FIG. 12B
Vmab–anti-CD20–SN-38
0.25 mg/dose
0.25 mg/dose
0.50 mg/dose
0.50 mg/dose
Tumor size (cm3)
0
1
2
3
0
4
8
12
FIG. 12C
Weeks
FIG. 12D

FIG. 13A

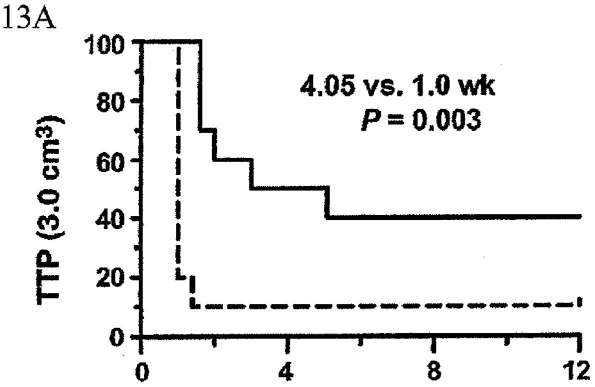


FIG. 13B

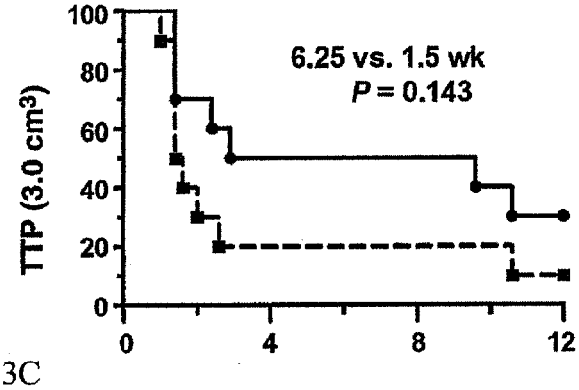


FIG. 13C

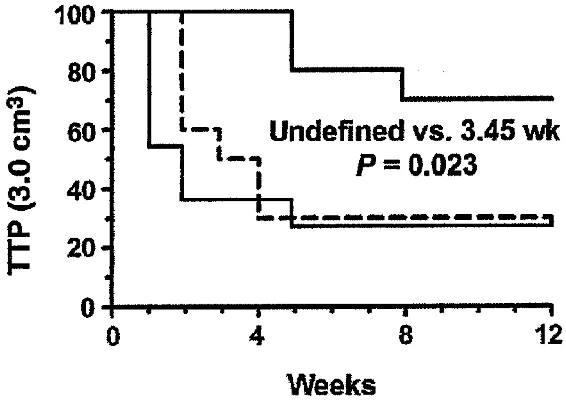

*FIG. 14*

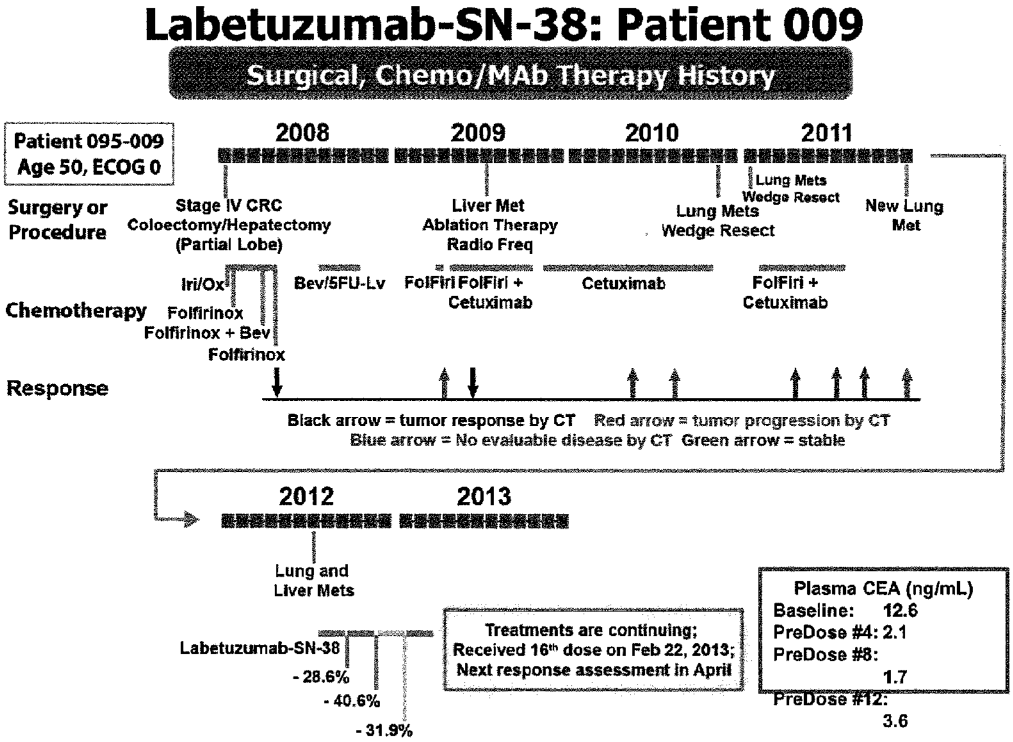
Labetuzumab-SN-38: Patient 009
Surgical, Chemo/MAb Therapy History
Patient 095-009
Age 50, ECOG 0
2008
2009
2010
2011
Surgery or Procedure
Stage IV CRC
Coloectomy/Hepatectomy
(Partial Lobe)
Liver Met
Ablation Therapy
Radio Freq
Lung Mets
Wedge Resect
Lung Mets
Wedge Resect
New Lung
Met
Chemotherapy
Iri/Ox
Folfirinox
Folfirinox + Bev
Folfirinox
Bev/5FU-Lv
FolFiri
FolFiri +
Cetuximab
Cetuximab
FolFiri +
Cetuximab
Response
Black arrow = tumor response by CT Red arrow = tumor progression by CT
Blue arrow = No evaluable disease by CT Green arrow = stable
2012
2013
Lung and
Liver Mets
Labetuzumab-SN-38
- 28.6%
- 40.6%
- 31.9%
Treatments are continuing;
Received 16th dose on Feb 22, 2013;
Next response assessment in April
Plasma CEA (ng/mL)
Baseline: 12.6
PreDose #4: 2.1
PreDose #8: 1.7
PreDose #12: 3.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015047510 A1
- US 68881212
- US 7282567 B
- US 7151164 B
- US 7109304 B
- US 7300655 B
- US 7312318 B
- US 5789554 A
- US 7387772 B
- US 7612180 B
- US 6676924 B
- US 8287865 B
- US 7238785 B
- US 7541440 B
- US 7138496 B
- US 983372
- WO 2009130575 A
- US 8333971 B
- US 5831034 A
- US 5911989 A
- US 5567610 A
- US 5229275 A
- US 7074403 B
- US 7060802 B
- US 7056509 B
- US 7049060 B
- US 7045132 B
- US 7041803 B
- US 7041802 B
- US 7041293 B
- US 7038018 B
- US 7037498 B
- US 7012133 B
- US 7001598 B
- US 6998468 B
- US 6994976 B
- US 6994852 B
- US 6989241 B
- US 6974863 B
- US 6965018 B
- US 6964854 B
- US 6962981 B
- US 6962813 B
- US 6956107 B
- US 6951924 B
- US 6949244 B
- US 6946129 B
- US 6943020 B
- US 6939547 B
- US 6921645 B
- US 6921533 B
- US 6919433 B
- US 6919078 B
- US 6916475 B
- US 6905681 B
- US 6899879 B
- US 6893625 B
- US 6887468 B
- US 6887466 B
- US 6884594 B
- US 6881405 B
- US 6878812 B
- US 6875580 B
- US 6872568 B
- US 6867006 B
- US 6864062 B
- US 6861511 B
- US 6861227 B
- US 6861226 B
- US 6838282 B
- US 6835549 B
- US 6835370 B
- US 6824780 B
- US 6824778 B
- US 6812206 B
- US 6793924 B
- US 6783758 B
- US 6770450 B
- US 6767711 B
- US 6764688 B
- US 6764681 B
- US 6764679 B
- US 6743898 B
- US 6733981 B
- US 6730307 B
- US 6720155 B
- US 6716966 B
- US 6709653 B
- US 6693176 B
- US 6692908 B
- US 6689607 B
- US 6689362 B
- US 6689355 B
- US 6682737 B
- US 6682736 B
- US 6682734 B
- US 6673344 B
- US 6653104 B

- US 6652852 B
- US 6635482 B
- US 6630144 B
- US 6610833 B
- US 6610294 B
- US 6605441 B
- US 6605279 B
- US 6596852 B
- US 6592868 B
- US 6576745 B
- US 6572856 B
- US 6566076 B
- US 6562618 B
- US 6545130 B
- US 6544749 B
- US 6534058 B
- US 6528625 B
- US 6528269 B
- US 6521227 B
- US 6518404 B
- US 6511665 B
- US 6491915 B
- US 6488930 B
- US 6482598 B
- US 6482408 B
- US 6479247 B
- US 6468531 B
- US 6468529 B
- US 6465173 B
- US 6461823 B
- US 6458356 B
- US 6455044 B
- US 6455040 B
- US 6451310 B
- US 6444206 B
- US 6441143 B
- US 6432404 B
- US 6432402 B
- US 6419928 B
- US 6413726 B
- US 6406694 B
- US 6403770 B
- US 6403091 B
- US 6395276 B
- US 6395274 B
- US 6387350 B
- US 6383759 B
- US 6383484 B
- US 6376654 B
- US 6372215 B
- US 6359126 B
- US 6355481 B
- US 6355444 B
- US 6355245 B
- US 6355244 B
- US 6346246 B
- US 6344198 B
- US 6340571 B
- US 6340459 B
- US 6331175 B
- US 6306393 B
- US 6254868 B
- US 6187287 B
- US 6183744 B
- US 6129914 B
- US 6120767 B
- US 6096289 B
- US 6077499 B
- US 5922302 B
- US 5874540 B
- US 5814440 B
- US 5798229 B
- US 5789554 B
- US 5776456 B
- US 5736119 B
- US 5716595 B
- US 5677136 B
- US 5587459 B
- US 5443953 B
- US 5525338 B
- WO 9111465 A, Goldenberg
- US 4036945 A
- US 4331647 A
- US 4946778 A
- US 7217797 B
- US 5686072 A
- US 5874540 A
- US 6107090 A
- US 6730300 B
- US 6899864 B
- US 6926893 B
- US 6962702 B
- US 7230084 B
- US 7238786 B
- US 7256004 B
- US 7585491 B
- US 7642239 B
- US 20050271671 A
- US 20060193865 A
- US 20060210475 A
- US 20070087001 A
- US 0611767 A
- US 42637902
- US 7405320 B
- EP 0453082 A
- US 5132405 A
- US 7521056 B
- US 7527787 B
- US 7534866 B
- US 7550143 B
- US 7666400 B
- US 7858070 B
- US 7871622 B
- US 7906121 B
- US 7906118 B
- US 8163291 B

- US 7901680 B
- US 7981398 B
- US 8003111 B
- US 8034352 B
- US 6083477 A
- US 20030124058
- US 20030219433 A
- US 20040001825 A
- US 20040202666 A
- US 20040219156 A
- US 20040219203 A
- US 20040235065 A
- US 20050002945 A
- US 20050014207 A
- US 20050025709 A
- US 20050079184 A
- US 20050169926 A
- US 20050175582 A
- US 20050249738 A
- US 20060014245 A
- US 20060034759 A
- US 90173713
- US 4554101 A
- US 20040175756
- US 20050048512 A
- US 20050053973 A
- US 20050089932 A
- US 20050221384 A
- US 5223409 A
- US 5622699 A
- US 6068829 A
- US 5840841 A
- US 5705610 A
- US 5670312 A
- US 5492807 A
- US 5582981 A
- US 5595877 A
- US 5637459 A
- US 5475096 A
- US 5270163 A
- US 7517964 B
- US 8084583 B
- US 7951369 B
- US 8267865 B
- US 7772373 B
- US 7919087 B
- US 7931903 B
- US 7641901 B
- US 7414121 B
- US 7553953 B
- US 7641891 B
- US 7670804 B
- US 7435803 B
- US 8287864 B
- US 7462352 B
- US 7902338 B
- US 8147831 B
- US 8337840 B
- US 6077499 A
- US 7999083 B
- US 175089
- US 8877202 B

**Non-patent literature cited in the description**


- **SHARKEY ; GOLDENBERG.** *CA Cancer J Clin.,* July 2006, vol. 56 (4), 226-243
- **DADACHOVA ; CASADEVALL.** *Q J Nucl Med Mol Imaging,* 2006, vol. 50 (3), 193-204
- **IYER ; RATAIN.** *Cancer Chemother. Phamacol.,* 1998, vol. 42, S31-S43
- **LIU et al.** The Camptothecins: Unfolding Their Anticancer Potential. NY Acad Sci, 2000, vol. 922, 1-10
- **VAN DER NEUT KOLFSCHOTEN et al.** *Science,* 2007, vol. 317, 1554-1557
- **SENSI et al.** *Clin Cancer Res,* 2006, vol. 12, 5023-32
- **PARMIANI et al.** *J Immunol,* 2007, vol. 178, 1975-79
- **NOVELLINO et al.** *Cancer Immunol Immunother,* 2005, vol. 54, 187-207
- **OFEI et al.** *Diabetes,* 2011, vol. 45, 881-85
- **KSIEZAK-REDING et al.** *J Biol Chem,* 1987, vol. 263, 7943-47
- **PAULIK et al.** *Biochem Pharmacol,* 1999, vol. 58, 1781-90
- **VCELAR et al.** *AIDS,* 2007, vol. 21 (16), 2161-2170
- **JOOS et al.** *Antimicrob. Agents Chemother.,* 2006, vol. 50 (5), 1773-9
- ACS symposium series. American Chemical Society, 1995, vol. 574
- **NAGY et al.** *Proc. Natl. Acad. Sci. USA,* 1996, vol. 93, 2464-2469
- **IYER ; RATAIN.** *Cancer Chemother Pharmacol.,* 1998, vol. 42, 31-43
- **MATHIJSSEN et al.** *Clin Cancer Res.,* 2002, vol. 7, 2182-2194
- **RIVORY.** *Ann NY Acad Sci.,* 2000, vol. 922, 205-215
- **SKOLNIK et al.** *Ther Drug Monit,* 2010, vol. 32, 741-48
- **DUMONTET ; JORDAN.** *Nat Rev Drug Discov,* 2010, vol. 9, 790-803
- **ROULEAU et al.** *Nat Rev Cancer,* 2010, vol. 10, 293-301
- **BAO et al.** *Oncotarget [Epub ahead of print,* 22 September 2015

- **VAN DER NEUT KOLFSCHOTEN et al.** *Science,* 14 September 2007, vol. 317, 1554-1557
- **GREEN et al.** *Nature Genet.,* 1994, vol. 7, 13
- **LONBERG et al.** *Nature,* 1994, vol. 368, 856
- **TAYLOR et al.** *Int. Immun.,* 1994, vol. 6, 579
- **MCCAFFERTY et al.** *Nature,* 1990, vol. 348, 552-553
- **JOHNSON ; CHISWELL.** *Current Opinion in Structural Biology,* 1993, vol. 3, 5564-571
- **KÖHLER ; MILSTEIN.** *Nature,* 1975, vol. 256, 495
- **COLIGAN et al.** CURRENT PROTOCOLS IN IMMUNOLOGY. John Wiley & Sons, 1991, vol. 1, 2.5.1-2.6.7
- Purification of Immunoglobulin G (IgG). **BAINES et al.** METHODS IN MOLECULAR BIOLOGY. The Humana Press, Inc, 1992, vol. 10, 79-104
- **LEUNG et al.** *Hybridoma,* 1994, vol. 13, 469
- **JONES et al.** *Nature,* 1986, vol. 321, 522
- **RIECHMANN et al.** *Nature,* 1988, vol. 332, 323
- **VERHOEYEN et al.** *Science,* 1988, vol. 239, 1534
- **CARTER et al.** *Proc. Nat'l Acad. Sci. USA,* 1992, vol. 89, 4285
- **SANDHU.** *Crit. Rev. Biotech.,* 1992, vol. 12, 437
- **TEMPEST et al.** *Biotechnology,* 1991, vol. 9, 266
- **SINGER et al.** *J. Immun.,* 1993, vol. 150, 2844
- **LOSMAN et al.** *Int. J. Cancer,* 1990, vol. 46, 310
- **MANCINI et al.** *New Microbiol.,* 2004, vol. 27, 315-28
- **CONRAD ; SCHELLER.** *Comb. Chem. High Throughput Screen.,* 2005, vol. 8, 117-26
- **BREKKE ; LOSET.** *Curr. Opin. Phamacol.,* 2003, vol. 3, 544-50
- **DANTAS-BARBOSA et al.** *Genet. Mol. Res.,* 2005, vol. 4, 126-40
- **MARKS et al.** *J. Mol. Biol.,* 1991, vol. 222, 581-97
- **ANDRIS-WIDHOPF et al.** Phage Display Laboratory Manual. Cold Spring Harbor Laboratory Press, 2000, 9.1-9.22
- **GREEN et al.** *J. Immunol. Methods,* 1999, vol. 231, 11-23
- **NISONOFF et al.** *Arch. Biochem. Biophys.,* 1960, vol. 89, 230
- **PORTER.** *Biochem. J.,* 1959, vol. 73, 119
- **EDELMAN et al.** METHODS IN ENZYMOLOGY. Academic Press, 1967, 422
- CURRENT PROTOCOLS IN IMMUNOLOGY. John Wiley & Sons, 1991
- **INBAR et al.** *Proc. Nat'l. Acad. Sci. USA,* 1972, vol. 69, 2659
- **WHITLOW et al.** *Methods: A Companion to Methods in Enzymology,* 1991, vol. 2, 97
- **BIRD et al.** *Science,* 1988, vol. 242, 423
- **PACK et al.** *Technology,* 1993, vol. 11, 1271
- **COSSINS et al.** *Protein Expression and Purification,* 2007, vol. 51, 253-59
- **SHUNTAO et al.** *Molec Immunol,* 2006, vol. 43, 1912-19
- **TANHA et al.** *J. Biol. Chem.,* 2001, vol. 276, 24774-780
- **LARRICK et al.** *Methods: A Companion to Methods in Enzymology,* 1991, vol. 2, 106
- MONOCLONAL ANTIBODIES: PRODUCTION, ENGINEERING AND CLINICAL APPLICATION. Cambridge University Press, 1995, 166-179
- MONOCLONAL ANTIBODIES: PRINCIPLES AND APPLICATIONS. Wiley-Liss, Inc, 1995, 137-185
- **SAMBROOK et al.** Molecular Cloning, A laboratory manual. 1989
- **HORNICK et al.** *J Nucl Med,* 2000, vol. 41, 355-62
- **HINTON et al.** *J Immunol,* 2006, vol. 176, 346-56
- **PETKOVA et al.** *Int Immunol,* 2006, vol. 18, 1759-69
- **HANSEN et al.** *Biochem J.,* 1996, vol. 320, 293-300
- **CRAIG ; FOON.** *Blood,* 15 January 2008
- **THEURILLAT et al.** *Int. J. Cancer,* 2007, vol. 120 (11), 2411-7
- **KOZLOV et al.** *Cancer Genet. Cytogenet.,* 2005, vol. 163 (1), 62-7
- **POISON et al.** *Blood,* vol. 110 (2), 616-623
- **HILL ; PERRIS.** *J. Natl. Cancer Inst.,* 2007, vol. 99, 1435-40
- **MAITLAND et al.** *Ernst Schering Found. Sympos. Proc.,* 2006, vol. 5, 155-79
- **DONNENBERG et al.** *J. Control Release,* 2007, vol. 122 (3), 385-91
- **BEIER et al.** *Cancer Res.,* 2007, vol. 67 (9), 4010-5
- **DALERBA.** *Proc. Natl. Acad. Sci. USA,* 2007, vol. 104 (24), 10158-63
- **LI et al.** *Cancer Res.,* 2007, vol. 67 (3), 1030-7
- **PRINCE et al.** *Proc. Natl. Acad. Sci. USA,* 2007, vol. 104 (3), 973-8
- **STEVENSON.** *Mol Med,* 2006, vol. 12 (11-12), 345-346
- **TASSONE et al.** *Blood,* 2004, vol. 104 (12), 3688-96
- **TAI et al.** *Blood,* 2008, vol. 112 (4), 1329-37
- **TAI et al.** *Cancer Res.,* 2005, vol. 65 (13), 5898-5906
- **LENG et al.** *J Exp Med,* 2003, vol. 197, 1467-76

- **MEYER-SIEGLER et al.** *BMC Cancer,* 2004, vol. 12, 34
- **SHACHAR ; HARAN.** *Leuk Lymphoma,* 2011, vol. 52, 1446-54
- **MORAND ; LEECH.** *Front Biosci,* 2005, vol. 10, 12-22
- **LAN.** *Nephron Exp Nephrol.,* 2008, vol. 109, e79-83
- **MEYER-SIEGLER et al.** *Mediators Inflamm,* 22 March 2009
- **TAKAHASHI et al.** *Respir Res,* 2009, vol. 10, 33
- **WINER et al.** *Nature Med,* 2011, vol. 17, 610-18
- **CERNEA et al.** *Diabetes Metab Rev,* 2010, vol. 26, 602-05
- **ONG et al.** *Immunology,* 1999, vol. 98, 296-302
- **STEIN et al.** *Clin Cancer Res.,* 15 September 2007, vol. 13 (2), 5556s-5563s
- **NITTA et al.** *Lancet.,* 1990, vol. 355, 368-371
- **MILSTEIN ; CUELLO.** *Nature,* 1983, vol. 305, 537-540
- **STAERZ et al.** *Nature,* 1985, vol. 314, 628-631
- **PEREZ et al.** *Nature,* 1985, vol. 316, 354-356
- **STAERZ ; BEVAN.** *Proc Natl Acad Sci U S A.,* 1986, vol. 83, 1453-1457
- **DEMONTE et al.** *Proc Natl Acad Sci U S A.,* 1990, vol. 87, 2941-2945
- **WALSH et al.** *J. Biol. Chem.,* 1968, vol. 243, 3763
- **TAYLOR.** *J. Biol. Chem.,* 1989, vol. 264, 8443
- **SCOTT.** *Pharmacol. Ther.,* 1991, vol. 50, 123
- **NEWLON et al.** *Nat. Struct. Biol.,* 1999, vol. 6, 222
- **SCOTT et al.** *J. Biol. Chem.,* 1990, vol. 265, 21561
- **LOHMANN et al.** *Proc. Natl. Acad. Sci USA.,* 1984, vol. 81, 6723
- **WONG ; SCOTT.** *Nat. Rev. Mol. Cell Biol.,* 2004, vol. 5, 959
- **CARR et al.** *J. Biol. Chem.,* 1991, vol. 266, 14188
- **ALTO et al.** *Proc. Natl. Acad. Sci. USA.,* 2003, vol. 100, 4445
- **COLLEDGE ; SCOTT.** *Trends Cell Biol.,* 1999, vol. 6, 216
- **NEWLON et al.** *EMBO J.,* 2001, vol. 20, 1651
- **CHMURA et al.** *Proc. Natl. Acad. Sci. USA.,* 2001, vol. 98, 8480
- **BURNS-HAMURO et al.** *Protein Sci,* 2005, vol. 14, 2982-92
- **CARR et al.** *J Biol Chem,* 2001, vol. 276, 17332-38
- **ALTO et al.** *Proc Natl Acad Sci USA,* 2003, vol. 100, 4445-50
- **HUNDSRUCKER et al.** *Biochem Y,* 2006, vol. 396, 297-306
- **STOKKA et al.** *Biochem J,* 2006, vol. 400, 493-99
- **GOLD et al.** *Mol Cell,* 2006, vol. 24, 383-95
- **KINDERMAN et al.** *Mol Cell,* 2006, vol. 24, 397-408
- **HUNDSRUCKER et al.** *Biochem J,* 2006, vol. 396, 297-306
- **BAERT et al.** *N Engl J Med,* 2003, vol. 348, 602-08
- **STICKLER et al.** *Genes and Immunity,* 2011, vol. 12, 213-21
- *J Immunol,* 1976, vol. 117, 1056-59
- **JEFFERIS ; LEFRANC.** *mAbs,* 2009, vol. 1, 1-7
- **MORCHHAUSER et al.** *J Clin Oncol,* 2009, vol. 27, 3346-53
- **GOLDENBERG et al.** *Blood,* 2009, vol. 113, 1062-70
- **ROBAK ; ROBAK.** *BioDrugs,* 2011, vol. 25, 13-25
- **KYTE ; DOOLITTLE.** *J. Mol. Biol.,* 1982, vol. 157, 105-132
- **CHOU ; FASMAN.** *Biochemistry,* 1974, vol. 13, 222-245
- *Ann. Rev. Biochem.,* 1978, vol. 47, 251-276
- *Biophys. J.,* 1979, vol. 26, 367-384
- **SILVERMAN et al.** *Nat. Biotechnol.,* 2005, vol. 23, 1493-94
- **SILVERMAN et al.** *Nat. Biotechnol.,* 2006, vol. 24, 220
- **SMITH ; SCOTT.** *Science,* 1985, vol. 228, 1315-1317
- **SMITH ; SCOTT.** *Meth. Enzymol.,* 1993, vol. 21, 228-257
- **ARAP et al.** *Science,* 1998, vol. 279, 377-380
- **PASQUALINI ; RUOSLAHTI.** *Nature,* 1996, vol. 380, 364-366
- **PASQUALINI.** *The Quart. J. Nucl. Med.,* 1999, vol. 43, 159-162
- **NORD et al.** *Protein Eng,* 1995, vol. 8, 601-8
- **NORD et al.** *Nat Biotechnol,* 1997, vol. 15, 772-77
- **PASQUALINI.** *Quart. J. Nucl. Med.,* 1999, vol. 43, 159-162
- **TOLMACHEV et al.** *Cancer Res,* 2007, vol. 67, 2773-82
- **TOLMACHEV et al.** *Bioconjugate Chem,* 2011, vol. 22, 894-902
- REMINGTON'S PHARMACEUTICAL SCIENCES. Mack Publishing Co, 1995
- GOODMAN AND GILMAN'S THE PHARMACOLOGICAL BASIS OF THERAPEUTICS. MacMillan Publishing Co, 1985
- **PASTAN. et al.** *Cell,* 1986, vol. 47, 641

- **SHARKEY ; GOLDENBERG.** *CA Cancer J Clin.,* July 2006, vol. 56 (4), 226-43
- **ANSEL et al.** PHARMACEUTICAL DOSAGE FORMS AND DRUG DELIVERY SYSTEMS. Lea & Febiger, 1990
- REMINGTON'S PHARMACEUTICAL SCIENCES. Mack Publishing Company, 1990
- **SHERWOOD et al.** *Bio/Technology,* 1992, vol. 10, 1446
- **SALTZMAN et al.** *Biophys. J.,* 1989, vol. 55, 163
- **ROBBINS ; ANGELL.** Basic Pathology. W. B. Saunders Co, 1976, 68-79
- **IBRAHIM et al.** *Cancer Discovery,* 2012, vol. 2, 1036-47
- **CHOU ; TALALAY.** *Adv Enzyme Regul,* 1984, vol. 22, 27-55
- **CARDILLO et al.** *Clin Cancer Res,* 2011, vol. 17, 3157-69
- **ALBERTI et al.** *Hybridoma,* 1992, vol. 11, 539-45
- **STEIN et al.** *Int J Cancer,* 1993, vol. 55, 938-46
- **STEIN et al.** *Int J Cancer Suppl.,* 1994, vol. 8, 98-102
- **CUBAS et al.** *Mol Cancer,* 2010, vol. 9, 253
- **STEIN.** *Int J Cancer,* 1993, vol. 55, 938-46
- **SHIH et al.** *Cancer Res,* 1995, vol. 55, 5857s-63s
- **STEIN et al.** *Cancer,* 1997, vol. 80, 2636-41
- **GOVINDAN et al.** *Breast Cancer Res Treat,* 2004, vol. 84, 173-82
- **MOON et al.** *J Med Chem,* 2008, vol. 51, 6916-26
- **GOVINDAN et al.** *Clin Cancer Res,* 2009, vol. 15, 6052-61
- **KOIZUMI et al.** *Cancer Res,* 2006, vol. 66, 10048-56
- **SAPRA et al.** *Clin Cancer Res,* 2008, vol. 14, 1888-96
- **ZHAO et al.** *Bioconjug Chem,* 2008, vol. 19, 849-59
- **HAMAGUCHI et al.** *Clin Cancer Res,* 2010, vol. 16, 5058-66
- **CHARI.** *Acc Chem Res,* 2008, vol. 41, 98-107
- **HAMBLETT et al.** *Clin Cancer Res,* 2004, vol. 10, 7063-70
- **SHARKEY et al.** *Mol Cancer Ther,* 2011, vol. 11, 224-34
- **GOVINDAN et al.** *Clin Cancer Res,* 2009, vol. 15, 6052-62
- **STEIN et al.** *Clin Cancer Res,* 2004, vol. 10, 2868-76
- **MATTES et al.** *Clin Cancer Res,* 2008, vol. 14, 6154-60
- **SHARKEY et al.** *J Nucl Med,* 2009, vol. 50, 444-53
- **RAVANDI.** *J Clin Oncol,* 2011, vol. 29, 349-51
- **SHIH et al.** *Int J Cancer,* 1994, vol. 56, 538-45
- **SHARKEY et al.** *Mol Cancer Ther,* 2011, vol. 10, 1072-81
- **SAPRA et al.** *Haematologica,* 2009, vol. 94, 1456-9
- **BAO et al.** *Oncotarget,* 22 September 2015
- **NAUJOKAS et al.** *Cell,* 1993, vol. 74, 257-68
- **BURTON et al.** *Clin Cancer Res,* 2004, vol. 10, 6606-11
- **STEIN et al.** *Blood,* 2004, vol. 104, 3705-11
- **ROCHE et al.** *PNAS USA,* 1993, vol. 90, 8581-85
- **HANSEN et al.** *Biochem,* 1996, vol. 320, 293-300
- **QU et al.** *Proc Am Assoc Cancer Res,* 2002, vol. 43, 255
- **FROLICH et al.** *Arthritis Res Ther,* 2012, vol. 14, R54
- **GRIFFITHS et al.** *Clin Cancer Res,* 2003, vol. 9, 6567-71
- **KAUFMAN et al.** *ASH Annual Meeting Abstracts,* 2008, vol. 112, 3697
- **GOLD et al.** *Int J Clin Exp Pathol,* 2010, vol. 4, 1-12
- **SAPRA et al.** *Clin Cancer Res,* 2005, vol. 11, 5257-64
- **BLUMENTHAL et al.** *BMC Cancer,* 2007, vol. 7, 2
- **GOVINDAN et al.** *Expert Opin Biol Ther,* 2012, vol. 12, 873-90
- **SAPRA et al.** *Expert Opin Biol Ther,* 2011, vol. 20, 1131-49
- **PRO et al.** *Expert Opin Biol Ther,* 2012, vol. 12, 1415-21
- **LORUSSO et al.** *Clin Cancer Res,* 2011, vol. 17, 437-47
- **THURBER et al.** *Adv Drug Del Rev,* 2008, vol. 60, 1421-34